# EUROPEAN PATENT APPLICATION

(11) **EP 2 128 157 A1**
(43) Date of publication of application: **02.12.2009**
(21) Application number: 08710655.5
(22) Date of filing: 24.01.2008
(51) Int. Cl.: C07D 413/12, A61K 31/4709, A61K 31/496, A61K 31/498, A61K 31/517, A61K 31/5355, A61K 31/536, A61K 31/538, A61K 31/5415, C07D 405/12, C07D 417/12

(54) **HETEROCYCLIDENE-N-(ARYL)ACETAMIDE DERIVATIVE**

(30) Priority: 24.01.2007 JP 2007014372
(71) Applicant: MOCHIDA PHARMACEUTICAL CO., LTD., Shinjuku-ku Tokyo 160-8515 (JP)
(72) Inventor: UCHIDA, Hideharu, Tokyo 160-8515 (JP); OGAWA, Shinichi, Tokyo 160-8515 (JP); MAKABE, Muneyoshi, Tokyo 160-8515 (JP); MAEDA, Yoshitaka, Tokyo 160-8515 (JP)
(74) Representative: HOFFMANN EITLE
(86) International application number: PCT/JP2008/051471
(87) International publication number: WO 2008/091021

(57) **Abstract**

The blow-described formula (I):
[Ch. 1]

a compound represented by formula (I) : 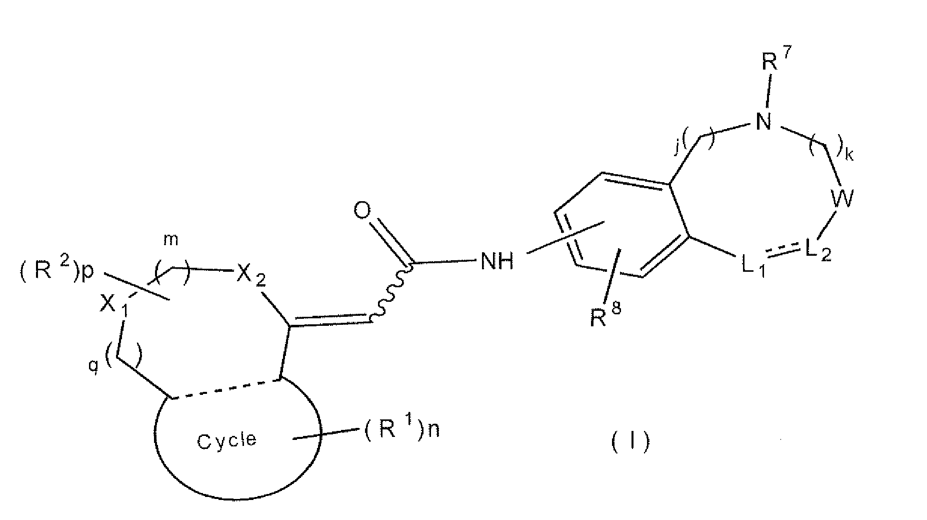 (wherein k, m, n, and p each represent 0 to 2; j and q represents 0 or 1; R¹ represents a halogen atom, a hydrocarbon group, a heterocyclic group, an alkoxy group, an alkoxycarbonyl group, NH₂, OH, a carboxyl group, an alkanoyl group, CN, NO₂, or the like; R² represents a halogen atom, an amino group, a hydrocarbon group, an aromatic heterocyclic group, an oxo group, or the like; X₁ represents an oxygen atom, -NR³-, or -S(O)r- (wherein r is an integer of 0 to 2); X₂ represents a methylene group, an oxygen atom, -NR³- (wherein R³ is a hydrogen atom, a hydrocarbon group, or the like), or -S(O)r- (wherein r is an integer of 0 to 2); W represents a methylene group, a carbonyl group, or a sulfonyl group; R⁷ represents a hydrogen atom, a hydrocarbon group, a heterocyclic group, or the like; R⁸ represents a hydrogen atom, a halogen atom, a hydrocarbon group, a heterocyclic group, the broken line in the ring containing X₁ and X₂ represents a condensation of two rings; cycle moiety represents a five- or six-memebered aryl ring or heteroaryl ring; and the solid line and the broken line between L₁ and L₂ are a single bond or double bond, and the wavy line represents an E-isomer or a Z-isomer), a salt thereof, or solvates thereof, and a pharmaceutical composition containing the compound as an active ingredient.

## Description

### Technical Field

The present invention relates to a medicine, in particular, a compound that modulates the function of having a transient receptor potential type I receptor (hereinafter referred to as "TRPV1 receptor"), in particular, to an N-(aryl) acetamide derivative having a heterocyclidene skeleton, a TRPV1 receptor antagonist comprising the derivative as an active ingredient, and an agent for preventing or treating diseases which cause pain and in which the TRPV1 receptor is involved, the preventive or treatable agent comprising the derivative as an active ingredient.

### Background Art

In a study related to the pain-producing mechanism, a receptor of capsaicin (8-methyl-N-vanillyl-6-nonenamide), which is a main pungent taste component of chili pepper, (TRPV1 receptor) was cloned in 1997 (Caterina MJ, Schumacher MA, Tominaga M, Rosen TA, Levine JD, and Julius D., Nature, Vol. 389, pp. 816-824, 1997). The TRPV1 receptor, which is a receptor that recognizes capsaicin, frequently expressed in primary sensory neurons involved in the sense of pain, and sensory afferent fibers containing C-fiber nerve ending. Thereafter, many TRP family receptors were cloned.

The structures of the TRP family receptors are similar to each other. The TRP family receptors each have a six transmembrane domain, and the N-terminal and the C-terminal of the molecule are disposed in a cell. In response to capsaicin stimulation, an acid (pH 6.0 or less), or heat (43°C or higher), the TRPV1 receptor allows cations such as a calcium ion and a sodium ion to flow into a cell. Accordingly, considering the expression sites of the TRPV1 receptor and the action of capsaicine, a marked contribution of the TRPV1 receptor to the excitement of nerve was assumed. Furthermore, contributions of the TRPV1 receptor to living organisms have been elucidated from information disclosed in many previous reports. In particular, in a mouse in which the TRPV1 receptor has been deleted (TRPV1 knockout mouse), enhancement of heat sensitivity due to neuropathic pain is not observed, development of edema is suppressed in a Complete Freund's Adjuvant (CFA)-induced inflammatory pain model (Szabo A, Helyes Z, Sandor K, Bite A, Pinter E, Nemeth J, Banvolgyi A, Bolcskei K, Elekes K, and Szolcsanyi J, Journal of Pharmacology And Experimental Therapeutics, Vol. 314, pp. 111-119, 2005), and desensitization action by a TRPV1 receptor agonist disclosed in a previous report exhibits an analgetic effect in a neuropathic pain model and an inflammatory pain model, and thus, an involvement of the TRPV1 receptor in pain has been suggested (Rashid MH, Inoue M, Kondo S, Kawashima T, Bakoshi S, and Ueda H, Journal of Pharmacology And Experimental Therapeutics, Vol. 304, pp. 940-948, 2003).

Application of capsaicin causes a temporary acute pain, but then induces desensitization to cause an analgetic effect. On the basis of this characteristic, many TRPV1 receptor agonists, such as a capsaicin cream, have been under development as analgetic drugs (Saper JR, Klapper J, Mathew NT, Rapoport A, Phillips SB, and Bernstein JE, Archives of Neurology, Vol. 59, pp. 990-994, 2002).

Recently, it has been reported that, in dorsal root ganglion cells of a diabetic pain model rat induced by administering streptozotocin, depolarization due to capsaicin stimulation is accelerated, that is, the sensitivity of the TRPV1 receptor is enhanced. Thus, an involvement of the TRPV1 receptor in diabetic pain has been suggested (Hong S and Wiley JW, The Journal of Biological Chemistry, Vol. 280, pp. 618-627, 2005). In addition, it has been reported that the desensitization action of capsaicin, which is a TRPV1 receptor agonist, is effective for improving the bladder function, and thus, a contribution to urination has also been suggested (Masayuki Takeda and Isao Araki, Nippon Yakurigaku zasshi (Folia Pharmacologica Japonica), Vol. 121, pp. 325-330, 2003). Furthermore, contraction of bronchia caused by capsaicin stimulation, an inhibition effect of a TRPV1 receptor antagonist for this action, and the like have also been reported, and thus, an involvement in respiratory organs has also been suggested. It has been elucidated that the TRPV1 receptor is involved in various diseases. From the information described above, TRPV1 receptor modulators that modulate the function of the TRPV1 receptor have been expected to be useful.

Among such TRPV1 modulators, agonists that stimulate the TRPV1 receptor to induce desensitization and antagonists are expected to be useful in treating various diseases. Among these agonists and antagonists, since the agonists cause pain involving temporary acute stimulation and so forth, TRPV1 receptor antagonists that do not induce such excitation due to stimulation have attracted attention. Currently, compounds having a TRPV1 receptor antagonism are expected to be widely useful for, for example, analgetic drugs, therapeutic drugs for urinary incontinence, and therapeutic drugs for respiratory diseases.

Pain is defined as "an unpleasant, sensory and emotional experience that is caused by a substantial or latent lesion of a tissue, and a sensory and emotional experience that is described using such an expression". Pain can be roughly divided into three categories: 1. nociceptive pain, 2. neuropathic pain, and 3. psychogenic pain.

The nociceptive pain is physiological pain caused by mechanical stimuli, thermal stimuli, or chemical stimuli. In general, the nociceptive pain acute pain and serves as a biosensor based on unpleasant sensory experiences to protect the body from danger. It has been thought that pain such as rheumatism is surely acute pain. However, a prolonged period from the onset thereof and the chronicity of inflammation bring about chronic pain.

Hyperalgesia to thermal to thermal stimuli or mechanical stimuli arises after tissue damage or during inflammation. The sensitization of receptors to a pain-inducing material and pain-inducing stimuli is reported in explanation of the hyperalgesia to thermal stimuli or mechanical stimuli. Examples thereof include sensitization of pain receptors due to inflammatory mediators occuring in local Inflammation and a decrease in the pH therein, an increase in reactivity to bradykinin and histamine due to an increase in the temperature of local inflammation, and sensitization due to nerve growth factor (NGF) (reference: Kazuo Hanaoka, Itami -Kiso, Shindan, Chiryo- (Pain -Base, Diagnosis, and Therapy-), Asakura Shoten, 2004). Specific examples thereof include chronic rheumatism and knee osteoarthiritis, which are typical examples. Non-steroidal anti-inflammatory drugs (NSAIDs) have been used for treatment of inflammatory pain due to pain chronic rheumatism and knee osteoarthiritis for a long period of time. However, the use thereof is restricted because of side effects due to a disorder of apparatus digestorius and renal disorder. Furthermore, although cyclooxygenase-2-selective inhibitors (COX2 inhibitors) have been developed for reducing the side effects of NSAIDs, there is concern abut side effect that can lead to cardiac insufficiency which has become a social problem. Accordingly, an inflammatory pain therapeutic agent having higher efficacy in oral administration and having fewer side effects is required.

Postoperative pain is basically inflammatory pain which tissue damage accompanies, and includes factors of neurogenic pain factor derived from nerve injury. Postoperative pain is broadly divided into somatic pain and visceral pain. Somatic pain is further divided into superficial pain and deep pain. Among these, when severe postoperative pain is left untreated, nerve sensitization occurs; hence, pain is also evoked by innocuous stimuli, such as a touch and a press (allodynia). When such pain occurs, there are many intractable cases that cannot be controller by nerve block therapy and the administration of drugs, such as NSAIDs, antiepileptic drugs, and opioid agonists. Furthermore, these drugs used have side effects. For example, the NSAIDs have side effects due to disorder of apparatus digestorius organs and renal disorder. In the antiepileptic drugs, carbamazepine and Phenytoin have side effects, such as tibutation, eruption, digestive symptoms, and cardiotoxicity; and Gabapentin has side effects such as somnolence and vertigo. The opioid agonists have side effects such as constipation. Accordingly, a postoperative pain therapeutic agent having higher efficacy and having fewer side effects is required.

Neuropathic pain is pain caused by primary damage of a certain portion in a neurotransmission system ranging from a periphery to center or caused by a malfunction thereof (Kenjiro Dan, Zusetsu Saishin Masuikagaku sirizu 4, Itami no rinsho (Textbook of anesthesiology 4, Fully illustrated) Chapter 1, 1998, Medical View Co., Ltd.).

Nerve injuries that cause neuropathic pain are typically external injuries or lesions on a peripheral nerve, a nerve plexus, or perineural soft-tissue. However, neuropathic pain is also caused by lesions on central somatosensory pathways (for example, ascending somatosensory pathways in spinal cord, brainstem, the thalamic or cortex level, and the like). For example, neuropathic pain is possibly caused by any of neurodegenerating diseases, osteolytic disease, metabolic disorder, cancer, infection, inflammation, after surgical operation, external injuries, radiotherapy, treatment using anticancer agents, and the like. However, the pathophysiological mechanism, or in particular, the molecular mechanism of the onset, has not yet been completely elucidated.

Allodynia is known as an example of an abnormal skin reaction characterizing neuropathic pain is allodynia. Allodynia is a state in which a person feels pain even with stimulation that would not result in normal person feeling pain. In allodynia, pain is evoked by tactile stimulus. That is, fundamental characteristics of allodynia are qualitative change in sensory responses and a low pain threshold. In postherpetic neuralgia, which is representative of neuropathic pain, it is confirmed that 87% of patients have allodynia. It is alleged that the strength of pain in postherpetic neuralgia is proportional to the degree of allodynia. Allodynia, which is a symptom that markedly constrains patients' freedom, draws attention as a therapeutic target of postherpetic neuralgia.

Herpes is a disease in which an infected herpes virus is neurons to cause onset, and 70% of herpes patients feel severe pain. This pain disappears as the disease is treated. However, about 10% of the patients suffers from so-called postherpetic neuralgia in which the pain remains for many years even after the disease is cured. On pathogenetic mechanism, it is said that the herpes virus proliferates again from a nerve ganglion, and nerve lesions generated during this proliferation accelerate reorganization of synapses, thus causing allodynia, which is neuropathic pain. In clinical settings, elderly people are more likely to develop the postherpetic neuralgia, and 70% or more of the cases of postherpetic neuralgia occur in patients 60 years old or older. Examples of a therapeutic agent used include anticonvulsant agents, non-steroidal anti-inflammatory agents, steroids, and the like, but there is no complete therapy (reference: Kazuo Hanaoka, Itami -Kiso, Shindan, Chiryo- (Pain -Base, Diagnosis, and Therapy-), Asakura Shoten, 2004).

Diabetic pain is broadly categorized into acute pain that occurs when hyperglycemia is rapidly remedied and chronic pain that occurs due to factors such as demyelination or nerve regeneration. Among these types of diabetic pain, the chronic pain is neuropathic pain due to inflammation of the dorsal root ganglion caused by a decrease in the bloodstream due to diabetes, and spontaneous firing of neurons and excitability caused by the subsequent regeneration of nerve fibers. Non-steroidal anti-inflammatory agents, antidepressant agents, capsaicin creams and the like are used for therapy. However, there is no perfect therapeutic agent for treatment of diabetic pain that can cure all the types of diabetic pain using a single agent (Reference: Iyaku no ayumi (Progress in Medicine)(Journal of Clinical and Experimental Medicine), Vol. 211, No. 5, 2004, Special feature "Itami shigunaru no seigyo kiko to saishin chiryo ebidensu" ("Control mechanisms of Pain Signal and Latest Evidence-based Therapy")).

In neuropathic pain, analgesic treatment for patients who complain of a chronic pain symptom that interferes with their daily life directly improves the quality of life. However, it is believed that central analgetic agents represented by morphine, non-steroidal anti-inflammatory analgesic agents, and steroids are not effective against neuropathic pain. In practical pharmacotherapy, antidepressant agents such as amitriptyline; antiepileptic drugs such as Gabapentin, Pregabalin, carbamazepine, and phenytoin; and antiarrhythmic agents such as mexiletine are also used and prescribed for the treatment of neuropathic pain. However, it is known that these drugs have the following side effects: Amitriptyline causes side effects such as dry mouth, drowsiness, sedation, constipation, and dysuria. Carbamazepine and phenytoin cause side effects such as light-headedness, eruption, digestive apparatus symptons, and cardiotoxicity. Gabapentin causes side effects such as somnolence and vertigo. Mexiletine causes side effects such as vertigo and digestive apparatus symptoms. These drugs, which are not specific neuropathic pain therapeutic agents, have poor dissociation between drug efficacy and side effect, thus, resulting in low treatment of satisfaction. Accordingly, a neuropathic pain therapeutic agent that exhibits a higher efficacy in oral administration and that have fewer side effects is required.

Recently, compounds having a TRPV1 receptor antagonism have been studied. Known heterocyclic compounds each having an amide bond are disclosed in, for example, PCT Publication No. 03/049702 pamphlet (Patent Document 1), PCT Publication No. 04/056774 pamphlet (Patent Document 2), PCT Publication No. 04/069792 pamphlet (Patent Document 3), PCT Publication No. 04/100865 pamphlet (Patent Document 4), PCT Publication No. 04/110986 pamphlet (Patent Document 5), PCT Publication No. 05/016922 pamphlet (Patent Document 6), PCT Publication No. 05/030766 pamphlet (Patent Document 7), PCT Publication No. 05/040121 pamphlet (Patent Document 8), PCT Publication No. 05/046683 pamphlet (Patent Document 9), PCT Publication No. 05/070885 pamphlet (Patent Document 10), PCT Publication No. 05/095329 pamphlet (Patent Document 11), PCT Publication No. 06/006741 pamphlet (Patent Document 12), PCT Publication No. 06/038871 pamphlet (Patent Document 13), and PCT Publication No. 06/058338 pamphlet (Patent Document 14). However, these patent documents do not disclose heterocyclidene acetamide derivatives.

Examples of the related art that disclose a compound having a heterocyclidene skeleton include that are PCT Publication No. 94/26692 pamphlet (Patent Document 15), PCT Publication No. 95/06035 pamphlet (Patent Document 16), PCT Publication No. 98/39325 pamphlet (Patent Document 17), PCT Publication No. 03/042181 pamphlet (Patent Document 18), Japanese Patent Application Laid-open No. 2001-213870 (Patent Document 19), PCT Publication No. 06/064075 pamphlet (Patent Document 20), PCT Publication No. 07/010383 pamphlet (Patent Document 21), Journal of Heterocyclic Chemistry, Vol. 22, No. 6, pp. 1511-18, 1985 (Non-Patent Document 1), Tetrahedron Letters, Vol. 42, No. 18, pp. 3227-3230, 2001 (Non-Patent Document 2), and Chemical & Pharmaceutical Bulletin, Vol. 47, No. 3, pp. 329-339, 1999 (Non-Patent Document 3).

Patent Document 15 discloses, as a muscle relaxant, a compound with a structure which has a 2H-1-benzopyran-4-ylidene skeleton or a 1,2,3,4-tetrahydro-4-quinolidene skeleton and in which a hydrogen atom, an alkyl group, or a cycloalkyl group is bonded to the N atom of the acetamide structure. However, a compound in which a substituted aryl group, heteroaryl group, or the like is bonded to the N atom is not disclosed. Patent Documents 16 to 18 disclose, as an arginine vasopressin antagonist or an oxytocin antagonist, a compound with a specific structure which has a 4,4-difluoro-2,3,4,5-tetrahydro-1H-1-benzodiazepine skeleton and in which an aryl carbonyl group substituted an aryl is bonded to the N atom of the 1-position of the skeleton.

Patent Document 19 discloses, as a 2-(1,2-benzisothiazol-3(2H)-ylidene 1,1-dioxide) acetamide derivative used as a novel charge-control agent for a toner for electrostatography, a specific compound in which the N atom of the acetamide has a substituted phenyl group.

Patent Document 20 discloses, as an amide derivative of a 2,3-dihydro-1-oxo-1H-isoquinolin-4-ylidene used as a calpain inhibitor, a compound with a specific structure which has a sec-butyl group at the 3-position.

Patent Document 21 discloses a nobel heterocycliden acetamide derivatives used as the TRPV1 receptor antagonist.

In a report related to the synthesis of an oxyindole derivative, Non-Patent Document 1 discloses 2-(1,2-dihydro-2-oxo-3H-indol-3-ylidene)-N,N-dimethyl-acetamide. However, a substituted aryl group or heteroaryl group, or the like is not bonded to the N atom.

Non-Patent Document 2 discloses, as a (1,2,3,4-tetrahydro-2-oxo-5H-1,4,-benzodiazepin-5-ylidene)acetamide derivative used for an N-methyl-D-aspartate (NMDA) antagonist, a compound with a specific structure in which a phenyl group is bonded to the N atom of the acetamide.

Non-Patent Document 3 discloses, as a (2,3,4,5-tetrahydro-1H-1-benzodiazepin-5-ylidene)acetamide derivative used as a nonpeptide arginine vasopressin antagonist, a compound with a specific structure in which a 2-pyridylmethyl group is bonded to the N atom of the acetamide, and the benzodiazepine skeleton does not have a substituent.

Patent Documents 15 to 20 and Non-Patent Documents 1 to 3 disclose compounds each having a heterocyclidene skeleton, but the antagonism of the TRPV1 receptor is not disclosed or suggested.

It was reported that rise of body temperature was caused by administration of TPRV1 receptor antagonist (Journal of Medicinal Chemistry, Vol. 48, No. 6, pp.1857-72, 2005 (Non-Patent Document 4) (Society Neuroscience Abstruct, 30, Program No. 890.24, 2004 (Non-Patent Document 5).

In the development of pharmaceuticals, it is required to satisfy strict criteria for not only target pharmacological activity but also absorption, distribution, metabolism, excretion, and the like. With respect to drug interactions, desensitization or tolerance, digestive absorption in oral administration, the rate of transfer to a small intestine, the rate of absorption and first-pass effect, an organ barrier, protein binding, induction of a drug-metabolizing enzyme, an excretion pathway and body clearance, a method of administration (an application site, a method, and purpose), and the like, various agenda are required. However, a drug that satisfies these requirements is selfdom discovered.

These comprehensive problems in drug development also exist for TRPV1 receptor antagonists, and TRPV1 receptor antagonists have not yet been released onto the market. More specifically, compounds having a TRPV1 receptor antagonism also include problems in terms of usefulness and safety. For example, these compounds have low metabolic stability and oral administration of these compounds is difficult; these compounds exhibit inhibitory activity of the human ether-a-go-go related gene (hERG) channel, which may cause arrhythmia, and pharmacokinetics of these compounds are not satisfactory. There are problems which will be understood at stages of clinical experiments.

For instance, the change in the body temperature according to administering the TRPV11 receptor antagonist is suggested, but no prior art has been found that discloses a method of inducing compounds to solve such problems. Accordingly, a compound in which these problems are solved and which has high activity has been desired.

In addition, a compound that causes fewer of the above-mentioned side effects than known drugs that are currently used in the treatment of pain including the above-described types of neuropathic pain has been desired.
(Patent Document 1) PCT Publication No. 03/049702 pamphlet
(Patent Document 2) PCT Publication No. 04/056774 pamphlet
(Patent Document 3) PCT Publication No. 04/069792 pamphlet
(Patent Document 4) PCT Publication No. 04/100865 pamphlet
(Patent Document 5) PCT Publication No. 04/110986 pamphlet
(Patent Document 6) PCT Publication No. 05/016922 pamphlet
(Patent Document 7) PCT Publication No. 05/030766 pamphlet
(Patent Document 8) PCT Publication No. 05/040121 pamphlet
(Patent Document 9) PCT Publication No. 05/046683 pamphlet
(Patent Document 10) PCT Publication No. 05/070885 pamphlet
(Patent Document 11) PCT Publication No. 05/095329 pamphlet
(Patent Document 12) PCT Publication No. 06/006741 pamphlet
(Patent Document 13) PCT Publication No. 06/038871 pamphlet
(Patent Document 14) PCT Publication No. 06/058338 pamphlet
(Patent Document 15) PCT Publication No. 94/26692 pamphlet
(Patent Document 16) PCT Publication No. 95/06035 pamphlet
(Patent Document 17) PCT Publication No. 98/39325 pamphlet
(Patent Document 18) PCT Publication No. 03/042181 pamphlet
(Patent Document 19) Japanese Patent Application Laid-open No. 2001-213870
(Patent Document 20) PCT Publication No. 06/064075 pamphlet
(Patent Document 21) PCT Publication No. 07/010383 pamphlet
(Non-Patent Document 1) Journal of Heterocyclic Chemistry, Vol. 22, No. 6, pp. 1511-18, 1985
(Non-Patent Document 2) Tetrahedron Letters, Vol. 42, No. 18, pp. 3227-3230, 2001
(Non-Patent Document 3) Chemical Pharmaceutical Bulletin, Vol. 47, No. 3, pp. 329-339, 1999
(Non-Patent Document 4) Journal of Medicinal Chemistry, Vol.48, No. 6, pp.1857-72, 2005
(Non-Patent Document 5) Society Neuroscience Abstruct, Program No. 890.20, 2004

### Disclosure of Invention

### Problems to be solved by the Invention

Under the above-described circumstances, a TRPV1 receptor modulator, in particular, a TRPV1 receptor antagonist that can be orally administered, that has high safety, and that has excellent effectiveness, an agent for preventing or treating diseases in which the TRPV1 receptor is involved, and in particular, an agent for preventing or treating pain have been desired. In the related art, amitriptyline causes side effects such as dry mouth, drowsiness, sedation, constipation, and dysuria; carbamazepine and phenytoin cause side effects such as eruption, digestive apparatus symptoms, and cardiotoxicity; gabapentin causes side effects such as somnolence and vertigo; mexiletine causes side effects such as vertigo and digestive apparatus symptoms; non-steroidal anti-inflammatory drugs cause side effects such as gastrointestinal damage; and COX2 inhibitors cause a side effect of heart failure. Accordingly, in particular, an agent for preventing or treating pain which can orally administered to mammals including humans, in particular, which can be clinically easily used (in particular, the change in the body temperature is very little), and in which at least one of the above-described problems of the related art is overcome, for example, one which causes fewer of the above-mentioned side effects than known drugs, one which does not have an inhibitory action of an hERG current, one which has satisfactory metabolic stability, one which can be orally administered, one which has satisfactory pharmacokinetics, one which has excellent in solubility, or one which does not cause the rise of the body temperature has been strongly desired.

### Means for Solving the Problems

The present invention provides a compound that modulates the function of a TRPV1 receptor, in particular, a heterocyclidene -N-(aryl) acetamide derivative represented by formula (I) where the benz ring (bicyclic ring system), which is condensed to nitrogen-containing ring (having, in particular, any of carbonyl group, sulfonyl group or oxygen atom), is bonded to amido-nitrogen atom, a pharmaceutically acceptable salt thereof, and a solvate thereof; a TRPV1 receptor modulator, in particular, a TRPV1 receptor antagonist, and an agent for preventing or treating pain, in particular, an agent for preventing or treating neuropathic pain, and an agent for preventing or treating inflammatory pain that contain the derivative as an active ingredient.

### Advantages of the Invention

In order to solve the above problems and to obtain a compound that modulates the function of having a TRPV1 receptor having high safety and excellent effectiveness, the present inventors have conducted intensive studies and found that N-(aryl)-acetamide derivatives having a heterocyclidene skeleton represented by formula (I) where the benz ring (bicyclic ring system), which is condensed to nitrogen-containing ring(having, in particular, any of carbonyl group, sulfonyl group or oxygen atom), is bonded to amido-nitrogen atom and, pharmaceutically acceptable salts thereof, and solvates thereof have an excellent activity that modulates the function of the TRPV1 receptor, and the group of these compounds has at least one of features that the compounds have high metabolic stability , excellent oral absorbability, or do not cause the rise of body temperature (in particular, the change in the body temperature is very little) . Accordingly, a pharmaceutical composition comprising one of the compounds as an active ingredient is promising as an agent for preventing or treating pain that can be orally administered, in particular, as an agent for preventing or treating neuropathic pain, or an agent for preventing or treating inflammatory pain.

### Best Mode for Carrying Out the Invention

The present invention provides a heterocyclidene-N-(aryl) acetamide derivative represented by formula (I) where the benz ring (bicyclic ring system) , which is condensed to nitrogen-containing ring(having, in particular, any of carbonyl group, sulfonyl group or oxygen atom),is bonded to amido-nitrogen atom, a salt thereof, a pharmaceutical composition comprising the derivative or a salt thereof; and pharmaceutical use of the derivative or a salt thereof.

Embodiments of the present invention will now be described. In the description related to the compounds of the present invention, for example, the expression "C₁₋₆" means, unless otherwise stated, "a linear or branched chain having 1 to 6 carbon atoms" for a linear group, and "the number of carbon atoms constituting a ring" for a cyclic group.

The molecular weight of a compound represented by formula (I) of the present invention is not particularly limited. However, the molecular weight is preferably 700 or less, and more preferably 550 or less. When the structure of a compound is specified in recent drug design, in addition to the basic skeleton having a pharmacological feature, a limitation such as that of the molecular weight is normally used as another significant limiting factor.

### [Embodiments of the present invention]

### [1] First embodiment of the present invention

A first embodiment of the present invention is a compound represented by formula (I): 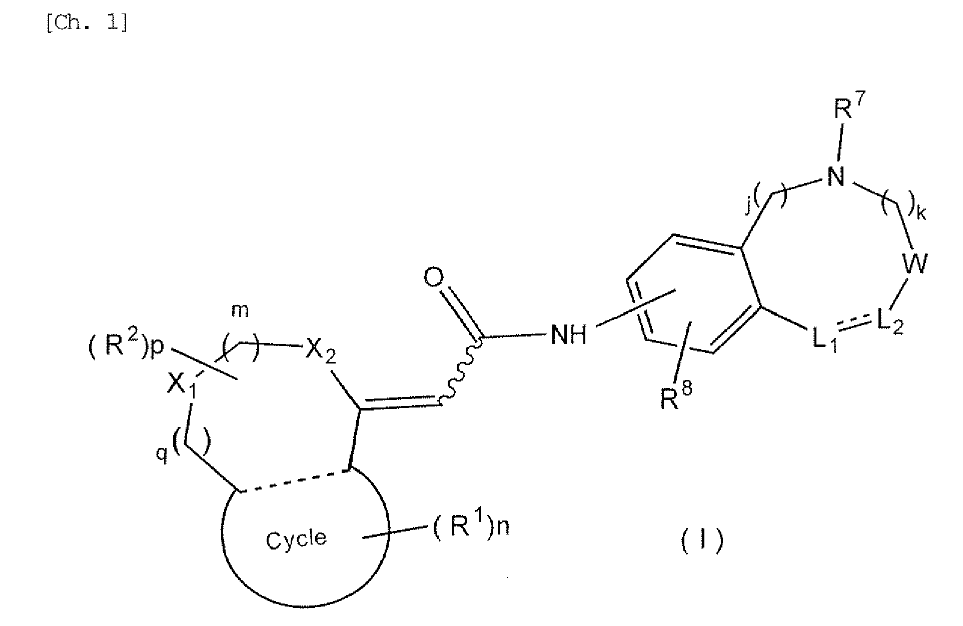 (wherein k, m, n, and p each independently represent an integer of 0 to 2; j and q represents an integer of 0 or 1; R¹ represents a group selected from a halogen atom, a substituted or unsubstituted hydrocarbon group, a substituted or unsubstituted heterocyclic group, a substituted or unsubstituted C₁₋₆ alkoxy group, a substituted or unsubstituted C₁₋₆ alkoxycarbonyl group, an amino group which may be mono- or di-substituted with a substituted or unsubstituted C₁₋₆ alkyl group, a protected or unprotected hydroxyl, group, a protected or unprotected carboxyl group, a carbamoyl group which may be mono- or di-substituted with a substituted or unsubstituted C₁₋₆ alkyl group, a C₁₋₆ alkanoyl group, a C₁₋₆ alkylthio group, a C₁₋₆ alkylsulfinyl group, a C₁₋₆ alkylsulfonyl group, a sulfamoyl group which may be mono- or di-substituted with a substituted or unsubstituted C₁₋₆ alkyl group, a cyano group, and a nitro group; R² represents a group selected from a halogen atom, a substituted or unsubstituted amino group, a substituted or unsubstituted hydrocarbon group, a substituted or unsubstituted aromatic heterocyclic group, and an oxo group, or two seminal or vicinal R² may bind to each other to form a C₂₋₆ alkylene group, and form a cycle ring group together with the carbon atom to which the two R² are bonded or the cyclo ring group may form non-aromatic heterocyclic groups containing an oxygen atom or a nitrogen atom; X₁ represents an oxygen atom, -NR³- (wherein R³ is a hydrogen atom, a substituted or unsubstituted hydrocarbon group, a substituted or unsubstituted heterocyclic group, or a substituted or unsubstituted acyl group), or -S(O)r- (wherein r is an integer of 0 to 2); X₂ represents a methylene group, an oxygen atom, -NR³- (wherein R³ is a hydrogen atom, a substituted or unsubstituted hydrocarbon group, a substituted or unsubstituted heterocyclic group, or a substituted or unsubstituted acyl group) or -S(O)r- (wherein r is an integer of 0 to 2); W represents a methylene group, a carbonyl group or a sulfonyl group;R⁷ represents a hydrogen atom, a substituted or unsubstituted hydrocarbon group, a substituted or unsubstituted heterocyclic group, or a substituted or unsubstituted acyl group; R⁸, R^{9A} and R^{9B} each independently represent a hydrogen atom, a halogen atom, a substituted or unsubstituted hydrocarbon group, a substituted or unsubstituted heterocyclic group, a substituted or unsubstituted C₁₋₆ alkoxy group, a substituted or unsubstituted C₁₋₆ alkoxycarbonyl group, an amino group which may be mono- or di-substituted by a substituted or unsubstituted C₁₋₆ alkyl group, a protected or unprotected hydroxyl grop, a protected or unprotected carboxyl group, a carbamoyl group which may be mono- or di-substituted by a substituted or unsubstituted C₁₋₆ alkyl group, a C₁₋₆ alkanoyl group, C₁₋₆ alkylthio group, a C₁₋₆ alkylsulfinyl group, C₁₋₆ alkylsulfonyl group, a sulfamoyl group which may be mono- or di-substitute by a substituted or unsubstituted C₁₋₆ alkyl group, a cyano group or a nitro group; L₁ and L₂ each independently represent a single bond, a -CR^{9A}R^{9B}-, an oxygen atom; -NP¹⁰- (R¹⁰ represents a hydrogen atom, a substituted or unsubstituted hydrocarbon group, a substituted or unsubstituted heterocyclic group or a substituted or unsubstituted acyl group) or -S(O)t- (t is an integer of 0 to 2), the broken line in the ring containing X₁ and X₂ represents a condensation of two rings; Cycle moiety represents a five- or six-membered aryl ring or heteroaryl ring; and the solid line and the broken line between L₁ and L₂ is a single bond or double bond, and the wavy line represents an E-isomer or a Z-isomer), provided that when W represents a methylene group L₁ is an oxygen atom and L₂ is a -CR^{9A}R^{9B}-,and that each of (E)-2-(8-trifluoromethyl-3,4-dihydrobenzo[b]oxepin-5(2H)-ylidene)-N-(3-hydroxy-2-oxo-1,2,3,4-tetrahydroquinolin-5- yl)acetamide; (E)-2-(7-trifluoromethyl-2,3-dihydro-1-pentanoylquinolin-4(1H)-ylidene)-N-(3,4-dihydro-3-hydroxy(1H)quinolin-2-on-5-yl)acetamide; (E)-N-(3-hydroxy-2-oxo-1,2,3,4-tetrahydroquinolin-5-yl)-2-(7-trifluoromethyl-chroman-4-ylidene)acetamide; (E)-2-(8-trifluoromethyl-3,4-dihydrobenzo[b]oxepin-5-(2H)- ylidene)-N-(3,4-dihydro-1H-quinolin-2-on-7-yl)acetamide; (e)-2-(8-trifluoromethyl-3,4-dihydrobenzo[b]oxepin-5(2H)- ylidene)-N-(2-quinclon-7-yl)acetamide; (E)-2-(8-trifluoromethyl-3,4-dihydrobenzo[b]oxepin-5(2H)- ylidene)-N-(2-oxoindolin-6-yl)acetamide; (E)-2-(B-trifluoromethyl-3,4-dihydrobenzo[b]oxepin-5(2H- ylidene)-N-(2H-benzo[1,4]oxazine-3(4H)-on-6-yl)acetamide; (E)-2-(8-trifluoromethyl-3,4-dihydrobenzo[b]oxepin-5(2H)- ylidene)-N-(3,4-dihydro-1H-quinolin-2-on-6-yl)acetamide; (E)-2-(8-trifluoromethyl-3,4-dihydrobenzo[b]oxepin-5(2H)- ylidene)-N-(2,3-dihydro-isoindol-1-on-6-yl)acetamide; (E)-2-(8-trifluoromethyl-3,4-dihydrobenzo[b]oxepin-5(2H)- ylidene)-N-(2-quinolon-8-yl)acetamide; (E)-2-(8-trifluoromethyl-3,4-dihydrobenzo[b]oxepin-5(2H)- ylidene)-N-(2-oxo-1,2,3,4-tetrahydroquinolin-8-yl)acetamide; (E)-2-(8-trifluoromethyl-3,4-dihydrobenzo[b]oxepin-5(2H)- ylidene)-N-(2-hydroxyethyl-2,3-dihydro-isoindol-1-on-6-yl) acetamide; (E)-2-(8-trifluoromethyl-3,4-dihydrobenzo[b]oxepin-5(2H)-ylidene)-N-(3,4-dihydro-(2H)-isoquinolin-1-on-7-yl) acetamide; (E)-2-(8-trifluoromethyl-3,4-dihydrobenzo[b]oxepin-5(2H)- ylidene)-N-(1-methyl-2-oxo-1,2,3,4-tetrahydroquinolin-7-yl) acetamide, (E)-2-(1-(2,2-difluorobutanoyl)-7-trifluoromethyl-2,3-dihydroquinolin-4(1H)-ylidene)-N-(3-hydroxy-2-oxo-1,2,3,4- tetrahydroquinolin-5-yl)acetamide and (E)-2-(8-trifluorometyl-3,4-dihydrobenzo[b]oxepin-5(2H)- ylidene)-N-(4-(2-hydroxyethyl)-2H-1,4-benzoxazin-3(4H)-on- 6-yl) acetamide is eliminated), a salt thereof, and solvates thereof.

Each of the groups in formula (I) used in the compound of embodiment [1] above will now be described specifically. In the following description, the expression "C₁₋₆" means that the number of carbon atoms is in the range of 1 to 6. For example, a C₁₋₆ alkyl group represents an alkyl group having 1 to 6 carbon atoms.

[1-1] In the compounds represented by formula (I), R¹ is a halogen atom, a substituted or unsubstituted hydrocarbon group, a substituted or unsubstituted heterocyclic group, a substituted or unsubstituted C₁₋₆ alkoxy group, a substituted or unsubstituted C₁₋₆ alkoxycarbonyl group, an amino group which may be mono- or di-substituted with a substituted or unsubstituted C₁₋₆ alkyl group, a protected or unprotected hydroxyl group, a protected or unprotected carboxyl group, a carbamoyl group which may be mono- or di-substituted with a substituted or unsubstituted C₁₋₆ alkyl group, a C₁₋₆ alkanoyl group, a C₁₋₆ alkylthio group, a C₁₋₆ alkylsulfinyl group, a C₁₋₆ alkylsulfonyl group, a sulfamoyl group which may be mono- or di-substituted with a substituted or unsubstituted C₁₋₆ alkyl group, a cyano group, or a nitro group. Among these, a substituted or unsubstituted hydrocarbon group is preferred.

Examples of the "halogen atom" include a fluorine atom, a chlorine atom, a bromine atom, and an iodine atom.

The "hydrocarbon groups" of the "substituted or unsubstituted hydrocarbon groups" include aliphatic hydrocarbon groups, alicyclic hydrocarbon groups, and aryl groups. Among these, aliphatic hydrocarbon groups are preferred.

Examples of the "aliphatic hydrocarbon groups" in the "substituted or unsubstituted aliphatic hydrocarbon groups" include linear or branched hydrocarbon groups such as alkyl groups, alkenyl groups, and alkynyl groups.

Examples of the "alkyl groups" include C₁₋₁₀ (more preferably C₁₋₆) alkyl groups such as methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2- trimethylpropyl, 1-ethyl-1-methylpropyl, 1-ethyl-2-methylpropyl, n-hexyl, 1-methyl-heptyl, and n-nonyl.

Examples of the "alkenyl groups" include C₂₋₆ alkenyl groups such as vinyl, allyl, isopropenyl, 2-methylallyl, butenyl, pentenyl, and hexenyl.

Examples of the "alkynyl groups" include C₂₋₆ alkynyl groups such as ethynyl, 1-propynyl, 2-propynyl, butynyl, pentynyl, and hexynyl.

Examples of the "alicyclic hydrocarbon groups" include saturated and unsaturated alicyclic hydrocarbon groups such as cycloalkyl groups, cycloalkenyl groups, and cycloalkanedienyl groups.

Examples of the "cycloalkyl groups" include C₃₋₉ cycloalkyl groups such as cyclopropyl, cyclobutyl, cyclopentyl, cyclohexyl, cycloheptyl, cyclooctyl, and cyclononyl.

Examples of the "cycloalkenyl groups" include C₃₋₆ cycloalkenyl groups such as 1-cyclopropen-1-yl, 1-cyclobuten-1-yl, 1-cyolopenten-1-yl, 2-cyclopenten-1-yl, 3-cyclopenten-1-yl, and 1-cyclohexen-1-yl.

Examples of the "cycloalkanedienyl groups" include C₄₋₆ cycloalkanedienyl groups such as 2,4-cyclopentadien-1-yl and 2,5-cyclohexadien-1-yl.

Examples of the "aryl groups" include C₆₋₁₄ aryl groups such as phenyl, naphthyl, biphenyl, 2-anthryl, phenanthryl, acenaphthyl, and 5,6,7,8-tetrahydronaphthalenyl; and partially hydrogenated fused aryl such as indanyl and tetrahydronaphthyl.

Examples of the heterocyclic groups of the "substituted or unsubstituted heterocyclic groups" in R¹ include aromatic heterocyclic groups and saturated or unsaturated non-aromatic heterocyclic groups. Example of the rings include five- to fourteen-membered rings, preferably five- to twelve-membered rings, containing at least one heteroatom (preferably, 1 to 4 heteroatoms) selected from N, O, and S in addition to the carbon atoms.

The "aromatic heterocyclic groups" include monocyclic aromatic heterocyclic groups and fused aromatic heterocyclic groups. Preferably, the monocyclic aromatic heterocyclic groups each have a five- or six-membered ring. Examples thereof include pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.

Preferably, the fused aromatic heterocyclic groups each have an eight- to twelve-membered ring. These groups include, for example, monovalent groups obtained by removing any hydrogen atom from a ring farmed by condensing the above-mentioned five- or six-membered aromatic ring with one or a plurality of (preferably 1 to 2) aromatic rings (such as benzene rings).

Specific examples thereof include indolyl, isoindolyl, 1H-indazolyl, benzofuranyl (-2-yl), isobenzofuranyl, benzothienyl (-2-yl), isobenzothienyl, benzindazolyl, benzoxazolyl (-2-yl), 1,2-benzisoxazolyl, benzothiazolyl (-2-yl), 1,2-benzisothiazolyl, 2H-benzopyranyl (-3-yl), (1H-)benzimidazolyl (-2-yl), 1H-benzotriazolyl, 4H-1,4-benzoxazinyl, 4H-1,4-benzothiazinyl, quinolyl, isoquinolyl, cinnolinyl, quinazolinyl, quinoxalinyl, phthalazinyl, naphthylizinyl, purinyl, pteridinyl, carbazolyl, carbolinyl, acridinyl, phenoxazinyl, phenothiazinyl, phenazinyl, phenoxathinyl, thianthrenyl, phenanthridinyl, phenanthrolinyl, indolizinyl, (4,5,6,7-)tetrahydrothiazolo[5,4-c]pyridyl (-2-yl), (4,5,6,7-)tetrahydrothieno[3,2-c]pyridyl, (1,2,3,4-)tetrahydroisoquinolyl (-6-yl), thiazolo[5,4-c]pyridyl (-2-yl), pyrrolo[1,2-b]pyridazinyl, pyrazo[1,5-a]pyridyl, imidazo[1,2-a]pyridyl, imidazo[1,5-a]pyridyl, imidazo[1,2-b]pyridazinyl, imidazo[1,5-a]pyrimidinyl, [1,2,4] triazolo[4,3-a]pyridyl, 1,2,4-triazolo[4,3-b]pyridazinyl, chromenyl (2H-chromenyl), 1H-pyrazolo[3,4-b]pyridyl, and [1,2,4] triazolo[1,5a]pyrimidinyl (Preferred embodiments are indicated in the parenthesis "( )").

Examples thereof also include partially hydrogenated fused aromatic heterocyclic groups and the like, such as tetrahydroquinolinyl, tetrahydroisoquinolinyl, tetrahydrobenzoxazepinyl, tetrahydrobenzoazepinyl, tetrahydronaphthpyridinyl, tetrahydroquinoxalinyl, chromanyl, dihydrobenzoxazinyl, 3,4-dihydro-2H-1,4-benzothiazinyl, dihydrobenzothiazolyl, 3,4-dihydro-2H-1,4-benzoxazinyl, isochromanyl, indolinyl, pteridinyl, 2,3-dihydrobenzo[b][1,4]dioxinyl, 1,2,3,4-tetrahydro-1-methylquinolinyl, 1,3-dihydro-1-oxoisobenzofuranyl, and 6,7,8,9-tetrahydro-5H-cyclohepta[b]pyridyl.

Examples of the "non-aromatic heterocyclic groups" include three-to eight-membered saturated and unsaturated non-aromatic heterocyclic groups such as azetidinyl, oxiranyl, oxepanyl, thietanyl, pyrrolidinyl, tetrahydrofuryl, thiolanyl, pyrazolinyl, pyrazolidinyl, piperidyl, tetrahydropyranyl, piperazinyl, morpholinyl, oxazolinyl, thiazolinyl, thiomorpholinyl, oxepanyl and quinuclidinyl.

In the "substituted or unsubstituted C₁₋₆ alkoxy group", examples of the C₁₋₆ alkoxy groups include a methoxy group, ethoxy group, propoxy group, isopropoxy group, butoxy group, isobutoxy group, sec-butoxy group, tert-butoxy group, pentyloxy group, isopentyloxy group, 3-pentyloxy group, tert-pentyloxy group, neopentyloxy group, 2-methylbutoxy group, 1,2-dimethylpropoxy group, 1-ethylpropoxy group, hexyloxy group, cyclopropyloxy group, cyclobutyloxy group, cyclopentyloxy group, cyclohexyloxy group, cyclopropylmethyloxy group, 1-cyclopropylethyloxy group, 2-cyclopropylethyloxy group, cyclobutylmethyloxy group, 2-cyclobutylethyloxy group, and cyclopentylmethyloxy group.

In the "substituted or unsubstituted C₁₋₆ alkoxycarbonyl group", examples of the C₁₋₆ alkoxycarbonyl groups include a methoxycarbonyl group, ethoxycarbonyl group, propoxycarbonyl group, isopropoxycarbonyl group, butoxycarbonyl group, isobutoxycarbonyl group, sec-butoxycarbonyl group, tert-butoxycarbonyl group, pentyloxycarbonyl group, isopentyloxycarbonyl group, neopentyloxycarbonyl group, tert-pentyloxycarbonyl group, hexyloxycarbonyl group, cyclopropyloxycarbonyl group, cyclobutyloxycarbonyl group, cyclopentyloxycarbonyl group, cyclohexyloxycarbonyl group, cyclopropylmethyloxycarbonyl group, 1-cyclopropylethyloxycarbonyl group, 2-cyclopropylethyloxycarbonyl group, cyclobutylmethyloxycarbonyl group, 2-cyclobutylethyloxycarbonyl group and cyclopentylmethyloxycarbonyl group.

In the "amino group which is arbitrarily mono- or di-substituted with a substituted or unsubstituted C₁₋₆ alkyl group", the amino group which may be mono- or di-substituted with a C₁₋₆ alkyl group means an amino group in which one or two hydrogen atoms of the amino group may be substituted with the above-mentioned "C₁₋₆ alkyl group". Specific examples thereof include an amino group, methylamino group, ethylamino group, propylamino group, isopropylamino group, butylamino group, isobutylamino group, pentylamino group, isopentylamino group, hexylamino group, isohexylamino group, dimethylamino group, diethylamino group, dipropylamino group, diisopropylamino group, dibutylamino group, dipentylamino group, ethylmethylamino group, methylpropylamino group, ethylpropylamino group, butylmethylamino group, butylethylamino group, and butylpropylamino group.

Examples of the protective group for the "protected or unprotected hydroxyl group" include alkyl protective groups such as a methyl group, tert-butyl group, benzyl group, trityl group, and methoxymethyl group; silyl protective groups such as a trimethylsilyl group and tert-butyldimethylsilyl group; acyl protective groups such as a formyl group, acetyl group, and benzoyl group; and carbonate protective groups such as a methoxycarbonyl group and benzyloxycarbonyl group.

Examples of the protective group for the "protected or unprotected carboxyl group" include alkylester protective groups such as a methyl group, ethyl group, tert-butyl group, benzyl group, diphenylmethyl group, and trityl group; and silyl ester protective groups such as a trimethylsilyl group and tert-butyldimethylsilyl group.

In the "carbamoyl group which is arbitrarily mono- or di-substituted with a substituted or unsubstituted C₁₋₆ alkyl group", the carbamoyl group which may be mono- or di-substituted with a C₁₋₆ alkyl group means a carbamoyl, group in which one or two hydrogen atoms bonded to the nitrogen atom of the carbamoyl group may be substituted with the above-mentioned "C₁₋₆ alkyl group". Specific examples thereof include a carbamoyl, group, methylcarbamoyl group, ethylcarbamoyl group, propylcarbamoyl group, isopropylcarbamoyl group, cyclopropylcarbamoyl group, butylcarbamoyl group, isobutylcarbamoyl group, pentylcarbamoyl group, isopentylcarbamoyl group, hexylcarbamoyl group, isohexylcarbamoyl group, dimethylcarbamoyl group, diethylcarbamoyl group, dipropylcarbamoyl group, diisopropylcarbamoyl group, dibutylcarbamoyl group, dipentylcarbamoyl group, ethylmethylcarbamoyl group, methylpropylcarbamoyl group, ethylpropylcarbamoyl group, butylmethylcarbamoyl group, butylethylcarbamoyl group, and butylpropylcarbamoyl group.

Examples of the "C₁₋₆ alkanoyl group" include a formyl group, acetyl group, propionyl group, butyryl group, isobutyryl group, valeryl group, isovaleryl group, pivaloyl group, and hexanoyl group.

Examples of the "C₁₋₆ alkylthio group" include a methylthio group, ethylthio group, propylthio group, isopropylthio group, butylthio group, isobutylthio group, sec-butylthio group, tert-butylthio group, pentylthio group, isopentylthio group, tert-pentylthio group, neopentylthio group, 2-methylbutylthio group, 1,2-dimethylpropylthio group, 1-ethylpropylthio group, hexylthio group, cyclopropylthio group, cyclobutylthio group, cyclopentylthio group, cyclohexylthio group, cyclopropylmethylthio group, 1-cyclopropylethylthio group, 2-cyclopropylethylthio group, cyclobutylmethylthio group, 2-cyclobutylethylthio group, and cyclopentylmethylthio group.

Examples of the "C₁₋₆ alkylsulfinyl group" include a methylsulfinyl group, ethylsulfinyl group, propylsulfinyl group, isopropylsulfinyl group, butylsulfinyl group, isobutylsulfinyl group, sec-butylsulfinyl group, tert-butylsulfinyl group, pentylsulfinyl group, isopentylsulfinyl group, tert-pentylsulfinyl group, neopentylsulfinyl group, 2-methylbutylsulfinyl group, 1,2-dimethylpropylsulfinyl group, 1-ethylpropylsulfinyl group, hexylsulfinyl group, cyclopropylsulfinyl group, cyclobutylsulfinyl group, cyclopentylsulfinyl group, cyclohexylsulfinyl group, cyclopropylmethylsulfinyl group, 1-cyclopropylethylsulfinyl group, 2-cyclopropylethylsulfinyl group, cyclobutylmethylsulfinyl group, 2-cyclobutylethylsulfinyl group, and cyclopentylmethylsulfinyl group.

Examples of the "C₁₋₆ alkylsulfonyl group" include a methylsulfonyl group, ethylsulfonyl group, propylsulfonyl group, isopropylsulfonyl group, butylsulfonyl group, isobutylsulfonyl group, sec-butylsulfonyl group, tert-butylsulfonyl group, pentylsulfonyl group, isopentylsulfonyl group, tert-pentylsulfonyl group, neopentylsulfonyl group, 2-methylbutylsulfonyl group, 1,2-dimethylpropylsulfonyl group, 1-ethylpropylsulfonyl group, hexylsulfonyl group, cyclopropylsulfonyl group, cyclobutylsulfonyl group, cyclopentylsulfonyl group, cyclohexylsulfonyl group, cyclopropylmethylsulfonyl group, 1-cyclopropylethylsulfonyl group, 2-cyclopropylethylsulfonyl group, cyclobutylmethylsulfonyl group, 2-cyclobutylethylsulfonyl group, and cyclopentylmethylsulfonyl group.

In the "sulfamoyl group which may be mono- or di-substztuted with a substituted or unsubstituted C₁₋₆ alkyl group", the sulfamoyl group which may be mono- or di-substituted with a C₁₋₆ alkyl group means a sulfamoyl group in which one or two hydrogen atoms bonded to the nitrogen atom of the sulfamoyl group may be substituted with the above-mentioned "C₁₋₆ alkyl group". Specific examples thereof include a sulfamoyl group, methylsulfamoyl group, ethylsulfamoyl group, propylsulfamoyl group, isopropylsulfamoyl group, cyclopropylsulfamoyl group, butylsulfamoyl group, isobutylsulfamoyl group, pentylsulfamoyl group, isopentylsulfamoyl group, hexylsulfamoyl group, isohexylsulfamoyl group, dimethylsulfamoyl group, diethylsulfamoyl group, dipropylsulfamoyl group, diisopropylsulfamoyl group, dibutylsulfamoyl group, dipentylsulfamoyl group, ethylmethylsulfamoyl group, methylpropylsulfamoyl group, ethylpropylsulfamoyl group, butylmethylsulfamoyl group, butylethylsulfamoyl group, and butylpropylsulfamoyl group.

Examples of the "substituents" of the "substituted or unsubstituted hydrocarbon group", the "substituted or unsubstituted heterocyclic group", the "substituted or unsubstituted C₁₋₆ alkoxy group", the "substituted or unsubstituted C₁₋₆ alkoxycarbonyl, group", the "amino group which may be mono- or di-substituted with a substituted or unsubstituted C₁₋₆ alkyl group", the "carbamoyl group which may be mono- or di-substituted with a substituted or unsubstituted C₁₋₆ alkyl group", or the "sulfamoyl group which may be mono- or di-substituted with a substituted or unsubstituted C₁₋₆ alkyl group" in R¹ include (a) alkyl, alkenyl, alkynyl, aryl, cycloalkyl, and cycloalkenyl; (b) heterocyclic groups; (c) amino; (d) imidoyl, amidino, hydroxyl, thiol, and oxo; (e) halogen atoms such as fluorine, chlorine, bromine, and iodine, cyano, and nitro; (f) carboxyl; and (g) carbamoyl, thiocarbamoyl, sulfonyl, sulfinyl, sulfide, and acyl. Among (a) to (g) mentioned above, the groups except for (e) may further have a substituent. The above groups in R¹ may be arbitrarily substituted with 1 to 5 such substituents as "substituent" of each of the "substituted or unsubstituted group" in R¹. Examples of the substituents (a) to (g) will now be described specifically.
(a) The alkyl, alkenyl, alkynyl, aryl, cycloalkyl, and cycloalkenyl groups may be any of the "alkyl, groups", "alkenyl groups", "alkynyl groups", "aryl groups", "cycloalkyl groups" and "cycloalkenyl groups" mentioned as examples of the "hydrocarbon group" for R¹. The preferred groups are C₁₋₆ alkyl groups, C₂₋₆ alkenyl groups, C₂₋₆ alkynyl groups, C₆₋₁₄ aryl groups, C₃₋₇ cycloalkyl groups, and C₃₋₆ cycloalkenyl groups.

These groups may further include an optional substituent RI (wherein RI represents a group selected from C₁₋₆ alkoxyl, C₁₋₆ alkoxycarbonyl, carboxyl, carbamoyl which may be mono- or di-substituted with C₁₋₆ alkyl, halogen, C₁₋₆ alkyl, halogenated C₁₋₆ alkyl, amino which may be mono- or di-substituted with C₁₋₆ alkyl, C₂₋₆ alkenoylamino, nitro, hydroxyl, phenyl, phenoxy, benzyl, pyridyl, oxo, cyano, and amidino).
(b) The heterocyclic group may be any of the "aromatic heterocyclic groups" and "non-aromatic heterocyclic groups" mentioned as examples of the "heterocyclic group" for R¹. More preferably, the heterocyclic groups include (i) "five- or six-membered, monocyclic aromatic heterocyclic groups", (ii) "eight- to twelve-membered, fused, aromatic heterocyclic groups", and (iii) "three- to eight-membered, saturated or unsaturated, non-aromatic heterocyclic groups" which contain 1 to 4 heteroatoms selected from a nitrogen atom, an oxygen atom, and a sulfur atom in addition to carbon atoms.
   These groups may further include 1 to 3 optional substituents RII (wherein RII represents a halogen atom such as fluorine, chlorine, bromine, or iodine; a C₁₋₆ alkyl group, a C₁₋₆ alkanoyl group, or a benzoyl group).
(c) The "substituted or unsubstituted amino group" may be, for example, an amino group which may be mono- or di-substituted with a substituent RIII (wherein RIII represents a group selected from C₁₋₆ alkyl, C₁₋₆ alkanoyl, C₂₋₆ alkenyl, benzoyl, benzyl, phenyl, pyridyl which may be substituted with a group selected from C₁₋₆ alkyl, halogen, and trifluoromethyl, and C₁₋₆ alkoxycarbonyl which may be substituted with 1 to 5 halogen atoms), or three- to eight-membered monocyclic amino group which may be substituted with a group selected from C₁₋₆ alkyl, C₇₋₁₀ aralkyl, and C₆₋₁₀ aryl.
(d) Examples of the substituents in "the substituted or unsubstituted imidoyl group, the substituted or unsubstituted amidino group, the substituted or unsubstituted hydroxyl group, and the substituted or unsubstituted thiol group" include RIII (wherein RIII represents a group selected from C₁₋₆ alkyl, C₁₋₆ alkanoyl, C₂₋₆ alkenyl, benzoyl, benzyl, phenyl, pyridyl which is arbitrarily substituted with a group selected from C₁₋₆ alkyl, halogen, and trifluoromethyl, and C₁₋₆ alkoxycarbonyl which may be substituted with 1 to 5 halogen atoms) described in (c) described above.
   Accordingly, examples of (d) include C₁₋₆ alkylimidoyl groups, a formimidoyl group, an amidino group, C₁₋₆ alkoxy groups, a benzyloxy group, C₁₋₆ alkanoyloxy groups, a phenoxy group, pyridyloxy groups which may be substituted with a group selected from C₁₋₆ alkyl, halogen, and trifluoromethyl, and an oxo group.
   Examples of (e) include halogen atoms such as fluorine, chlorine, bromine, and iodine; a cyano group; and a nitro group.
(f) The "substituted or unsubstituted carboxyl groups" include a carboxyl group, C₁₋₆ alkoxycarbonyl groups, C₇₋₁₂ aryloxycarbonyl groups, and C₆₋₁₀ aryl-C₁₋₄ alkoxycarbonyl groups. The aryl group in such (f) may be further substituted with a substituent RIV. RIV represents an amino group which may be mono- or di-substituted with a substituent RIII' (wherein RII' represents a C₁₋₆ alkyl group, a C₁₋₆ alkanoyl group, or a benzoyl group); a halogen atom; a hydroxyl group; a nitro group; a cyano group; a C₁₋₆ alkyl group which may be substituted with 1 to 5 halogen atoms; or an alkoxy group which may be substituted with 1 to 5 halogen atoms.
(g) Examples of "the substituted or unsubstituted carbamoyl group, the substituted or unsubstituted thiocarbamoyl group, the substituted or unsubstituted sulfonyl group, the substituted or unsubstituted sulfinyl group, the substituted or unsubstituted sulfide group, and the substituted or unsubstituted acyl group" include groups represented by -CONRgRg', -CSNRgRg', -SO_{y}-Rg, or -CO-Rg, wherein Rg represents a hydrogen atom or a substituent RV (wherein RV represents C₁₋₆ alkyl, C₃₋₆ cycloalkyl, C₆₋₁₀ aryl, C₇₋₁₀ aralkyl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain 1 to 4 heteroatoms selected from an oxygen atom, a sulfur atom, and a nitrogen atom in addition to the carbon atoms; and the alkyl, the cycloalkyl, the aryl, the aralkyl, or the heterocyclic group may be further substituted with 1 to 5 substituents RIV of (f) described above); Rg' is a hydrogen atom or a group selected from C₁₋₆ alkyl groups, C₃₋₆ cycloalkyl groups, and C₇₋₁₀ aralkyl groups; and y is 0, 1, or 2.

[1-1-a] In the compounds represented by formula (I) of embodiment [1], examples of R¹ preferably include halogen atoms, substituted or unsubstituted hydrocarbon groups, substituted or unsubstituted heterocyclic groups, and substituted or unsubstituted C₁₋₆ alkoxy groups. Examples of the "substituted or unsubstituted hydrocarbon group" and the "substituted or unsubstituted heterocyclic group" include (1) C₁₋₁₀ alkyl groups; (2) C₂₋₆ alkenyl groups; (3) C₂₋₆ alkynyl groups; (4) C₃₋₉ cycloalkyl groups; (5) C₃₋₆ cycloalkenyl groups; (6) C₄₋₆ cycloalkanedienyl groups; (7) C₆₋₁₄ aryl groups; (8) heterocyclic groups each containing 1 to 4 hetero-atoms selected from an oxygen atom, a sulfur atom, and a nitrogen atom in addition to the carbon atoms, the heterocyclic groups being selected from (i) five- or six-membered, monocyclic aromatic heterocyclic groups, (ii) eight- to twelve-membered, fused aromatic heterocyclic groups, and (iii) "three- to eight-membered, saturated or unsaturated, non-aromatic heterocyclic groups; and (9) substituted or unsubstituted C₁₋₆ alkoxy groups. Each of the groups in (1) to (9) may be either unsubstituted or substituted with 1 to 5 substituents in a class selected from (a-1) to (g-1) as described below.

The classes are as follows.
(a-1): Substituents include C₁₋₆ alkyl groups, C₂₋₆ alkenyl groups, C₂₋₆ alkynyl groups, C₆₋₁₄ aryl groups, C₃₋₇ cycloalkyl groups, and C₃₋₆ cycloalkenyl groups. These substituents may be further substituted with a substituent RI (wherein RI represents a group selected from C₁₋₆ alkoxy, C₁₋₆ alkoxycarbonyl, carboxyl, carbamoyl which is arbitrarily mono- or di-substituted with C₁₋₆ alkyl, halogen, C₁₋₆ alkyl, halogenated C₁₋₆ alkyl, amino which is arbitrarily mono- or di-substituted with C₁₋₆ alkyl, C₂₋₆ alkenoylamino, nitro, hydroxy, pyridyl, oxo, cyano, and amidino).
(b-1): Substituents are any one of heterocyclic groups of (i) five- or six-membered, monocyclic aromatic heterocyclic groups, (ii) eight- to twelve-membered, fused aromatic heterocyclic groups, and (iii) "three- to eight-membered, saturated or unsaturated, non-aromatic heterocyclic groups which contain 1 to heteroatoms selected from an oxygen atom, a sulfur atom, and a nitrogen atom in addition to the carbon atoms,. These heterocyclic groups may be further substituted with a substituent RII (wherein RII represents a group selected from halogen atoms such as fluorine, chlorine, bromine, and iodine; C₁₋₆ alkyl, C₁₋₆ alkanoyl, and benzyl).
(c-1): Substituents in (c-1) include an amino group which may be substituted with a substituent RIII (wherein RIII represents a group selected from C₁₋₆ alkyl, C₁₋₆ alkanoyl, C₂₋₆ alkenoyl, benzoyl, benzyl, phenyl, pyridyl which may be substituted with a group selected from C₁₋₆ alkyl, halogen, and trifluoromethyl, and C₁₋₆ alkoxycarbonyl which may be substituted with to 5 halogen atoms), or a three- to eight-membered monocyclic amino group which may be substituted with a group selected from C₁₋₆ alkyl, C₇₋₁₀ aralkyl, and C₆₋₁₀ aryl.
(d-1): Substituents in (d-1) include an imidoyl group, an amidino group, a hydroxyl group, a thiol group, and an oxo group. These substituents may be substituted with groups selected from the substituents RIII described in (c-1) described above.
(e-1): Substituents in (e-1) include halogen atoms such as fluorine, chlorine, bromine, and iodine, a cyano group, and a nitro group.
(f-1): Substituents in (f-1) include a carboxyl group, C₁₋₆ alkoxycarbonyl groups, C₇₋₁₂ aryloxycarbonyl groups, and C₆₋₁₀ aryl-C₁₋₄ alkoxycarbonyl groups. The aryl groups in (f-1) may be further substituted with a substituent RIV' (wherein RIV' represents amino which may be mono- or di-substituted with groups selected from RIII described in (c-1) described above; C₁₋₆ alkyl or C₁₋₆ alkoxy which may be substituted with 1 to 5 halogen atoms; halogen atoms; hydroxyl; nitro; and cyano).
(g-1): Substituents in (g-1) include groups represented by - CONRgRg', -CSNRgRg', -CO-Rg, and -SO_{y}-Rg wherein Rg represents a hydrogen atom or a substituent RV (wherein RV represents C₁₋₆ alkyl, C₃₋₆ cycloalkyl, C₆₋₁₀ aryl, C₇₋₁₀ aralkyl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain 1 to 4 heteroatoms selected from an oxygen atom, a sulfur atom, and a nitrogen atom in addition to the carbon atoms; and the alkyl, the cycloalkyl, the aryl, the aralkyl, or the heterocyclic group may be further substituted with 1 to 5 substituents RIV of (f) described above); Rg' is a hydrogen atom or a group selected from C₁₋₆ alkyl groups, C₃₋₆ cycloalkyl groups, and C₇₋₁₀ aralkyl groups; and y is 0, 1, or 2.

In the groups listed in (a-1) to (g-1) described above, "particularly preferable groups" include substituents such as C₁₋₆ alkyl, C₂₋₆ alkenyl, C₂₋₆ alkynyl, halogen atoms, halogenated C₁₋₆ alkyl, cyano, amino, hydroxyl, carbamoyl, C₁₋₆ alkoxy, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, oxo, morpholinylcarbonyl, morpholinylsulfonyl, 5-trifluoromethylpyridin-2-yloxy, quinoxalin-2-yl, (pyridin-4-yl)methyl, 1,2,3-thiadiazolo-4-yl, 1H-pyrazolo-1-yl, 4-chlorophenyl, tetrahydrofuranyl and oxyranyl. The aromatic rings in these substituents may be further substituted with 1 to 5 substituents selected from halogen atoms, trifluoromethyl, cyano, hydroxyl, amino, nitro, carboxyl, carbamoyl, C₁₋₆ alkyl, C₁₋₆ alkoxy, mono/di C₁₋₆ alkylamino, di-C₁₋₆ alkylcarbamoyl, C₁₋₆ alkoxycarbonyl, N-C₁₋₆ alkylcarbamoyl, N,N-di C₁₋₆ alkylcarbamoyl, and C₂₋₆ alkenoylamino.

[1-1-b] Preferably, R¹ is a halogen atom, and (1) a C₁₋₆ alkyl group, (2) a C₂₋₆ alkenyl group, (7) a C₆₋₁₄ aryl group, and (9) a C₁₋₆ alkoxy group. Each group in (1), (2), (7), and (9) is arbitrarily substituted with 1 to 5 substituents in a class selected from (a-1) to (g-1) in [1-1] described above (in particular, the substituents listed as "particularly preferable groups").

[1-1-c] More preferably, R¹ is a halogen atom (a fluorine atom, a chlorine atom, a bromine atom, or an iodine atom), and a C₁₋₆ alkyl group (in particular, C₁₋₄ alkyl group) or C₁₋₆ alkoxy group (in particular, C₁₋₄ alkoxy group) which may be substituted with 1 to 5 halogen atoms.

[1-1-d] Further preferably, R¹ is a halogen atom (particularly preferably, a fluorine atom or a chlorine atom), and a C₁₋₄ alkyl group or C₁₋₄ alkoxy group which is arbitrarily substituted with 1 to 5 halogen atoms. More specifically, examples thereof include a fluorine atom, a chlorine atom, methyl, ethyl, propyl, isopropyl, butyl, isobutyl, sec-butyl, tert-butyl, trifluoromethyl, methoxy, ethoxy, propoxy, isopropoxy, butoxy, sec-butoxy, tert-butoxy, trifluoromethoxy, and tetrafluoroethoxy.

[1-1-e] Particularly preferably, R¹ is a fluorine atom, a chlorine atom, isobutyl, tert-butyl, trifluoromethyl, or tetrafluoroethoxy. Still more preferably, R¹ is trifluoromethyl.

[1-2] In the compounds represented by formula (I) of embodiment [1], n is an integer of 0 to 2. Preferably, n is 1 or 2, and more preferably, n is 1.

The substitution position of R¹ may be any position except for the condensation position of the five- or six-membered aryl ring or heteroaryl ring represented by "Cycle" in formula (I).

### [1-2-1]

More preferably, when the "Cycle" is a six-membered ring, at least one of R¹'s is preferably bonded to the 4th position (A₂) in the clockwise direction from the condensation position close to the carbon atom of the cyclidene in the partial structural formula (wherein each of A₁ to A₄ is either CH or N) below.

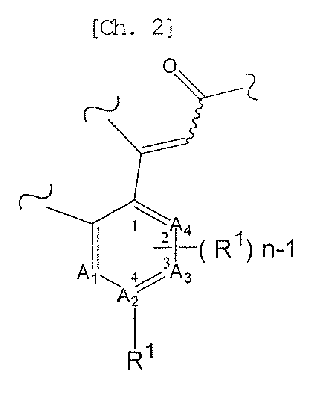

[1-2-1a] For example, this position corresponds to the 7th position of a chroman ring, a pyridochroman ring, a 2,3-dihydroquinoline ring, or the like, which belongs to a skeleton in which m = 1 and q = 0, or an isochroman ring or the like, which belongs to a skeleton in which m = 0 and q = 1.

[1-2-1b] This position corresponds to the 8th position of a 3,4-dihydrobenzo[b]oxepine ring or a 1,2,3,4-tetrahydrobenzo[b]azepine ring, which belongs to a skeleton in which m = 2 and q = 0, or a 3,4-dihydrobenzo[b]isooxepine ring or the like, which belongs to a skeleton in which m = 1 and q = 1.

[1-2-2] When the "Cycle" is a five-membered ring, at least one of R¹'s is preferably bonded to the 3rd position (B₂) in the clockwise direction from the condensation position close to the carbon atom of the cyclidene in the partial structural formula (wherein each of B₁ to B₃ is any one of CH, N, O, and S) below.

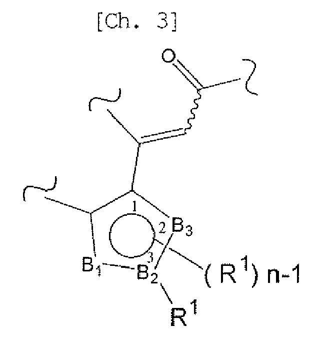

[1-2-2a] For example, this position corresponds to the 6th position of a 2,3-dihydro-4H-pyrano[2,3b]pyrrole ring or a 2,3-dihydro-thieno[2,3-b]pyran ring, which belongs to a skeleton in which m = 1 and q = 0. This position corresponds to the 2nd position of a 5,6-dihydro-furo[2,3-b]pyran ring, which belongs to a skeleton in which m = 1 and q = 0. In the all embodiments [1-2] to [1-2-2b], at least one of R¹'s is preferably a fluorine atom, a chlorine atom, isobutyl, tert-butyl, trifluoromethyl, or tetrafluoroethoxy. More preferably, at least R¹ bonded to A₂ or B₂ is a fluorine atom, a chlorine atom, isobutyl, tert-butyl, trifluoromethyl, or tetrafluoroethoxy, and particularly preferably, trifluoromethyl.

[1-3] In the compounds represented by formula (I) of embodiment [1], R² is a halogen atom, a substituted or unsubstituted amino group, a substituted or unsubstituted hydrocarbon group, a substituted or unsubstituted aromatic heterocyclic group, or an oxo group.

Examples of the "halogen atom" include a fluorine atom, a chlorine atom, a bromine atom, and an iodine atom.

Examples of the "substituted or unsubstituted amino group" include amino groups which may be mono- or di-substituted with a substituent RIII (wherein RIII represents a group selected from C₁₋₆ alkyl, C₁₋₆ alkanoyl, C₂₋₆ alkenoyl, benzyl, and C₁₋₆ alkoxycarbonyl which is arbitrarily substituted with 1 to 5 halogen atoms) , or three- to eight-membered monocyclic amino group which may be substituted with a group selected from C₁₋₆ alkyl, C₇₋₁₀ aralkyl, and C₆₋₁₀ aryl.

Aromatic rings of these substituents may further include 1 to 3 optional substituents selected from halogen atoms, trifluoromethyl, cyano, hydroxyl, amino, nitro, carboxyl, carbamoyl, C₁₋₆ alkyl, C₁₋₆ alkoxy, mono/di C₁₋₆ alkylamino, di-C₁₋₆ alkylcarbamoyl, C₁₋₆ alkoxycarbonyl, N-C₁₋₆ alkylcarbamoyl, N, N-di C₁₋₆ alkylcarbamoyl, and C₂₋₆ alkenoylamino.

The "substituted or unsubstituted hydrocarbon group" represents the same meaning as described in R¹ of embodiment [1-1] described above. Examples of the "hydrocarbon group" include alkyl groups (for example, C₁₋₁₀ (more preferably C₁₋₆) alkyl groups), alkenyl groups (for example, C₂₋₆ alkenyl groups), cycloalkyl groups (for example, C₃₋₉ cycloalkyl groups), cycloalkenyl groups (for example, C₃₋₆ cycloalkenyl groups), and aryl groups.

The "aromatic heterocyclic group" of the "substituted or unsubstituted aromatic heterocyclic group" represents the same meaning as described in R¹ described above.

Substituents of these groups are the same groups as those listed as "particularly preferable groups" in the groups described in (a-1) to (g-1) in R¹ described above.

[1-3-a] In the compounds represented by formula (I) of embodiment [1], R² is preferably a fluorine atom, a chlorine atom, an amino group which is arbitrarily mono-substituted with a substituent RIII,a C₁₋₆ alkyl group which is arbitrarily mono-substituted with a group selected from a C₁₋₆ alkoxy, amino and mono/di C₁₋₆ alkylamino, or a phenyl group. More preferably, R² is a C₁₋₆ alkyl group which is arbitrarily mono-substituted with a group selected from a C₁₋₆ alkoxy, amino and mono/di C₁₋₆ alkylamino (in particular, a C₁₋₄ alkyl group such as methyl, ethyl, propyl, isopropyl, butyl, isobutyl, sec-butyl, or tert-butyl, methoxymethyl, 2-methoxyethyl). Further preferably, R² is methyl, ethyl, methoxyethyl.

[1-4] In the compounds represented by formula (I) of embodiment [1], p is an integer of 0 to 2. Preferably, p is 0 or 2 except cases raised in the following [1-4-a] to [1-4-c].

[1-4-a] However, in the compounds represented by formula (I), when R² is a C₁₋₆ alkyl group (in particular, a C₁₋₄ alkyl group such as methyl, ethyl, propyl, isopropyl, butyl, isobutyl, sec-butyl, or tert-butyl), p is preferably 1 or 2, and more preferably 2 and is bonded to geminal position. Alternatively, two geminal or vicinal R² may bind to each other to form a C₂₋₆ alkylene group respectively, and form a cyclo ring group together with the carbon atom to which the two R² are bonded, or the cyclo ring group may form non-aromatic heterocyclic groups containing an oxygen atom or a nitrogen atom. Three to eight-membered rings are preferable. For example, a cyclopropane ring, a cyclobutane ring, a cyclopentane ring, a cyclohexane ring, oxirane ring, oxetane ring, tetrahydrofuran ring, tetrahydropyran ring, aziridine ring, azetidine ring, pyrrolidine ring or piperizine ring can be formed.

[1-4-b] However, in the compounds represented by formula (I), when R² is a fluorine atom, p is preferably 1 or 2, and more preferably 2.

[1-4-c] In the compounds represented by formula (I), when R² is an amino group which may be mono-substituted with a substituent RIII or an oxo group, p is preferably 1 or 2, and more preferably 1.

[1-5] In the compounds represented by formula (I) of embodiment [1], m is 0 to 2, and preferably 1 or 2. In either case, the carbon atom or atoms located at the position corresponding to m may be substituted with R².

[1-6] In the compounds represented by formula (I) of embodiment [1], X₁ represents an oxygen atom, -NR³- (wherein R³ is a hydrogen atom, a substituted or unsubstituted hydrocarbon group, a substituted or unsubstituted heterocyclic group, or a substituted or unsubstituted acyl group), or -S(O)r- (wherein r is an integer of 0 to 2).

When R³ is a substituted or unsubstituted hydrocarbon group or a substituted or unsubstituted heterocyclic group, examples of the hydrocarbon group or the heterocyclic group include those listed in the "substituted or unsubstituted hydrocarbon groups" or the "substituted or unsubstituted heterocyclic groups", respectively, in [1-1] mentioned above. These groups may be substituted with 1 to 3 "substituents" listed in (a) to (g).

When R³ is a "substituted or unsubstituted acyl group", R³ is a group represented by -CO-Rg (wherein Rg is the same as the above) in (g) of [1-1] described above.

[1-6-a] In the compounds represented by formula (I) of embodiment [1], preferably, X₁ is an oxygen atom or -NR³'- (wherein R³' is a substituted or unsubstituted hydrocarbon group, a substituted or unsubstituted heterocyclic group, or a substituted or unsubstituted acyl group all of which is defined in R³). More preferably, X₁ is an oxygen atom.

[1-6-b] When X₁ is -NR³'-, examples of the "substituted or unsubstituted hydrocarbon group" or the "substituted or unsubstituted heterocyclic group" of R³' preferably include (1) C₁₋₁₀ alkyl groups; (2) C₂₋₆ alkenyl groups; (3) C₂₋₆ alkynyl groups; (4) C₃₋₉ cycloalkyl groups; (5) C₃₋₆ cycloalkenyl groups; (6) C₄₋₆ cycloalkanedienyl groups; (7) C₆₋₁₄ aryl groups; and (8) heterocyclic groups each containing 1 to 4 hetero-atoms selected from an oxygen atom, a sulfur atom, and a nitrogen atom in addition to the carbon atoms, the heterocyclic groups being selected from (i) five- or six-membered, monocyclic aromatic heterocyclic groups, (ii) eight- to twelve-membered, fused aromatic heterocyclic groups, and (iii) "three- to eight-membered, saturated or unsaturated, non-aromatic heterocyclic groups, and each of the groups in (1) to (8) may be either unsubstituted or arbitrarily substituted with 1 to 5 substituents in a class selected from (a-1) to (g-1) described in [1-1-a] above.

When X₁ is -NR³'-, examples of the "substituted or unsubstituted acyl group" of R³' preferably include groups represented by -CO-Rg'' (wherein Rg" represents a substituent RV (wherein RV represents C₁₋₆ alkyl, C₃₋₆ cycloalkyl, C₆₋₁₀ aryl, C₇₋₁₀ aralkyl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain 1 to 4 heteroatoms selected from an oxygen atom, a sulfur atom, and a nitrogen atom in addition to the carbon atoms; and the alkyl, the cycloalkyl, the aryl, the aralkyl, or the heterocyclic group may be further substituted with 1 to 5 substituents RIV of (f) described above).

[1-6-c] More preferably, when X₁ is -NR³'-, examples of the "substituted or unsubstituted hydrocarbon groups" or the "substituted or unsubstituted heterocyclic group" of R³' include (1') C₁₋₆ alkyl groups; (2') C₂₋₆ alkenyl groups; (4') C₃₋₆ cycloalkyl groups; (7') C₆₋₁₄ aryl groups; and (8') heterocyclic groups each containing 1 heteroatom or 2 heteroatoms selected from an oxygen atom, a sulfur atom, and a nitrogen atom in addition to the carbon atoms, the heterocyclic groups being selected from (i) five- or six-membered, monocyclic aromatic heterocyclic groups, (ii) eight- to twelve-membered, fused aromatic heterocyclic groups, and (iii) "three- to eight-membered, saturated or unsaturated, non-aromatic heterocyclic groups, and each of the groups in (1'), (2'), (4'), (7'), and (8') may be mono-substituted with a substituent in a class selected from the substituents (a-1) to (g-1) (in particular, the substituents listed as "particularly preferable groups" in (a-1) to (g-1)).

More preferably, when X₁ is -NR³'-, examples of the "substituted or unsubstituted acyl group" of R³' include groups represented by -CO-Rg''' (wherein Rg''' represents a substituent RV' (wherein RV' represents C₁₋₆ alkyl, C₃₋₆ cycloalkyl, C₆₋₁₀ aryl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain 1 heteroatom or 2 heteroatoms selected from an oxygen atom, a sulfur atom, and a nitrogen atom in addition to the carbon atoms; and the alkyl, the cycloalkyl, the aryl, or the heterocyclic group may be further substituted with 1 to 5 substituents RIV of (f) described above).

[1-6-d] Further preferably, when X₁ is -NR³'-, examples of the "substituted or unsubstituted hydrocarbon group" or the "substituted or unsubstituted heterocyclic group" of R³' include (1'') C₁₋₆ alkyl groups; (4'') C₃₋₆ cycloalkyl groups; (7'') C₆₋₁₄ aryl groups; and (8'') heterocyclic groups each containing a heteroatom selected from an oxygen atom, a sulfur atom, and a nitrogen atom in addition to the carbon atoms, the heterocyclic groups being selected from (i) five- or six-membered, monocyclic aromatic heterocyclic groups, (ii) eight- to twelve-membered, fused aromatic heterocyclic groups, and (iii) "three-to eight-membered, saturated or unsaturated, non-aromatic heterocyclic groups, and each of the groups in (1''), (4''), (7''), and (8'') may be mono-substituted with a substituent in a class selected from the substituents (a-1) to (g-1) (in particular, the substituents listed as "particularly preferable groups" in (a-1) to (g-1)).

Further preferably, when X₁ is -NR³'-, examples of the "substituted or unsubstituted acyl group" of R³' include groups represented by -CO-Rg'''' (wherein Rg'''' represents a substituent RV'' (wherein RV'' represents C₁₋₆ alkyl, C₃₋₆ cycloalkyl, C₆₋₁₀ aryl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain a heteroatom selected from an oxygen atom, a sulfur atom, and a nitrogen atom in addition to the carbon atoms; and the alkyl, the cycloalkyl, the aryl, or the heterocyclic group may be further substituted with 1 to 3 substituents RIV of (f) described above).

[1-6-e] Particularly preferably, when X₁ is -NR³'-, examples of the "substituted or unsubstituted hydrocarbon group" or the "substituted or unsubstituted heterocyclic group" of R³' include (1''') methyl and (1''') ethyl, (4''') cyclohexyl, (7''') phenyl and (7''') naphthyl (e.g., naphthalen-1-yl and naphthalen-2-yl), and (8''') pyridyl (e.g., pyridin-2-yl, pyridin-3-yl, and pyridin-4-yl) which may be substituted with a halogen atom. More specifically, examples thereof include methyl, trifluoromethyl, ethyl, cyclohexyl, 2-chlorophenyl, 3-chlorophenyl, 4-chlorophenyl, naphthalen-1-yl, naphthalen-2-yl, and 3-chloro-pyridin-2-yl.

Particularly preferably, when X₁ is -NR³'-, examples of the "substituted or unsubstituted acyl group" of R³' include groups represented by -CO-Rg''''' (wherein Rg''''' represents a substituent RV''' (wherein RV''' represents methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethylpropyl, 1-ethyl-1-methylpropyl, 1-ethyl-2-methylpropyl, n-hexyl, cyclopropyl, cyclobutyl, cyclopentyl, cyclohexyl, phenyl, heptyl, naphthyl, tetrahydropyran-4-yl, pyridyl (e.g., pyridin-2-yl, pyridin-3-yl, and pyridin-4-yl), 2,2-dimethylpropyl, 2-methylpropyl, 3-methylbutyl, 2-methylbutyl, 1-methylbutyl, 1,1-dimethylbutyl, 4,4-difluorocyclohexyl, 3-fluorocyclopentyl, 1-methylcyclopropyl, 1-methylcyclobutyl, 3,3,3-trifluoropropyl, 2,2,2-trifluoroethyl, 4,4,4-trifluorobutyl, phenylmethyl, 1,1-difluoropropyl, and 1-fluoro-1-methylethyl; and the alkyl, the cycloalkyl, the aryl, or the heterocyclic group may be further substituted with a substituent RIV of (f) described above).

More specifically, examples of the groups represented by -CO-Rg''''' include acyl groups which may be halogenated, such as acetyl, pentanoyl, 2-ethylbutanoyl, cyclohexanecarbonyl, 4-pyranoyl, benzoyl, nicotinoyl, cyclopentanecarbonyl, pentanoyl, cyclobutanecarbonyl, 3,3-dimethylbutanoyl, 3-methylbutanoyl, 4-methylpentanoyl, 3-methylpentanoyl, 2-methylpentanoyl, 2,2-dimethylpentanoyl, 4,4-difluorocyclohexanecarbonyl, 3-cyclopentanecarbonyl, 1-methylcyclopropanecarbonyl, 1-methylcyclobutanecarbonyl, 4,4,4-trifluorobutanoyl, 3,3,3-trifluoropropanoyl, 5,5,5-trifluoropentanoyl, 1-phenylacetyl, 2,2-difluorobutanoyl, and 2-fluoro-2-methylpropanoyl.

[1-7] X₂ represents a methylene group, an oxygen atom, -NR⁴-(wherein R⁴ is a hydrogen atom, a C₁₋₆ alkyl group (in particular, a C₁₋₄ alkyl group such as methyl, ethyl, propyl, isopropyl, butyl, isobutyl, sec-butyl, or tert-butyl), or -S(O)r- (wherein r is an integer of 0 to 2).

[1-7-a] In the compounds represented by formula (I) of embodiment [1], X₂ is preferably a methylene group or an -NH- group. More preferably, X₂ is a methylene group.

[1-8] In the compounds represented by formula (I) of embodiment [1], r is an integer of 0 or 1. Preferably, r is 0.

[1-9] In the compounds represented by formula (I) of embodiment [1], examples of the Cycle moiety include the rings described as "aryl groups" in R¹ and the five- to fourteen-membered rings, preferably five- to twelve-membered rings, containing at least one heteroatom (preferably, 1 to 4 heteroatoms) selected from N, O, and S in addition to the carbon atoms, which are described as "aromatic heterocyclic groups".

[1-9-a] More preferably, examples of the Cycle moiety include monocyclic, five- or six-membered rings. A benzene ring and some of the groups described as examples of the monocyclic aromatic heterocyclic groups in R¹ of embodiment [1-1] above correspond to such rings. Specific examples thereof include a benzene ring, a pyridine ring, a pyrimidine ring, a pyridazine ring, a pyrrole ring, a thiophene ring, a furan ring, an imidazole ring, a thiazole ring, and an isothiazole ring.

Regarding the condensation form of the monocyclic aromatic heterocyclic groups, at least one heteroatom is preferably located at positions selected from A₁, A₂, and A₃, or B₁, B₂, and B₃ in the following formulae. More preferably, at least one heteroatom is located at the position of A₁ or B₁. 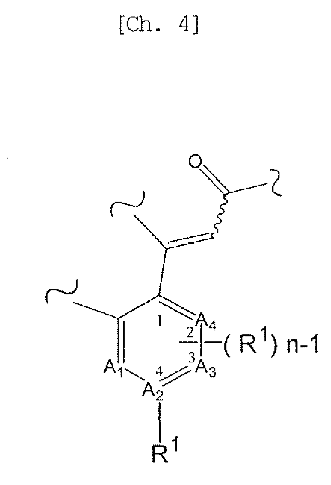 or

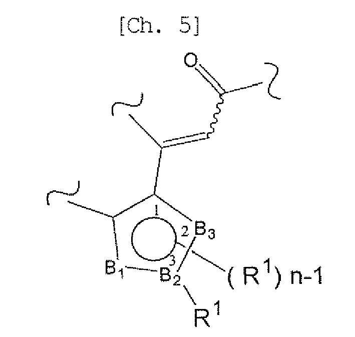

[1-9-b] Zero to two R¹'s described above can be bonded to the Cycle moiety. More specifically, n represents an integer of 0 to 2. Preferably, n is an integer of 1 or 2, and more preferably, n is 1.

[1-9-c] When n is 1, the substitution position of R¹ corresponds to the 7th position of a chroman ring, a pyridochroman ring, a 2,3-dihydroquinoline ring, or the like, which belongs to a skeleton in which m = 1 and q = 0, or an isochroman ring or the like, which belongs to a skeleton in which m = 0 and q = 1. This position also corresponds to the 8th position of a 3,4-dihydrobenzo[b]oxepine ring or a 1,2,3,4-tetrahydrobenzo[b]azepine ring, which belongs to a skeleton in which m = 2 and q = 0, or a 3,4-dihydrobenzo[b]isooxepine ring or the like, which belongs to a skeleton in which m = 1 and q = 1. In the substitution positions of R¹'s, at least one of R¹'s is preferably a fluorine atom, a chlorine atom, isobutyl, tert-butyl, trifluoromethyl, or tetrafluoroethoxy. More preferably, at least R¹ bonded to A₂ or B₂ is a fluorine atom, a chlorine atom, isobutyl, tert-butyl, trifluoromethyl, or tetrafluoroethoxy, and particularly preferably, trifluoromethyl.

[1-10] In the compounds represented by formula (I) of embodiment [1], j is 0 or 1, and preferably 0.

[1-11] In the compounds represented by formula (I) of embodiment [1], k is 0 to 2, and preferably 0 or 2, and more preferably 0.

When j or k is not 0 in the embodiments [1-10] and [1-11], i.e., when j=1 or k=1 or 2, carbon atoms defined by the number of j or k may be mono-substituted by the substituents indicated as "particularly preferable substituent" in the groups shown in (a-1) to (g-1) in the embodiment [1-a].

[1-12] In the compounds represented by formula (I) of embodiment (1), W represents a methylene group, a carboxyl group or a sulfonyl group. W represents preferably carboxyl group or a sulfonyl group. When w represents a methylene group, L₁ is an oxygen atom and L₂ is a - CR^{9A}R^{9B}-.

[1-13] In the compounds represented by formula (I) of embodiment [1], R⁷ represents a hydrogen atom, a substituted or unsubstituted hydrocarbon group, a substituted or unsubstituted heterocyclic group or a substituted or unsubstituted acyl group. When R⁷ is the substituted or unsubstituted hydrocarbon atom or the substituted or unsubstituted heterocyclic group, R⁷ has the same meaning with the "substituted or unsubstituted hydrocarbon group" and the "substituted or unsubstituted heterocyclic group" listed in the [1-1] mentioned above and these groups may be substituted by 1 to 3 "subsituents" listed in (a) to (g).

When R⁷ represents the "substituted or unsubstituted acyl group", R⁷ means -CO-Rg (Rg has the same meaning mentioned above) of (g) in the [1-1] mentioned above.

[1-13-a] In the compounds represented by formula (I) of embodiment [1], R⁷ represents preferably a hydrogen atom, a substituted or unsubstituted hydrocarbon group, or a substituted or unsubstituted heterocyclic group.

[1-13-a-1] Examples of the "substituted or unsubstituted carbonhydrogen group" or the "substituted or unsubstituted heterocyclic group" raised as the preferable R⁷ are:
(1) C₁₋₁₀ alkyl group, (2) C₂₋₆ alkenyl group or (3) C₂₋₆ alkynyl group, (4) C₃₋₉ cycloalkyl group, (5) C₃₋₆ cycloalkenyl group, (6) C₄₋₆ cylcoalcanedienyl group, (7) C₆₋₁₄ aryl group, (8) any one of heterocyclic groups which contain 1 to 4 heterocarbon atoms selected from an oxygen atom, a sulfur atom or a nitrogen atom other than carbon atom, the heterocyclic groups being selected from (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group. The above-mentioned (1) to (8) may be arbitrarily substituted with 1 to 5 substituents in the classes of the subsitutents (a-1) to (g-1) in [1-1-a] mentioned above and the following.

[1-13-a-2] Preferable examples of the "substituted or unsubstituted hydrocarbon group" or the "substituted or unsubstituted heterocyclic group" raised as the preferable R⁷ are:
(1') C₁₋₁₀ alkyl group, (7') C₆₋₁₄ aryl group or (8') any one of heterocyclic groups of (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono- or di-substituted by substituents in the classes of the subsitutents (a-1) to (g-1) (especially, the substituents listed as "particularly preferable").

[1-13-b] In the compounds represented by formula (I) of embodiment [1], more preferably, R⁷ represents a hydrogen atom or (1') C₁₋₁₀ alkyl group, or (8') any one of heterocyclic groups of (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono-or di-substituted by substituents in the classes of the subsitutents (a-1) to (g-1) (especially, the substituents listed as "particularly preferable").

[1-13-c] In the compounds represented by formula (I) of embodiment [1], more preferably, R⁷ represents a hydrogen atom, or C₁₋₆ alkyl group or tetrahydropyraniy (preferably teotrahydropyran-4-yl group) which may be mono- or di-substituted by a substituent such as halogen atom, halogenated C₁₋₆ alkyl, cyano, amino, hydroxyl, carbamoyl, C₁₋₆ alkoxyl group, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, piperazino, oxo, oxiranyl, or tetrahydrofuryl.

[1-13-d] In the compounds represented by formula (I) of embodiment [1], particularily preferably, R⁷ represents a hydrogen atom, or C₁₋₆ alkyl group which may be mono- or di-substituted by a substituent such as amino, hydroxyl, C₁₋₆ alkoxyl, mono/di C₁₋₆ alkylamino, morpholino, piperazino, oxo, oxiranyl, or tetrahydrofuryl.

[1-13-d-1] Examples of the "C₁₋₆ alkyl group" in the substituents of the particularly preferable R⁷ are methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl. Methyl, ethyl , propyl, isopropyl, butyl, isobutyl, or sec-butyl is preferable.

[1-13-e] In the compounds represented by formula (I) of embodiment [1], particularly preferably, R⁷ represents a hydrogen atom, or a methyl group , a ethyl group, a propyl group, isopropyl group, butyl group which may be mono- or di-substituted by a substituent such as amino, hydroxyl, C₁₋₆ alkoxy, mono/di C₁₋₆ alkylamino, phenyl. More concretely, hydrogen atom, methyl group, ethyl group, propyl group, isopropyl group, butyl group, isobutyl, sec-butyl, aminomethyl group, (2-)aminoethyl group, hydroxymethyl group, (2-)hydroxyethyl group, (3-)hydroxypropane-1-yl group, (4-)hydroxybuthyl group, 2-hydroxy-2,2-dimethylethyl group, 1,3-dihydroxy-propane-2-yl group, 1-methyl-2-hydroxyethyl group, 2-hydroxy-propane-1-yl group, methoxyethyl group, (2-)ethoxyethyl group, (2-)N,N-dimethylaminoethyl group, (2-)N,N-diethylaminoethyl group, benzyl group, phenethyl group, oxiranylmethyl group, (2-)tetrahydrofuranylmethyl group etc. (Preferred embodiments are indicated in the parenthesis "( )").

[1-14] In the compounds represented by formula (I) of embodiment [1], R⁸, R^{9A} and R^{9B} each independently represent a substituent arbitrarily selected from a hydrogen atom, a halogen atom, a substituted or unsubstituted hydrocarbon group, a substituted or unsubstituted heterocyclic group, a substituted or unsubstituted C₁₋₆ alkoxy group, a substituted or unsubstituted C₁₋₆ alkoxycarbonyl group, an amino group which may be mono- or di-substituted by a substituted or unsubstituted C₁₋₆ alky group, a protected or unprotected hydroxyl grop, a protected or unprotected carboxyl group, a carbamoyl group which may be mono- or di-substituted by a substituted or unsubstituted C₁₋₆ alky group, a C₁₋₆ alkanoyl, group, C₁₋₆ alkylthio group, a C₁₋₆ alkylsulfinyol group, C₁₋₆ alkylsulfonyl group, a sulfamoyl group which may be mono- or di-substituted by a substituted or unsubstituted C₁₋₆ alky group, a cyano group or a nitro group. Preferably, R⁸, R^{9A} and R^{9B} each independently represent a substituent selected from a hydrogen atom, a substituted or unsubstituted C₁₋₆ alkyl group, a substituted or unsubstituted heterocyclic group, a substituted or unsubstituted C₁₋₆ alkoxy group, an amino group which may be mono- or di-substituted by a substituted or unsubstituted C₁₋₆ alky group, a protected or unprotected hydroxyl group. The definition of each substituent in R⁸, R^{9A} and R^{9B} has the same meaning as defined in the embodiment [1-1] mentioned above.

[1-14-a] In the compounds represented by formula (I) of embodiment [1], preferably, R⁸ represent a hydrogen atom, a substituted or unsubstituted C₁₋₄ alky group, a substituted or unsubstituted non-aromatic heterocyclic group, a substituted or unsubstituted C₁₋₄ alkoxy group, an amino group which may be mono- or di-substituted by a substituted or unsubstituted C₁₋₄ alkyl group. Example of non-aromatic substituents of "substituted or unsubstituted non-aromatic heterocyclic group" are azetidinyl, morpholinyl, piperidinyl, piperazinyl, pyrrolidinyl, thiazolinyl, oxepanyl, thiomorpholinyl. These substituents arbitrarily substituted with 1 to 3 substituents in a class selected from (a-1) to (g-1) in [1-1] described above (in particular, the substituents listed as "particularly preferable groups").

[1-14-a-1] Examples of more preferable R⁸ are a hydrogen atom, or a group selected from the group consisting of a methyl group, an ethyl group, a methoxy group, an ethoxy group, an n-propoxy group, an azetidinyl group, a morpholinyl group, a piperidinyl group, a piperazinyl group, a pyrrolidinyl group, a thiazolinyl group, an oxepanyl group, a thiomorpholinyl group or amino group which may be substituted by a substituted or unsubstituted C₁₋₂ alkyl group. Each of these groups may be substituted by substituents such as C₁₋₆ alkyl, halogen, amino, hydroxyl, C₁₋₆ alkoxyl, mono-/di-C₁₋₆ alkylamino, oxo which are listed in [1-1] mentioned above as "particularly preferable group". Examples of substituents in "substituted or unsubstituted C₁₋₂ alkyl" are halogen, amino, hydroxyl, C₁₋₆ alkoxy, mono-/di- C₁₋₆ alkylamino, oxo, 4-pyranoyl.

[1-14-a-2] Examples of further preferable R⁸ are, concretely, a hydrogen atom, a methyl group, an ethyl group, a hydroxymethyl group, a hydroxyethyl group, a methoxymethyl group, a methoxyethyl group, 3-hydroxypropoxy group; 4-morpholinyl group, 2,6-dimethyl-4-morpholinyl group, a 1-piperidinyl group, 4-oxo-1-piperidinyl group, a 4-hydroxy-1-piperidinyl group, 4-methoxy-1-piperidinyl group, 4,4-difluoro-1-piperidinyl group, 1-piperazinyl group, 4-methyl-piperazinyl group, a pyrrolidinyl group, a 3S-fluoro-pyrrolidinyl group, a 3S-hydroxy-pyrodinyl group, a thiazolinyl group, an oxepanyl group, a thiomorpholinyl group, a 2S-hydroxymethyl-pyrrolidinyl, a 2S-methoxymethyl-pyrrolidinyl group; an N,N-dimethylamino group, an N,N-diethylamino group, an N,N-ethylmethylamino group, an N,N-bis(2-methoxyethyl) amino group, an N-methyl-N-(2-methoxyethyl)amino group, an N-methyl-N-cyclohexylamino group, an N-methyl-N-(2-dimethylaminoethyl)amino, an N-methyl-N-(2-hydroxyethyl)amino group, an N-methyl-N-(2-methoxyethyl)amino group, an N-methyl,N-(4-pyranoyl)amino.

[1-14-a-3] Particularly preferable R⁸ is hydrogen atom.

[1-14-b] In the compounds represented by formula (I) of embodiment [1], preferably, R^{9A} and R^{9B} are a substituent arbitrarily selected from the group of a hydrogen atom, a substituted or unsubstituted C₁₋₄ alky group, a substituted or unsubstituted non-aromatic heterocyclic group, a substituted or unsubstituted C₁₋₆ alkoxy group, or an amino group which may be mono- or di-substituted by a substituted or unsubstituted C₁₋₄ alky group. Non-aromatic substituents of the "substituted or unsubstituted non-aromatic heterocyclic group" have the same meaning as defined in the embodiment [1-1] mentioned above, and ,for example, azetidinyl group, morpholinyl group, piperidinyl group, piperazinyl group, pyrrolidinyl group, thiazolinyl group, oxepanyl group, thiomorpholinyl group and these substituents are arbitrarily substituted with 1 to 3 substituents in a class selected from (a-1) to (g-1) in [1-1] described above (in particular, the substituents listed as "particularly preferable groups").

[1-14-b-1] R^{9A} and R^{9B} may be same or different, but more preferable R^{9A} and R^{9B} are a substituent selected from a group of a hydrogen atom, or a methyl group, an ethyl group, a methoxy group, an ethoxyl group, an azetidinyl group, a morpholinyl group, a piperidinyl group, a piperazinyl group, a pyrrolidinyl group, a thiazolinyl group, an oxepanyl group, a thiomorpholinyl group or amino group which may be substituted by a substituted or unsubstituted C₁₋₂ alkyl group. These substituents are arbitrarily substituted with substituents listed as "particularly preferable substituent" in [1-1] mentioned above, for example, C₁₋₆ alkyl, halogen, amino, hydroxy, C₁₋₆ alkoxyl group, mono-/di-C₁₋₆ alkylamino, oxo. Examples of the substituents in "substituted or unsubstituted C₁₋₂ alkyl" are halogen, amino, hydroxyl, C₁₋₆ alkoxy, mono-/di- C₁₋₆ alkylamino, oxo, 4-pyranoyl.

[1-14-b-2] Examples of further preferable R^{9A} and R^{9B} are, concretely, a hydrogen atom, a methyl group, an ethyl group, a hydroxymethyl group, a hydroxyethyl group, a methoxyethyl group, a methoxyethyl group; 4-morpholinyl group, 2,6-dimethyl-4-morpholinyl group, a 1-piperidinyl group, 4-oxo-1-piperidinyl group, a 4-hydroxy-1-piperidinyl group, 4-methoxy-1-piperidinyl group, 4,4-difluoro-1-piperidinyl group, 1-piperazinyl group, 4-methyl-piperazinyl group, a pyrrolidinyl group, a 3S-fluoro-pyrrolidinyl group, a 3S-hydroxy-pyrrolidinyl group, a thiazolinyl group, an oxepanyl group, a thiomorpholinyl group, a 2S-hydroxymethyl-pyrrolidinyl, a 2S-methoxymethyl-pyrrolidinyl group; an N,N-dimethylamino group, an N,N-diethylamino group, an N,N-ethylmethylamino group, an N,N-bis(2-methoxyethyl)amino group, an N-methyl-N-(2-methoxyethyl)amino group, an N-methyl-N-cyclohexylamino group, an N-methyl-N-(2-dimethylaminoethyl)amino, an N-methyl-N-(2-hydroxyethyl)amino group, an N-methyl-N-(2-methoxyethyl)amino group, an N-methyl-N-(4-pyranoyl)amino.

[1-14-b-3] Particularly preferable R^{9A} and R^{9B} are hydrogen atom or methyl group when they are the; and one of them represents the hydrogen atom and the other presents a group (except the hydrogen atom) listed in [1-14-b-2] mentioned above.

[1-15] In the compound of formula (I) used for the compound of Embodiment [1], L₁ and L₂ each independently represent single bond, - CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or - S(O)t- (t is an integer of 0 to 2), and L₁ and L₂ may be identical with or different from each other.

[1-15-a] Preferable L₁ and L₂ are as follows : in a case where L₁ and L₂ are identical with each other, they are selected from single bond or -CR^{9A}R^{9B}-, and in a case where L₁ and L₂ are different from each other, one is -CR^{9A}R^{9B}-, and the other is oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). When W represents a methylene group, L₁ is an oxygen atom and L₂ is a -CR^{9A}R^{9B}-.

[1-15-b] More preferable L₁ and L₂ are as follows: in a case where L₁ is -CR^{9A}R^{9B}-, L₂ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). More preferable L₁ and L₂ are as follow: in a case where L₂ is -CR^{9A}R^{9B}-, L₁ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). More specifically, in a case where the solid line and broken line between L₁ and L₂ are single bonds, the moiety of L₁ and L₂ can be represented by the following formula:: 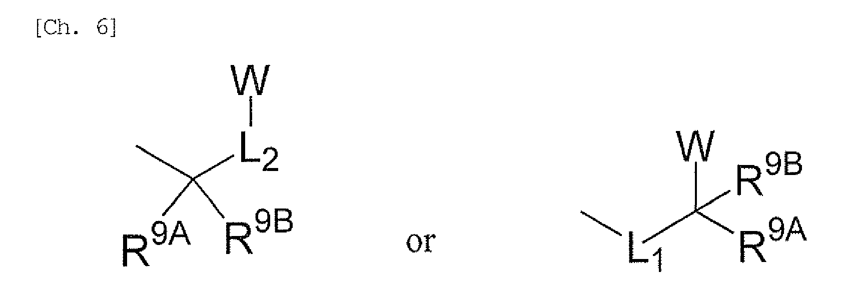 and it is more preferable that R^{9B} is hydrogen atom. Further, in a case where the solid line and broken line between L₁ and L₂ are double bonds, the moiety of L₁ and L₂ can be represented by the following formula: 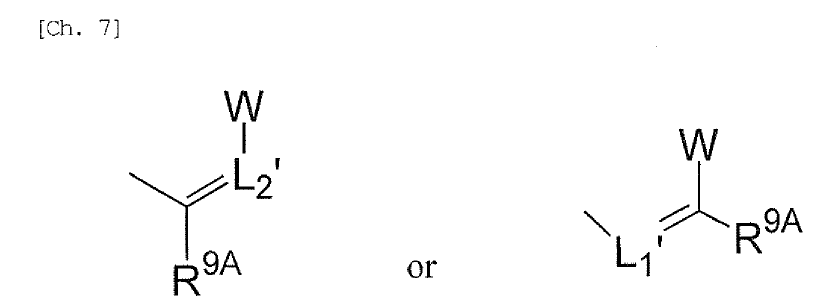 wherein L₁' and L₂' represent -CR^{9B}= or -N=.

[1-15-b-1] In these cases, preferable R^{9A} and R^{9B} can include hydrogen atom, methyl group, ethyl group, hydroxymethyl group, hydroxyethyl group, methoxyethyl group, methoxymethyl group; 4-morpholinyl group, 2,6-dimethyl-4-morpholinyl group, 1-piperidinyl group, 4-oxo-1-piperidinyl group, 4-hydroxy-1-piperidinyl group, 4-methoxy-1-piperidinyl group, 4,4-difluoro-1-piperidinyl group, 1-piperadinyl group, 4-methyl-piperadinyl group, pyrrolidinyl group, 3S-fluoro-pyrrolidinyl group, 3S-hydroxy-pyrrolidinyl group, thiazolinyl group, oxepanyl group, thiomorpholinyl group, 2S-hydroxymethyl-pyrrolidinyl group, 2S-methoxymethyl-pyrrolidinyl group; N,N-dimethylamino group, N,N-diethylamino group, an N,N-ethylmethylamino group, N,N-bis(2-methoxyethyl)amino group, N-methyl-N-(2-methoxyethyl)amino group, N-methyl-N-cyclohexylamino group, N-methyl-N-(2-dimethylaminoethyl)amino group, N-methyl-N-(2-hydroxyethyl)amino group, an N-methyl-N-(2-methoxyethyl)amino group, N-methyl-N-(4-pyranoyl)amino group, and the like that are mentioned in [1-14-b-2].

[1-15-c] Further preferable L₁ and L₂ are as follows: in a case where L₂ is -CR^{9A}R^{9B}-, L₁ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). The solid line and broken line between L₁ and L₂ are single bonds or double bonds, the moiety of L₁ and L₂ can be represented by the following formula: 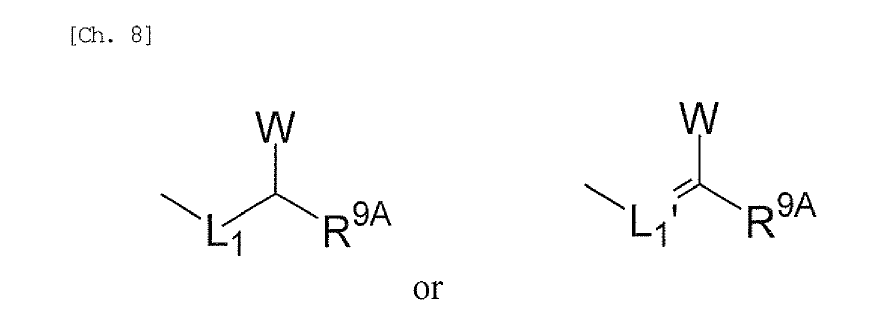 wherein L₁' represents -CR^{9B}= or -N=.

[1-15-c-1] In these cases, preferable R^{9A} and R^{9B} can include hydrogen atom, methyl group, ethyl group, hydroxyethyl group, hydroxyethyl group, methoxyethyl group, methoxyethyl group; 4-morpholinyl group, 2,6-dimethyl-4-morpholinyl group, 1-piperidinyl group, 4-oxo-1-piperidinyl group, 4-hydroxy-1-piperidinyl group, 4-methoxy-1-piperidinyl group, 4,4-difluoro-1-piperidinyl group, 1-piperadinyl group, 4-methyl-piperadinyl group, pyrrolidinyl group, 3S-fluoro-pyrrolidinyl group, 3S-hydroxy-pyrrolidinyl group, thiazolinyl group, oxepanyl group, thiomorpholinyl group, 2S-hydroxymethyl-pyrrolidinyl group, 2S-methoxymethyl-pyrrolidinyl group; N,N-dimethylamino group, N,N-diethylamino group, an N,N-ethylmethylamino group, N,N-bis(2-methoxyethyl)amino group, N-methyl-N-(2-methoxyethyl)amino group, N-methyl-N-cyclohexylamino group, N-methyl-N-(2-dimethylaminoethyl)amino group, N-methyl-N-(2-hydroxyethyl)amino group, an N-methyl-N-(2-methoxyethyl)amino group, N-methyl-N-(4-pyranoyl)amino group, and the like that are mentioned in [1-14-b-2].
More preferably R^{9B}- in the L₂' represents a hydrogen atom.

[1-15-d] Particularly preferable L₁ and L₂ are as follows: in a case where L₁ is -CH₂-, L₂ is -CR^{9A}H-, or L₁ is -CH=, L₂ is =CR^{9A}-. In this case, it is particularly preferable that R^{9A} is morpholino group. For example, the solid line and broken line between L₁ and L₂ are single bonds or double bonds, and the moiety of L₁ and L₂ can be represented by the following formula:

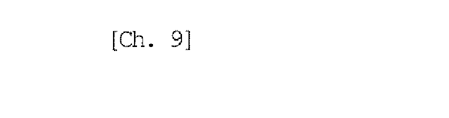

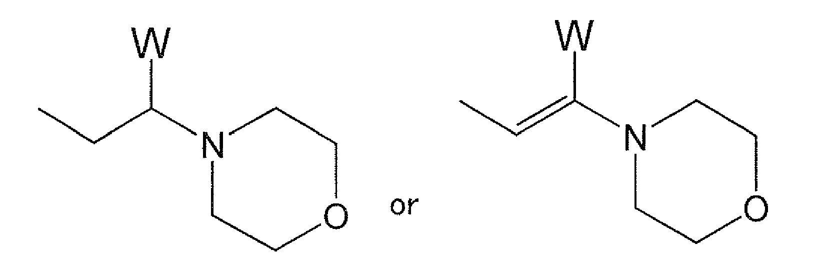

[1-15-e] In L₁ and L₂, t is an integer of 0 to 2, and it is preferable that t is 0 or 2.

[1-16] In the compound of formula (I) used for the compound of Embodiment [1], R¹⁰ represents hydrogen atom, a substituted or unsubstituted hydrocarbon group, a substituted or unsubstituted heterocyclic group, or a substituted or unsubstituted acyl group, or - S(O)t- (t is an integer of 0 to 2), which has the same meaning as that in the above-mentioned [1-13]. When R¹⁰ is the substituted or unsubstituted hydrocarbon atom or the substituted or unsubstituted heterocyclic group, R¹⁰ has the same meaning with the "substituted or unsubstituted hydrocarbon group" and the "substituted or unsubstituted heterocyclic group" listed in the [1-1] mentioned above and these groups may be substituted by 1 to 3 "subsituents" listed in (a) to (g).
When R¹⁰ represents the "substituted or unsubstituted acyl group", R¹⁰ means -CO-Rg (Rg has the same meaning mentioned above) of (g) in the [1-1] mentioned above.

[1-16-a] In the compounds represented by formula (I) of embodiment [1], R¹⁰ represents a hydrogen atom, a substituted or unsubstituted hydrocarbon group, or a substituted or unsubstituted heterocyclic group.

[1-16-a-1] Examples of the "substituted or unsubstituted carbonhydrogen group" or the "substituted or unsubstituted heterocyclic group" raised as the preferable R¹⁰ are: (1) C₁₋₁₀ alkyl group, (2) C₂₋₆ alkenyl group or (3) C₂₋₆ alkynyl group, (4) C₃₋₉ cycloalkyl group, (5) C₃₋₆ cycloalkenyl group, (6) C₄₋₆ cylcoalcanedienyl group, (7) C₆₋₁₄ aryl group, (8) any one of heterocyclic groups of (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group which contain 1 to 4 heterocarbon atoms selected from an oxygen atom, a sulfur atom or a nitrogen atom other than carbon atom. The above-mentioned (1) to (8) may be arbitrarily substituted with 1 to 5 substituents in the classes of the subsitutents (a-1) to (g-1) in [1-1-a] mentioned above and the following.

[1-16-a-2] Preferable examples of the "substituted or unsubstituted hydrocarbon group" or the "substituted or unsubstituted heterocyclic group" raised as the preferable R¹⁰ are:
(1') C₁₋₁₀ alkyl group, (7') C₆₋₁₄ aryl group or (8') any one of heterocyclic groups of (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono-or di-substituted by substituents in the classes of the subsitutents (a-1) to (g-1) (especially, the substituents listed as "particularly preferable").

[1-16-b] In the compounds represented by formula (I) of embodiment [1], more preferably, R¹⁰ represents a hydrogen atom or (1') C₁₋₁₀ alkyl group, or (8') any one of heterocyclic groups of (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono-or di-substituted by substituents in the classes of the subsitutents (a-1) to (g-1) (especially, the substituents listed as "particularly preferable").

[1-16-c] In the compounds represented by formula (I) of embodiment [1], more preferably, R¹⁰ represents a hydrogen atom, or C₁₋₆ alkyl group or tetrahydropyraniy (preferably teotrahydropyran-4-yl group) which may be mono- or di-substituted by a substituent such as halogen atom, halogenated C₁₋₆ alkyl, cyano, amino, hydroxyl, carbamoyl, C₁₋₆ alkoxyl group, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, piperazino, oxo, oxiranyl, or tetrahydrofuryl.

[1-16-d] In the compounds represented by formula (I) of embodiment [1], particularily preferably, R¹⁰ represents a hydrogen atom, or C₁₋₆ alkyl group which may be mono- or di-substituted by a substituent such as amino, hydroxyl, C₁₋₆ alkoxyl, mono/di C₁₋₆ alkylamino, morpholino, piperazino, oxo, oxiranyl, or tetrahydrofuryl.

[1-16-d-1] Examples of the "C₁₋₆ alkyl group" in the substituents of the particularly preferable R¹⁰ are methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl. Methyl, ethy, propyl, isopropyl, butyl, isobutyl, or sec-butyl is preferable.

[1-16-e] In the compounds represented by formula (I) of embodiment [1], particularly preferably, R¹⁰ represents a hydrogen atom, or a methyl group, a ethyl group, a propyl group, isopropyl group, butyl group which may be mono- or di-substituted by a substituent such as amino, hydroxyl, C₁₋₆ alkoxyl, mono/di C₁₋₆ alkylamino, phenyl. More concretely, hydrogen atom, methyl group, ethyl group, propyl group, isopropyl group, butyl group, isobutyl, sec-butyl, aminomethyl group, (2-)aminoethyl group, hydroxyethyl group, (2-)hydroxyethyl group, (3-)hydroxypropane-1-yl group, (4-)hydroxybuthyl group, 2-hydroxy-2,2-dimethylethyl group, 1,3-dihydroxy-propane-2-yl group, 1-methyl-2-hydroxyethyl group, 2-hydroxy-propane-1-yl group, methoxyethyl group, (2-)ethoxyethyl group, (2-)N,N-dimethylaminoethyl group, (2-)N,N-diethylaminoethyl group, benzyl group, phenethyl group, oxiranylmethyl group, (2-)tetrahydrofuranylmethyl group etc. (Preferred embodiments are indicated in the parenthesis "( )").

[1-16-f] Most preferable R¹⁰ includes hydrogen atom, methyl group, ethyl group, hydroxymethyl group, hydroxyethyl group or methoxyethyl group.

[1-17] In the compounds represented by formula (I) in embodiment [1], solid line and broken line between L1 and L2 represents as a whole a single bond or a double bond, preferably a single bond.

[1-18] 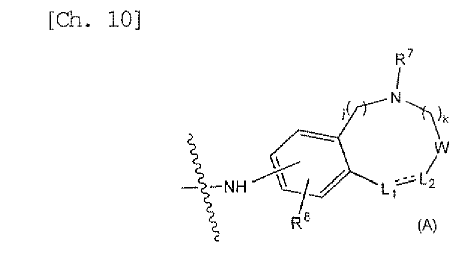 In the compounds represented by formula (I) in embodiment [1], examples of group represented by formula (A) include more preferable group represented by formula (a). 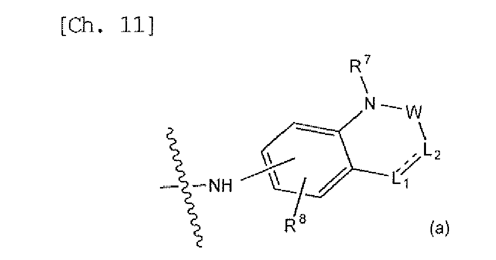 (In formula (A), the definitions of k, j, t, W, R7, R8, R9A, R9B, R10, L1, and L2 are the same as those described in one of embodiments [1-10] to [1-17], and in formula (a), the definitions of k, j, t, W, R7, R8, R9A, R9B, R10, L1, and L2 are the same as those described in one of embodiments [1-10] to [1-17]).
In formula (A) and (a), the substitution position of -NH- or R8 may be any position of carbon atoms of G1 to G4 represented in the partial structural formula (wherein each of G1 to G4 is CH) below. -NH- is preferably bonded to the 1st position (G4) or 3rd position (G2) in the clockwise direction from the condensation position close to the L1, and more preferably bonded to the third position (G2). When -NH- is bonded to the carbon atom of G2 position, R8 is preferably bonded to the carbon atom of G4 position.

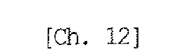

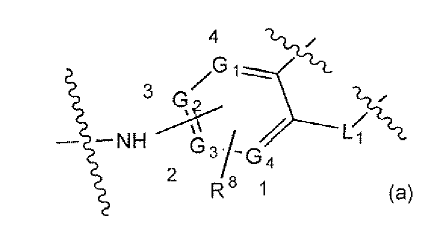

Specific examples of formula (a) are those described in the embodiments of [1-10] to [1-17], more specifically, further preferable examples of each substituents are amino groups described below or formula (a1) to (a141).

(2,2-dimethyl-4H-benzo[1,4]oxazin-3-on-5-yl) amino group, (2,2-dimethyl-4H-benzo[1,4]oxazin-3-on-6-yl)amino group, (2,2-dimethyl-4H-benzo[1,4]oxazin-3-on-7-yl)amino group, (2,2-dimethyl-4H-benzo[1,4]oxazin-3-on-8-yl)amino group, (2-methyl-4H-benzo[1,4]oxazin-3-on-5-yl)amino group, (2-methyl-4H-benzo[1,4]oxazin-3-on-6-yl)amino group, (2-methyl-4H-benzo[1,4]oxazin-3-on-7-yl)amino group, (2-methyl-4H-benzo[1,4]oxazin-3-on-8-yl)amino group, (2-(2-hydroxyethyl)-4H-benzo[1,4]oxazin-3-on-5-yl)amino group, (2-(2-hydroxyethyl)-4H-benzo[1,4]oxazin-3-on-6-yl)amino group, (2-(2-hydroxyethyl)-4H-benzo[1,4]oxazin-3-on-7-yl)amino group, (2-(2-hydroxyethyl)-4H-benzo[1,4]oxazin-3-on-8-yl)amino group, (2H-benzo[b][1,4]thiazin-3(4H)-on-5-yl)amino group, (2H-benzo[b][1,4]thiazin-3(4H)-on-6-yl)amino group, (2H-benzo[b][1,4]thiazin-3(4H)-on-7-yl)amino group, (2H-benzo[b][1,4]thiazin-3(4H)-on-8-yl)amino group, (1-oxo-2H-benzo[b][1,4]thiazin-3(4H)-on-5-yl)amino group, (1-oxo-2H-benzo[b][1,4]thiazin-3(4H)-on-6-yl)amino group, (1-oxo-2H-benzo[b][1,4]thiazin-3(4H)-on-7-yl)amino group, (1-oxo-2H-benzo[b][1,4]thiazin-3(4H)-on-8-yl)amino group, (1,1-dioxo-2H-benzo[b][1,4]thiazin-3(4H)-on-5-yl)amino group, (1,1-dioxo-2H-benzo[b][1,4]thiazin-3(4H)-on-6-yl)amino group, (1,1-dioxo-2H-benzo[b][1,4]thiazin-3(4H)-on-7-yl)amino group, (1,1-dioxo-2H-benzo[b][1,4]thiazin-3(4H)-on-8-yl)amino group, (3,4-dihydro-2(1H)-quinoxalinon-5-yl)amino group, (3,4-dihydro-2(1H)-quinoxalinon-6-yl)amino group, (3,4-dihydro-2(1H)-quinoxalinone-7-yl)amino group, (3,4-dihydro-2(1H)-quinoxalinon-8-yl)amino group, (4-methyl-3,4-dihydro-2(1H)-quinoxalinon-5-yl)amino group, (4-methyl-3,4-dihydro-2(1H)-quinoxalinon-6-yl)amino group, (4-methyl-3,4-dihydro-2(1H)-quinoxalinon-7-yl)amino group, (4-methyl-3,4-dihydro-2(1H)-quinoxalinon-8-yl) amino group, (3-hydroxymethyl-3,4-dihydro-2(1H)-quinoxalinon-5-yl)amino group, (3-hydroxymethyl-3,4-dihydro-2(1H)-quinoxalinon-6-yl)amino group, (3-hydroxymethyl-3,4-dihydro-2(1H)-quinoxalinon-7-yl)amino group, (3-hydroxymethyl-3,4-dihydro-2(1H)-quinoxalinon-8-yl)amino group, (3,3-dimethyl-3,4-dihydro-2(1H)-quinoxalinon-5-yl)amino group, (3,3-dimethyl-3,4-dihydro-2(1H)-quinoxalinon-6-yl) amino group, (3,3-dimethyl-3,4-dihydro-2(1H)-quinoxalinon-7-yl)amino group, (3,3-dimethyl-3,4-dihydro-2(1H)-quinoxalinon-8-yl)amino group, (3,3-dimethyl-4-methyl-3,4-dihydro-2(1H)-quinoxalinon-5-yl)amino group, (3,3-dimethyl-4-methyl-3,4-dihydro-2(1H)-quinoxalinon-6-yl)amino group, (3,3-dimethyl-4-methyl-3,4-dihydro-2(1H)-quinoxalinon-7-yl)amino group, (3,3-dimethyl-4-methyl-3,4-dihydro-2(1H)-quinoxalinon-8-yl)amino group,(1,4-dihydro-2H-3,1-benzoxazin-2-on-5-yl)amino group, (1,4-dihydro-2H-3,1-benzoxazin-2-on-6-yl)amino group, (1,4-dihydro-2H-3,1-benzoxazin-2-on-7-yl)amino group, (1,4-dihydro-2H-3,1-benzoxazin-2-on-8-yl)amino group, (3,4-dihydro-1H-qunazolin-2-on-5-yl)amino group, (3,4-dihydro-1H-qunazolin-2-on-6-yl)amino group, (3,4-dihydro-1H-qunazolin-2-on-7-yl)amino group, (3,4-dihydro-1H-qunazolin-2-on-8-yl)amino group, (3-methyl-3,4-dihydro-2(1H)quinazolinon-5-yl) amino group, (3-methyl-3,4-dihydro-2(1H)quinazolinon-6-yl)amino group, (3-methyl-3,4-dihydro-2(1H)quinazolinon-7-yl)amino group, (3-methyl-3,4-dihydro-2(1H)quinazolinon-8-yl)amino group, (3-(2-hydroxyethyl)-3,4-dihydro-2(1H)quinazolinon-5-yl)amino group, (3-(2-hydroxyethyl)-3,4-dihydro-2(1H)quinazolinon-6-yl)amino group, (3-(2-hydroxyethyl)-3,4-dihydro-2(1H)quinazolinon-7-yl)amino group, (3-(2-hydroxyethyl)-3,4-dihydro-2(1H)quinazolinon-8-yl)amino group, (3-(2-methoxyethyl)-3,4-dihydro-2(1H)quinazolinon-5-yl)amino group, (3-(2-methoxyethyl)-3,4-dihydro-2(1H)quinazolinon-6-yl)amino group, (3-(2-methoxyethyl)-3,4-dihydro-2(1H)quinazolinon-7-yl)amino group, (3-(2-methoxyethyl)-3,4-dihydro-2(1H)quinazolinon-8-yl)amino group, (3,4-dihydro-2,2-dioxo-1H-2,1,3-benzothiazin-5-yl)amino group, (3,4-dihydro-2,2-dioxo-1H-2,1,3-benzothiazin-6-yl)amino group, (3,4-dihydro-2,2-dioxo-1H-2,1,3-benzothiazin-7-yl)amino group, (3,4-dihydro-2,2-dioxo-1H-2,1,3-benzothiazin-8-yl)amino group, (3,4-dihydro-2(1H)-quinolinon-5-yl)amino group, (3,4-dihydro-2(1H)-quinolinon-6-yl)amino group, (3,4-dihydro-2(1H)-quinolinon-7-yl)amino group, (3,4-dihydro-2(1H)-quinolinon-8-yl)amino group, (1-(2-hydroxyethyl)-3,4-dihydro-2(1H)-quinolinon-5-yl) amino group, (1-(2-hydroxyethyl)-3,4-dihydro-2(1H)-quinolinon-6-yl)amino group, (1-(2-hydroxyethyl)-3,4-dihydro-2(1H)-quinolinon-7-yl)amino group, (1-(2-hydroxyethyl)-3,4-dihydro-2(1H)-quinolinon-8-yl)amino group, (2H-benzo[1,4]oxazin-3(4H)-on-5-yl)amino group, (2H-benzo[1,4]oxazin-3(4H)-on-6-yl)amino group, (2H-benzo[1,4]oxazin-3(4H)-on-7-yl)amino group, (2H-benzo[1,4]oxazin-3(4H)-on-8-yl)amino group, (3,4-dihydro-2(1H)-quinoxalinon-5-yl)amino group, (3,4-dihydro-2(1H)-quinoxalinon-6-yl)amino group, (3,4-dihydro-2(1H)-quinoxalinon-7-yl)amino group, (3,4-dihydro-2(1H)-quinoxalinon-8-yl)amino group, (3,4-dihydro-4-methyl-2(1H)-quinoxalinon-5-yl)amino group, (3,4-dihydro-4-methyl-2(1H)-quinoxalinon-6-yl)amino group, (3,4-dihydro-4-methyl-2(1H)-quinoxalinon-7-yl)amino group, (3,4-dihydro-4-methyl-2(1H)-quinoxalinon-8-yl)amino group, (3,4-dihydroquinolin-2(1H)-on-5-yl)amino group, (3,4-dihydroquinolin-2(1H)-on-6-yl)amino group, (3,4-dihydroquinolin-2(1H)-on-7-yl)amino group, (3,4-dihydroquinolin-2(1H)-on-8-yl)amino group, (1-methyl-3,4-dihydroquinolin-2(1H)-on-5-yl)amino group, (1-methyl-3,4-dihydroquinolin-2(1H)-on-6-yl)amino group, (1-methyl-3,4-dihydroquinolin-2(1H)-on-7-yl)amino group, (1-methyl-3,4-dihydroquinolin-2(1H)-on-8-yl)amino group, (3-(hydroxymethyl)-3,4-dihydro-2(1H)-quinolinon-5-yl)amino group, (3-(hydroxymethyl)-3,4-dihydro-2(1H)-quinolinon-6-yl)amino group, (3-(hydroxymethyl)-3,4-dihydro-2(1H)-quinolinon-7-yl)amino group, (3-(hydroxymethyl)-3,4-dihydro-2(1H)-quinolinon-8-yl)amino group, (3,3-dimethyl-3,4-dihydro-2(1H)-quinolinon-5-yl)-amino group, (3,3-dimethyl-3,4-dihydro-2(1H)-quinolinon-6-yl)amino group, (3,3-dimethyl-3,4-dihydro-2(1H)-quinolinon-7-yl)amino group, (3,3-dimethyl-3,4-dihydro-2(1H)-quinolinon-8-yl)amino group, (8-(4-morpholinyl)-8,4-dihydro-2(1H)-quinolinan-5-yl)amino group, (3-(4-morpholinyl)-3,4-dihydro-2(1H)-quinolinon-6-yl)amino group, (3-(4-morpholinyl)-3,4-dihydro-2(1H)-quinolinon-7-yl)amino group, (3-(4-morpholinyl)-3,4-dihydro-2(1H)-quinolinon-8-yl)amino group, (3-(1-piperidinyl)-3,4-dihydro-2(1H)-quinolinon-5-yl)amino group, (3-(1-piperidinyl)-3,4-dihydro-2(1H)-quinolinon-6-yl)amino group, (3-(1-piperidinyl)-3,4-dihydro-2(1H)-quinolinon-7-yl)amino group, (3-(1-piperidinyl)-3,4-dihydro-2(1H)-quinolinon-8-yl)amino group, (3-(4-methyl-1-piperazinyl)-3,4-dihydro-2(1H)-quinolinon-5-yl)amino group, (3-(4-methyl-1-piperazinyl)-3,4-dihydro-2(1H)-quinolinon-6-yl)amino group, (3-(4-methyl-1-piperazinyl)-3,4-dihydro-2(1H)-quinolinon-7-yl)amino group, (3-(4-methyl-1-piperazinyl)-3,4-dihydro-2(1H)-quinolinon-8-yl)amino group, (3-(N,N-dimethylamino)-3,4-dihydro-2(1H)-quinolinon-5-yl)amino group, (3-(N,N-dimethylamino)-3,4-dihydro-2(1H)-quinolinon-6-yl)amino group, (3-(N,N-dimethylamino)-3,4-dihydro-2(1H)-quinolinon-7-yl)amino group, (3-(N,N-dimethylamino)-3,4-dihydro-2(1H)-quinolinon-8-yl)amino group, (3-(N,N-diethylamino)-3,4-dihydro-2(1H)-quinolinon-5-yl)amino group, (3-(N,N-diethylamino)-3,4-dihydro-2(1H)-quinolinon-6-yl)amino group, (3-(N,N-diethylamino)-3,4-dihydro-2(1H)-quinolinon-7-yl)amino group, (3-(N,N-diethylamino)-3,4-dihydro-2(1H)-quinolinon-8-yl)amino group, (3-(N,N-(bis(2-methoxydiethyl)amino)-3,4-dihydro-2(1H)-quinolinon-5-yl)amino group, (3-(N,N-(bis(2-methoxydiethyl)amino)-3,4-dihydro-2(1H)-quinolinon-6-yl)amino group, (3-(N,N-(bis(2-methoxydiethyl)amino)-3,4-dihydro-2(1H)-quinolinon-7-yl)amino group, (3-(N,N-(bis(2-methoxydiethyl)amino)-3,4-dihydro-2(1H)-quinolinon-8-yl)amino group, (3-(N-methyl-N-(2-methoxyethyl)amino)-3,4-dihydro-2(1H)-quinolinon-5-yl)amino group, (3-(N-methyl-N-(2-methoxyethyl)amino)-3,4-dihydro-2(1H)-quinolinon-6-yl)amino group, (3-(N-methyl-N-(2-methoxyethyl)amino)-3,4-dihydro-2(1H)-quinolinon-7-yl)amino group, (3-(N-methyl-N-(2-methoxyethyl)amino)-3,4-dihydro-2(1H)-quinolinon-8-yl)amino group, (3-pyrrolidinyl-3,4-dihydro-2(1H)-quinolinon-5-yl)amino group, (3-pyrrolidinyl-3,4-dihydro-2(1H)-quinolinon-6-yl)amino group, (3-pyrrolidinyl-3,4-dihydro-2(1H)-quinolinon-7-yl)amino group, (3-pyrrolidinyl-3,4-dihydro-2(1H)-quinolinon-8-yl)amino group, (3-((S)-3-fluoropyrrolidin-1-yl)-3,4-dihydro-2(1H)-quinolinon-5-yl) amino group, (3-((S)-3-fluoropyrrolidin-1-yl)-3,4-dihydro-2(1H)-quinolinon-6-yl)amino group, (3-((S)-3-fluoropyrrolidin-1-yl)-3,4-dihydro-2(1H)-quinolinon-7-yl)amino group, (3-((S)-3-fluoropyrrolidin-1-yl)-3,4-dihydro-2(1H)-quinolinon-8-yl)amino group, (3-((S)-3-hydroxypyrrolidin-1-yl)-3,4-dihydro-2(1H)-quinolinon-5-yl)amino group, (3-((S)-3-hydroxypyrrolidin-1-yl)-3,4-dihydro-2(1H)-quinolinon-6-yl)amino group, (3-((S)-3-hydroxypyrrolidin-1-yl)-3,4-dihydro-2(1H)-quinolinon-7-yl)amino group, (3-((S)-3-hydroxypyrrolidin-1-yl)-3,4-dihydro-2(1H)-quinolinon-8-yl)amino group, (3-((S)-2-(hydroxymethyl)pyrrolidin-1-yl)-3,4-dihydro-2(1H)-quinolinon-5-yl)amino group, (3-((S)-2-(hydroxymethyl)pyrrolidin-1-yl)-3,4-dihydro-2(1H)-quinolinon-6-yl) amino group, (3-((S)-2-(hydroxymethyl)pyrrolidin-1-yl)-3,4-dihydro-2(1H)-quinolinon-7-yl)amino group, (3-((S)-2-(hydroxymethyl)pyrrolidin-1-yl)-3,4-dihydro-2(1H)-quinolinon-8-yl)amino group, (3-((S)-2-(methoxymethyl)pyrrolidin-1-yl)-3,4-dihydro-2(1H)-quinolinon-5-yl) amino group, (3-((S)-2-(methoxymethyl)pyrrolidin-1-yl)-3,4-dihydro-2(1H)-quinolinon-6-yl)amino group, (3-((S)-2-(methoxymethyl)pyrrolidin-1-yl)-3,4-dihydro-2(1H)-quinolinon-7-yl)amino group, (3-((S)-2-(methoxymethyl)pyrrolidin-1-yl)-3,4-dihydro-2(1H)-quinolinon-8-yl)amino group, (3-(3-thiazolidinyl)-3,4-dihydro-2(1H)-quinolinon-5-yl)amino group, (3-(3- thiazolidinyl)-3,4-dihydro-2(1H)-quinolinon-6-yl)amino group, (3-(3- thiazolidinyl)-3,4-dihydro-2(1H)-quinolinon-7-yl)amino group, (3-(3- thiazolidinyl)-3,4-dihydro-2(1H)-quinolinon-8-yl)amino group, (3-(N-methyl-N-cyclohexylamino)-3,4-dihydro-2(1H)-quinolinon-5-yl)amino group, (3-(N-methyl-N-cyclohexylamino)-3,4-dihydro-2(1H)-quinolinon-6-yl)amino group, (3-(N-methyl-N-cyclohexylamino)-3,4-dihydro-2(1H)-quinolinon-7-yl)amino group, (3-(N-methyl-N-cyclohexylamino)-3,4-dihydro-2(1H)-quinolinon-8-yl)amino group, (3-(1-piperazinyl)-3,4-dihydro-2(1H)-quinolinon-5-yl)amino group, (3-(1-piperazinyl)-3,4-dihydro-2(1H)-quinolinon-6-yl)amino group, (3-(1-piperazinyl)-3,4-dihydro-2(1H)-quinolinon-7-yl)-amino group, (3-(1-piperazinyl)-3,4-dihydro-2(1H)-quinolinon-8-yl)amino group, (3-([1,4]oxepan-4-yl)-3,4-dihydro-2(1H)-quinolinon-5-yl)amino group, (3-([1,4]oxepan-4-yl)-3,4-dihydro-2(1H)-quinolinon-6-yl)amino group, (3-([1,4]oxepan-4-yl)-3,4-dihydro-2(1H)-quinolinon-7-yl)amino group, (3-([1,4]oxepan-4-yl)-3,4-dihydro-2(1H)-quinolinon-8-yl)amino group, (3-(4-oxo-piperidin-1-yl)-3,4-dihydro-2(1H)-quinolinon-5-yl)amino group, (3-(4-oxo-piperidin-1-yl)-3,4-dihydro-2(1H)-quinolinon-6-yl)amino group, (3-(4-oxo-piperidin-1-yl)-3,4-dihydro-2(1H)-quinolinon-7-yl)amino group, (3-(4-oxo-piperidin-1-yl)-3,4-dihydro-2(1H)-quinolinon-8-yl)amino group, (3-(4-hydroxypiperidin-1-yl)-3,4-dihydro-2(1H)-quinolinon-5-yl) amino group, (3-(4-hydroxypiperidin-1-yl)-3,4-dihydro-2(1H)-quinolinon-6-yl) amino group, (3-(4-hydroxypiperidin-1-yl)-3,4-dihydro-2(1H)-quinolinon-7-yl)amino group, (3-(4-hydroxypiperidin-1-yl)-3,4-dihydro-2(1H)-quinolinon-8-yl)amino group, (3-(4-thiomorpholinyl)-3,4-dihydro-2(1H)-quinolinon-5-yl)amino group, (3-(4-thiomorpholinyl)-3,4-dihydro-2(1H)-quinolinon-6-yl)amino group, (3-(4-thiomorpholinyl)-3,4-dihydro-2(1H)-quinolinon-7-yl)amino group, (3-(4-thiomorpholinyl)-3,4-dihydro-2(1H)-quinolinon-8-yl)amino group, (3-(1,1-dioxothiomorpholin-4-yl)-3,4-dihydro-2(1H)-quinolinon-5-yl)amino group, (3-(1,1-dioxothiomorpholin-4-yl)-3,4-dihydro-2(1H)-quinolinon-6-yl)amino group, (3-(1,1-dioxothiomorpholin-4-yl)-3,4-dihydro-2(1H)-quinolinon-7-yl)amino group, (3-(1,1-dioxothiomorpholin-4-yl)-3,4-dihydro-2(1H)-quinolinon-8-yl)amino group, (3-(4-methoxy-1-piperidinyl)-3,4-dihydro-2(1H)-quinolinon-5-yl)amino group, (3-(4-methoxy-1-piperidinyl)-3,4-dihydro-2(1H)-quinolinon-6-yl)amino group, (3-(4-methoxy-1-piperidinyl)-3,4-dihydro-2(1H)-quinolinon-7-yl)amino group, (3-(4-methoxy-1-piperidinyl)-3,4-dihydro-2(1H)-quinolinon-8-yl)amino group, (3-((S)-3-methoxy-1-pyrrolidinyl-3,4-dihydro-2(1H)-quinolinon-5-yl)amino group, (3-((S)-3-methoxy-1-pyrrolidinyl-3,4-dihydro-2(1H)-quinolinon-6-yl)amino group, (3-((S)-3-methoxy-1-pyrrolidinyl-3,4-dihydro-2(1H)-quinolinon-7-yl)amino group, (3-((S)-3-methoxy-1-pyrrolidinyl-3,4-dihydro-2(1H)-quinolinon-8-yl)amino group, ((S)-2-methyl-4H-benzo[1,4]oxadin-3-on-5-yl-amino group, ((S)-2-methyl-4H-benzo[1,4]oxadin-3-on-6-yl)amino group, ((S)-2-methyl-4H-benzo[1,4]oxadin-3-on-7-yl)amino group, ((S)-2-methyl-4H-benzo[1,4]oxadin-3-on-8-yl)amino group, ((R)-2-methyl-4H-benzo[1,4]oxadin-3-on-5-yl)amino group, ((R)-2-methyl-4H-benzo[1,4]oxadin-3-on-6-yl)amino group, ((R)-2-methyl-4H-benzo[1,4]oxadin-3-on-7-yl)amino group, ((R)-2-methyl-4H-benzo[1,4]oxadin-3-on-8-yl)amino group, (3-(4-morpholinyl)quinolin-2(1H)-on-5-yl)amino group, (3-(4-morpholinyl)quinolin-2(1H)-on-6-yl)amino group, (3-(4-morpholinyl)quinolin-2(1H)-on-7-yl)amino group, (3-(4-morpholinyl)quinolin-2(1H)-on-8-yl)amino group, 3-(1-azetidinyl) -3,4-dihydro-2(1H)-qulnolinon-5-yl)amino group, 3-(1-azetidinyl)-3,4-dihydro-2(1H)-quinolinon-6-yl)amino group, 3-(1-azetidinyl)-3,4-dihydro-2(1H)-quinolinon-7-yl)amino group, 3-(1-azetidinyl)-3,4-dihydro-2(1H)-quinolinon-8-yl)amino group, (3-(N-methyl-N-(2-(dimethylamino)ethyl)amino)-3,4-dihydro-2(1H)-quinolinon-5-yl)amino group, (3-(N-methyl-N-(2-(dimethylamino)ethyl)amino)-3,4-dihydro-2(1H) - quinolinon-6-yl)amino group, (3-(N-methyl-N-(2-(dimethylamino)ethyl) amino)-3,4-dihydro-2(1H)-quinolinon-7-yl)amino group, (3-(N-methyl-N-(2-(dimethylamino)ethyl)amino)-3,4-dihydro-2(1H)-quinolinon-8-yl)amino group, (3-(N-methyl-N-(hydroxyethyl)amino)-3,4-dihydro-2(1H)-quinolinon-5-yl)amino group, (3-(N-methyl-N-(hydroxyethyl)amino)-3,4-dihydro-2(1H)-quinolinon-6-yl)amino group, (3-(N-methyl-N-(hydroxyethyl)amino)-3,4-dihydro-2(1H)-quinolinon-7-yl)amino group, (3-(N-methyl-N-(hydroxyethyl)amino)-3,4-dihydro-2(1H)-quinolinon-8-yl)amino group, (3-(N-methyl-N-(4-tetrahydropiranyl)amino)-3,4-dihydro-2(1H)-quinolinon-5-yl)amino group, (3-(N-methyl-N-(4-tetrahydropiranyl)amino)-3,4-dihydro-2(1H)-quinolinon-6-yl)amino group, (3-(N-methyl-N-(4-tetrahydropiranyl)amino)-3,4-dihydro-2(1H)-quinolinon-7-yl)amino group, (3-(N-methyl-N-(4-tetrahydropiranyl)amino)-3,4-dihydro-2(1H)-quinolinon-8-yl)amino group, (2,2-dioxo-3,4-dihydro-1H-2,1-benzothiazin-5-yl)amino group, (2,2-dioxo-3,4-dihydro-1H-2,1-benzothiazin-6-yl)amino group, (2,2-dioxo-3,4-dihydro-1H-2,1-benzothiazin-7-yl)amino group, (2,2-dioxo-3,4-dihydro-1H-2,1-benzothiazin-8-yl)amino group.

Further preferable examples of each substituents are formula (a1) to (a141).

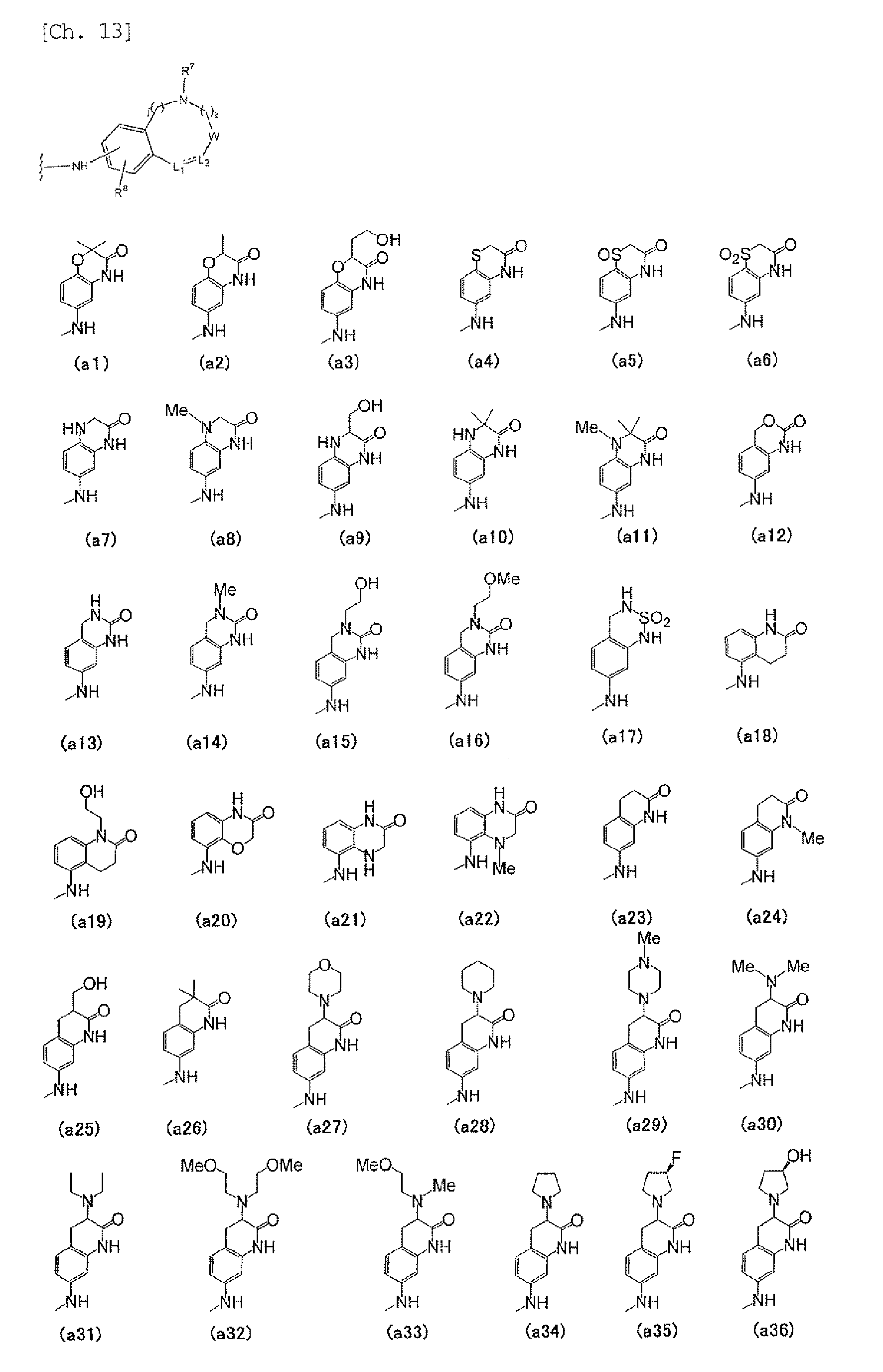

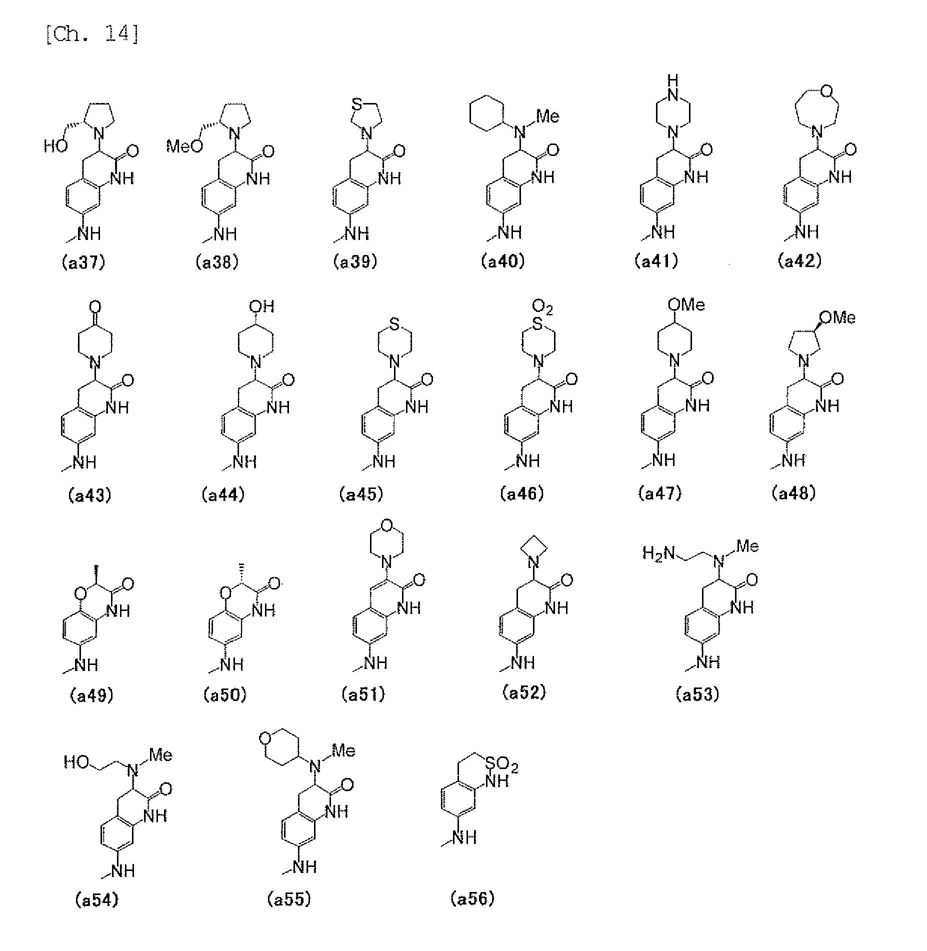

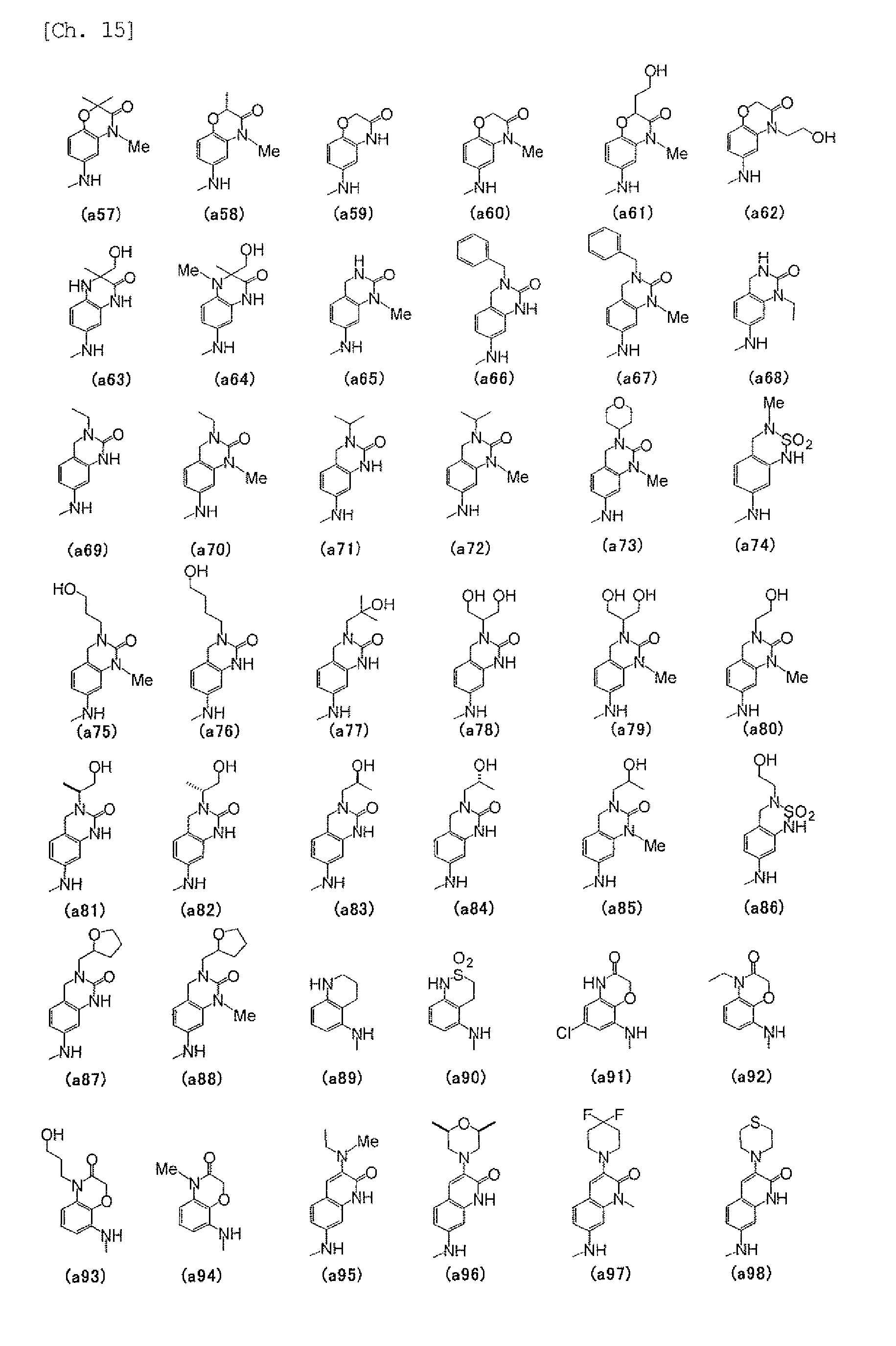

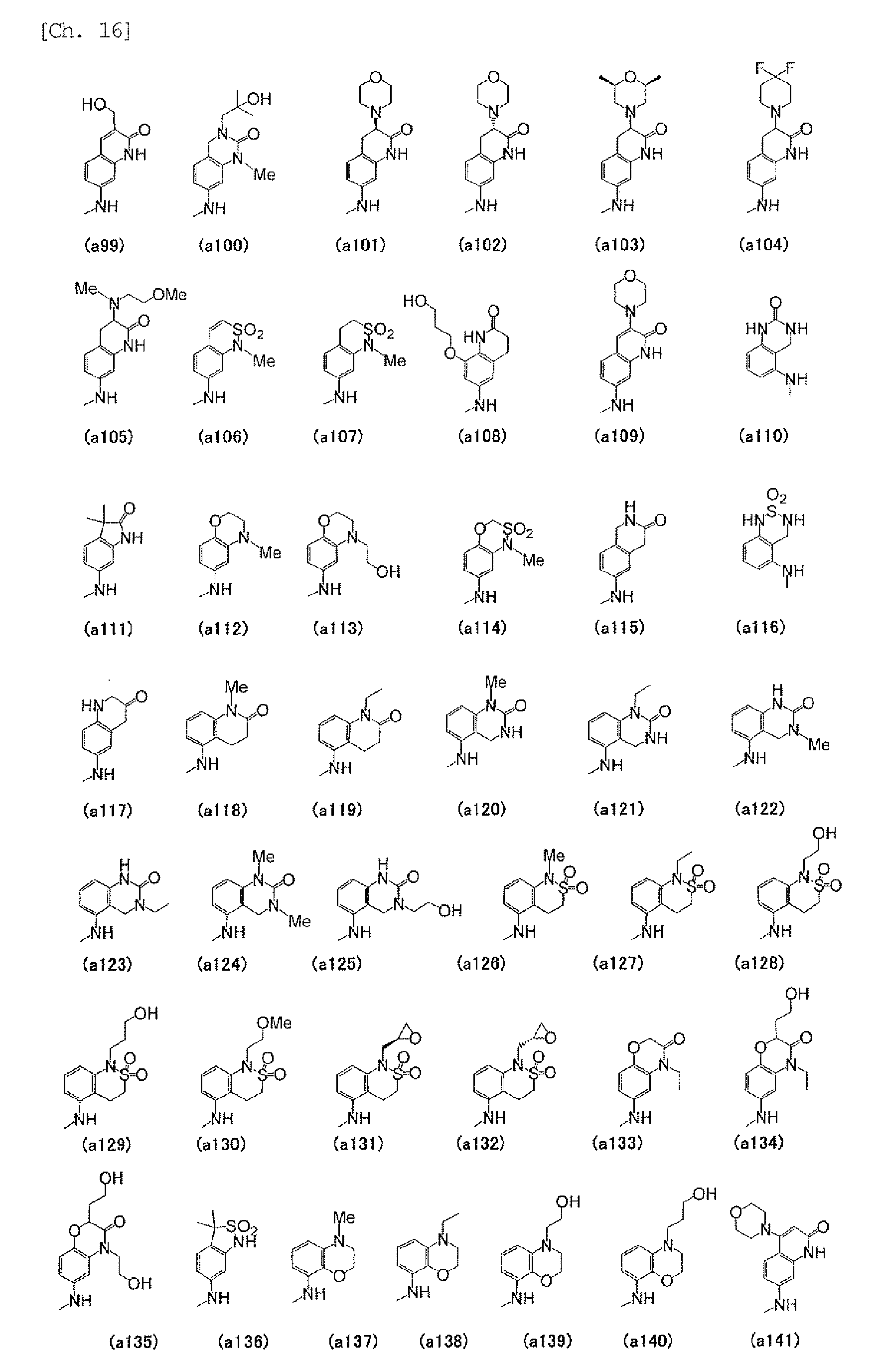

Preferably, each of the groups (a1) to (a141) in the embodiment of [1-18] may be either unsubstituted or substituted with 1 to 2 subst ituents in a class selected from (a-1) to (g-1) described in [1-1-a] ab ove, or arbitrarily exchanged for any of the substituent in (a1) to (a1 41).

Particularly preferable substituents include C₁₋₆ alkyl, C₂₋₆ alkenyl, C₂₋₆ alkynyl, halogen atoms, halogenated C₁₋₆ alkyl, cyano, amino, hydroxyl, carbamoyl, C₁₋₆ alkoxy, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, oxo, morpholinylcarbonyl, morpholinylsulfonyl, 5-trifluoromethylpyridin-2-yloxy, quinoxalin-2-yl, (pyridin-4-yl)methyl, 1,2,3-thiadiazolo-4-yl, 1H-pyrazolo-1-yl, and 4-chlorophenyl. The aromatic rings in these substituents may be substituted with a halogen atom, trifluoromethyl, cyano, hydroxy, amino, nitro, carboxyl, carbamoyl, C₁₋₆ alkyl, C₁₋₆ alkoxy, mono/di C₁₋₆ alkylamino, di-C₁₋₆ alkylcarbamoyl, C₁₋₆ alkoxycarbonyl, N-C₁₋₆ alkylcarbamoyl, N,N-di C₁₋₆ alkylcarbamoyl, or C₂₋₆ alkenoylamino.

[1-19] The wavy line to which "CO-NH" in formula (I) of the present invention is bonded represents a bond of an E-isomer (anti-isomer or trans-isomer) or a Z-isomer (syn-isomer or cis-isomer). This means that the compounds represented by formula (I) include E-isomers(anti-isomer or trans-isomer) and Z-isomers(syn-isomer or cis-isomer). The compounds represented by formula (I) are preferably E-isomers(anti-isomer or trans-isomer). Hereinafter, wavy lines in formulae in this description represent the same meaning.

[1-20] In the compounds represented by formula (I) in embodiment [1], the ring containing X1 and X2 is preferably five- to eight-membered, more preferably six- or seven-membered. The ring containing W is preferably five- to eight-membered, more preferably five- to seven-membered, and most preferably five- or six-membered. When L1 and L2 are both single bond, W connects to the phenyl ring.

In the compounds represented by formula (I), preferable compounds can be determined by optional combinations of [1-1] to [1-20] described above. Examples of the compounds having specific combinations are described in [1-21].

[1-21] In formula (I),

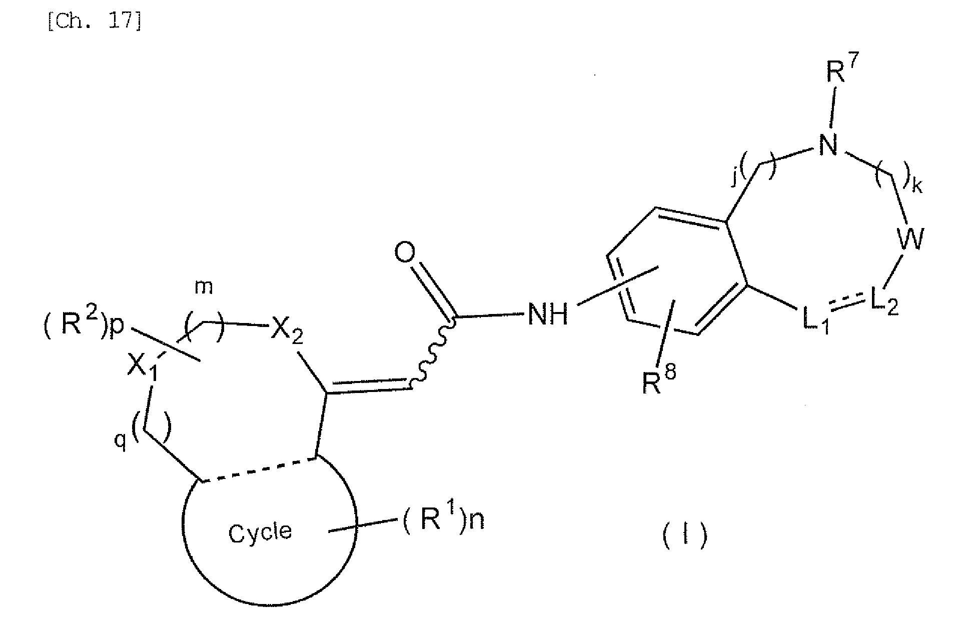

R¹ is a halogen atom, and (1) a C₁₋₆ alkyl group, (2) a C₂-₆ alkenyl group, (7) a C₆₋₁₄ aryl group, and (9) a C₁₋₆ alkoxy group. Each group in (1), (2), (7), and (9) is arbitrarily substituted with 1 to 3 substituents in a class selected from (a-1) to (g-1) in [1-1] described above (in particular, the substituents listed as "particularly preferable groups").

More preferably, R¹ is a halogen atom (a fluorine atom, a chlorine atom, a bromine atom, or an iodine atom), and a C₁₋₆ alkyl group (in particular, C₁₋₄ alkyl group) or C₁₋₆ alkoxy group (in particular, C₁₋₄ alkoxy group) which may be substituted with 1 to 3 halogen atoms.

More specifically, examples thereof include a fluorine atom, a chlorine atom, methyl, ethyl, propyl, isopropyl, butyl, isobutyl, sec-butyl, tert-butyl, trifluoromethyl, methoxy, ethoxy, propoxy, isopropoxy, butoxy, sec-butoxy, tert-butoxy, trifluoromethoxy, and tetrafluoroethoxy.

Particularly preferably, R¹ is a fluorine atom, a chlorine atom, isobutyl, tert-butyl, trifluoromethyl, or tetrafluoroethoxy. Still more preferably, R¹ is trifluoromethyl.
n is an integer of 0 to 2. Preferably, n is 1 or 2, and more preferably, n is 1.
R² is a halogen atom, a substituted or unsubstituted amino group, a substituted or unsubstituted hydrocarbon group, a substituted or unsubstituted aromatic heterocyclic group, or an oxo group.
R² is preferably a fluorine atom, a chlorine atom, an amino grou p which is arbitrarily mono-substituted with a substituent RIII, a C₁₋₆ alkyl group which is arbitrarily mono-substituted with a group selected from a C₁₋₆ alkoxy, amino and mono/di C₁₋₆ alkylamino, or a phenyl group

More preferably, R² is a C₁₋₆ alkyl group which is arbitrarily mono-s ubstituted with a group selected from a C₁₋₆ alkoxy, amino and mono/di C ₁₋₆ alkylamino (in particular, a C₁₋₄ alkyl group such as methyl, ethyl, propyl, isopropyl, butyl, isobutyl, sec-butyl, or tert-butyl, methoxyme thyl, 2-methoxyethyl). Further preferably, R² is methyl, ethyl, methox ymethyl.

p is an integer of 0 to 2. Preferably, p is 0 or 2. However, in the compounds represented by formula (I), when R² is a C₁₋₆ alkyl group (in particular, a C₁₋₄ alkyl group such as methyl, ethyl, propyl, isopropyl, butyl, isobutyl, sec-butyl, or tert-butyl), p is preferably 1 or 2, and more preferably 2 and is bonded to geminal position.

Alternatively, two geminal or vicinal R² may bind to each other to form a C₂₋₆ alkylene group respectively, and form a cyclo ring group together with the carbon atom to which the two R² are bonded, or the cyclo ring group may form non-aromatic heterocyclic groups containing an oxygen atom or a nitrogen atom. Three to eight-membered rings are preferable. For example, a cyclopropane ring, a cyclobutane ring, a cyclopentane ring, or a cyclohexane ring, oxirane ring, oxetane ring, tetrahydrofuran ring, tetrahydropyran ring, aziridine ring, azetidine ring, pyrrolidine ring or piperizine ring can be formed.

When R² is a fluorine atom, p is preferably 1 or 2, and more preferably 2. When R² is an amino group which may be mono-substituted with a substituent RIII or an oxo group, p is preferably 1 or 2. m is 0 to 2, and preferably 1 or 2.

X₁ represents an oxygen atom, -NR³'- (wherein R³' is a substituted or unsubstituted hydrocarbon group, a substituted or unsubstituted heterocyclic group, or a substituted or unsubstituted acyl group), or preferably, X₁ is an oxygen atom.

When X₁ is -NR³'-, examples of the "substituted or unsubstituted hydrocarbon group" or the "substituted or unsubstituted heterocyclic group" of R³' include (1') C₁₋₆ alkyl groups; (2') C₂₋₆ alkenyl groups; (4') C₃₋₆ cycloalkyl groups; (7') C₆₋₁₄ aryl groups; and (8') heterocyclic groups each containing 1 heteroatom or 2 heteroatoms selected from an oxygen atom, a sulfur atom, and a nitrogen atom in addition to the carbon atoms, the heterocyclic groups being selected from (i) five- or six-membered, monocyclic aromatic heterocyclic groups, (ii) eight- to twelve-membered, fused aromatic heterocyclic groups, and (iii) "three- to eight-membered, saturated or unsaturated, non-aromatic heterocyclic groups, and each of the groups in (1'), (2'), (4'), (7'), and (8') may be mono-substituted with a substituent in a class selected from the substituents (a-1) to (g-1) (in particular, the substituents listed as "particularly preferable groups" in (a-1) to (g-1)).

Examples of the "substituted or unsubstituted acyl group" of R³' include groups represented by -CO-Rg''' (wherein Rg"' represents a substituent RV' (wherein RV' represents C₁₋₆ alkyl, C₃₋₆ cycloalkyl, C₆₋₁₀ aryl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain 1 heteroatom or 2 heteroatoms selected from an oxygen atom, a sulfur atom, and a nitrogen atom in addition to the carbon atoms; and the alkyl, the aryl, or the heterocyclic group may be further substituted with 1 to 5 substituents RIV of (f) described above).

Further preferably, when X₁ is -NR³'-, examples of the "substituted or unsubstituted hydrocarbon group" or the "substituted or unsubstituted heterocyclic group" of R³' include (7") C₆₋₁₄ aryl groups and (8'') heterocyclic groups each containing a heteroatom selected from an oxygen atom, a sulfur atom, and a nitrogen atom in addition to the carbon atoms, the heterocyclic groups being selected from (i) five-or six-membered, monocyclic aromatic heterocyclic groups, (ii) eight-to twelve-membered, fused aromatic heterocyclic groups, and (iii) three- to eight-membered, saturated or unsaturated, non-aromatic heterocyclic groups, and each of the groups in (7") and (8") may be mono-substituted with a substituent in a class selected from the substituents (a-1) to (g-1) (in particular, the substituents listed as "particularly preferable groups" in (a-1) to (g-1)).

Examples of the "substituted or unsubstituted acyl group" of R³' include groups represented by -CO-Rg'''' (wherein Rg'''' represents a substituent RV" (wherein RV" represents C₁₋₆ alkyl, C₃₋₆ cycloalkyl, C₆₋₁₀ aryl, or a heterocyclic group; the heterocyclic group is any one of (i) five- or six-membered monocyclic aromatic heterocyclic groups, (ii) eight- to twelve-membered fused aromatic heterocyclic groups, and (iii) three- to eight-membered saturated or unsaturated non-aromatic heterocyclic groups which contain a heteroatom selected from an oxygen atom, a sulfur atom, and a nitrogen atom in addition to the carbon atoms; and the alkyl, the cycloalkyl, the aryl, or the heterocyclic group may be further substituted with 1 to 3 substituents RIV of (f) described above).

Particularly preferably, when X₁ is -NR³'-, examples of the "substituted or unsubstituted hydrocarbon group" or the "substituted or unsubstituted heterocyclic group" of R³' include (1"') methyl and (1"') ethyl, (4"') cyclohexyl, (7"') phenyl and (7"') naphthyl (e.g., naphthalen-1-yl and naphthalen-2-yl), and (8"') pyridyl (e.g., pyridin-2-yl, pyridin-3-yl, and pyridin-4-yl) which may be substituted with a halogen atom. More specifically, examples thereof include methyl, trifluoromethyl, ethyl, cyclohexyl, 2-chlorophenyl, 3-chlorophenyl, 4-chlorophenyl, naphthalen-1-yl, naphthalen-2-yl, and 3-chloro-pyridin-2-yl.

Examples of the "substituted or unsubstituted acyl group" include groups represented by -CO-Rg"" (wherein Rg""' represents a substituent RV"' (wherein RV"' represents methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl, cyclopropyl, cyclobutyl, cyclopentyl, cyclohexyl, phenyl, naphthyl, pyridyl (e.g., pyridin-2-yl, pyridin-3-yl, and pyridin-4-yl), 2,2-dimethylpropyl, 2-methylpropyl, 3-methylbutyl, 2-methylbutyl, 1-methylbutyl, 1,1-dimethylbutyl, 4,4-difluorocyclohexyl, 3-fluorocyclopentyl, 1-methylcyclopropyl, 1-methylcyclobutyl, 3,3,3-trifluoropropyl, 2,2,2-trifluoroethyl, 4,4,4-trifluorobutyl, phenylmethyl, 1,1-difluoropropyl, and 1-fluoro-1-methylethyl; and the alkyl, the cycloalkyl, the aryl, or the heterocyclic group may be further substituted with a substituent RIV of (f) described above).

More specifically, examples of the groups represented by -CO-Rg''''' include acyl groups which may be halogenated, such as acetyl, pentanoyl, 2-ethylbutanoyl, cyclohexanecarbonyl, 4-pyranoyl, benzoyl, nicotinoyl, cyclopentanecarbonyl, pentanoyl, cyclobutanecarbonyl, 3,3-dimethylbutanoyl, 3-methylbutanoyl, 4-methylpentanoyl, 3-methylpentanoyl, 2-methylpentanoyl, 2,2-dimethylpentanoyl, 4,4-difluorocyclohexanecarbonyl, 3-fluorocyclopentanecarbonyl, 1-methylcyclopropanecarbonyl, I-methylcyclobutanecarbonyl, 4,4,4-trifluorobutanoyl, 3,3,3-trifluoropropanoyl, 5,5,5-trifluoropentanoyl, 1-phenylacetyl, 2,2-difluorobutanoyl, and 2-fluoro-2-methylpropanoyl.

X₂ is preferably a methylene group or an -NH- group. More preferably, X₂ is a methylene group.
r is an integer of 0 or 1. Preferably, r is 0.

Examples of the Cycle moiety include monocyclic, five- or six-membered rings. Specific examples thereof include a benzene ring, a pyridine ring, a thiophene ring.

Zero to two R¹'s described above can be bonded to the Cycle moiety. More specifically, n represents an integer of 0 to 2. Preferably, n is an integer of 1 or 2, and more preferably, n is 1.

When n is 1, the substitution position of R¹ corresponds to the 7th position of a chroman ring, a pyridochroman ring, a 2,3-dihydroquinoline ring, or the like, which belongs to a skeleton in which m = 1 and q = 0, or an isochroman ring or the like, which belongs to a skeleton in which m = 0 and q = 1. This position also corresponds to the 8th position of a 3,4-dihydrobenzo[b]oxepine ring or a 1,2,3,4-tetrahydrobenzo[b]azepine ring, which belongs to a skeleton in which m = 2 and q = 0, or a 3,4-dihydrobenzo[b]isooxepine ring or the like, which belongs to a skeleton in which m = 1 and q = 1. In the substitution positions of R¹'s, at least one of R¹'s is preferably a fluorine atom, a chlorine atoms, isobutyl, tert-butyl, trifluoromethyl, or tetrafluoroethoxy. More preferably, at least R¹ bonded to A₂ or B₂ is a fluorine atom, a chlorine atom, isobutyl, tert-butyl, trifluoromethyl, or tetrafluoroethoxy, and particularly preferably, trifluoromethyl.
j is 0 or 1, and preferably 0.
k is 0 to 2, and preferably 0.

When j or k is not 0, i.e., when j=1 or k=1 or 2, carbon atoms defined by the number of j or k may be mono-substituted by the substituents indicated as "particularly preferable substituent" in the groups shown in (a-1) to (g-1) in the embodiment [1-a].

W represents a methylene group, a carboxyl group or a sulfonyl group. W represents preferably carboxyl group or a sulfonyl group. When w represents a methylene group, L₁ is an oxygen atom and L₂ is a - CR^{9A}R^{9B}-_{.}

R⁷ represents hydrogen, a substituted or unsubstituted hydrocarbon group, or a substituted or unsubstituted heterocyclic group.

Examples of the "substituted or unsubstituted carbonhydrogen group" or the "substituted or unsubstituted heterocyclic group" raised as the preferable R⁷ are:
(1) C₁₋₁₀ alkyl group, (2) C₂₋₆ alkenyl group or (3) C₂₋₆ alkynyl group, (4) C₃₋₉ cycloalkyl group, (5) C₃₋₆ cycloalkenyl group, (6) C₄₋₆ cylcoalcanedienyl group, (7) C₆₋₁₄ aryl group, (8) any one of heterocyclic groups of (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group which contain 1 to 4 heterocarbon atoms selected from an oxygen atom, a sulfur atom or a nitrogen atom other than carbon atom.

The above-mentioned (1) to (8) may be arbitrarily substituted with 1 to 5 substituents in the classes of the subsitutents (a-1) to (g-1) in [1-1-a] mentioned above and the following.

Preferable examples of the "substituted or unsubstituted hydrocarbon group" or the "substituted or unsubstituted heterocyclic group" raised as the preferable R⁷ are:
(1') C₁₋₁₀ alkyl group, (7') C₆₋₁₄ aryl group or (8') any one of heterocyclic groups of (i) five- to six-membered monocyclic aromatic heterocyclic groups (ii) eight- to twelve-membered fused aromatic heterocyclic groups and (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono- or di-substituted by substituents in the classes of the subsitutents (a-1) to (g-1) (especially, the substituents listed as "particularly preferable").

More preferably, R⁷ represents a hydrogen atom or (1') C₁₋₁₀ alkyl group, or (8') any one of heterocyclic groups of (iii) three- to eight-membered saturated or unsaturated non-aromatic heterocyclic group which contain 1 to 2 heterocarbon atoms selected from an oxygen atom, a sulfur atom or a nitrogen atom other than carbon atom which may be mono-or di-substituted by substituents in the classes of the subsitutents (a-1) to (g-1) (especially, the substituents listed as "particularly preferable").

Example of the "substituted or unsubstituted hydrocargon group" raised as more preferable R⁷ is:
(1') C₁₋₆ alkyl group which may be mono-substituted by substituents in the classes of the subsitutents (a-1) to (g-1) in [1-1-a] (especially, the substituents listed as "particularly preferable").

More preferably, R⁷ represents a hydrogen atom, or C₁₋₆ alkyl group or tetrahydropyraniy (preferably teotrahydropyran-4-yl group) which may be mono- or di-substituted by a substituent such as halogen atom, halogenated C₁₋₆ alkyl, cyano, amino, hydroxy, carbamoyl, C₁₋₆ alkoxyl group, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C_{1- 6} alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alky, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, piperazino, oxo, oxiranyl, or tetrahydrofuryl..

Particularily preferably, R⁷ represents a hydrogen atom, or C₁₋₆ alkyl group which may be mono- or di-substituted by a substituent such as amino, hydroxyl, C₁₋₆ alkoxyl, mono/di C₁₋₆ alkylamino, morpholino, piperazino, oxo, oxiranyl, or tetrahydrofuryl.

Examples of the "C₁₋₆ alkyl group" in the substituents of the particularly preferable R⁷ are methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl. Methyl ,ethy, propyl, isopropyl, butyl, isobutyl, or sec-butyl is preferable.

Particularly preferably, R⁷ represents a hydrogen atom, or a methyl group, a ethyl group, a propyl group, isopropyl group, butyl group which may be mono- or di-substituted by a substituent such as amino, hydroxyl, C₁₋₆ alkoxy, mono/di C₁₋₆ alkylamino, phenyl. More concretely, hydrogen atom, methyl group, ethyl group, propyl group, isopropyl group, butyl group, isobutyl, sec-butyl, aminomethyl group, (2-)aminoethyl group, hydroxymethyl group, (2-)hydroxyethyl group, (3-)hydroxypropane-1-yl group, (4-)hydroxybuthyl group, 2-hydroxy-2,2-dimethylethyl group, 1,3-dihydroxy-propane-2-yl group, 1-methyl-2-hydroxyethyl group, 2-hydroxy-propane-1-yl group, methoxyethyl group, (2-)ethoxyethyl group, (2-)N,N-dimethylaminoethyl group, (2-)N,N-diethylaminoethyl group, benzyl group, phenethyl group, oxiranylmethyl group, (2-)tetrahydrofuranylmethyl group etc. (Preferred embodiments are indicated in the parenthesis "( )").

Preferably, R⁸, R^{9A} and R^{9B} each independently represent a substituent selected from a hydrogen atom, a substituted or unsubstituted C₁₋₆ alkyl group, a substituted or unsubstituted heterocyclic group, a substituted or unsubstituted C₁₋₆ alkoxy group, an amino group which may be mono- or di-substituted by a substituted or unsubstituted C₁₋₆ alky group, a protected or unprotected hydroxyl group. The definition of each substituent in R⁸, R^{9A} and R^{9B} has the same meaning as defined in the embodiment [1-1] mentioned above.

Preferably, R⁸ represent a hydrogen atom, a substituted or unsubstituted C₁₋₄ alky group, a substituted or unsubstituted non-aromatic heterocyclic group, a substituted or unsubstituted C₁₋₄ alkoxy group, an amino group which may be mono- or di-substituted by a substituted or unsubstituted C₁₋₄ alkyl group. Example of non-aromatic substituents of "substituted or unsubstituted non-aromatic heterocyclic group" are azetidinyl, morpholinyl, piperidinyl, piperazinyl, pyrrolidinyl, thiazolinyl, oxepanyl, thiomorpholinyl. These substituents arbitrarily substituted with 1 to 3 substituents in a class selected from (a-1) to (g-1) in [1-1] described above (in particular, the substituents listed as "particularly preferable groups").

Examples of more preferable R⁸ are a hydrogen atom, or a group selected from the group consisting of a methyl group, an ethyl group, a methoxy group, an ethoxy group, an n-propoxy group, an azetidinyl group, a morpholinyl group, a piperidinyl group, a piperazinyl group, a pyrrolidinyl group, a thiazolinyl group, an oxepanyl group, a thiomorpholinyl group or amino group which may be substituted by a substituted or unsubstituted C₁₋₂ alkyl group. Each of these groups may be substituted by substituents such as C₁₋₆ alkyl, halogen, amino, hydroxyl, C₁₋₆ alkoxyl, mono-/di-C₁₋₆ alkylamino, or oxo which are listed in [1-1] mentioned above as "particularly preferable group". Examples of substituents in "substituted or unsubstituted C₁₋₂ alkyl" are halogen, amino, hydroxyl, C₁₋₆ alkoxy, mono-/di- C₁₋₆ alkylamino, oxo, 4-pyranoyl.

Examples of further preferable R⁸ are, concretely, a hydrogen atom, a methyl group, an ethyl group, a hydroxyethyl group, a hydroxyethyl group, a methoxyethyl group, a methoxyethyl group, 3-hydroxypropoxy group; 4-morpholinyl group, 2,6-dimethyl-4-morpholinyl group,a 1-piperidinyl group, 4-oxo-1-piperidinyl group, a 4-hydroxy-1-piperidinyl group, 4-methoxy-1-piperidinyl group, 4,4-difluoro-1-piperidinyl group, 1-piperazinyl group, 4-methyl-piperazinyl group, a pyrrolidinyl group, a 3S-fluoro-pyrrolidinyl group, a 3S-hydroxy-pyrodinyl group, a thiazolinyl group, an oxepanyl group, a thiomorpholinyl group, a 2S-hydroxymethyl-pyrrolidinyl, a 2S-methoxymethyl-pyrrolidinyl group; an N,N-dimethylamino group, an N,N-diethylamino group, an N,N-ethylmethylamino group, an N,N-bis(2-methoxyethyl)amino group, an N-methyl-N-(2-methoxyethyl)amino group, an N-methyl-N-cyclohexylamino group, an N-methyl-N-(2-dimethylaminoethyl)amino, an N-methyl-N-(2-hydroxyethyl)amino group, an N-methyl-N-(2-methoxyethyl)amino group, an N-methyl-N-(4-pyranoyl)amino.

Particularly preferable R⁸ hydrogen atom.

Preferably, R^{9A} and R^{9B} are a substituent arbitrarily selected from the group of a hydrogen atom, a substituted or unsubstituted C₁₋₄ alky group, a substituted or unsubstituted non-aromatic heterocyclic group, a substituted or unsubstituted C₁₋₆ alkoxy group, or an amino group which may be mono- or di-substituted by a substituted or unsubstituted C₁₋₄ alky group. Non-aromatic substituents of the "substituted or unsubstituted non-aromatic heterocyclic group" have the same meaning as defined in the embodiment [1-1] mentioned above, and ,for example, azetidinyl group, morpholinyl group, piperidinyl group, piperazinyl group, pyrrolidinyl group, thiazolinyl group, oxepanyl group, thiomorpholinyl group and these substituents are arbitrarily substituted with 1 to 3 substituents in a class selected from (a-1) to (g-1) in [1-1] described above (in particular, the substituents listed as "particularly preferable groups").

R^{9A} and R^{9B} may be same or different, but more preferable R^{9A} and R^{9B} are a substituent selected from a group of a hydrogen atom, or a methyl group, an ethyl group, a methoxy group, an ethoxyl group, an azetidinyl group, a morpholinyl group, a piperidinyl group, a piperazinyl group, a pyrrolidinyl group, a thiazolinyl group, an oxepanyl group, a thiomorpholinyl group or amino group which may be substituted by a substituted or unsubstituted C₁₋₂ alkyl group. These substituents are arbitrarily substituted with substituents listed as "particularly preferable substituent" in [1-1] mentioned above, for example, C₁₋₆ alkyl, halogen, amino, hydroxyl, C₁₋₆ alkoxyl group, mono-/di-C₁₋₆ alkylamino, oxo. Examples of the substituents in "substituted or unsubstituted C₁₋₂ alkyl" are halogen, amino, hydroxyl, C₁₋₆ alkoxyl, mono-/di- C₁₋₆ alkylamino, oxo, 4-pyranoyl.

Examples of further preferable R^{9A} and R^{9B} are, concretely, a hydrogen atom, a methyl group, an ethyl group, a hydroxyethyl group, a hydroxyethyl group, a methoxymethyl group, a methoxymethyl group; 4-morpholinyl group, 2,6-dimethyl-4-morpholinyl group, a 1-piperidinyl group, 4-oxo-1-piperidinyl group, a 4-hydroxy-1-piperidinyl group, 4-methoxy-1-piperidinyl group, 4,4-difluoro-1-piperidinyl group, 1-piperazinyl group, 4-methyl-piperazinyl group, a pyrrolidinyl group, a 3S-fluoro-pyrrolidinyl group, a 3S-hydroxy-pyrrolidinyl group, a thiazolinyl group, an oxepanyl group, a thiomorpholinyl group, a 2S-hydroxymethyl-pyrrolidinyl, a 2S-methoxymethyl-pyrrolidinyl group; an N,N-dimethylamino group, an N,N-diethylamino group, an N,N-ethylmethylamino group, an N,N-bis(2-methoxyethyl)amino group, an N-methyl-N-(2-methoxyethyl)amino group, an N-methyl-N-cyclohexylamino group, an N-methyl-N-(2-dimethylaminoethyl)amino, an N-methyl-N-(2-hydroxyethyl)amino group, an N-methyl-N-(2-methoxyethyl)amino group, an N-methyl-N-(4-pyranoyl)amino.

Particularly preferable R^{9A} and R^{9B} are hydrogen atom or methyl group when they are the same; and one of them represents the hydrogen atom and the other presents a group (except the hydrogen atom) listed in [1-14-b-2] mentioned above.

L₁ and L₂ each independently represent single bond, -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or - S(O)t- (t is an integer of 0 to 2), and L₁ and L₂ may be identical with or different from each other.

Preferable L₁ and L₂ are as follows: in a case where L₁ and L₂ are identical with each other, they are each independently single bond or -CR^{9A}R^{9B}-, and in a case where L₁ and L₂ are different from each other, one is -CR^{9A}R^{9B}-, and the other is oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). When w represents a methylene group, L₁ is an oxygen atom and L₂ is a - CR^{9A}R^{9B}-.

More preferable L₁ and L₂ are as follows: in a case where L₁ is - CR^{9A}R^{9B}-, L₂ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2). More preferable L₁ and L₂ are as follows: in a case where L₂ is -CR^{9A}R^{9B}-, L₁ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or - S(O)t- (t is an integer of 0 to 2).

More specifically, in a case where the solid line and broken line between L₁ and L₂ are single bonds, the moiety of L₁ and L₂ can be represented by the following formula: 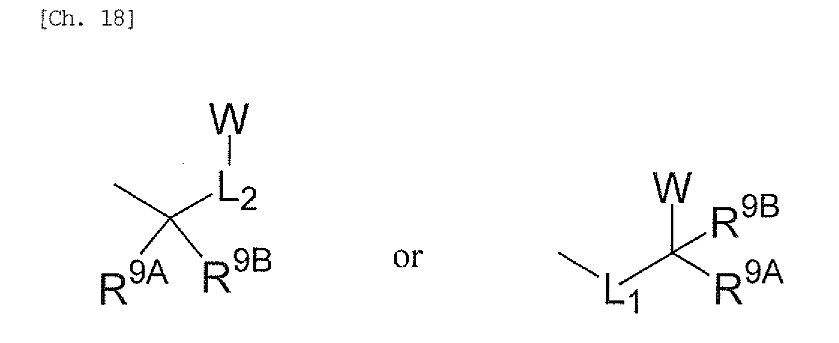 and it is more preferable that R^{9B} is hydrogen atom. Further, in a case where the solid line and broken line between L₁ and L₂ are double bonds, the moiety of L₁ and L₂ can be represented by the following formula: 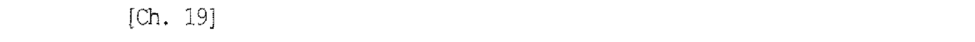 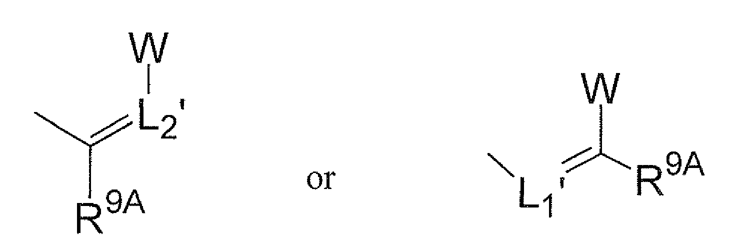 wherein L₁' and L₂' represent -CR^{9B}= or -N=.

In these cases, preferable R^{9A} and R^{9B} can include hydrogen atom, methyl group, ethyl group, hydroxymethyl group, hydroxyethyl group, methoxymethyl group, methoxyethyl group; 4-morpholinyl group, 2,6-dimethyl-4-morpholinyl group, 1-piperidinyl group, 4-oxo-1-piperidinyl group, 4-hydroxy-1-piperidinyl group, 4-methoxy-1-piperidinyl group, 4,4-difluoro-1-piperidinyl group, 1-piperadinyl, group, 4-methyl-piperadinyl group, pyrrolidinyl group, 3S-fluoro-pyrrolidinyl group, 3S-hydroxy-pyrrolidinyl group, thiazolinyl group, oxepanyl group, thiomorpholinyl group, 2S-hydroxymethyl-pyrrolidinyl group, 2S-methoxymethyl-pyrrolidinyl group; N,N-dimethylamino group, N,N-diethylamino group, an N,N-ethylmethylamino group, N,N-bis(2-methoxyethyl)amino group, N-methyl-N-(2-methoxyethyl)amino group, N-methyl-N-cyclohexylamino group, N-methyl-N-(2-dimethylaminoethyl)amino group, N-methyl-N-(2-hydroxyethyl)amino group, an N-methyl-N-(2-methoxyethyl)amino group, N-methyl-N-(4-pyranoyl)amino group, and the like that are mentioned in [1-14-b-2].

Further preferable L₁ and L₂ are as follows: in a case where L₂ is -CR^{9A}R^{9B}-, L₁ is -CR^{9A}R^{9B}-, oxygen atom, -NR¹⁰- (R¹⁰ represents hydrogen atom, a substituted or unsubstituted hydrocarbon group, a substituted or unsubstituted heterocyclic group, or a substituted or unsubstituted acyl group), or -S(O)t- (t is an integer of 0 to 2).

The solid line and broken line between L₁ and L₂ are single bonds or double bonds, the moiety of L₁ and L₂ can be represented by the following formula: 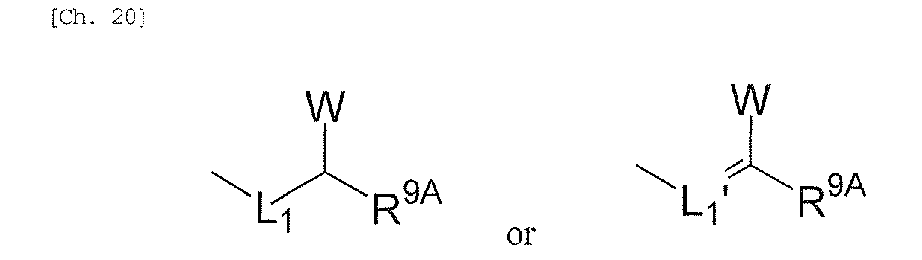 wherein L₁' represents -CR^{9B}= or -N=.

In these cases, preferable R^{9A} and R^{9B} can include hydrogen atom, methyl group, ethyl group, hydroxymethyl group, hydroxyethyl group, methoxymethyl group, methoxymethyl group; 4-morpholinyl group, 2,6-dimethyl-4-morpholinyl group, 1-piperidinyl group, 4-oxo-1-piperidinyl group, 4-hydroxy-1-piperidinyl group, 4-methoxy-1-piperidinyl group, 4,4-difluoro-1-piperidinyl group, 1-piperadinyl group, 4-methyl-piperadinyl group, pyrrolidinyl group, 3S-fluoro-pyrrolidinyl group, 3S-hydroxy-pyrrolidinyl group, thiazolinyl group, oxepanyl group, thiomorpholinyl group, 2S-hydroxymethyl-pyrrolidinyl group, 2S-methoxymethyl-pyrrolidinyl group; N,N-dimethylamino group, N,N-diethylamino group, an N,N-ethylmethylamino group, N,N-bis(2-methoxyethyl)amino group, N-methyl-N-(2-methoxyethyl)amino group, N-methyl-N-cyclohexylamino group, N-methyl-N-(2-dimethylaminoethyl)amino group, N-methyl-N-(2-hydroxyethyl)amino group, an N-methyl-N-(2-methoxyethyl)amino group, N-methyl-N-(4-pyranoyl)amino group, and the like that are mentioned in [1-14-b-2]. More preferably, R9B in L2' is hydrogen atom.

Particularly preferable L₁ and L₂ are as follows: in a case where L₁ is -CH₂-, L₂ is -CR^{9A}H-, or L₁ is -CH=, L₂ is =CR^{9A}-. In this case, it is particularly preferable that R^{9A} is morpholino group. For example, the solid line and broken line between L₁ and L₂ are single bonds or double bonds, and the moiety of L₁ and L₂ can be represented by the following formula:

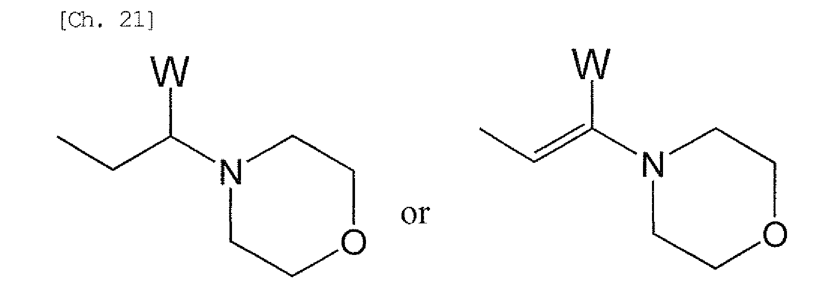

In L₁ and L₂, t is an integer of 0 to 2, and it is preferable that t is 0 or 2.

Preferable R¹⁰ includes a hydrogen atom, or C₁₋₆ alkyl group or tetrahydropyraniy (preferably teotrahydropyran-4-yl group) which may be mono- or di-substituted by a substituent such as halogen atom, halogenated C₁₋₆ alkyl, cyano, amino, hydroxyl, carbamoyl, C₁₋₆ alkoxyl group, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-C₁₋₆ alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, benzoyl, morpholino, piperazino, oxo, oxiranyl, or tetrahydrofuryl and the like.

More preferable R¹⁰ includes a hydrogen atom, or C₁₋₆ alkyl group which may be mono- or di-substituted by a substituent such as amino, hydroxyl, C₁₋₆ alkoxyl, mono/di C₁₋₆ alkylamino, morpholino, piperazino, oxo, oxiranyl, or tetrahydrofuryl and the like.

"C₁₋₆ alkyl group" in the substituents of the particularly preferable R¹⁰ are methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl. Methyl, ethy, propyl., isopropyl, butyl, isobutyl, or sec-butyl is preferable.

Particularly preferably, R¹⁰ represents a hydrogen atom, or a methyl group, a ethyl group, a propyl group, isopropyl group, butyl group which may be mono- or di-substituted by a substituent such as amino, hydroxyl, C₁₋₆ alkoxy, mono/di C₁₋₆ alkylamino, phenyl. More concretely, hydrogen atom, methyl group, ethyl group, propyl group, isopropyl group, butyl group, isobutyl, sec-butyl, aminomethyl group, (2-)aminoethyl group, hydroxymethyl group, (2-)hydroxyethyl group, (3-)hydroxypropane-1-yl group, (4-)hydroxybuthyl group, 2-hydroxy-2,2-dimethylethyl group, 1,3-dihydroxy-propane-2-yl group, 1-methyl-2-hydroxyethyl group, 2-hydroxy-propane-1-yl group, methoxyethyl group, (2-)ethoxyethyl group, (2-)N,N-dimethylaminoethyl group, (2-)N,N-diethylaminoethyl group, benzyl group, phenethyl group, oxiranylmethyl group, (2-)tetrahydrofuranylmethyl group etc. (Preferred embodiments are indicated in the parenthesis "( )").

Most preferable R¹⁰ includes hydrogen atom, methyl group, ethyl group, hydroxymethyl group, hydroxyethyl group or methoxyethyl group. The arylamine group in formula (I) is represented by formula (A) : 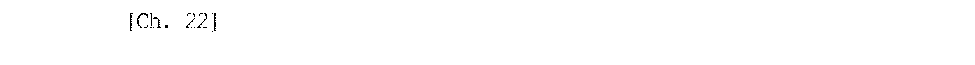 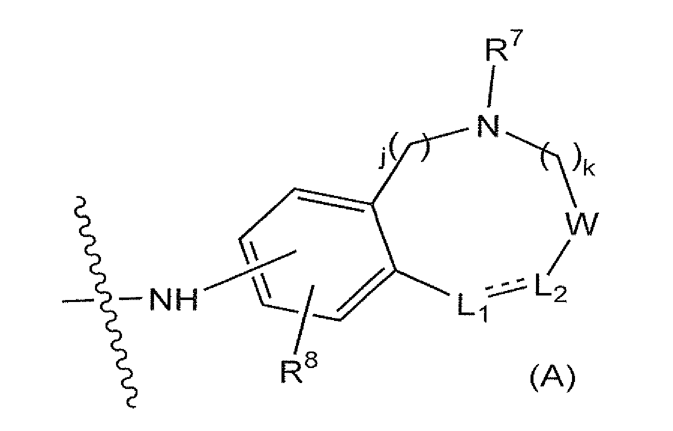 (wherein the definitions of k, j, t, W, R7, R8, R9A, R9B, R10, L1 and L2 are the same as those described in one of embodiments [1-1] to [1-17]), and preferably, formula (a) : 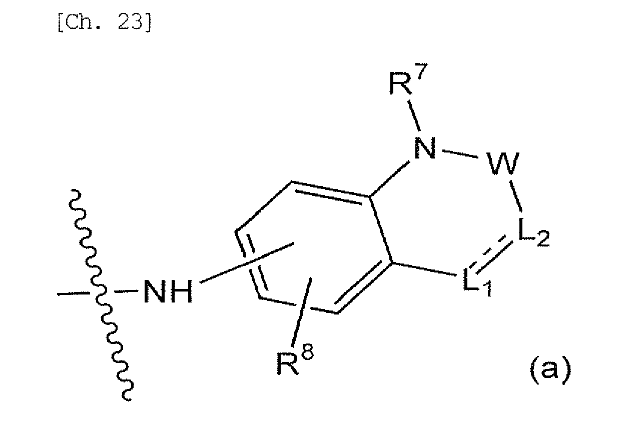 (wherein the definitions of k, j, t, W, R7, R8, R9A, R9B, R10, L1 and L2 are the same as those described in one of embodiments [1-10] to [1-17]), in formula (A) and formula (a), -NH- or R8 is bonded to the positions of G1 to G4 of the phenyl ring described below. -NH- is preferably bonded to the first position (G4) or third position (G2) in the clockwise direction from the condensation position close to the L1, and more preferably bonded to the third position (G2). When -NH- is bonded to the G2 position, R8 is preferably bonded to the G4 position.

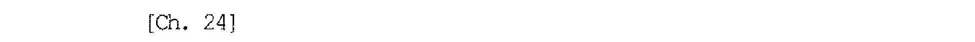

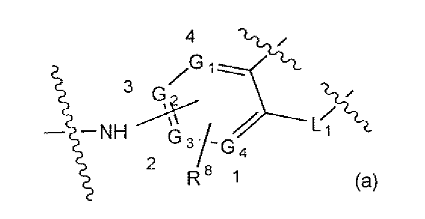

Preferable examples of each substituents are the as those described previously in embodiment of [1-10] to [1-17], more specifically, formula (a) represents formula (a1) to (a141) described below.

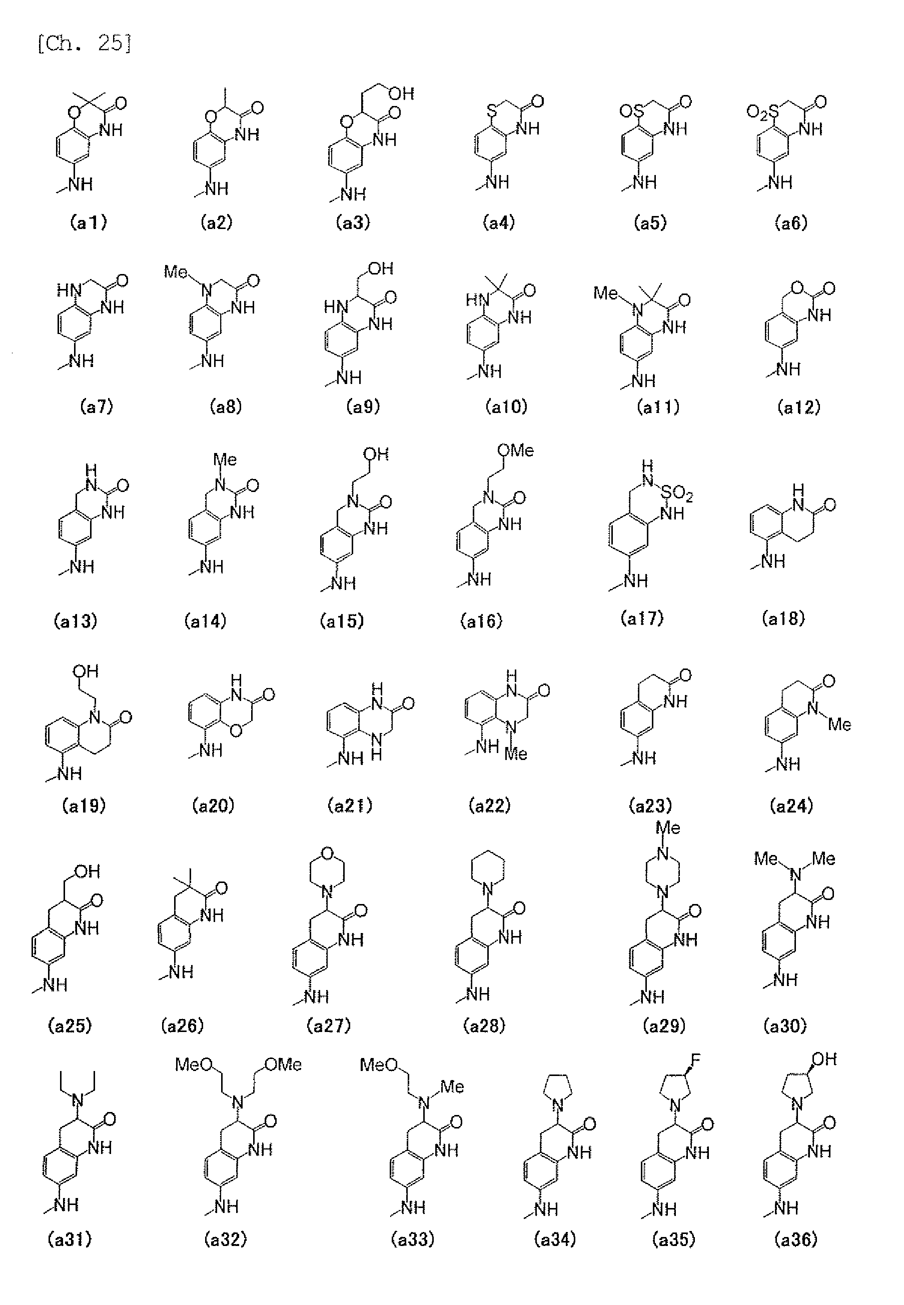

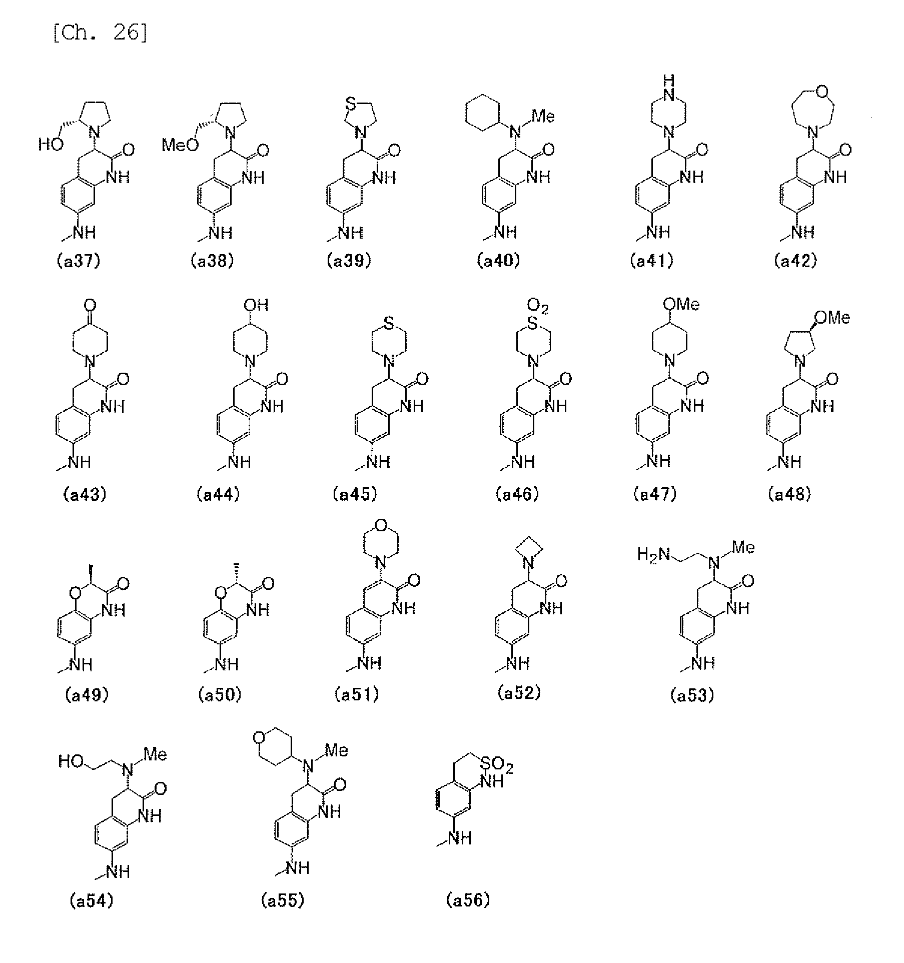

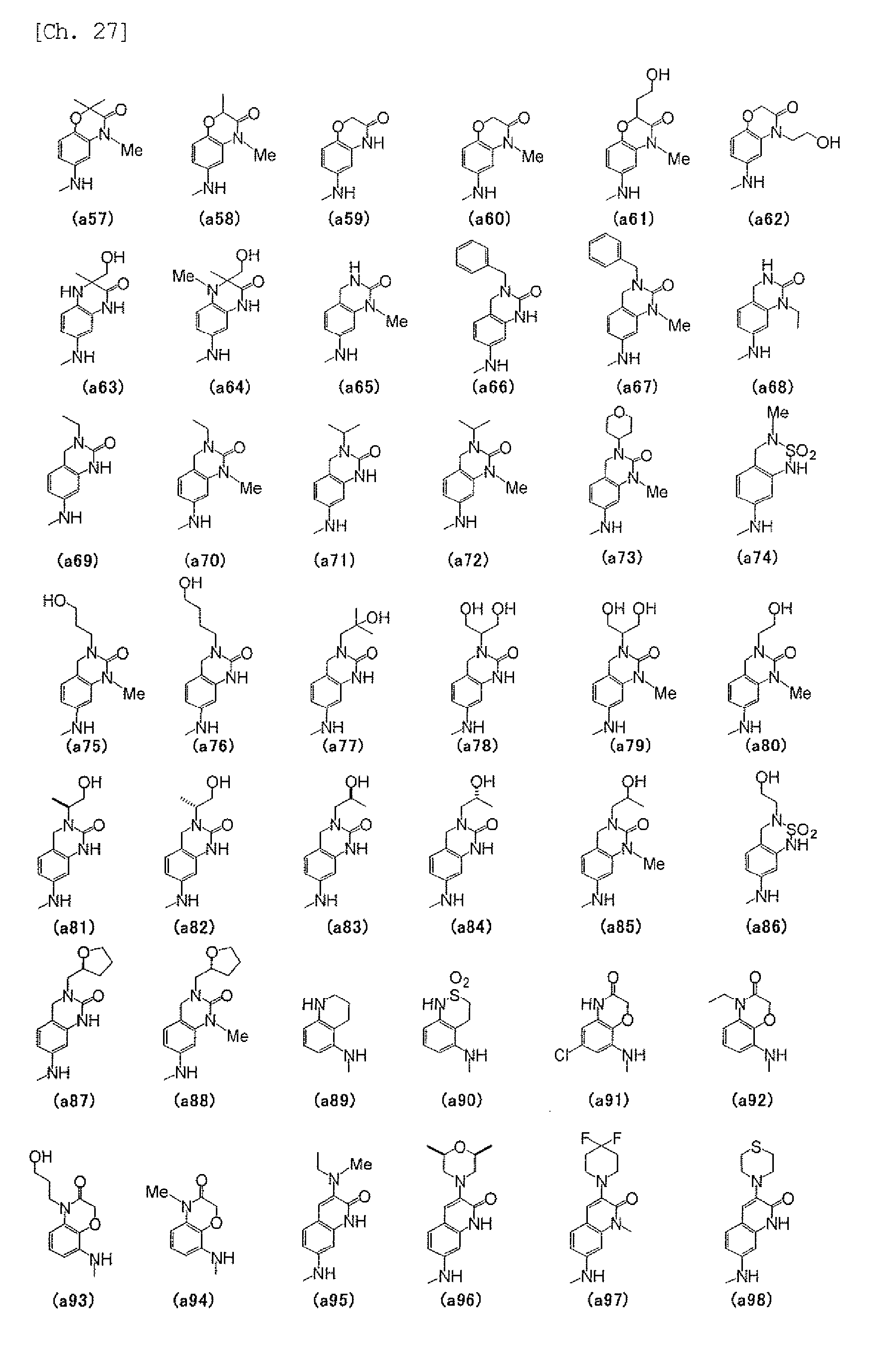

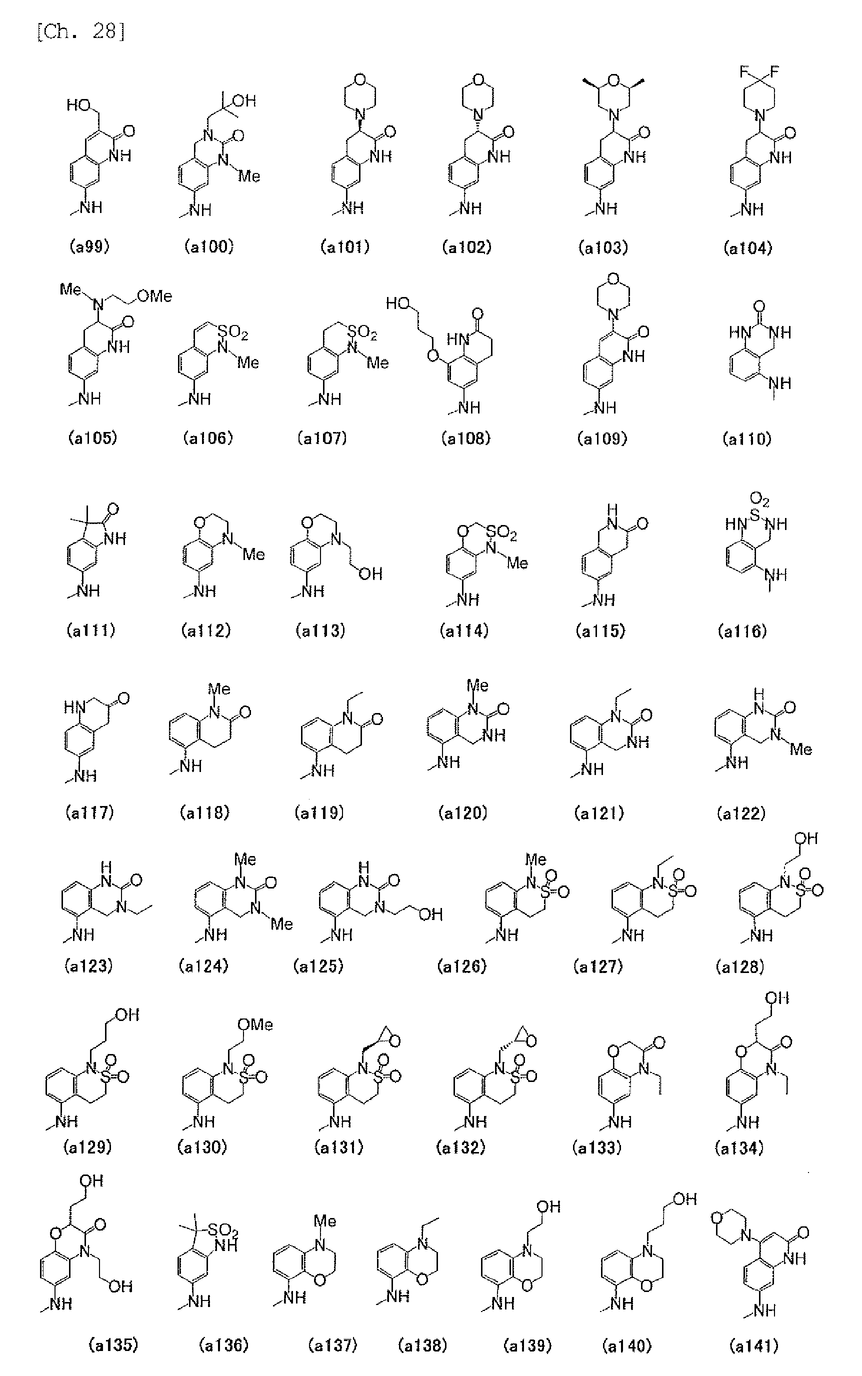

The wavy line to which "CO-NH" in formula (I) of the present invention bonded represents a bond of an E-isomer (anti-isomer or trans-isomer) or a Z-isomer (syn-isomer or cis-isomer). This means that the compounds represented by formula (I) include E-isomers(anti-isomer or trans-isomer) and Z-isomers(syn-isomer or cis-isomer). The compounds represented by formula (I) are preferably E-isomers(anti-isomer or trans-isomer). Hereinafter, wavy lines in formulae in this description represent the same meaning.

In the compounds represented by formula (I) in embodiment [1], the ring containing X1 and X2 is preferably five- to eight-membered, more preferably six- or seven-membered. The ring containing W is preferably five- to eight-membered, more preferably five- to seven-membered, and most preferably five- or six-membered. When L1 and L2 are both single bond, W connects to the phenyl ring.

### Examples of preferable compounds include:

(E)-2-(8-trifluoromethyl-3,4-dihydrobenzo[b]oxepin-5(2H)-ylidene)-N-(2,2-dimethyl-4H-benzo [1,4]oxazin-3-on-6-yl)acetamide(EXAMPLE 1) ;
Synthesis of (E)-2-(8-trifluoromethyl-3,4-dihydrobenzo[b]oxepin-5(2H)-ylidene)-N-(2-methyl-4H-benzo [1,4]oxazin-3-on-6-yl)acetamide(EXAMPLE 2) ;
(E)-2-(8-trifluoromethyl-3,4-dihydrobenzo[b]oxepin-5(2H)-ylidene)-N-(2-(2-hydroxyethyl)-4H-benzo[1,4]oxazin-3-on-6-yl)acetamide (EXAMPLE 3) ;
(E)-2-(8-trifluoromethyl-3,4-dihydrobenzo[b]oxepin-5(2H)-ylidene)-N-(2H-benzo [1,4]thiazin-3(4H)-on-6-yl)acetamide(EXAMPLE 4) ;
(E)-2-(8-trifluoromethyl-3,4-dihydrobenzo[b]oxepin-5(2H)-ylidene)-N-(1-oxy-2H-benzol,4]thiazin-3(4H)-on-6-yl)acetamide(EXAMPLE 5) ;
(E)-2-(8-trifluoromethyl-3,4-dihydrobenzo[b]oxepin-5(2H)-ylidene)-N-(sulfazon-6-yl)acetamide(EXAMPLE 6) ;
(E)-2-(8-trifluoromethyl-3,4-dihydrobenzo[b]oxepin-5(2H)-ylidene)-N-(3,4-dihydro-2(1H)-quinoxalinon-7-yl)acetamide(EXAMPLE 7) ;
(E)-2-(8-trifluoromethyl-3,4-dihydrobenzo[b]oxepin-5(2H)-ylidene)-N-(4-methyl-3,4-dihydro-2(1H)-quinoxalinon-7-yl)acetamide(EXAMPLE 8) ;
(E)-2-(8-trifluoromethyl-3,4-dihydrobenzo[b]oxepin-5(2H)-ylidene)-N-(3-hydroxymethyl-3,4-dihydro-2(1H)-quinoxalinon-7-yl)acetamide(EXAMPLE 9) ;
(E)-2-(8-trifluoromethyl-3,4-dihydrobenzo[b]oxepin-5(2H)-ylidene)-N-(3,3-dimethyl-3,4-dihydro-2(1H)-quinoxalinon-7-yl)acetamide(EXAMPLE 10) ;
(E)-2-(8-trifluoromethyl-3,4-dihydrobenzo[b]oxepin-5(2H)-ylidene)-N-(3,3-dimethyl-4-methyl-3,4-dihydro-2(1H)-quinoxalinon-7-yl)acetamide(EXAMPLE 11) ;
(E)-2-(8-trifluoromethyl-3,4-dihydrobenzo[b]oxepin-5(2H)-ylidene)-N-(1,4-dihydro-2H-3,1-benzoxadin-2-on-7-yl)acetamide(EXAMPLE 12) ;
(E)-2-(8-trifluoromethyl-3,4-dihydrobenzo[b]oxepin-5(2H)-ylidene)-N-(3,4-dihydro-1H-quinazolin-2-on-7-yl)acetamide(EXAMPLE 13) ;
(E)-2-(8-trifluoromethyl-3,4-dihydrobenzo[b]oxepin-5(2H)-ylidene)-N-(3-methyl-3,4-dihydro-2(1H)-quinazolinon-7-yl)acetamide(EXAMPLE 14) ;
(E)-2-(8-trifluoromethyl-3,4-dihydrobenzo[b]oxepin-5(2H)-ylidene)-N-(3- (2-hydroxyehtyl)-3,4-dihydro-2(1H)-quinazolinon-7-yl)acetamide(EXAMPLE 15) ;
(E)-2-(8-trifluoromethyl-3,4-dihydrobenzo[b]oxepin-5(2H)-ylidene)-N-(3- (2-methoxyehtyl)-3,4-dihydro-2(1H)-quinazolinon-7-yl)acetamide(EXAMPLE 16) ;
(E)-2-(8-trifluoromethyl-3,4-dihydrobenzo[b]oxepin-5(2H)-ylidene)-N-(3,4-dihydro-2,2-dioxo-1H-2,1,3-benzothiadiazin-7-yl)acetamide <Step 1> Synthesis of 7-nitro-3,4-dihydro-2,2-dioxy-1H-2,1,3-benzothiadine(EXAMPLE 17) ;
(E)-2-(8-trifluoromethyl-3,4-dihydrobenzo[b]oxepin-5(2H)-ylidene)-N-(3,4-dihydro-2(1H)-quinolinon-5-yl)acetamide(Example 18) ;
(E)-2-(8-trifluoromethyl-3,4-dihydrobenzo[b]oxepin-5(2H)-ylidene)-N-(1-(2-hydroxyethyl)-3,4-dihydro-2(1H)-quinolinon-5-yl)acetamide(EXAMPLE 19) ;
(E)-2-(8-trifluoromethyl-3,4-dihydrobenzo[b]oxepin-5(2H)-ylidene)-N-(2H-1,4-benzoxazin-3(4H)-on-8-yl)acetamide(EXAMPLE 20) ;
(E)-2-(8-trifluoromethyl-3,4-dihydrobenzo[b]oxepin-5(2H)-ylidene)-N-(3,4-dihydro-2 (1H)-quinoxalinon-5-ylacetamide(EXAMPLE 21) ;
(E)-2-(8-trifluoromethyl-3,4-dihydrobenzo[b]oxepin-5(2H)-ylidene)-N-(3,4-dihydro-4-methyl-2 (1H)-quinoxalinon-5-yl)acetamide(EXAMPLE 22) ;
(E)-2-(7-trifluoromethylchroman-4-ylidene)-N-(3,4-dihydroquinolin-2(1H)-on-7-yl)acetamide(EXAMPLE 23) ;
(E)-2-(7-trifluoromethyl-2,2-dimethylchroman-4-ylidene)-N-(3,4-dihydroquinolin-2(1H)-on-7-yl)acetamide(EXAMPLE 24) ;
(E)-2-(7-trifluoromethyl-2,2-dimethylchroman-4-ylidene)-N-(1-methyl-3,4-dihydro-2(1H)-quinolinon-7-yl)acetamide(EXAMPLE 25) ;
(Z)-2-(6-trifluoromethyl-3,3-dimethyl-4-oxa-3,4-dihydroisoquinolin-1(2H)-ylidene)-N-(3,4-dihydroquinolin-2(1H)-on-7-yl)acetamide(EXAMPLE 26) ;
(Z)-2-(8-trifluoromethyl-3,4-dihydrobenzo[f][1,4]oxazepin-5(2H)-ylidene)-N-(3,4-dihydroquinolin-2(1H)-on-7-yl)acetamide(EXAMPLE 27) ;
(E)-2-(8-trifluoromethyl-3,4-dihydrobenzo[b]oxepin-5(2H)-ylidene)-N-(3-hydroxymethyl-3,4-dihydro-2(1H)-quinolinon-7-yl)acetamide(EXAMPLE 28) ;
(E)-2-(8-trifluoromethyl-3,4-dihydrobenzo[b]oxepin-5(2H)-ylidene)-N-(3,3-di-methyl-3,4-dihydro-2(1H)-quinoiinon-7-yl)acetamide(EXAMPLE 29) ;
(E)-2-(8-trifruoromethyl-3,4-dihydrobenzo[b]oxepin-5(2H)-ylidene)-N-(3-(4-morpholinyl)-3,4-dihydro-2(1H)-quinolinon-7-yl)acetamide(EXAMPLE 30) ;
(E)-2-(8-trifluoromethyl-3,4-dihydrobenzo[b]oxepin-5(2H)-ylidene)-N-(3-(1-piperidinyl)-3,4-dihydro-2(1H)-quinolinon-7-yl)acetamide(EXAMPLE 31) ;
(E)-2-(7-trifluoromethyl-3,4-dihydrobenzo[b]oxepin-5(2H)-ylidene)-N-(3-(4-methyl-1-piperazinyl)-3,4-dihydro-2(1H)-quinolinon-7-yl)acetamide(EXAMPLE 32) ;
(E)-2-(7-trifluoromethyl-2,2-dimethylchroman-4-ylidene)-N-(3-(4-morpholinyl)-3,4-dihydro-2(1H)-quinolinon-7-yl)acetamide(EXAMPLE 33) ;
(E)-2-(8-trifluoromthyl-3,4-dihydrobenzo[b]oxepin-5(2H)-ylidene)-N-(3-(4-morpholinyl)-3,4-dihydro-2(1H)-quinolinon-7-yl)acetamide(EXAMPLE 34, 35) ;
(E)-2-(8-trifluoromethyl-3,4-dihydrobenzo[b]oxepin-5(2H)-yliden)-N-(3-oxo-1,2,3,4-tetrahydroquinolin-5-yl)acetamide(EXAMPLE 36) ;
(E)-2-(8-trifluoromethyl-3,4-dihydrobenzo[b]oxepin-5(2H)-ylidene)-N-(8-(3-hydroxypropoxy)-3,4-dihydro-2(1H)-quinolinon-5-yl)acetamide(EXAMPLE 37) ;
(E)-2-(8-trifluoromethyl-3,4-dihydrobenzo[b]oxepin-5(2H)-ylidene)-N-(3-benzyl-3,4-dihydro-2(1H)-quinazolinon-7-yl)acetamide(EXAMPLE 38) ;
(E)-2-(8-trifluoromethyl-3,4-dihydrobenzo[b]oxepin-5(2H)-ylidene)-N-(3-benzyl-1-methyl-3,4-dihydro-2(1H)-quinazolinon-7-yl)acetamide(EXAMPLE 39) ;
(E)-2-(8-trifluoromethyl-3,4-dihydrobenzo[b]oxepin-5(2H)-ylidene)-N-(3-hydroxymethyl-3-methyl-3,4-dihydro-2(1H)-quinoxalinon-7-yl)acetamide(EXAMPLE 40) ;
(E)-2-(8-trifluoromethyl-3,4-dihydrobenzo[b]oxepin-5(2H)-ylidene)-N-(3-hydroxymethyl-3,4-dimethyl-3,4-dihydro-2(1H)-quinoxalinon-7-yl)acetamide(EXAMPLE 41) ;
(E)-2-(8-trifluoromethyl-3,4-dihydrobenzo[b]oxepin-5(2H)-ylidene)-N-(3- (N,N-dimethylamino) -3,4-dihydro-2(1H)-quinolinon-7-yl)acetamide(EXAMPLE 42) ;
(E)-2-(8-trifluoromethyl-3,4-dihydrobenzo[b]oxepin-5(2H)-ylidene)-N-(3- (N,N-diethylamino) -3,4-dihydro-2(1H)-quinolinon-7-yl)acetamide(EXAMPLE 43) ;
(E)-2-(8-trifluoromethyl-3,4-dihydrobenzo[b]oxepin-5-(2H)-ylidene)-N-(3-(N,N-bis(2-methoxyethyl)amino))3,4-dihydro-2(1H)-quinolinon-7-yl)acetamide(EXAMPLE 44) ;
(E)-2-(8-trifluoromethyl-3,4-dihydrobenzo[b]oxepin-5-(2H)-ylidene)-N-(3-(N-methyl-N-(2-methoxyethyl)amino)-3,4-dihydro-2(1H)-quinolinon-7-yl)acetamide(EXAMPLE 45) ;
(E)-2-(8-trifluoromethyl-3,4-dihydrobenzo[b]oxepin-5-(2H)-ylidene)-N-(3-((pyrrolidin-1-yl)-3,4-dihydro-2(1H)-quinolinon-7-yl)acetamide(EXAMPLE 46) ;
(E)-2-(8-trifluoromethyl-3,4-dihydrobenzo[b]oxepin-5(2H)-ylidene)-N-(3-((3S)-fluoropyrrolidin-1-yl)-3,4-dihydro-2(1H)-quinolinon-7-yl)acetamide(EXAMPLE 47) ;
(E)-2-(8-trifluoromethyl-3,4-dihydrobenzo[b]oxepin-5(2H)-ylidene)-N-(3-((3S)-hydroxypyrrolidin-1-yl)-3,4-dihydro-2(1H)-quinolinon-7-yl)acetamide(EXAMPLE 48) ;
(E)-2-(8-trifluoromethyl-3,4-dihydrobenzo[b]oxepin-5(2H)-ylidene)-N-(3-((2S)-hydroxymethylpyrrolidin-1-yl)-3,4-dihydro-2(1H)-quinolinon-7-yl)acetamide(EXAMPLE 49) ;
(E)-2-(8-trifluoromethyl-3,4-dihydrobenzo[b]oxepin-5(2H)-ylidene)-N-(3-((2S)-methoxymethylpyrrolidin-1-yl)-3,4-dihydro-2(1H)-quinolinon-7-yl) acetamide (EXAMPLE 50) ;
(E)-2-(8-trifluoromethyl-3,4-dihydrobenzo[b]oxepin-5(2H)-ylidene)-N-(3-(N-methyl-N-cyclohexylamino)-3,4-dihydro-2(1H)-quinolinon-7-yl)acetamide(EXAMPLE 51) ;
(E)-2-(8-trifluoromethyl-3,4-dihydrobenzo[b]oxepin-5(2H)-ylidene)-N-(3-(1-piperazinyl)-3,4-dihydro-2(1H)-quinolinon-7-yl)acetamide(EXAMPLE 52) ;
(E)-2-(8-trifluoromethyl-3,4-dihydrobenzo[b]oxepin-5(2H)-ylidene)-N-(3-([1,4]oxazepan-4-yl)-3,4-dihydro-2(1H)-quinolinon-7-yl)acetamide(EXAMPLE 53) ;
(E)-2-(8-trifluoromethyl-3,4-dihydrobenzo[b]oxepin-5(2H)-ylidene)-N-(3-(4-thiomorpholinyl)-3,4-dihydro-2(1H)-quinolinon-7-yl)acetamide(EXAMPLE 54) ;
(E)-2-(8-trifluoromethyl-3,4-dihydrobenzo[b]oxepin-5(2H)-ylidene)-N-(3-(4-methoxy-1-piperidinyl)-3,4-dihydro-2(1H)-quinolinon-7-yl)acetamide(EXAMPLE 55) ;
(E)-2-(8-trifluoromethyl-3,4-dihydrobenzo[b]oxepin-5(2H)-ylidene)-N-(3-((3S)-methoxy pyrrolidin-1-yl)-3,4-dihydro-2(1H)-quinolinon-7-yl)acetamide(EXAMPLE 56) ;
(E)-2-(8-trifluoromethyl-3,4-dihydrobenzo[b]oxepin-5(2H)-ylidene)-N-(3-(N-methyl-N-(4- tetrahydropyranyl)amino)-3,4-dihydro-2(1H)-quinolinon-7-yl)acetamide(EXAMPLE 57) ;
   and the compound described below example 58-301 ; or
   examples of pharmaceutically acceptable salts thereof, solvate thereof and optical isomers thereof.

More preferably, the compound of the group A, B, C or D described below.

### Grpoup A:

The compounds of EXAMPLE
9,13,14,15,23,26,28,30,33,34,35,40,45,53,59,64,65,74,77,81,88,89,93,94, 95,107,109,110,112,113,114,115,151,154,161,180,181,182,183,196,200,210, 211,212 and 213.

### Grpoup B:

The compounds of EXAMPLE
61,62,73,75,76,78,79,80,82,93,96,97,117,118,119,134,136,137,138,139,140 ,141,152,153,162,163,176,187,188,189,191 and 193.

### Group C : 71,83,104,121,160,166,169,185,186,194,195,197 and 206.

### Group D :

The compounds of EXAMPLE
66,68,69,70,84,85,87,106,108,120,122,123,124,125,126,127,128,129,130,13 1,132,133,142,143,144,145,146,147,148,149,150,156,157,158,164,167,168,1 72,173,174,177,179,190,200,202,203,208 and 209.

The compound of the group A or B is Further preferable, the compound of the group A is Particularly preferable. Thses preferable compounds of group A, B, C, or D also include pharmaceutically acceptable salts thereof, solvate thereof and optical isomers thereof.

[1-22] In the compounds represented by formula (I) in embodiment [1], examples of more preferable compounds include compounds represented by formula (I-A).

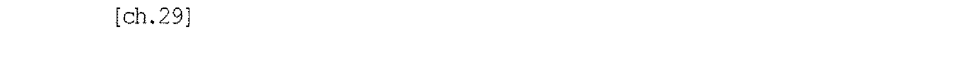

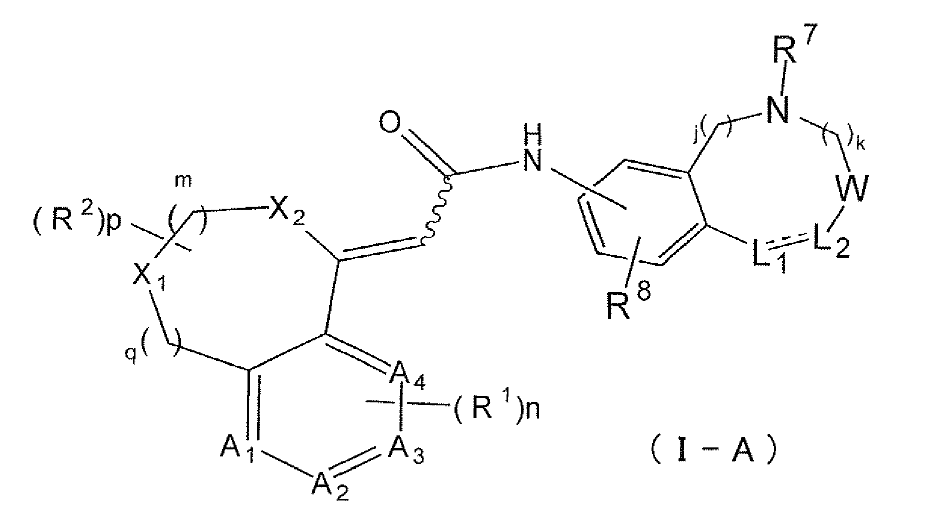

In the compounds represented by formula (I-A), A1, A2, A3 and A4 represents each independly -N= or -CH=, and R1, R2, X1, X2, m, n, p, q, R7, R8, W, L1, L2, j, k, and t are the same as those described in one of embodiments [1-1] to [1-20], preferably the same as those described in [1-21]. The wavy line to which "CO-NH" in formula (I) of the present invention is bonded preferably represents a bond of an E-isomer (anti-isomer or trans-isomer). Here, q is an integer of 0 or 1. When q is 0, the compounds can be represented by formula (I-A-1). When q is 1, the compounds can be represented by formula (I-A-2). Preferable formula (A) in formula (I-A), an arylamine group, is represented by formula (a) or (a1) to (a141) as those described in embodiment of [1-18].

[1-23] In the compounds represented by formula (I) in embodiment [1], examples of more preferable compounds represented by formula (I-A) include compounds represented by formula (I-B).

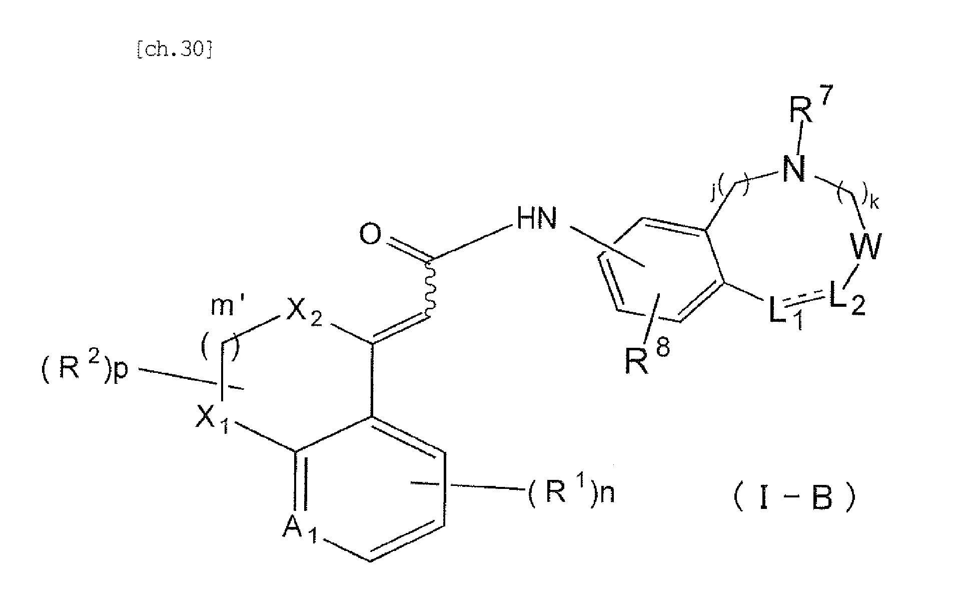

In formula (I-B), A₁ representss -N= or -CH=, m' is an integer of 1 or 2, the definitions in of R¹, R², X₁, X₂, m, n, p, q, R⁷, R⁸, W, L₁, L₂, j, k and p are the same as those described in one of embodiments [1-1] to [1-20], and preferably, the same as the definitions in embodiment [1-21]. The wavy line to which "CO-NH" in formula (I) of the present invention is bonded preferably represents a bond of an E-isomer (anti-isomer or trans-isomer). Here, m' is an integer of 1 or 2. When m' is 1, the compounds can be represented by formula (I-B-1). When m' is 2, the compounds can be represented by formula (I-B-2). Preferable formula (A) in formula (I-B), an arylamine group, is represented by formula (a) or (a1) to (a141) as those described in embodiment of [1-18].

[1-24] In the compounds represented by formula (I) in embodiment [1], examples of more preferable compounds represented by formula (I-B) include compounds represented by formula (I-C). 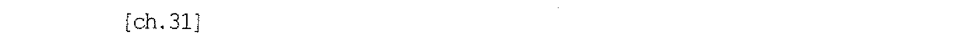 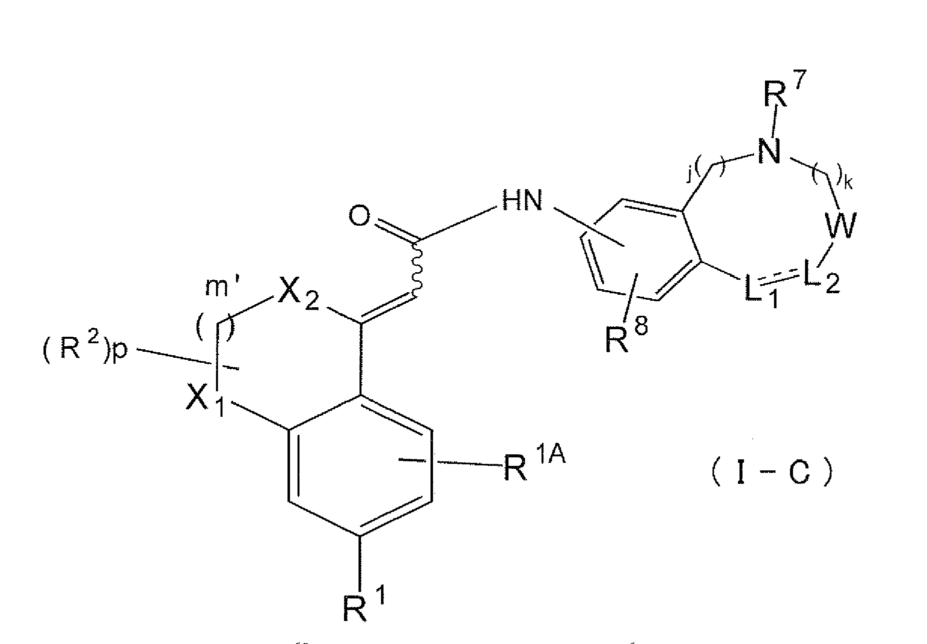 In formula (I-C), R^{1A} represents hydrogen or R¹ described before, m' is an integer of 1 or 2, and the definitions of R¹, R², X₁, X₂, R⁷, R⁸, W, L₁, L₂, j, k, and p are the same as those described in one of embodiments [1-1] to [1-20], and preferably, the same as the definitions in embodiment [1-21]. The wavy line to which "CO-NH" in formula (I) of the present invention is bonded preferably represents a bond of an E-isomer (anti-isomer or trans-isomer). Here, m' is an integer of 1 or 2. When m' is 1, the compounds can be represented by formula (I-C-1). When m' is 2, the compounds can be represented by formula (I-C-2). Preferable formula (A) in formula (I-C), an arylamine group, is represented by formula (a) or (a1) to (a141) as those described in embodiment of [1-18].

In formula (I-C), formula(B): 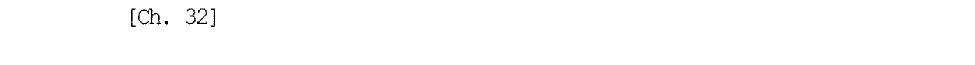 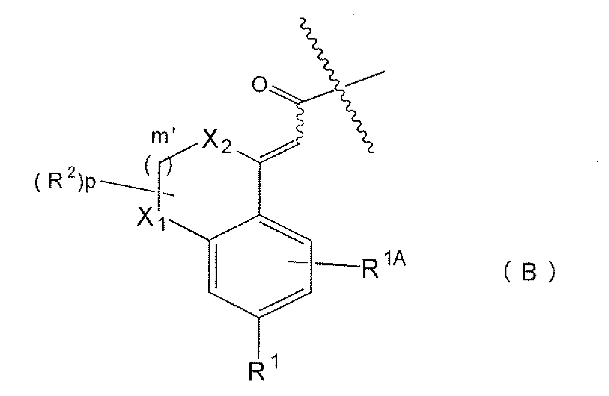 (wherein, definitions of R1A, m', R1, R2, X1, and X2 are the same as those described above), further preferable examples of each substituents are the same as those described previously in embodiment of [1-1] to [1-9], more specifically, formula (b1) to (b18) described below.

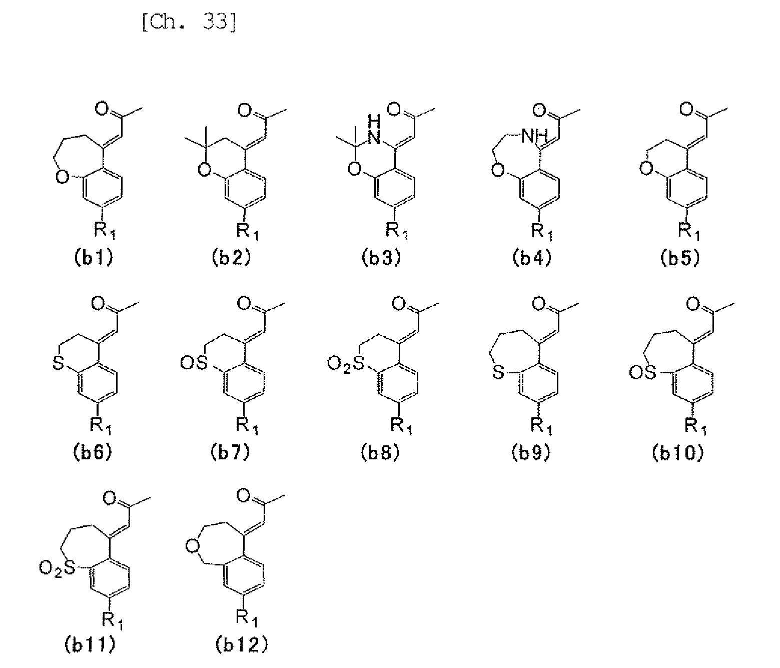

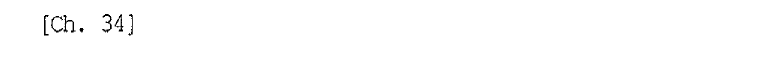

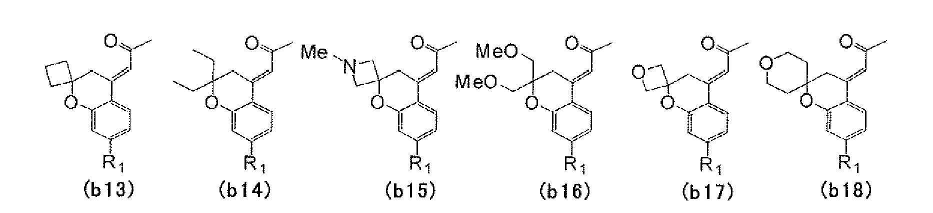

[2] A second embodiment of the present invention provides a pharmaceutical composition comprising the compounds represented by formula (I), pharmaceutically acceptable salts thereof, or solvates thereof as an active ingredient.

### More specifically, the following embodiments are preferred.

[2-1] An embodiment 2-1 of the present invention provides a pharmaceutical composition comprising at least one of the compounds represented by formula (I-A), pharmaceutically acceptable salts thereof, and solvates thereof as an active ingredient.

[2-2] An embodiment 2-2 of the present invention provides a pharmaceutical composition comprising at least one of the compounds represented by formula (I-B), pharmaceutically acceptable salts thereof, and solvates thereof as an active ingredient.

[2-3] An embodiment 2-3 of the present invention provides a pharmaceutical composition comprising at least one of the compounds represented by formula (I-C), pharmaceutically acceptable salts thereof, and solvates thereof as an active ingredient.

[2-4] An embodiment 2-4 of the present invention provides a pharmaceutical composition comprising at least one of the compounds described as the preferable compounds in embodiment [1-21], pharmaceutically acceptable salts thereof, and solvates thereof as an active ingredient.

[3] A third embodiment of the present invention provides a pharmaceutical composition comprising the compounds represented by formula (I), pharmaceutically acceptable salts thereof, or solvates thereof as TRPV1 receptor antagonists.

### More specifically, the following embodiments are preferred.

[3-1] An embodiment 3-1 of the present invention provides a pharmaceutical composition comprising at least one of the compounds represented by formula (I-A), pharmaceutically acceptable salts thereof, or solvates thereof as TRPV1 receptor antagonists.

[3-2] An embodiment 3-2 of the present invention provides a pharmaceutical composition comprising at least one of the compounds represented by formula (I-B), pharmaceutically acceptable salts thereof, and solvates thereof as TRPV1 receptor antagonists.

[3-3] An embodiment 3-3 of the present invention provides a pharmaceutical composition comprising at least one of the compounds represented by formula (I-C), pharmaceutically acceptable salts thereof, and solvates thereof as TRPV1 receptor antagonists.

[3-4] An embodiment 3-4 of the present invention provides an agent for preventing or treating pain comprising at least one of the compounds described as the preferable compounds in embodiment [1-21], pharmaceutically acceptable salts thereof, and solvates thereof as TRPV1 receptor antagonists.

In this description, in particular, in the third embodiment of the present invention, the "TRPV1 receptor antagonist," is an embodiment of a "TRPV1 receptor modulator". The term "TRPV1 receptor modulator" means an agent comprising a compound that modulates the function of the TRPV1 receptor. More specifically, the term "TRPV1 receptor modulator" means an agent comprising a compound that suppresses activation of the TRPV1 receptor. The compound may be a compound (TRPV1 receptor antagonist) that binds to the TRPV1 receptor and that antagonizes an endogenous ligand, thereby suppressing activation of the TRPV1 receptor, or a compound (TRPV1 receptor agonist) that continuously activates the TRPV1 receptor and that desensitizes nerves in which the receptor is present, thereby suppressing activation of the receptor thereafter. Accordingly, the term "TRPV1 receptor modulator" is a generic name for the TRPV1 receptor antagonists and the TRPV1 receptor agonists. Antagonists include neutral antagonists and inverse agonists, and agonists include full agonists and partial agonists. Partial agonists show the action of antagonists in some conditions.

The TRPV1 receptor modulator of the present invention is preferably a TRPV1 receptor antagonist. The TRPV1 antagonists of the present invention include neutral antagonists, inverse agonists and partial agonist. It is expected that the TRPV1 antagonist of the present invention has a promising effect of preventing or trating various diseases and conditions. Examples thereof include acute pain; chronic pain; neuropathic pain; postherpetic neuralgia; trigeminal neuralgia; lower-back pain; pain after spinal cord injury; leg pain; causalgia; diabetic neuralgia; pain caused by edema, burns, sprains, bone fractures, and the like; pain after surgical operations; scapulohumeral periarthritis; osteoarthritis; arthritis; rheumatic arthritis pain; inflammatory pain; cancer pain; migraines; headaches; toothaches; neuralgia; muscle pain; hyperalgesia; pain caused by angina pectoris, menstruation, and the like; neuropathy; nerve damage; neurodegeneration; chronic obstructive pulmonary disease (COPD); asthma; airway hypersensitivity; stridor; cough; rhinitis; inflammation of mucosa such as eyes; nervous dermatitis; inflammatory skin complaint such as psoriasis and eczema; edema; allergic diseases; gastroduodenal ulcer; ulcerative colitis; irritable colon syndrome; Crohn disease; urinary incontinence; urge urinary incontinence; overactive bladder; cystitis; nephritis; pancreatitis; uveitis; splanchnopathy; ischemia; apoplexy; dystonia; obesity; sepsis; pruritus; and diabetes. In particular, a promising effect for neuropathic pain, inflammatory pain, and urinary incontinence can be expected.

[4] A fourth embodiment of the present invention provides an agent for preventing or treating pain comprising at least one of the compounds represented by formula (I), pharmaceutically acceptable salts thereof, and solvates thereof as an active ingredient.

### More specifically, the following embodiments are preferred.

[4-1] An embodiment 4-1 of the present invention provides an agent for preventing or treating pain comprising at least one of the compounds represented by formula (I-A), pharmaceutically acceptable salts thereof, and solvates thereof as an active ingredient.

[4-2] An embodiment 4-2 of the present invention provides an agent for preventing or treating pain comprising at least one of the compounds represented by formula (I-B), pharmaceutically acceptable salts thereof, and solvates thereof as an active ingredient.

[4-3] An embodiment 4-3 of the present invention provides an agent for preventing or treating pain comprising at least one of the compounds represented by formula (I-C), pharmaceutically acceptable salts thereof, and solvates thereof as an active ingredient.

[4-4] An embodiment 4-4 of the present invention provides an agent for preventing or treating pain comprising at least one of the compounds described as the preferable compounds in embodiment [1-21], pharmaceutically acceptable salts thereof, and solvates thereof as an active ingredient.

[5] A fifth embodiment of the present invention provides an agent for preventing or treating neuropathic pain comprising at least one of the compounds represented by formula (I), pharmaceutically acceptable salts thereof, and solvates thereof as an active ingredient. More specifically, the following embodiments are preferred.

[5-1] An embodiment 5-1 of the present invention provides an agent for preventing or treating neuropathic pain comprising at least one of the compounds represented by formula (I-A), pharmaceutically acceptable salts thereof, and solvates thereof as an active ingredient.

[5-2] An embodiment 5-2 of the present invention provides an agent for preventing or treating neuropathic pain comprising at least one of the compounds represented by formula (I-B), pharmaceutically acceptable salts thereof, and solvates thereof as an active ingredient.

[5-3] An embodiment 5-3 of the present invention provides an agent for preventing or treating neuropathic pain comprising at least one of the compounds represented by formula (I-C), pharmaceutically acceptable salts thereof, and solvates thereof as an active ingredient.

[5-4] An embodiment 5-4 of the present invention provides an agent for preventing or treating neuropathic pain comprising at least one of the compounds described as the preferable compounds in embodiment [1-21], pharmaceutically acceptable salts thereof, and solvates thereof as an active ingredient.

[6] A sixth embodiment of the present invention provides an agent for preventing or treating inflammatory pain comprising at least one of the compounds represented by formula (I), pharmaceutically acceptable salts thereof, and solvates thereof as an active ingredient. More specifically, the following embodiments are preferred.

[6-1] An embodiment 6-1 of the present invention provides an agent for preventing or treating inflammatory pain comprising at least one of the compounds represented by formula (I-A), pharmaceutically acceptable salts thereof, and solvates thereof as an active ingredient.

[6-2] An embodiment 6-2 of the present invention provides an agent for preventing or treating inflammatory pain comprising at least one of the compounds represented by formula (I-B), pharmaceutically acceptable salts thereof, and solvates thereof as an active ingredient.

[6-3] An embodiment 6-3 of the present invention provides an agent for preventing or treating inflammatory pain comprising at least one of the compounds represented by formula (I-C), pharmaceutically acceptable salts thereof, and solvates thereof as an active ingredient.

[6-4] An embodiment 6-4 of the present invention provides an agent for preventing or treating inflammatory pain comprising at least one of the compounds described as the preferable compounds in embodiment [1-21], pharmaceutically acceptable salts thereof, and solvates thereof as an active ingredient.

In any one of the second embodiment to the sixth embodiment, and preferable embodiments thereof, in the compounds represented by formulae (I), (I-A), (I-B), and (I-C), preferable substituents and combinations thereof are described in the first embodiment.

[7] A seventh embodiment of the present invention provides a compound which is obtainable by the processes and identified with at least one of the analytical data of each example disclosed as EXAMPLE 1 through EXAMPLE 301, a salt thereof, and solvates thereof.
The analytical data are listed in Table 11-13(LC-MS) and Table 14-16(NMR) for final compounds or in Table 17-18(NMR) for intermediates. The analytical date is preferably NMR.

[7-1] An embodiment 7-1 of the present invention provides a compound which is obtainable by the processes and identified with at least one of the analytical data of each example disclosed as EXAMPLE 30, 31, 32, 33, 34, 35, 42, 43, 44, 45, 46, 47, 48, 49, 50, 51, 52, 53, 54, 55, 56, 57 and 58, a salt thereof, and solvates thereof. The analytical date is preferably NMR.

[7-2] An embodiment 7-2 of the present invention provides a pharmaceutical composition comprising at least one of the compounds of the embodiment 7, pharmaceutically acceptable salts thereof and solvates thereof as an active ingredient.

[7-3] An embodiment 7-3 of the present invention provides an agent for preventing or treating pain comprising at least one of the compounds of the embodiment 7, pharmaceutically acceptable salts thereof and solvates thereof as an active ingredient.

In the embodiments described in [1] to [7] of the present invention, compounds having TRPV1 receptor antagonistic activity (determined by, for example, experimental example (2) described below: a measurement of Ca-influx using FDSS-6000) of 1 µM or less, preferably 100 nM or less, and more preferably 30 nM or less in terms of an A2 value are preferably used.

In the embodiments described above, "agent" means improvement of disease or symptom, not only treatment of disease or symptom.

In all the above embodiments, when the term "compound" is used, the term also refers to pharmaceutically acceptable salts thereof. The compounds of the present invention may have an asymmetric carbon atom. Accordingly, the compounds of the present invention include mixtures of various stereoisomers, such as geometrical isomers, tautomers, and optical isomers, and isolated isomers. The isolation and the purification of such stereoisomers can be performed by those skilled in the art with a known technique such as optical resolution using preferential crystallization or column chromatography, or asymmetric synthesis.

The compounds represented by formulae (I), (I-A), (I-B), and (I-C) of the present invention may form acid addition salts. Alternatively, these compounds may form salts with a base according to the type of substituent. These salts are not particularly limited as long as the salts are pharmaceutically acceptable salts. Specific examples of the salts include acid addition salts with a mineral acid such as hydrochloric acid, hydrobromic acid, hydroiodic acid, sulfuric acid, nitric acid, or phosphoric acid; an organic carboxylic acid such as an aliphatic monocarboxylic acid, e.g., formic acid, acetic acid, propionic acid, butyric acid, valeric acid, enanthic acid, capric acid, myristic acid, palmitic acid, stearic acid, lactic acid, sorbic acid, or mandelic acid, an aromatic monocarboxylic acid, e.g., benzoic acid or salicylic acid, an aliphatic dicarboxylic acid, e.g., oxalic acid, malonic acid, succinic acid, fumaric acid, maleic acid, malic acid, or tartaric acid, and an aliphatic tricarboxylic acid e.g., citric acid; an organic sulfonic acid such as an aliphatic sulfonic acid, e.g., methanesulfonic acid, ethanesulfonic acid, or 2-hydroxyethanesulionic acid, or an aromatic sulfonic acid, e.g., benzenesulfonic acid or p-toluenesulfonic acid; or an acidic amino acid, e.g., aspartic acid or glutamic acid; salts with a metal such as an alkali metal, e.g., sodium or potassium, or an alkaline earth metal, e.g., magnesium or calcium; salts with an organic base such as methylamine, ethylamine, ethanolamine, pyridine, lysine, arginine, or ornithine; and ammonium salts.

These salts can be obtained by a known method, for example, by mixing a compound of the present invention with an equivalent amount and a solution containing a desired acid, base, or the like, and then collecting the desired salt by filtering the salt or distilling off the solvent. The compounds of the present invention and salts thereof can form solvates with a solvent such as water, ethanol, or glycerol.

The salts of a compound of the present invention include monosalts and di-salts. The compounds of the present invention can form an acid addition salt and a salt with a base at the same time according to the type of substituent of the side chain.

Furthermore, the present invention includes hydrates, pharmaceutically acceptable various solvates, and crystal polymorphism of the compounds represented by formulae (I), (I-A), (I-B), and (I-C) of the present invention. The present invention is not Limited to the compounds described in examples below and includes all compounds represented by formulae (I), (I-A), (I-B), and (I-C) of the present invention and pharmaceutically acceptable salts thereof.

[Process of producing compound of the present invention] Compounds represented by formulae (I), (I-A), (I-B), (I-C), (I'), (I"), (I"'), (I""), (II), (IV), (V), (V-a), (V-a-1), (V-a-2), (V-b), (VI), (VI-a), or (VIII) which is used in the present invention, and related compounds can be obtained by production processes described below. Each of reaction steps will now be described.

Unless otherwise stated, the reaction conditions employed in the production processes are as described below. The reaction temperature is in the range of -78°C to the solvent-reflux temperature, and the reaction time is the time sufficient for required progress of the reaction. Examples of solvents which are inactive to the reaction include aromatic hydrocarbon solvents such as toluene, xylene, and benzene; polar solvents such as alcohols, e.g., methanol and ethanol, N,N-dimethylformamide, dimethyl sulfoxide, acetonitrile, and water; basic solvents such as triethylamine and pyridine; organic acidic solvents such as acetic acid; halogenated solvents such as chloroform, dichloromethane, and 1,2-dichloroethane; ethereal solvents such as diethyl ether, tetrahydrofuran, dioxane, and dimethoxyethane; and mixed solvents thereof, and the solvent used may be adequately selected according to the reaction conditions. Examples of bases include inorganic bases such as potassium carbonate, sodium carbonate, cesium carbonate, sodium hydroxide, potassium hydroxide, sodium hydride, and sodium hydrogencarbonate; and organic bases such as triethylamine, diethylamine, pyridine, N,N-dialkylanilines, lithium diisopropylamide, and lithium bis(trimethylsilyl)amide. Examples of acids include inorganic acids such as hydrochloric acid and sulfuric acid; and organic acids such as acetic acid, trifluoroacetic acid, methanesulfonic acid, and p-toluenesulfonic acid. The solvents, the bases, and the acids are not necessarily limited to those mentioned above.

The compounds represented by formula (I) and salts thereof, which are the compounds of the present invention can be readily produced from known compounds or commercially available compounds by, for example, known processes described in published documents, and produced by production processes described below.

The present invention is not limited to the production processes described below.

The production processes will now be described in detail.

In the description below, unless otherwise stated, the definitions of R¹, R², R³, R⁷, R⁸, R^{9A}, R^{9B}, R¹⁰, X₁, X₂, X₁', m, m', n, p, q, k, j, L₁, L₂, W and cycle in formulae of the compound represented by formula (I), (I'), (I"), (I"'), (I""), (II), (IV), (V), (V-a), (V-a-1), (V-a-2), (V-b), (VI), (VI-a), or (VIII)is the same as those in formula (I). R⁴ represents a hydrogen atom or a alkyl group; R⁵ represents an alkyl group, R⁶ represents a protective group such as an arylsulfonyl group, an acyl group, a carbamoyl group (for example, a tert-butoxycarbonyl group or a benzyloxycarbonyl group), or a p-toluenesulfonyl group; R^{10'} represents the same substituents as R¹, "a group : -NR¹¹R¹¹ "represents a nitrogen containing group defined into R^{9A} or R^{9B}, formed a linear or branched chain, or cyclic ring. R¹² represents an alkyl group. R¹³ represents a NO₂ or NHCOOR⁵, Y and Z each represent a leaving group such as halogen; Y and Z each represent a leaving group such as halogen; and M represents a metal such as Li, Na, or K; r represents an integral number 1 or 2.

A compound represented by formula (I) can be obtained by a condensation reaction of a carboxylic acid represented by formula (VIII) and an arylamine represented by formula (A-H) which described (A) in [1-18] above-mentioned. 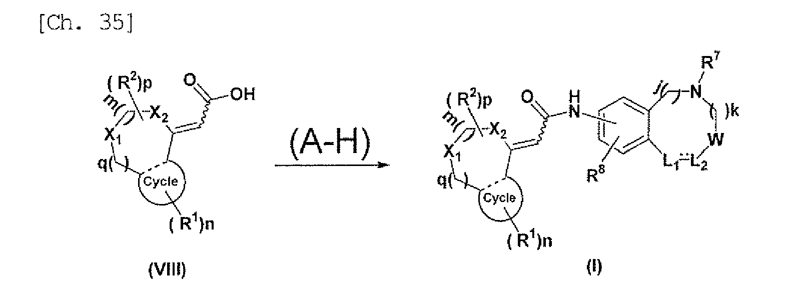 And, formula (A-H) represents Q-NH2 (= formula (IX)) in reaction scheme and production processes described below.

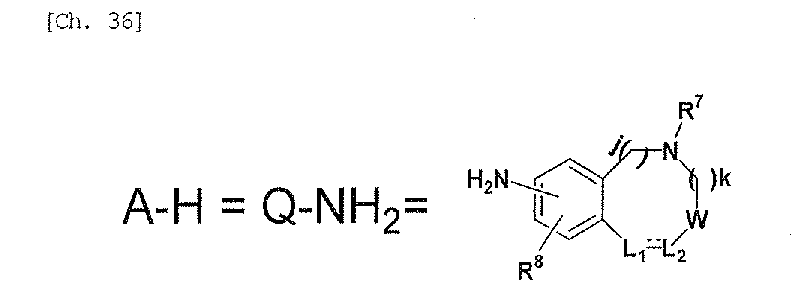

### (Reaction scheme)

### <The case where q is 0 and X₂ is CH₂, and X₁' is O, N-R³, or S.>

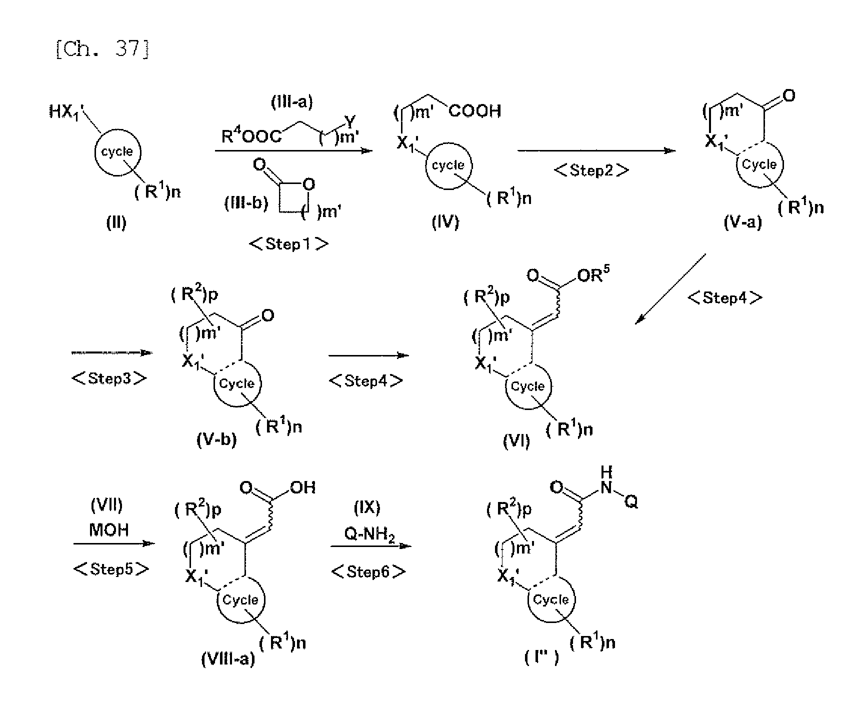

### (Reaction scheme) <Step 1>

When R⁴ is H (a hydrogen atom), a compound represented by formula (IV) can be produced by allowing a compound represented by formula (II) to react with a compound represented by formula (ITI-a) by a process similar to that described in published documents, for example, Journal of Medicinal Chemistry, 31(1), pp. 230-243, 1988, in the presence of a base such as sodium hydride, lithium hydroxide, sodium hydroxide, potassium hydroxide, lithium carbonate, sodium carbonate, or potassium carbonate using a solvent which is inactive to the reaction, such as methanol, ethanol, acetone, N,N-dimethylformamide, dioxane, tetrahydrofuran, or water, or a mixed solvent thereof at a temperature in the range of room temperature to the solvent-reflux temperature.

Alternatively, the compound represented by formula (IV) can be produced by conducting a reaction using a compound represented by formula (III-b) by a process similar to that described in published documents, for example, PCT Publication No. 01/036381 pamphlet, pp. 360-361, in the presence of a base such as sodium hydride, lithium hydroxide, sodium hydroxide, potassium hydroxide, lithium carbonate, sodium carbonate, or potassium carbonate with a solvent which is inactive to the reaction, such as methanol, ethanol, acetone, N,N-dimethylformamide, dioxane, tetrahydrofuran, or water, or a mixed solvent thereof at a temperature in the range of room temperature to the solvent-reflux temperature.

When R⁴ is an alkyl group (e.g., a methyl group or an ethyl group), the compound represented by formula (IV) can be produced from an ester, produced by the same reaction as that conducted in the case where R⁴ is H, by a process similar to that described in published documents, for example, Jikken Kagaku Koza (Experimental Chemistry Series), 4th edition, 22, Organic synthesis IV, Acids, amino acids, and peptides, pp. 1-43, 1992, Maruzen Co., Ltd., in the presence of a base such as lithium hydroxide, sodium hydroxide, potassium hydroxide, lithium carbonate, sodium carbonate, or potassium carbonate using water and a solvent which is inactive to the reaction, such as methanol, ethanol, 2-propanol, N,N-dimethylformamide, dioxane, or tetrahydrofuran, or a mixed solvent thereof at a temperature in the range of 0°C to the solvent-reflux temperature.

### (Reaction scheme) <Step 2>

A compound represented by formula (V-a) can be produced by conducting a reaction using the compound represented by formula (IV) by a process similar to that described in published documents, for example, Journal of Medicinal Chemistry, 31(1), pp. 230-243, 1988, in a cyclization-dehydrating agent such as polyphosphoric acid (PPA), polyphosphoric acid ethyl ester (PPE), diphosphorus pentaoxide (P₂O₅), or Eaton's reagent (a mixture of methanesulfonic acid and phosphorus pentoxide) or, and in a solvent which is inactive to the reaction, such as a halogenated solvent, e.g., dichloromethane or chloroform, an ethereal solvent, e.g., diethyl ether or tetrahydrofuran, or an aromatic hydrocarbon solvent, e.g., toluene or benzene in the presence of a cyclization-dehydrating agent described above at a temperature in the range of 0°C to the solvent-reflux temperature. Alternatively, the compound represented by formula (V-a) can be similarly produced by conducting the reaction in the presence of a Lewis acid such as aluminum trichloride or tin tetrachloride in a solvent which is inactive to the reaction, such as a halogenated solvent, e.g., dichloromethane or chloroform at a temperature in the range of 0°C to the solvent-reflux temperature.

### (Reaction scheme) <Step 3>

A compound represented by formula (V-b) (wherein p represents 1 or 2) can be produced as follows. When R² is a halogen atom, for example, a fluorine atom (F), the compound represented by formula (V-a) is converted to a trimethylsilyl enol ether by a process similar to that described in published documents, for example, Tetrahedron Letters, 25(51), pp. 5953-5956, 1984. The resulting compound is then treated by a process similar to that described in published documents, for example, Organic Letters, 1(10), pp. 1591-1594, 1998, in the presence of a fluorinating reagent such as xenon difluoride (XeF₂), fluorine (F₂), 1-fluoro-4-methyl-1,4-diazabicyclo[2, 2, 2]octane trifluoromethanesulfonate, N-fluoro-O-benzenesulfonimide, N-fluorobenzenesulfonimide, hypofluorous acid trifluoromethyl ether, or 1-fluoropyridine trifluoromethanesulfonate in a solvent which is inactive to the reaction, such as a halogenated solvent, e.g., dichloromethane or chloroform, an ethereal solvent, e.g., diethyl ether or tetrahydrofuran, or an aromatic hydrocarbon solvent, e.g., toluene or benzene at a temperature in the range of -78°C to the solvent-reflux temperature, thereby producing the compound represented by formula (V-b). When R² is an amino group, the above-mentioned trimethylsilyl enol ether is allowed to react with sodium azide by a process similar to that described in published documents, for example, Tetrahedron, 51(41), pp. 11075-11086, 1995, in the presence of diammonium cerium hexanitrate in a solvent which is inactive to the reaction, such as a halogenated solvent, e.g., dichloromethane or chloroform, an ethereal solvent, e.g., diethyl ether or tetrahydrofuran, a polar solvent, e.g., acetonitrile, or an aromatic hydrocarbon solvent, e.g., toluene or benzene to produce an azide compound. Subsequently, hydrogen gas is added to the azide compound by a process similar to that described in published documents, for example, Jikken Kagaku Koza (Experimental Chemistry Series), 4th edition, 26, Organic synthesis VIII, Asymmetric synthesis, reduction, sugar, and labeled compound, pp. 251-266, 1992, Maruzen Co., Ltd., in the presence of a catalyst such as palladium-carbon (Pd-C), Raney-Ni, or platinum oxide (PtO₂) in a solvent which is inactive to the reaction, such as an alcoholic solvent, e.g., methanol, ethanol, or 2-propanol, a halogenated solvent, e.g., dichloromethane or chloroform, an ethereal solvent, e.g., diethyl ether or tetrahydrofuran, a polar solvent, e.g., ethyl acetate or acetonitrile, an aromatic hydrocarbon solvent, e.g., toluene or benzene, or an acid solvent, e.g., acetic acid at a temperature in the range of room temperature to the solvent-reflux temperature, thereby producing the compound represented by formula (V-b). When R² is an hydroxy group, the above-mentioned trimethylsilyl enol ether is allowed to react with 3-chloroperbenzoic acid, aqueous hydrogen peroxide, by a process similar to that described in published documents, for example, Jikken Kagaku Koza (Experimental Chemistry Series), 4th edition, 23, Organic synthesis V, Oxidative reaction, pp. 225-298, 1992, Maruzen Co., Ltd., in a solvent which is inactive to the reaction, such as water, an alcoholic solvent, e.g., methanol, ethanol, or 2-propanol, a halogenated solvent, e.g., dichloromethane or chloroform, or an aromatic hydrocarbon solvent, e.g., toluene or benzene to produce an epoxy compound. Subsequently, the trimethylsilyl group is removed by a process described in published textbooks, for example, Greene et al., Protective Groups in Organic Synthesis, (the United States), 3rd edition, 1999, thereby producing the compound represented by formula (V-b).

### (Reaction scheme) <Step 4>

A compound represented by formula (VI) can be produced by conducting a reaction using the compound represented by formula (V-a) or (V-b) by a process similar to that described in published documents, for example, Jikken Kagaku Koza (Experimental Chemistry Series), 4th edition, 19, Organic synthesis I, Hydrocarbons and halogenated compounds, pp. 53-298, 1992, Maruzen Co., Ltd., in the presence of a Wittig reagent or a Horner-Emmons reagent, such as (ethoxycarbonylmethyl)triphenylphosphonium chloride, (ethoxycarbonylmethyl)triphenylphosphonium bromide, ethyl triphenylphosphoranylidene acetate, bis-2,2,2-trifluoroethoxy phosphinyl acetate, ethyl di-ortho-tolylphosphonoacetate, ethyl dimethylphosphonoacetate, ethyl diethylphosphonoacetate, or ethyl 1-trimethylsilyl acetate, and a base such as sodium hydride, butyllithium, piperazine, morpholine, triethylamine, lithium diisopropylamide, lithium bis (trimethylsilyl) amide, sodium bis (trimethylsilyl) amide, potassium bis(trimethylsilyl)amide, or phosphazene base-P4-tert-butyl, using a solvent which is inactive to the reaction, such as methanol, ethanol, N,N-dimethylformamide, dioxane, tetrahydrofuran, or an aromatic hydrocarbon solvent, e.g., benzene, toluene, or xylene, or a mixed solvent thereof at a temperature in the range of -78°C to the solvent-reflux temperature.

### (Reaction scheme) <Step 5>

A compound represented by formula (VIII-a) can be produced by conducting a reaction by the same process as that used in <Step 1> of (Reaction scheme) (in the case where R⁴ is an alkyl group (e.g., a methyl group or an ethyl group)) using the compound represented by formula (VI) and a compound represented by formula (VII).

### (Reaction scheme) <Step 6>

A compound represented by formula (I") can be produced by conducting a reaction using the compound represented by formula (VIII-a) and a compound represented by formula (IX) (for example, a known amine) as follows. When the compound represented by formula (VIII-a) is a carboxylic acid, the compound represented by formula (I") can be produced by allowing the compound represented by formula (VIII-a) to react with the compound represented by formula (IX) by a process similar to that described in published documents, for example, Jikken Kagaku Koza (Experimental Chemistry Series), 4th edition, 22, Organic synthesis IV, Acids, amino acids, and peptides, pp. 191-309, 1992, Maruzen Co., Ltd., in the presence of a condensing agent such as 1,3-dicyclohexylcarbodiimide (DCC), 1-ethyl-3-(3'-dimethylaminopropyl)carbodiimide hydrochloride (WSC·HCl), benzotriazol-1-yloxy tris(dimethylamino)phosphonium hexafluorophosphate (BOP reagent), bis(2-oxo-3-oxazolidinyl)phosphinic chloride (BOP-C1), 2-chloro-1,3-dimethylimidazolinium hexafluorophosphate (CIP), or 4-(4,6-dimethoxy-1,3,5-triazin-2-yl)-4-methylmorpholinium chloride, in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, a polar solvent, e.g., N,N-dimethylformamide, or an alcoholic solvent, e.g., methanol, ethanol, or 2-propanol, in the presence or absence of a base such as triethylamine or pyridine at a temperature in the range of 0°C to the solvent-reflux temperature. When the compound represented by formula (VIII-a) is converted to an acid halide, the compound represented by formula (I") can be similarly produced by conducting a reaction by a process similar to that described in, for example, Jikken Kagaku Koza (Experimental Chemistry Series), 4th edition, 22, Organic synthesis IV, Acids, amino acids, and peptides, pp. 144-146, 1992, Maruzen Co., Ltd., in the presence of a base such as triethylamine or pyridine in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, or a polar solvent, e.g., N,N-dimethylformamide at a temperature in the range of 0°C to the solvent-reflux temperature.

The compound represented by formula (V-a) or a compound represented by formula (VI-a) (a compound in which p is 0 in formula (VI)), which is an intermediate in the above reaction scheme, can also be produced by Production processes A to D described below. In the formulae, X₁' is O, N-R³, or S.

### (Production process A)

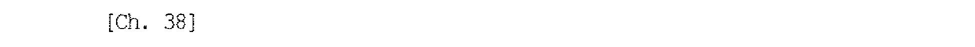

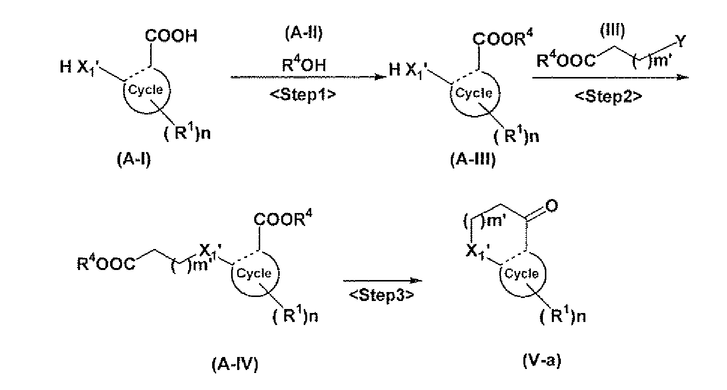

### <Step 1>

A compound represented by formula (A-III) can be produced by allowing a compound represented by formula (A-I) to react with a compound represented by formula (A-II) by a process similar to that described in published documents, for example, Jikken Kagaku Koza (Experimental Chemistry, Series), 4th edition, 22, Organic synthesis IV, Acids, amino acids, and peptides, pp. 1-82, 1992, Maruzen Co., Ltd., in the presence of an acidic reagent such as hydrochloric acid, sulfuric acid, thionyl chloride, or acetyl chloride, using a solvent such as methanol, ethanol, or 2-propanol at a temperature in the range of 0°C to the solvent-reflux temperature.

### <Step 2>

A compound represented by formula (A-IV) can be produced by the same process as that used in <Step 1> of (Reaction scheme) using the compound represented by formula (A-III) and a compound represented by formula (III).

### <Step 3>

The compound represented by formula (V-a) can be produced by conducting a reaction using the compound represented by formula (A-IV) by a process similar to that described in published documents, for example, Organic Reactions, 1, p. 274, 1942, in the presence of a basic reagent such as sodium methoxide, sodium ethoxide, potassium tert-butoxide, sodium hydride, sodium hydroxide, or potassium hydroxide with a solvent which is inactive to the reaction, such as methanol, ethanol, dimethyl sulfoxide, benzene, toluene, or xylene at a temperature in the range of 0°C to the solvent-reflux temperature, followed by a reaction in a mixed solvent containing a solvent which is inactive to the reaction, such as dimethyl sulfoxide, benzene, toluene, or xylene, and water or an acidic aqueous solution such as an aqueous hydrochloric acid solution or an aqueous acetic acid solution at a temperature in the range of room temperature to the solvent-reflux temperature.

### (Production process B)

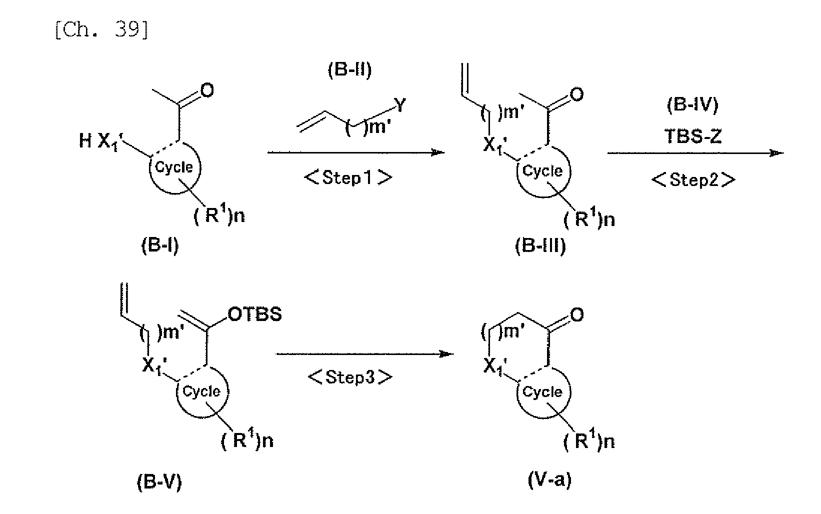

### <Step 1>

A compound represented by formula (B-III) can be produced by the same process as that used in <Step 1> of (Reaction scheme) using a compound represented by formula (B-I) and a compound represented by formula (B-II).

### <Step 2>

A compound represented by formula (B-V) can be produced by allowing the compound represented by formula (B-III) to react with a compound represented by formula (B-IV) by a process similar to that described in published documents, for example, Tetrahedron Letters, 25(51), pp. 5953-5956, 1984, in the presence of a silylation agent such as tert-butyldimethylsilyl chloride (TBSCl) or tert-butyldimethylsilyl trifluoromethanesulfonate (TBSOTf) and a base such as sodium hydride, piperazine, morpholine, triethylamine, lithium diisopropylamide, lithium bis(trimethylsilyl)amide, sodium bis(trimethylsilyl)amide, or potassium bis(trimethylsilyl)amide using a solvent which is inactive to the reaction, such as a halogen-containing solvent, e.g., methylene chloride or chloroform, an ethereal solvent, e.g., dioxane or tetrahydrofuran, or an aromatic hydrocarbon solvent, e.g., benzene, toluene, or xylene, or a mixed solvent thereof at a temperature in the range of -78°C to the solvent-reflux temperature.

### <Step 3>

The compound represented by formula (V-a) can be produced by conducting a reaction using the compound represented by formula (B-V) by a process similar to that described in published documents, for example, Tetrahedron, 60(13), pp. 3017-3035, 2004, in the presence of a ruthenium catalyst such as benzylidene bistricyclohexylphosphineruthenium dichloride, tricyclohexylphosphine-1,3-bis-2,4,6-trimethylphenyl-4,5-dihydroimidazol-2-ylidene benzylideneruthenium dichloride, or ruthenium-1,3-bis-2,4,6-trimethylphenyl-2-imidazolidinylylidenedichloro-2-1-methylethoxy phenyl methylene with a solvent which is inactive to the reaction, such as a halogenated solvent, e.g., dichloromethane or chloroform, an ethereal solvent, e.g., dioxane or tetrahydrofuran, or an aromatic hydrocarbon solvent, e.g., benzene, toluene, or xylene, or a mixed solvent thereof at a temperature in the range of room temperature to the solvent-reflux temperature.

### (Production process C)

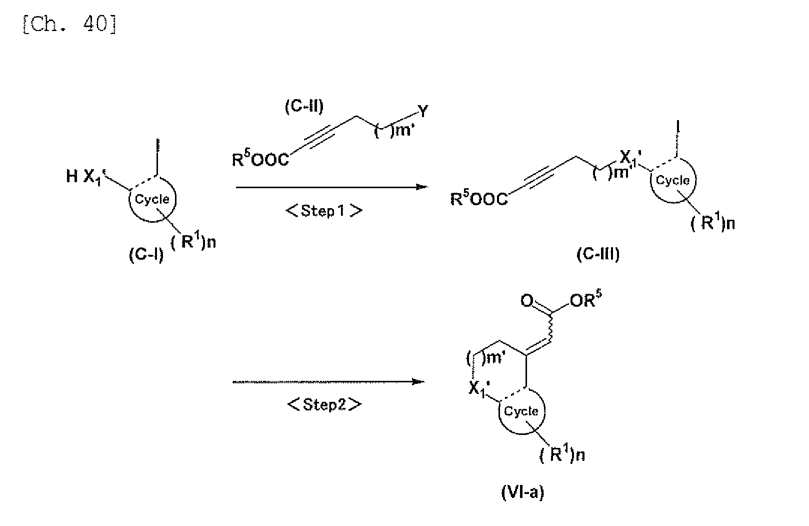

### <Step 1>

A compound represented by formula (C-III) can be produced by the same process as that used in <Step 1> of (Reaction scheme) using a compound represented by formula (C-I) and a compound represented by formula (C-II).

### <Step 2>

A compound represented by formula (VI-a) (a compound in which p is 0 in formula (VI)) can be produced by conducting a reaction using the compound represented by formula (C-III) by a process similar to that described in published documents, for example, Tetrahedron Letters, 28(44), pp. 5291-5294, 1987, in the presence of a palladium catalyst such as palladium diacetate, tetrakis triphenylphosphine palladium, or tris dibenzylideneacetone dipalladium with a solvent which is inactive to the reaction, such as acetonitrile, dioxane, tetrahydrofuran, benzene, toluene, dimethyl sulfoxide, or N,N-dimethylformamide, or a mixed solvent thereof at a temperature in the range of room temperature to the solvent-reflux temperature.

### (Production process D)

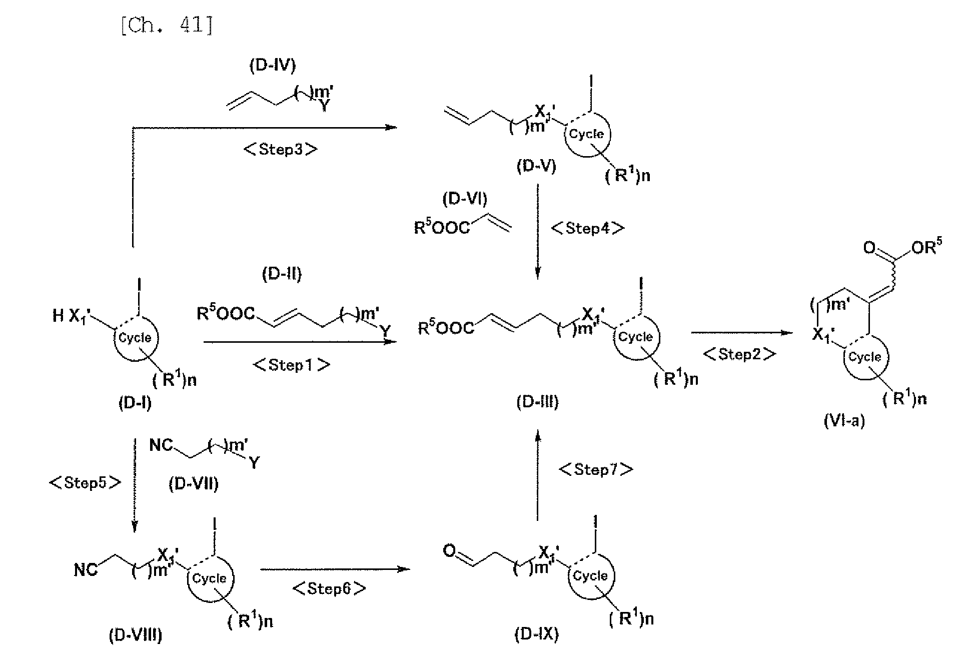

### <Step 1>

A compound represented by formula (D-III) can be produced by the same process as that used in <Step 1> of (Reaction scheme) using a compound represented by formula (D-I) and a compound represented by formula (D-II).

### <Step 2>

The compound represented by formula (VI-a) (the compound in which p is 0 in formula (VI)) can be produced by conducting a reaction using the compound represented by formula (D-III) by a process similar to that described in published documents, for example, Synlett, No. 6, pp. 848-850, 2001, in the presence of a palladium catalyst such as palladium diacetate, tetrakis triphenylphosphine palladium, or tris dibenzylideneacetone dipalladium, and a base such as silver carbonate with a solvent which is inactive to the reaction, such as acetonitrile, dioxane, tetrahydrofuran, benzene, toluene, dimethyl sulfoxide, or N,N-dimethylformamide, or a mixed solvent thereof at a temperature in the range of room temperature to the solvent-reflux temperature.

Alternatively, the compound represented by formula (D-III), which is an intermediate, can be produced by the following process.

### <Step 3>

A compound represented by formula (D-V) can be produced by the same process as that used in <Step 1> of (Reaction scheme) using the compound represented by formula (D-I) and a compound represented by formula (D-IV).

### <Step 4>

The compound represented by formula (D-III) can be produced by the same process as that used in <Step 3> of (Production process B) using the compound represented by formula (D-V) and a compound represented by formula (D-VI).

### <Step 5>

A compound represented by formula (D-VIII) can be produced by the same process as that used in <Step 1> of (Reaction scheme) using the compound represented by formula (D-I) and a compound represented by formula (D-VII).

### <Step 6>

A compound represented by formula (D-IX) can be produced by conducting a reaction using the compound represented by formula (D-VIII) by a process similar to that described in published documents, for example, Jikken Kagaku Koza (Experimental Chemistry Series), 4th edition, 26, Organic synthesis VIII, Asymmetric synthesis, reduction, sugar, and labeled compound, pp. 159-266, 1992, Maruzen Co., Ltd., in the presence of a reducing agent such as diisobutylaluminum hydride (DIBAH), lithium triethoxyaluminum hydride, sodium bis-2-methoxyethoxy aluminum hydride, or Raney-Ni-formic acid, with a solvent which is inactive to the reaction, such as diethyl ether, 1,2-dimethoxyethane, dioxane, tetrahydrofuran, benzene, or toluene, or a mixed solvent thereof at a temperature in the range of -78°C to the solvent-reflux temperature.

### <Step 7>

The compound represented by formula (D-III) can be produced by the same process as that used in <Step 4> of (Reaction scheme) using the compound represented by formula (D-IX).

A compound represented by formula (V-a-1), in which m' is 1 and X₁' is NH in the compound represented by formula (V-a), or a compound represented by formula (V-a-2), in which m' is 1 and X₁' is N-R³' (wherein R³' is a substituted or unsubstituted hydrocarbon group, a substituted or unsubstituted heterocyclic group, or a substituted or unsubstituted acyl group which is defined in R³) in the compound represented by formula (V-a) can also be produced by Production process E below.

### (Production Process E)

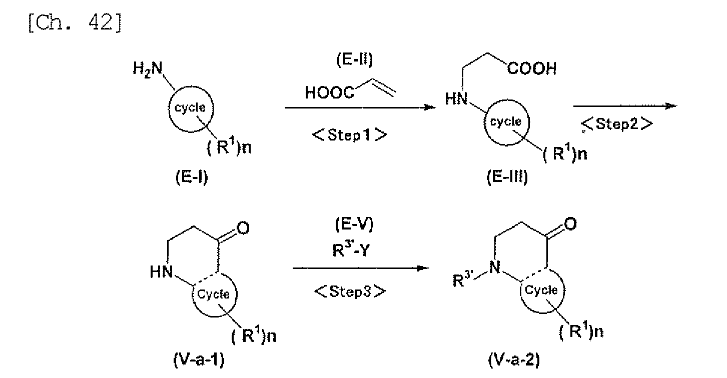

### <Step 1>

A compound represented by formula (E-III) can be produced by allowing a compound represented by formula (E-I) to react with a compound represented by formula (E-II) by a process similar to that described in published documents, for example, Jikken Kagaku Koza (Experimental Chemistry Series), 4th edition, 20, Organic synthesis II, Alcohols and amines, pp. 280-372, 1992, Maruzen Co., Ltd., using a solvent which is inactive to the reaction, such as acetonitrile, dioxane, tetrahydrofuran, benzene, toluene, dimethyl sulfoxide, N,N-dimethylformamide, or water, or a mixed solvent thereof at a temperature in the range of room temperature to the solvent-reflux temperature.

### <Step 2>

The compound represented by formula (V-a-1) (the compound in which X₁' is N-R³, R³ is H, and m' is 1 in the compound represented by formula (V-a)) can be produced by the same process as that used in <Step 2> of (Reaction scheme) using the compound represented by formula (E-III).

### <Step 3>

The compound represented by formula (V-a-2) (compound in which X₁' is N-R³', R³' is a substituted or unsubstituted hydrocarbon group, a substituted or unsubstituted heterocyclic group, or a substituted or unsubstituted acyl group which is defined in R³, and m' is 1 in the compound represented by formula (V-a)) can be produced using the compound represented by formula (V-a-1) and a compound represented by formula (E-V) (for example, a desired alkyl halide, acyl halide, aryl halide, or heteroaryl halide, wherein R³' is a substituted or unsubstituted hydrocarbon group, a substituted or unsubstituted heterocyclic group, or a substituted or unsubstituted acyl group which is defined in R³). For example, when R³' is alkyl, the compound represented by formula (V-a-2) can be produced by conducting a reaction by a process similar to that described in published documents, for example, Jikken Kagaku Koza (Experimental Chemistry Series), 4th edition, 20, Organic synthesis II, Alcohols and amines, pp. 280-372, 1992, Maruzen Co., Ltd., using a solvent which is inactive to the reaction, such as acetonitrile, dioxane, tetrahydrofuran, benzene, toluene, dimethyl sulfoxide, or N,N-dimethylformamide, or a mixed solvent thereof at a temperature in the range of room temperature to the solvent-reflux temperature. When R³' is acyl, the compound represented by formula (V-a-2) can be produced by the same process as that used in <Step 6> of (Reaction scheme). When R³' is aryl or a heterocycle, the compound represented by formula (V-a-2) can be produced by conducting a reaction by a process similar to that described in published documents, for example, Jikken Kagaku Koza (Experimental Chemistry Series), 4th edition, 20, Organic synthesis II, Alcohols and amines, pp. 187-243, 1992, Maruzen Co., Ltd., using a solvent which is inactive to the reaction, such as acetonitrile, dioxane, tetrahydrofuran, benzene, toluene, dimethyl sulfoxide, or N,N-dimethylformamide, or a mixed solvent thereof at a temperature in the range of room temperature to the solvent-reflux temperature.

In the above reaction scheme, the compound represented by formula (VIII-a) can also be produced from a compound represented by formula (V) (including the compounds represented by formulae (V-a) and (V-b) in the reaction scheme) by Production process F below.

### (Production process F)

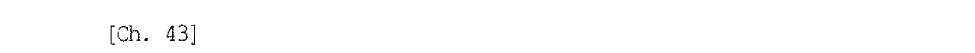

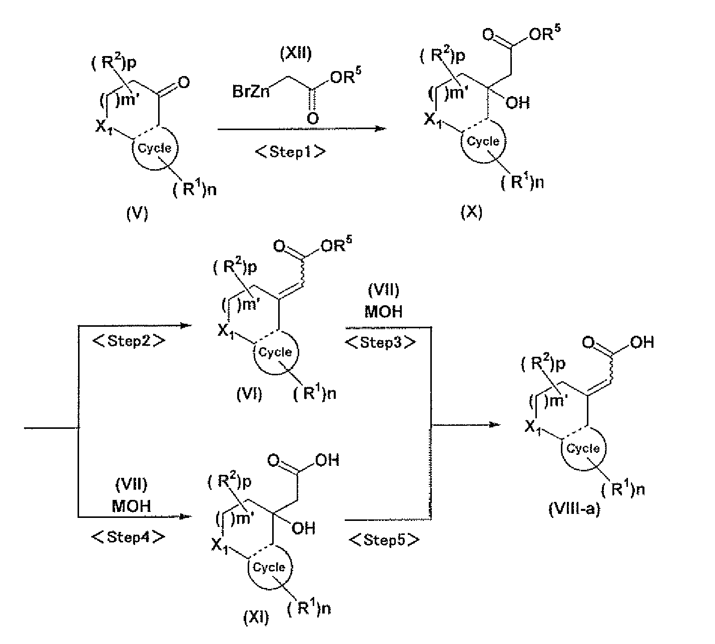

### <Step 1>

A compound represented by formula (X) can be produced by a process similar to that described in published documents, for example, Jikken Kagaku Koza (Experimental Chemistry Series), 4th edition, 20, Organic synthesis II, Alcohols, pp. 82-94, 1992, Maruzen Co., Ltd., by allowing the compound represented by formula (Vb) to react with a Reformatsky reagent (a compound represented by formula (XII)), which is prepared from an α-haloacetate such as ethyl bromoacetate or tert-butyl bromoacetate in the presence of zinc, or by allowing the compound represented by formula (V) to react with a silyl acetate such as ethyl (trimethylsilyl)acetate in the presence of a base such as phosphazene base-P4-tert-butyl using a solvent which is inactive to the reaction, such as an ethereal solvent, e.g., dioxane or tetrahydrofuran, or an aromatic hydrocarbon solvent, e.g., benzene, toluene, or xylene, or a mixed solvent thereof at a temperature in the range of -78°C to the solvent-reflux temperature.

### <Step 2>

The compound represented by formula (VI) can be produced by performing a reaction using the compound represented by formula (X) by a process similar to that described in published documents, for example, Jikken Kagaku Koza (Experimental Chemistry Series), 4th edition, 19, Organic synthesis I, Hydrocarbons, pp. 194-236, 1992, Maruzen Co., Ltd., in the presence of a dehydrating agent such as potassium hydrogensulfate; an inorganic acid, e.g., concentrated sulfuric acid; an organic acid, e.g., p-toluenesulfonic acid, methanesulfonic acid, or trifluoroacetic acid; thionyl chloride; or phosphorus oxychloride using a solvent which is inactive to the reaction, such as an ethereal solvent, e.g., dioxane or tetrahydrofuran, or an aromatic hydrocarbon solvent, e.g., benzene, toluene, or xylene, or a mixed solvent thereof at a temperature in the range of -78°C to the solvent-reflux temperature.

### <Step 3>

The compound represented by formula (VIII-a) can be produced by conducting a reaction by the same process as that used in <Step 5> of (Reaction scheme) (in the case where R⁵ is an alkyl group (e.g., a methyl group or an ethyl group)) using the compound represented by formula (VI) and the compound represented by formula (VII). When R⁵ is a tert-butyl group, the compound represented by formula (VIII-a) can be produced by conducting a reaction using an acid such as hydrochloric acid or trifluoroacetic acid.

### <Step 4>

A compound represented by formula (XI) can be produced by conducting a reaction by the same process as that used in <Step 5> of (Reaction scheme) using the compound represented by formula (X) and the compound represented by formula (VII).

### <Step 5>

The compound represented by formula (VIII-a) can be produced by conducting a reaction by the same process as that used in <Step 2> of (Production process F) using the compound represented by formula (XI).

A compound represented by formula (I)-e-1, in which X₁' is N-R³, R³ is H, p is 0 and m' is 1 in the compound represented by formula (I") in the reaction scheme, and a compound represented by formula (I)-e-2, in which X₁' is N-R³', R³' is a substituted or unsubstituted hydrocarbon group, a substituted or unsubstituted heterocyclic group, or a substituted or unsubstituted acyl group which is defined in R³, p is 0 and m' is 1 in the compound represented by formula (I"), can also be produced by Production process G below.

### (Production process G)

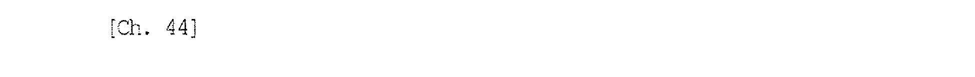

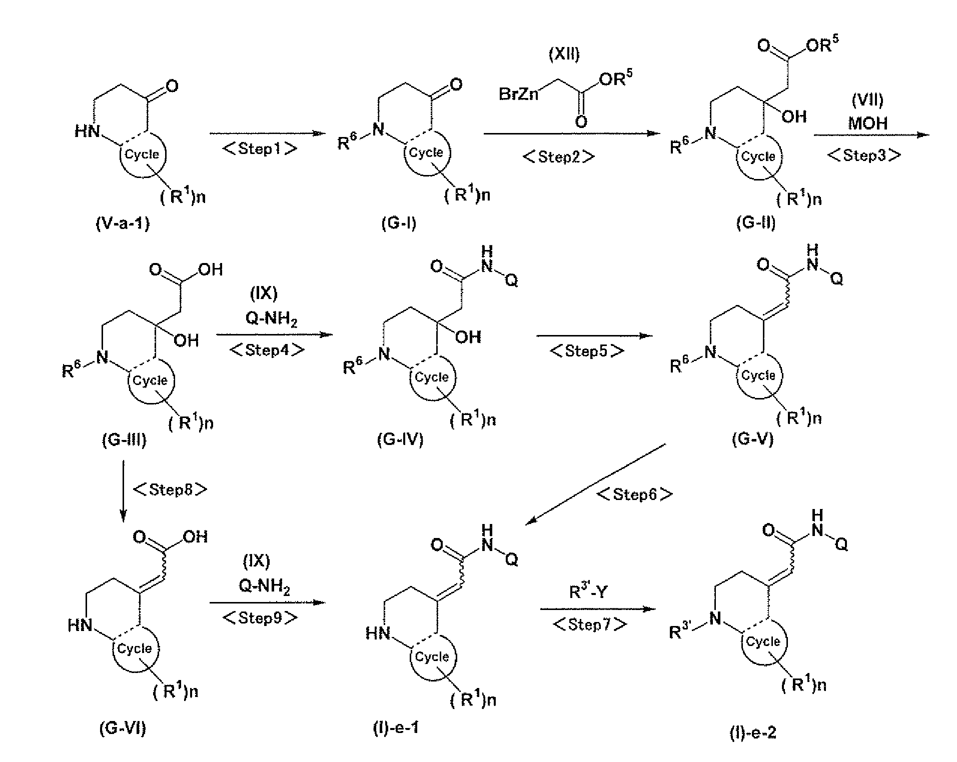

### <Step 1>

A compound represented by formula (G-I) can be produced by introducing a protective group such as a tert-butoxycarbonyl group, a benzyloxycarbonyl group, or a p-toluenesulfonyl group into the compound represented by formula (V-a-1) by a process described in published textbooks, for example, Greene et al., Protective Groups in Organic Synthesis, (the United States), 3rd edition, 1999.

### <Step 2>

A compound represented by formula (G-II) can be produced in accordance with the process described in <Step 1> of (Production process F) using the compound represented by formula (G-I).

### <Step 3>

A compound represented by formula (G-III) can be produced in accordance with the process described in <Step 3> of (Production process F) using the compound represented by formula (G-II) and the compound represented by formula (VII).

### <Step 4>

A compound represented by formula (G-IV) can be produced in accordance with the process described in <Step 6> of (Reaction scheme) using the compound represented by formula (G-III) and the compound represented by formula (IX).

### <Step 5>

A compound represented by formula (G-V) can be produced by the same process as that used in <Step 5> of (Production process F) using the compound represented by formula (G-IV).

### <Step 6>

The compound represented by formula (I)-e-1 can be produced by removing the introduced protective group from the compound represented by formula (G-V) by a process described in published textbooks, for example, Greene et al., Protective Groups in Organic Synthesis, (the United States), 3rd edition, 1999.

### <Step 7>

The compound represented by formula (I)-e-2 can be produced by the same process as that used in <Step 3> of (Production process E) using the compound represented by formula (I)-e-1.

### <Step 8>

A compound represented by formula (G-VI) can be produced by conducting a reaction as in <Step 5> of (Production process G) using the compound represented by formula (G-III).

### <Step 9>

The compound represented by formula (I)-e-1 can be produced by conducting a reaction as in <Step 4> of (Production process G) using the compound represented by formula (G-VI).

### (Production process H)

### <In formula (I), the case where X₁ is O, N-R³, or S (which is represented by X₁'), X₂ is CH₂, and p is 0.>

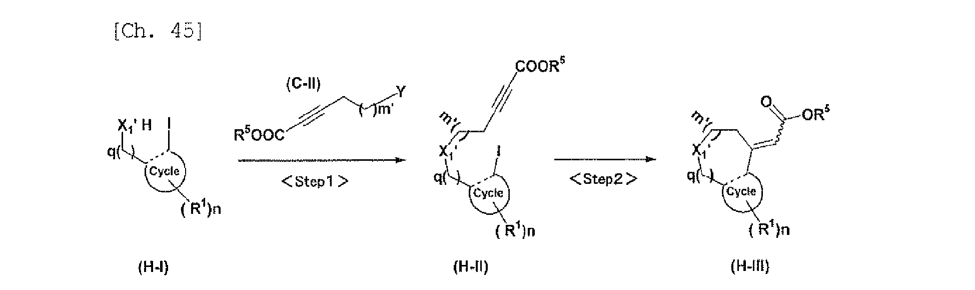

### <Step 1>

A compound represented by formula (H-II) can be produced by the same process as that used in <Step 1> of (Reaction scheme) using a compound represented by formula (H-I) and the compound represented by formula (C-II).

### <Step 2>

A compound represented by formula (H-III) can be produced by the same process as that used in <Step 2> of (Production process C) using the compound represented by formula (H-II).

Alternatively, the compound represented by formula (H-III) can be produced by the following process.

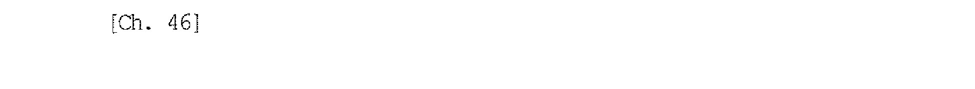

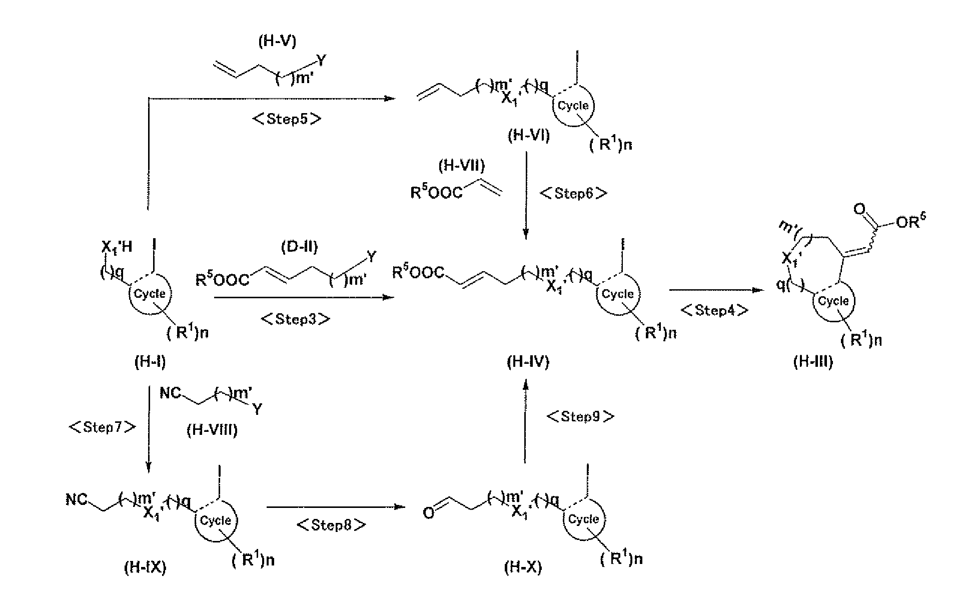

### <Step 3>

A compound represented by formula (H-IV) can be produced by the same process as that used in <Step 1> of (Reaction scheme) using the compound represented by formula (H-I) and the compound represented by formula (D-II).

### <Step 4>

The compound represented by formula (H-III) can be produced by the same process as that used in <Step 2> of (Production process D) using the compound represented by formula (H-IV).

Furthermore, the compound represented by formula (H-IV), which is an intermediate, can be produced by the following process.

### <Step 5>

A compound represented by formula (H-VI) can be produced by the same process as that used in <Step 1> of (Reaction scheme) using the compound represented by formula (H-I) and a compound represented by formula (H-V).

The compound represented by formula (H-IV) can be produced by the same process as that used in <Step 3> of (Production process B) using the compound represented by formula (H-VI) and a compound represented by formula (H-VII).

### <Step 7>

A compound represented by formula (H-IX) can be produced by the same process as that used in <Step 1> of (Reaction scheme) using the compound represented by formula (H-I) and a compound represented by formula (H-VIII).

### <Step 8>

A compound represented by formula (H-X) can be produced by the same process as that used in <Step 6> of (Production process D) using the compound represented by formula (H-IX).

### <Step 9>

The compound represented by formula (H-IV) can be produced by the same process as that used in <Step 4> of (Reaction scheme) using the compound represented by formula (H-X).

### (Production process I)

### <In formula (I), the case where X₁ is O, N-R³, or S (which is represented by X₁'), X₂ is CH₂, q is 0, m is 1, R² is alkyl, and p is 2.>

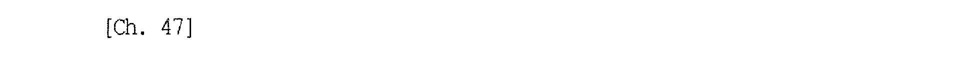

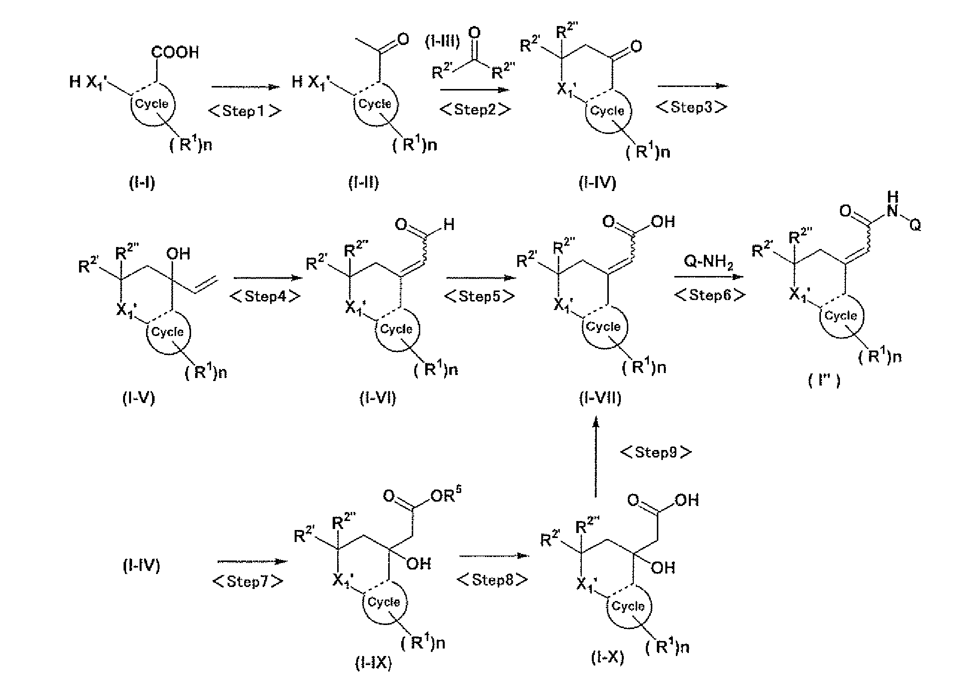

### <Step 1>

A compound represented by formula (I-II) can be produced by conducting a reaction using a compound represented by formula (I-I) by a process similar to that described in published documents, for example, Journal of Medicinal Chemistry, 46(13), pp. 2683-2696, 2003, in the presence of methyllithium (MeLi) with a solvent which is inactive to the reaction, such as diethyl ether, 1,2-dimethoxyethane, dioxane, or tetrahydrofuran, or a mixed solvent thereof at a temperature in the range of -78°C to the solvent-reflux temperature.

### <Step 2>

A compound represented by formula (I-IV) can be produced by reacting the compound represented by formula (I-II) with a compound represented by formula (I-III) by a process similar to that described in published documents, for example, Journal of Heterocyclic Chemistry, 32, pp. 1393-1395, 1995, in the presence of a base such as pyrrolidine, piperazine, morpholine, triethylamine, N,N-diisopropylethylamine, or pyridine using a solvent which is inactive to the reaction, such as an alcoholic solvent, e.g., methanol, ethanol, or 2-propanol, or a mixed solvent thereof at a temperature in the range of 0°C to the solvent-reflux temperature. In the formulae, each of R²' and R²" is an alkyl group such as methyl, ethyl, propyl , or isopropyl, and R²' and R²" may be the same or independent each other. R²' and R²" may form a ring such as cyclopropyl, cyclobutyl, cyclopentyl, or cyclohexyl, and the ring may include a heteroatom such as S, O, or N.

### <Step 3>

A compound represented by formula (I-V) can be produced by conducting a reaction using the compound represented by formula (I-IV) by a process similar to that described in published documents, for example, Jikken Kagaku Koza (Experimental Chemistry Series), 4th edition, 25, Organic synthesis VII, Synthesis using organometallic reagent, pp. 59-72, 1992, Maruzen Co., Ltd., in the presence of vinyl magnesium chloride or vinyl magnesium bromide with a solvent which is inactive to the reaction, such as diethyl ether, 1,2-dimethoxyethane, dioxane, or tetrahydrofuran, or a mixed solvent thereof at a temperature in the range of -78°C to the solvent-reflux temperature.

### <Step 4>

A compound represented by formula (I-VI) can be produced by conducting a reaction using the compound represented by formula (I-V) by a process similar to that described in published documents, for example, Tetrahedron Letters, 30(9), pp. 1033-1036, 1989, in the presence of an oxidizing agent such as pyridinium dichromate (PDC), pyridinium chlorochromate (PCC), or chromium oxide (CrO₃) with a solvent which is inactive to the reaction, such as dichloromethane, 1,2-dichloroethane, or benzene, or a mixed solvent thereof at a temperature in the range of 0°C to the solvent-reflux temperature.

### <Step 5>

A compound represented by formula (I-VII) can be produced by conducting a reaction using the compound represented by formula (I-VI) by a process similar to that described in published documents, for example, Jikken Kagaku Koza (Experimental Chemistry Series), 4th edition, 23, Organic synthesis V, Oxidative reaction, pp. 472-513, 1992, Maruzen Co., Ltd., in the presence of an oxidizing agent such as sodium hypochlorite or calcium hypochlorite with a solvent which is inactive to the reaction, such as dichloromethane, 1,2-dichloroethane, acetonitrile, or water, or a mixed solvent thereof at a temperature in the range of 0°C to the solvent-reflux temperature.

### <Step 6>

A compound represented by formula (I") can be produced by the same process as that used in <Step 6> of (Reaction scheme) using the compound represented by formula (I-VII) and the compound represented by formula (IX).

Alternatively, the compound represented by formula (I-VII), which is an intermediate, can be produced by the following process.

### <Step 7>

A compound represented by formula (I-IX) can be produced by a process similar to that described in <Step 1> of (Production process F) using the compound represented by formula (I-IV).

### <Step 8>

A compound represented by formula (I-X) can be produced by the same process as that used in <Step 4> of (Production process F) using the compound represented by formula (I-IX).

### <Step 9>

The compound represented by formula (I-VII) can be produced by the same process as that used in <Step 2> of (Production process F) using the compound represented by formula (I-X).

### (Production process J)

### <In formula (I), the case where X₁ is O, N-R³, or S (which is represented by X₁'), X₂ is NH, m is 1, R² is alkyl, q is 0 and p is 2.>

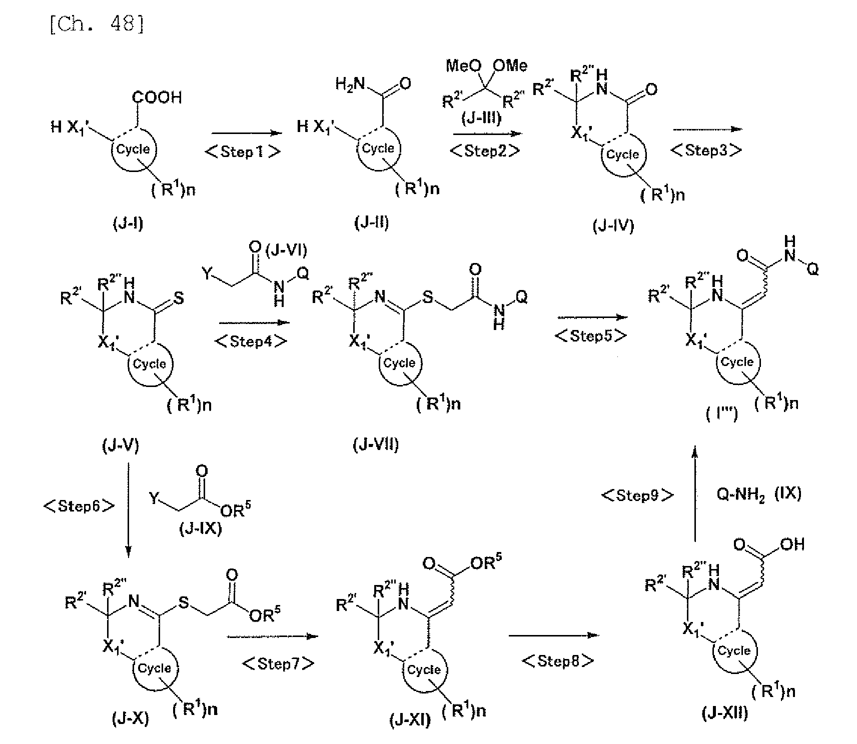

### <Step 1>

A compound represented by formula (J-II) can be produced by a process similar to that described in <Step 6> of (Reaction scheme) using a compound represented by formula (J-I).

### <Step 2>

A compound represented by formula (J-IV) can be produced by allowing the compound represented by formula (J-II) to react with a compound represented by formula (J-III) by a process described in published textbooks, for example, Greene et al., Protective Groups in Organic Synthesis, (the United States), 3rd edition, 1999. In the formulae, each of R²' and R²" is an alkyl group such as methyl, ethyl, propyl, or isopropyl, and R²' and R²" may be the same or independent each other. R²' and R²" may form a ring such as cyclopropyl, cyclobutyl, cyclopentyl, or cyclohexyl, and the ring may include a heteroatom such as S, O, or N.

### <Step 3>

A compound represented by formula (J-V) can be produced by conducting a reaction using the compound represented by formula (J-IV) by a process similar to that described in published documents, for example, Bulletin des Societes Chimiques Belges, 87, p. 229, 1978, in the presence of the Lawesson's reagent (2,4-bis(4-methoxyphenyl)-1,3,2,4-dithiadiphosphetane-2,4-disulfide) with a solvent which is inactive to the reaction, such as toluene, benzene, xylene, 1,2-dimethoxyethane, dichloromethane, 1,2-dichloroethane, chloroform, or hexamethylphosphoric triamide, or a mixed solvent thereof at a temperature in the range of 0°C to the solvent-reflux temperature.

### <Step 4>

A compound represented by formula (J-VII) can be produced by allowing the compound represented by formula (J-V) to react with a compound represented by formula (J-VI) by a process similar to that described in published documents, for example, Synlett, No. 11, pp. 1117-1118, 1996, in the presence of a base such as triethylamine, N,N-diisopropylethylamine, or N,N-dimethylaminopyridine using a solvent which is inactive to the reaction, such as acetonitrile, dioxane, tetrahydrofuran, benzene, toluene, dichloromethane, 1,2-dichloroethane, or chloroform, or a mixed solvent thereof at a temperature in the range of room temperature to the solvent-reflux temperature.

### <Step 5>

A compound represented by formula (I"') can be produced by conducting a reaction using the compound represented by formula (J-VII) by a process similar to that described in published documents, for example, Synlett, No. 11, pp. 1117-1118, 1996, in the presence of a phosphine reagent such as triphenylphosphine or tributylphosphine; a phosphite reagent such as trimethyl phosphite, triethyl phosphite, tripropyl phosphite, or tributyl phosphite; and a base such as triethylamine, N,N-diisopropylethylamine, or N,N-dimethylaminopyridine at a temperature in the range of room temperature to the solvent-reflux temperature.

### <Step 6>

A compound represented by formula (J-X) can be produced by the same process as that used in <Step 4> of (Production process J) using the compound represented by formula (J-V) and a compound represented by formula (J-IX).

### <Step 7>

A compound represented by formula (J-XI) can be produced by the same process as that used in <Step 5> of (Production process J) using the compound represented by formula (J-X).

### <Step 8>

A compound represented by formula (J-XII) can be produced by the same process as that used in <Step 5> of (Reaction scheme) using the compound represented by formula (J-XI).

### <Step 9>

A compound represented by formula (I"') can be produced by the same process as that used in <Step 6> of (Reaction scheme) using the compound represented by formula (J-XII) and the compound represented by formula (IX).

### (Production process K)

### <In formula (I), the case where X₁ is O, N-R³, or S (which is represented by X₁'), X₂ is NH, m is 2, q is 0 and p is 0.>

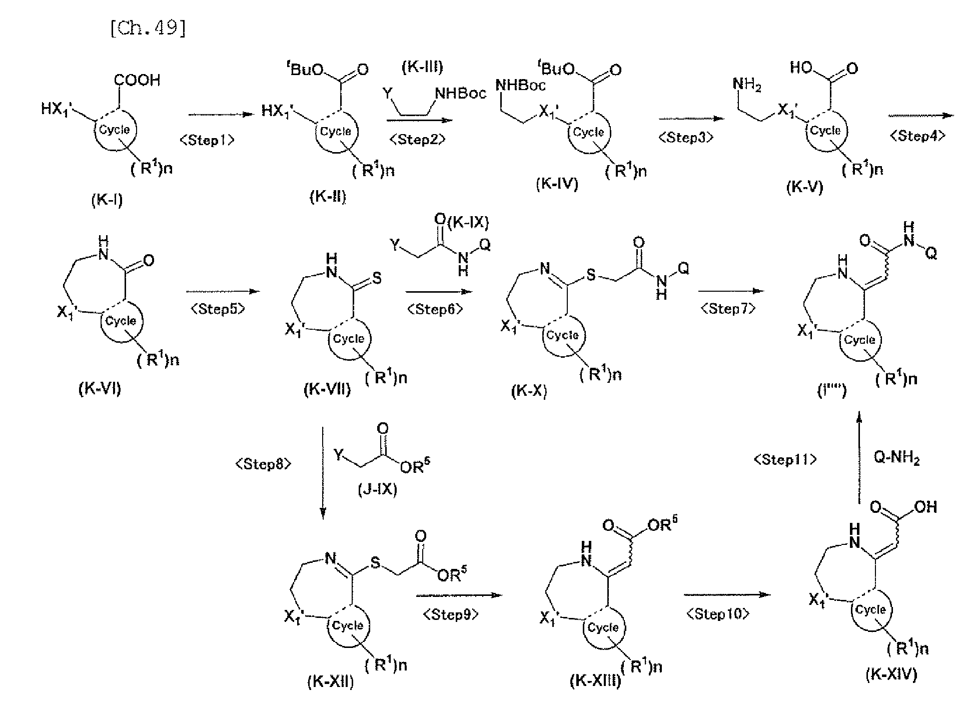

### <Step 1>

A compound represented by formula (K-II) can be produced by the same process as that used in <Step 1> of (Production process A) using the compound represented by formula (K-I), and t-BuOH.

### <Step 2>

A compound represented by formula (K-IV) can be produced by the same process as that used in <Step 2> of (Production process A) using the compound represented by formula (K-II), and (K-III).

### <Step 3>

A compound represented by formula (K-V) can be produced by the same process as that used in <Step 6> of (Production process G) using the compound represented by formula (K-IV)

### <Step 4>

A compound represented by formula (K-VI) can be produced by the same process as that used in <Step 6> of (Reaction scheme) using the compound represented by formula (K-V).

### <Step 5>

A compound represented by formula (K-VII) can be produced by the same process as that used in <Step 3> of (Production process J) using the compound represented by formula (K-VI).

### <Step 6>

A compound represented by formula (K-X) can be produced by the same process as that used in <Step 4> of (Production process J) using the compound represented by formula (K-VII), and (K-IX).

### <Step 7>

A compound represented by formula (I"") can be produced by the same process as that used in <Step 5> of (Production process J) using the compound represented by formula (K-X).

### <Step 8>

A compound represented by formula (K-XII) can be produced by the same process as that used in <Step 4> of (Production process J) using the compound represented by formula (K-VII), and (J-IX).

### <Step 9>

A compound represented by formula (K-XIII) can be produced by the same process as that used in <Step 5> of (Production process J) using the compound represented by formula (K-XII).

### <Step 10>

A compound represented by formula (K-XIV) can be produced by the same process as that used in <Step 5> of (Reaction scheme) using the compound represented by formula (K-XIII).

### <Step 11>

A compound represented by formula (I"") can be produced by the same process as that used in <Step 6> of (Reaction scheme) using the compound represented by formula (K-XIV).

An amine parts represented by formula A-H(=Q-NH2) can be produced by below process.

### (Production process L)

### <In formula A-H, the case where j=0, k=0, L₁=O, W=CO>

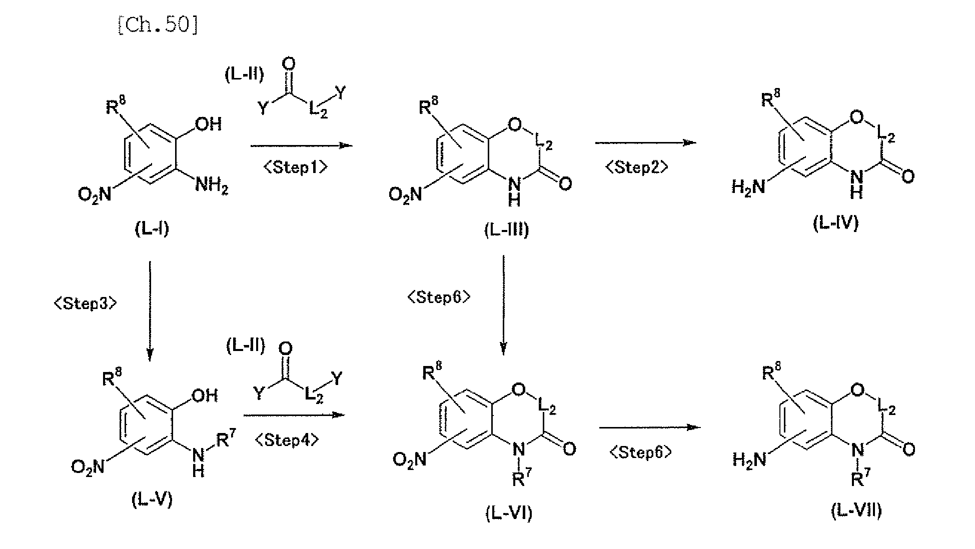

### <Step 1>

A compound represented by formula (L-III) can be produced by allowing a compound represented by formula (L-I) to react with a compound represented by formula (L-II) by a process similar to that described in published documents, for example, Bioorganic and Medicinal Chemistry, 10(8), pp. 2663-2669, 2002, in the presence of a base such as sodium hydrogen carbonate, sodium carbonate, sodium hydroxide, potassium hydrogen carbonate, potassium carbonate, potassium hydroxide, cesium carbonate, or potassium fluoride using a solvent which is inactive to the reaction, such as a halogenated solvent, e.g., dichloromethane or chloroform, an ethereal solvent, e.g., diethyl ether or tetrahydrofuran, or an aromatic hydrocarbon solvent, e.g., toluene or benzene, or a polar solvent, e.g., N,N-dimethylformamide, acetone, 4-methyl-2-pentanone, 2,6-dimethylheptanone, or a mixed solvent thereof at a temperature in the range of room temperature to the solvent-reflux temperature.

### <Step 2>

A compound represented by formula (L-IV) can be produced by conducting a reaction using the compound represented by formula (L-III) by a process similar to that described in published documents, for example, Jikken Kagaku Koza (Experimental Chemistry Series), 4th edition, 26, Organic synthesis VIII, Asymmetric synthesis, reduction, sugar, and labeled compound, pp. 159-266, 1992, Maruzen Co., Ltd., in the presence of a catalyst such as palladium-carbon (Pd-C), Raney-Ni, platinum oxide (PtO2), or dichloro triphenyl phosphine ruthenium, under hydrogen atmosphere, using a solvent which is inactive to the reaction, such as an alcoholic solvent, e.g., methanol, ethanol, or 2-propanol, an ethereal solvent, e.g., diethyl ether or tetrahydrofuran, 1,2-dimethoxyethane, or 1,4-dioxane, a polar solvent, e.g., ethyl acetate or methyl acetate, or a mixed solvent thereof at a temperature in the range of room temperature to the solvent-reflux temperature.

And alternatively, a compound represented by formula (L-IV) can be produced by using Fe, or Sn, in hydrochloric acid or acetic acid, at a temperature in the range of 0 °C to the solvent-reflux temperature. Further more, a compound represented by formula (L-IV) can be produced also by using sodium borohydride in the presence of Lewis Acid, e.g., Nickel(II)chloride (NiCl₂), Tin(II)chloride (SnCl₂) using a solvent which is inactive to the reaction, such as an alcoholic solvent, e.g., methanol, ethanol, or 2-propanol, an ethereal solvent, e.g., diethyl ether or tetrahydrofuran, 1,2-dimethoxyethane, or 1,4-dioxane, or a mixed solvent thereof at a temperature in the range of 0 °C to the solvent-reflux temperature.

### <Step 3>

A compound represented by formula (L-V) can be produced by the same process as that used in <Step 3> of (Production process E) using the compound represented by formula (L-I).

### <Step 4>

A compound represented by formula (L-VI) can be produced by the same process as that used in <Step 1> of (Production process L) using the compound represented by formula (L-V) and (L-II).

### <Step 5>

A compound represented by formula (L-VI) can be produced by the same process as that used in <Step 3> of (Production process E) using the compound represented by formula (L-III).

### <Step 6>

A compound represented by formula (L-VII) can be produced by the same process as that used in <Step 2> of (Production process L) using the compound represented by formula (L-VI).

### (Production process M)

### <In formula A-H, the case where j=0, k=0, L1=NR¹⁰, NH, or S, W=CO>

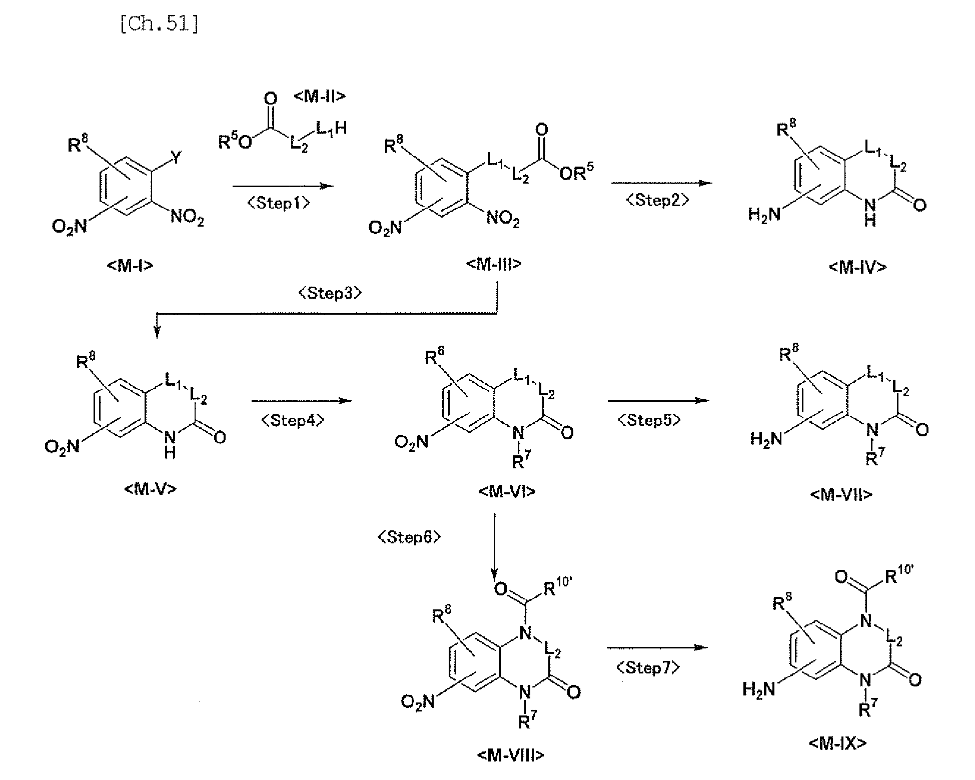

### <Step 1>

A compound represented by formula (M-III) can be produced by allowing a compound represented by formula (M-I) to react with a compound represented by formula (M-II) by a process similar to that described in published documents, for example, Journal of the Chemical Society, Perkin Transactions I, (3), pp. 681-689, 1988, in the presence of a base such as sodium hydrogen carbonate, sodium carbonate, sodium hydroxide, potassium hydrogen carbonate, potassium carbonate, potassium hydroxide, cesium carbonate, or potassium fluoride using a solvent which is inactive to the reaction, such as a halogenated solvent, e.g., dichloromethane or chloroform, an ethereal solvent, e.g., diethyl ether or tetrahydrofuran, or an aromatic hydrocarbon solvent, e.g., toluene or benzene, or a polar solvent, e.g., N,N-dimethylformamide, acetone or a mixed solvent thereof at a temperature in the range of room temperature to the solvent-reflux temperature.

### <Step 2>

A compound represented by formula (M-IV) can be produced by the same process as that used in <Step 6> of (Production process L) using the compound represented by formula (M-III).

### <Step 3>

A compound represented by formula (M-V) can be produced by conducting a reaction using the compound represented by formula (M-III) by a process similar to that described in published documents, for example, Journal of Medical Chemistry, 32(1), pp. 23-30, 1989, in the presence of sodium sulfide/Sulfur using a solvent which is inactive to the reaction, such as an ethereal solvent, e.g., diethyl ether, tetrahydrofuran, 1,2-dimethoxyethane, 1,4-dioxane, or an alcoholic solvent, e.g., methanol, ethanol, 2-propanol, or an aromatic hydrocarbon solvent, e.g., toluene or benzene, or a polar solvent, e.g., acetonitrile, N,N-dimethylformamide, dimethylsulfoxide or a mixed solvent thereof at a temperature in the range of 0 °C to the solvent-reflux temperature.

### <Step 4>

A compound represented by formula (M-VI) can be produced by the same process as that used in <Step 3> of (Production process E) using the compound represented by formula (M-V).

### <Step 5>

A compound represented by formula (M-VII) can be produced by the same process as that used in <Step 2> of (Production process L) using the compound represented by formula (M-VI).

### <In particularly, the case where L1=NCOR^{10'}, W=CO>

### <Step 6>

A compound represented by formula (M-VIII) can be produced by the same process as that used in <Step 6> of (Reaction scheme) using the compound represented by formula (M-VI).

### <Step 7>

A compound represented by formula (M-IX) can be produced by the same process as that used in <Step 2> of (Production process L) using the compound represented by formula (M-VI).

### (Production process N)

### <In formula A-H, the case where L₁=S(O)ₜ, t = 1 or 2 W=CO>

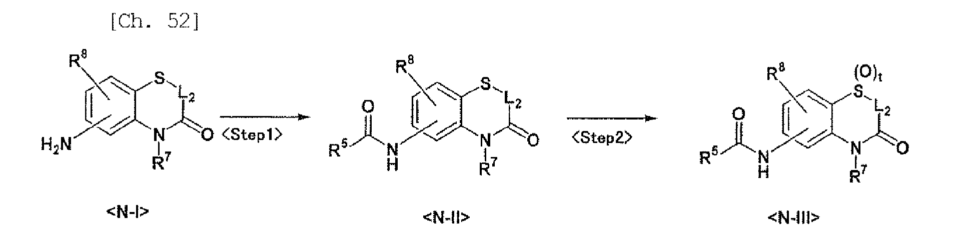

### <Step 1>

A compound represented by formula (N-II) can be produced by the same process as that used in <Step 6> of (Reaction scheme) using the compound represented by formula (N-I).

### <Step 2>

A compound represented by formula (N-III) can be produced by conducting a reaction using the compound represented by formula (N-II) by a process similar to that described in published documents, for example, Jikken Kagaku Koza (Experimental Chemistry Series), 4th edition, 23, Organic synthesis V, Oxidative reaction, pp. 472-513, 1992, Maruzen Co., Ltd., in the presence of a peroxyacid such as m-chloro perbenzoic acid, peracetic acid, trifluoromethyl peracetic acid, hydrogen peroxide, using a solvent which is inactive to the reaction, such as a halogenated solvent, e.g., dichloromethane or chloroform, an alcoholic solvent, e.g., methanol, ethanol, 2-propanol, an ethereal solvent, e.g., diethyl ether, tetrahydrofuran, 1,2-dimethoxyethane, 1,4-dioxane, or an aromatic hydrocarbon solvent, e.g., toluene or benzene, or a mixed solvent thereof at a temperature in the range of 0 °C to the solvent-reflux temperature.

### (Production process O)

### <In formula A-H, the case where j=0, k=0, L₂=O, W=CO>

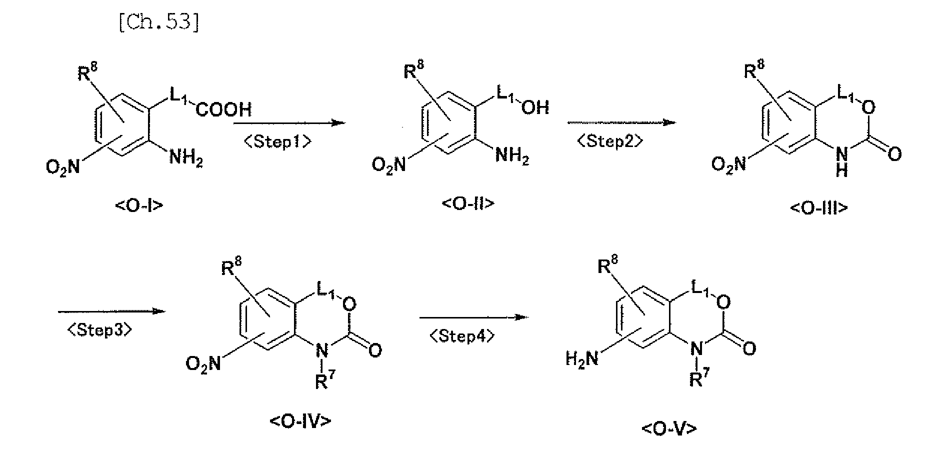

### <Step 1>

A compound represented by formula (O-II) can be produced by conducting a reaction using the compound represented by formula (O-I) by a process similar to that described in published documents, for example, Jikken Kagaku Koza (Experimental Chemistry Series), 4th edition, 26, Organic synthesis VIII, Asymmetric synthesis, reduction, sugar, and labeled compound, pp. 234-245, 1992, Maruzen Co., Ltd., in the presence of a borane reagent such as borane-tetrahydrofurane complex (BH₃-THF), borane-dimethylsulfide complex (BH₃-Me₂S) using a solvent which is inactive to the reaction, such an ethereal solvent, e.g., diethyl ether or tetrahydrofuran, 1,2-dimethoxyethane, or 1,4-dioxane, a halogenated solvent, e.g., dichloromethane or chloroform, a polar solvent, or a mixed solvent thereof at a temperature in the range of 0 °C to the solvent-reflux temperature.

### <Step 2>

A compound represented by formula (O-III) can be produced by conducting a reaction using the compound represented by formula (O-II) by a process similar to that described in published documents, for example, Journal of Medical Chemistry, 25(6), pp. 735-742, 1982, in the presence of a carbonylation reagent such as urea, 1,1-carbonylbis-1H-imidazole, triphosgen and a base such as sodium hydride, lithium hydroxyde, sodium hydroxide, potassium hydroxide, lithium carbonate, sodium carbonate, potassium carbonate, triethylamine, N,N-diisopropylethylamine, pyridine using a solvent which is inactive to the reaction, such as an ethereal solvent, e.g., diethyl ether, tetrahydrofuran, 1,2-dimethoxyethane, 1,4-dioxane, or a polar solvent, e.g., N,N-dimethylformamide or a mixed solvent thereof at a temperature in the range of 0 °C to the solvent-reflux temperature.

### <Step 3>

A compound represented by formula (O-IV) can be produced by the same process as that used in <Step 3> of (Production process E) using the compound represented by formula (O-III).

### <Step 4>

A compound represented by formula (O-V) can be produced by the same process as that used in <Step 2> of (Production process L) using the compound represented by formula (O-IV)

### (Production process P)

### <In formula A-H, the case where j=0, k=0, L₂=NR¹⁰, W=CO>

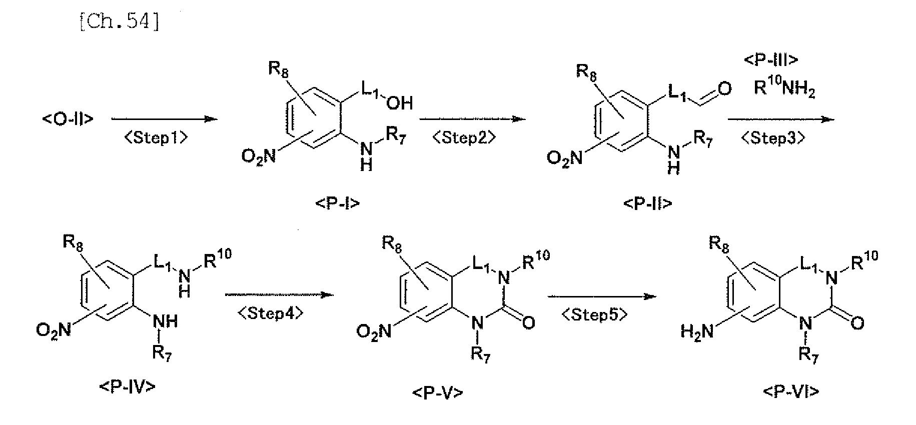

### <Step 1>

A compound represented by formula (P-I) can be produced by the same process as that used in <Step 3> of (Production process E) using the compound represented by formula (O-II)

### <Step 2>

A compound represented by formula (P-II) can be produced by conducting a reaction using the compound represented by formula (P-I) by a process similar to that described in published documents, for example, Jikken Kagaku Koza (Experimental Chemistry Series), 4th edition, 21, Organic synthesis III, aldehyde, ketone, and quinone, pp. 1-148, 1992, Maruzen Co., Ltd., in the presence of a oxidant such as pyridinium chlorochlomate (PCC), activated manganese dioxide (MnO₂), Dess-Martin reagent using a solvent which is inactive to the reaction, such a halogenated solvent, e.g., dichloromethane or chloroform, an ethereal solvent, e.g., diethyl ether or tetrahydrofuran, 1,2-dimethoxyethane, or 1,4-dioxane or a mixed solvent thereof at a temperature in the range of 0 °C to the solvent-reflux temperature.

### <Step 3>

After the compound represented by formula (P-II) and (P-III) are converted to an imine, using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0 °C to the solvent-reflux temperature, A compound represented by formula (P-IV) can be produced by a process similar to that described in published documents, for example, Journal of Medical Chemistry, 23(12), pp. 1405-1410, 1980 in the presence of a reductive reagent such as sodium borohydride using a solvent which is inactive to the reaction, such as an alcoholic solvent, e.g., methanol, ethanol, 2-propanol, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0 °C to the solvent-reflux temperature.

### <Step 4>

A compound represented by formula (P-V) can be produced by the same process as that used in <Step 3> of (Production process O) using the compound represented by formula (P-IV).

### <Step 5>

A compound represented by formula (P-VI) can be produced by the same process as that used in <Step 2> of (Production process L) using the compound represented by formula (P-V).

### (Production process Q)

### <In formula A-H, the case where j=0, k=0, L₂=NR¹⁰, M=SO₂>

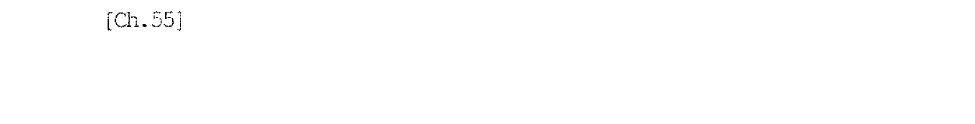

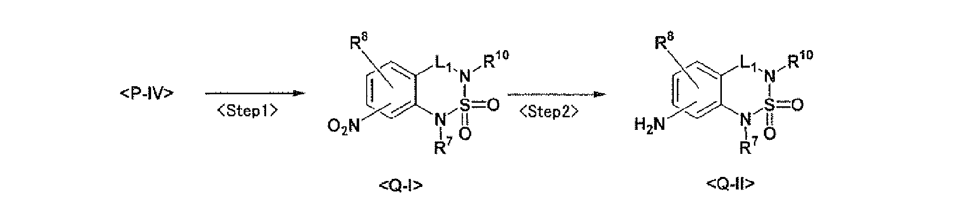

### <Step 1>

A compound represented by formula (Q-1) can be produced by conducting a reaction using the compound represented by formula (P-IV) by a process similar to that described in published documents, for example, Journal of Medical Chemistry, 44(12), pp. 1847-1852, 2001, in the presence of a sulfonylation reagent such as sulfamide using a solvent which is inactive to the reaction, such as a basic solvent e.g., triethylamine, N,N-diisopropylethylamine, pyridine or a mixed solvent thereof at a temperature in the range of 0 °C to the solvent-reflux temperature.

### <Step 2>

A compound represented by formula (Q-II) can be produced by the same process as that used in <Step 2> of (Production process L) using the compound represented by formula (Q-I).

### (Production process R)

### <In formula A-H, the case where j=0, k=0, L₁-L₂=-CH₂CH(NR¹¹R¹¹)- or L₁-L₂=-CH=C(NR¹¹R¹¹)-, W=CO>

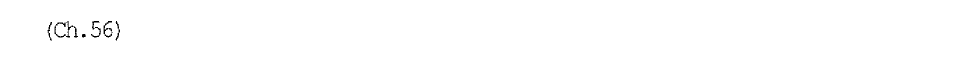

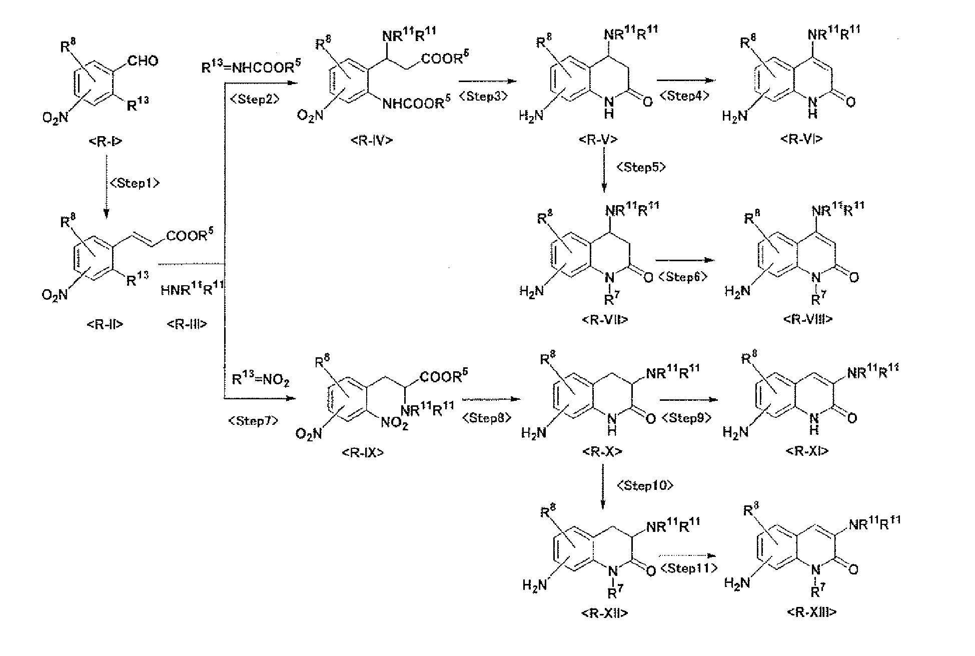

### <Step 1>

A compound represented by formula (R-II) can be produced by the same process as that used in <Step 4> of (Reaction scheme) using the compound represented by formula (R-I).

### <Step 2> (In the case where R¹³=NHCOOR⁵)

A compound represented by formula (R-IV) can be produced by allowing a compound represented by formula (R-II) to react with a compound represented by formula (R-III) by a process similar to that described in published documents, for example, Tetrahedron, 60(2), pp. 383-387, 2004, in the presence of a Lewis Acid such as aluminum(III)chlride, titanium(IV)chloride, tin(IV)chloride, lithium perchlorate using a solvent which would not take part in the reaction, such as a halogenated solvent, e.g., dichloromethane or chloroform, an alcoholic solvent, e.g., methanol, ethanol, 2-propanol, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0 °C to the solvent-reflux temperature.

### <Step 3>

A compound represented by formula (R-V) can be produced, first, by conducting a reaction of deprotection using the compound represented by formula (R-IV) and acid catalyst by a process similar to that described in published textbooks, for example, Greene et al., Protective Groups in Organic Synthesis, (the United States), 3rd edition, 1999., then, by the same process as that used in <Step 2> of (Production process L).

### <Step 4>

A compound represented by formula (R-VI) can be produced by conducting a reaction using the compound represented by formula (R-V) by a process similar to that described in published documents, for example, Heterocyclic Communications, 11(6), pp. 485-490, 2005, in the presence of 2,3-dichloro-5,6-dicyano-p-benzoquinone using a solvent which would not take part in the reaction, such as an ethereal solvent, e.g., diethyl ether, tetrahydrofuran, 1,2-dimethoxyethane, 1,4-dioxane, or a polar solvents e.g., acetonitril or a mixed solvent thereof at a temperature in the range of 0 °C to the solvent-reflux temperature.

### <Step 5>

A compound represented by formula (R-VII) can be produced by the same process as that used in <Step 3> of (Production process E) using the compound represented by formula (R-V).

### <Step 6>

A compound represented by formula (R-VIII) can be produced by the same process as that used in <Step 4> of (Production process R) using the compound represented by formula (R-VII).

### <Step 7> (In the case where R¹³=NO₂)

A compound represented by formula (R-IX) can be produced by the same process as that used in <Step 2> of (Production process R) using the compound represented by formula (R-II).

### <Step 8>

A compound represented by formula (R-X) can be produced by the same process as that used in <Step 2> of (Production process L) using the compound represented by formula (R-IX).

### <Step 9>

A compound represented by formula (R-XI) can be produced by the same process as that used in <Step 4> of (Production process R) using the compound represented by formula (R-X).

### <Step 10>

A compound represented by formula (R-XII) can be produced by the same process as that used in <Step 3> of (Production process E) using the compound represented by formula (R-X).

### <Step 11>

A compound represented by formula (R-XIII) can be produced by the same process as that used in <Step 4> of (Production process R) using the compound represented by formula (R-XII).

Regarding the Production process R, the original products, such as EXAMPLE 30 of the basic patent application JP2007-014372, obtained from the series of 2,4-dinitrocinnamate through the step 7 and step 8 (originally step 2 and step 3 or step 4 of the Production process R in the basic application) have been reassigned and confirmed this time as alpha(α)-addition products. This addition position corresponds to the 3-position of the 3,4-dihydro- 2(1H)-quinolinone ring. From the view point, the reassigned EXAMPLES are No.30, 31, 32, 33, 34, 35, 42, 43, 44, 45, 46, 47, 48, 49, 50, 51, 52, 53, 54, 55, 56, 57and 58.

During the investigation of the step 7, since the Michael reaction seemed to undergo in the step, the inventors misassigned the addition position as beta(p), which corresponds to 4-position of the 3,4-dihydro- 2(1H)-quinolinone ring. Then, the inventors happened to recognize a literature^{*1} which reports that "ethyl 2-nitrocinnamate undergoes standard β-addition, however, ethyl 2,4-dinitrocinnamate undergoes α-addition" and tried the reassignment of the original products.

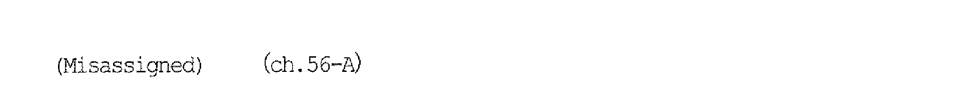

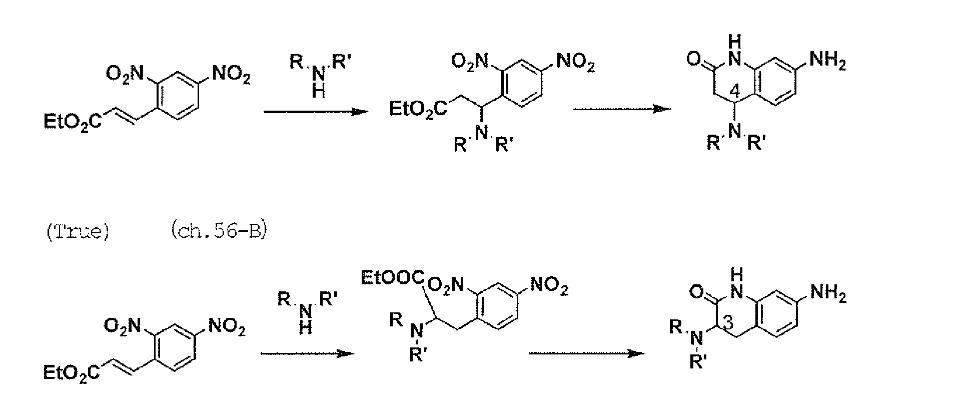

Since the misassignment took place in a series of intermediates, the assignment of the positions of a series of following final products were also affected. Therefore, all wrong description "4-" should be reassigned as true position "3-" of the 3,4-dihydro-2(1H)-quinolinone ring in the chemical structures or chemical names of the above series of intermediates and related final products. For example, regarding the EXAMPLE 30, the addition position of 4-morpholinyl group has been reassigned from 4-(4-morpholinyl) to 3-(4-morpholinyl) in this application. The same reassignments have been done in the chemical structure or partial structure of related intermediates as formula 30-3 or (a27). The reassignmets in the other EXAMPLES have also been done in the same way.

As the above explanation, there were the series of misassignments in the examples of basic patent application JP 2007-014372. And in the present application, these examples are described with reassigned results. However, there is no substantial difference as real products between the products or intermediates of above mentioned EXAMPLES described in the specifications of both patent applications, that is apparent since the analytical data are really identical.
*1: Canadian J. of Chemistry (2002), 80(2), 192-199 (Scheme 4/ Procedure E)

### (Production process S)

### <In formula A-H, the case where j=0, k=0, L₁-L₂=CH=CNR¹¹R¹¹, W=CO>

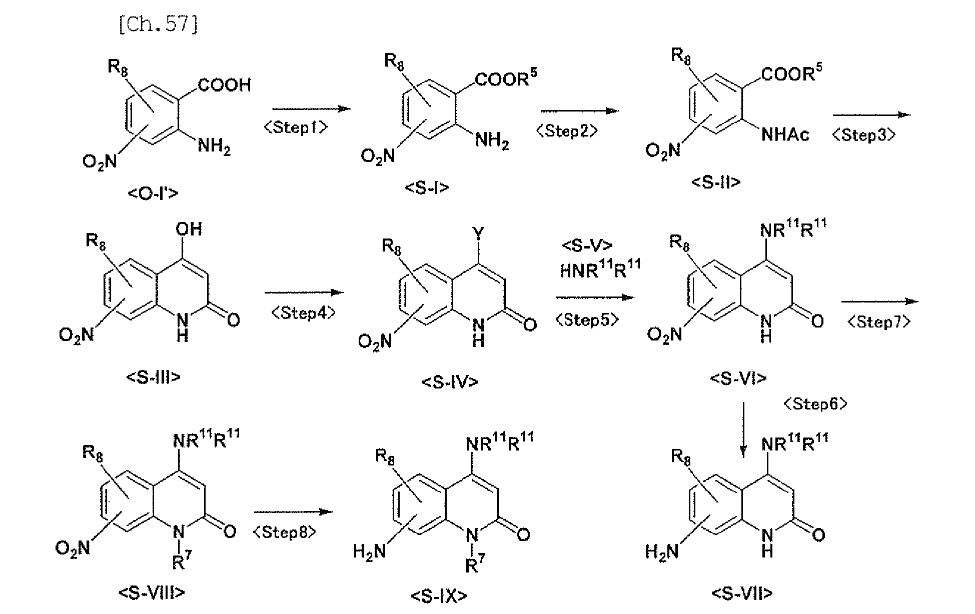

### <Step 1>

A compound represented by formula (S-I) can be produced by the same process as that used in <Step 1> of (Production process A) using the compound represented by formula (0-1'), and an alcoholic solvent, e.g., methanol, ethanol, t-butanol, benzylalcohol.

### <Step 2>

A compound represented by formula (S-II) can be produced by conducting a reaction using the compound represented by formula (S-I) by a process similar to that described in published documents, for example, European Journal of Medicinal Chemistry, 40(9), pp. 897-907, 2005, in the presence of acetic anhydride using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, or a mixed solvent thereof at a temperature in the range of 0 °C to the solvent-reflux temperature.

### <Step 3>

A compound represented by formula (S-III) can be produced by conducting a reaction using the compound represented by formula (S-II) by a process similar to that described in published documents, for example, European Journal of Medicinal Chemistry, 40(9), pp. 897-907, 2005, in the presence of basic reagent such as sodium hydride, butyllithium, piperazine, morpholine, triethylamine, lithium diisopropylamide, lithium bistrimethylsilylamide, sodium bistrimethylsilylamide, potassium bistrimethylsilylamide, using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, or a mixed solvent thereof at a temperature in the range of -78 °C to the solvent-reflux temperature.

### <Step 4>

A compound represented by formula (S-IV) can be produced by conducting a reaction using the compound represented by formula (S-III)and phosphoryl chloride by a process similar to that described in published documents, for example, Journal of Medicinal Chemistry, 31(7), pp. 1347-1351, 1988, using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, or a mixed solvent thereof at a temperature in the range of 0 °C to the solvent-reflux temperature.

### <Step 5>

A compound represented by formula (S-VI) can be produced by allowing a compound represented by formula (S-IV) to react with a compound represented by formula (S-V) by a process similar to that described in published documents, for example, Journal of Medicinal Chemistry, 31(7), pp. 1347-1351, 1988, using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, a polar solvent, e.g., acetonitril, N,N-dimethylformamide, dimethylsulfoxide, or a mixed solvent thereof at a temperature in the range of 0 °C to the solvent-reflux temperature.

### <Step 6>

A compound represented by formula (S-VII) can be produced by the same process as that used in <Step 2> of (Production process L) using the compound represented by formula (S-VI).

### <Step 7>

A compound represented by formula (S-VIII) can be produced by the same process as that used in <Step 3> of (Production process E) using the compound represented by formula (S-VI).

### <Step 8>

A compound represented by formula (S-IX) can be produced by the same process as that used in <Step 2> of (Production process L) using the compound represented by formula (S-VIII).

### (Production process T)

### <In formula A-H, the case where j=0, k=0, L₁-L₂=CH₂CH₂, R⁸=NR¹¹R¹¹, W=CO>

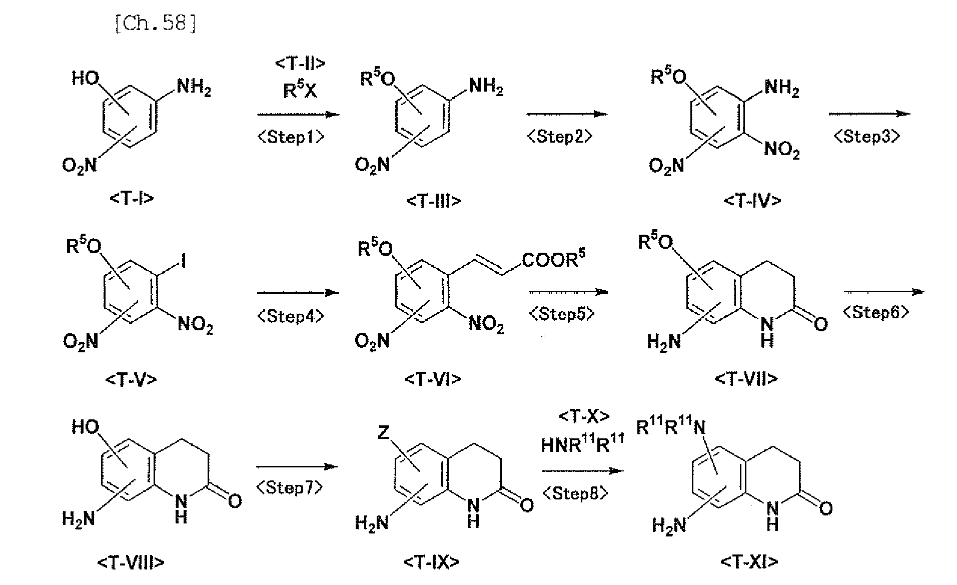

### <Step 1>

A compound represented by formula (T-III) can be produced by allowing a compound represented by formula (T-I) to react with a compound represented by formula (T-II) by a process similar to that described in published documents, for example, Jikken Kagaku Koza (Experimental Chemistry Series), 4th edition, 20, Organic synthesis II, Alcohols and amines, pp. 280-372, 1992, Maruzen Co., Ltd., in the presence of a basic reagent such as sodium hydrogen carbonate, sodium carbonate, sodium hydroxide, potassium hydrogen carbonate, potassium carbonate, potassium hydroxide, cesium carbonate, or potassium fluoride, using a solvent which is inactive to the reaction, such as acetonitrile, dioxane, tetrahydrofurane, benzene, toluene, dimethylsulfoxide, N,N-dimethylformamide, or a mixed solvent thereof at a temperature in the range of room temperature to the solvent-reflux temperature.

### <Step 2>

A compound represented by formula (T-IV) can be produced by conducting a reaction using the compound represented by formula (T-III) and nitrating reagent such as nitric acid, nitric acid / sulfonic acid, nitric acid / acetic anhydride, potassium nitrate / sulfonic acid, sodium nitrate / sulfonic acid, potassium nitrate / acetic anhydride, nitric acid /trifluoromethansulfonic acid by a process similar to that described in published documents, for example, Jikken Kagaku Koza (Experimental Chemistry Series), 4th edition, 20, Organic synthesis II, Alcohols and amines, pp. 394-405, 1992, Maruzen Co., Ltd., at a temperature in the range of 0 °C to the solvent-reflux temperature.

### <Step 3>

A compound represented by formula (T-V) can be produced by conducting a reaction using potassium iodide and the diazo compound which converted from a compound represented by formula (T-IV) with sodium nitrite / sulfuric acid / acetic acid, by a process similar to that described in published documents, for example, Tetrahedron, 61(52), pp. 12300-12338, 2005, at a temperature in the range of 0 °C to room temperature.

### <Step 4>

A compound represented by formula (T-VI) can be produced by the same process as that used in <Step 2> of (Production process D) using the compound represented by formula (T-V).

### <Step 5>

A compound represented by formula (T-VII) can be produced by the same process as that used in <Step 2> of (Production process L) using the compound represented by formula (T-VI).

### <Step 6>

A compound represented by formula (T-VIII) can be produced by conducting a reaction of deprotection using the compound represented by formula (T-VII) and acid catalyst such as 48% hydrobromide / acetic acid, aluminum (III) chloride by a process similar to that described in published textbooks, for example, Green et al., Protective Groups in Organic Synthesis, (the United States), 3rd edition, 1999.

### <Step 7>

A compound represented by formula (T-IX) can be produced by conducting a reaction using the compound represented by formula (T-VIII) and trifluoromethansulfonic acid anhydride, or trifluoromethansulfonic acid chloride by a process similar to that described in published documents, for example, Synthesis, (4), pp. 547-550, 2005, in the presence of the basic reagent such as triethylamine, N,N-diisopropylethylamine, pyridine using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, or a mixed solvent thereof at a temperature in the range of -78 °C to the solvent-reflux temperature.

### <Step 8>

A compound represented by formula (T-XI) can be produced by allowing a compound represented by formula (T-IX) to react with a compound represented by formula (T-X) by a process similar to that described in published documents, for example, Synlett, (12), pp. 1400-1402, 1997, using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene, a polar solvent, e.g., acetonitril, N,N-dimethylformamide, dimethylsulfoxide, or a mixed solvent thereof at a temperature in the range of 0 □ to the solvent-reflux temperature.

### (Production process U)

### <In formula A-H, the case where j=1, k=0, L₁-L₂=CH₂, W=CO>

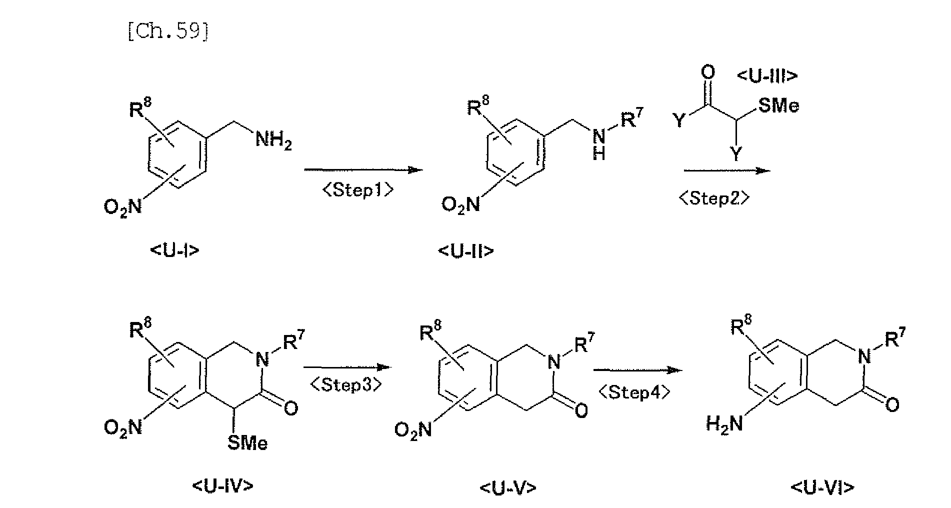

### <Step 1>

A compound represented by formula (U-II) can be produced by the same process as that used in <Step 3> of (Production process E) using the compound represented by formula (U-I).

### <Step 2>

A compound represented by formula (U-IV) can be produced by allowing a compound represented by formula (U-II) to react with a compound represented by formula (U-III) by a process similar to that described in published documents, for example, Synthesis, (7), pp. 534-537, 1981, in the presence of Tin (IV) chloride using a solvent which is inactive to the reaction, such as a halogenated solvent, e.g., dichloromethane or chloroform, at a temperature in the range of 0 □ to the solvent-reflux temperature.

### <Step 3>

A compound represented by formula (U-V) can be produced by conducting a reaction using the compound represented by formula (U-IV) by a process similar to that described in published documents, for example, Tetrahedron Letters, 28(21), pp. 2399-2402, 1987, in the presence of a catalyst such as Raney-Ni, under hydrogen atmosphere, in a solvent which is inactive to the reaction, such as an alcoholic solvent, e.g., methanol, ethanol, or 2-propanol, an ethereal solvent, e.g., diethyl ether or tetrahydrofuran, 1,2-dimethoxyethane, or 1,4-dioxane, a polar solvent, e.g., ethyl acetate or methyl acetate, or a mixed solvent thereof at a temperature in the range of room temperature to the solvent-reflux temperature.

### <Step 4>

A compound represented by formula (U-VI) can be produced by the same process as that used in <Step 2> of (Production process L) using the compound represented by formula (U-V).

### (Production process V)

### <In formula A-H, the case where j=0, k=0, L₁=L₂≠O, NR, S(O)_{t=0∼2}, W=CO>

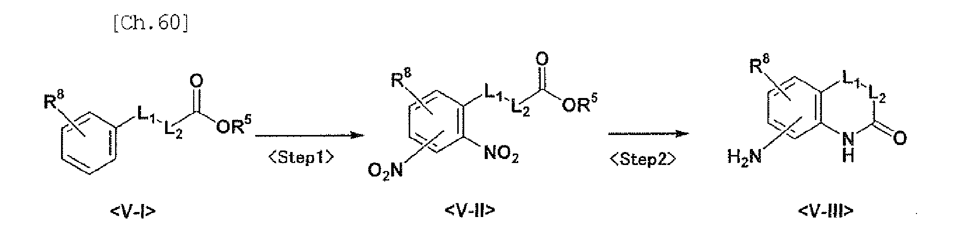

### <Step 1>

A compound represented by formula (V-II) can be produced by the same process as that used in <Step 2> of (Production process T) using the compound represented by formula (V-I).

### <Step 2>

A compound represented by formula (V-III) can be produced by the same process as that used in <Step 2> of (Production process L) using the compound represented by formula (V-II).

### (Production process W)

### <In formula A-H, the case where j=1, k=0, L₁=L₂=CH_{r=1∼2}, W=CO>

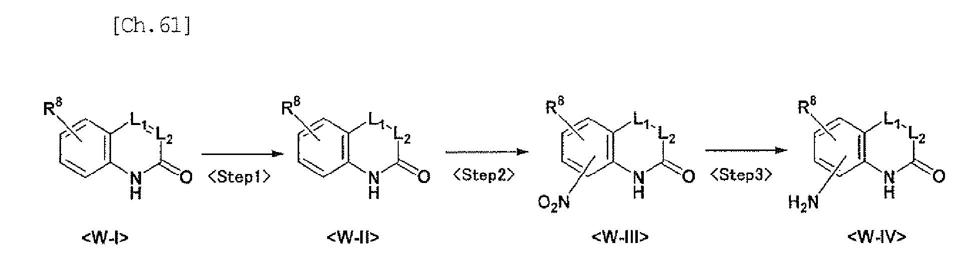

### <Step 1>

A compound represented by formula (W-II) can be produced by the same process as that used in <Step 2> of (Production process L) using the compound represented by formula (W-I).

### <Step 2>

A compound represented by formula (W-III) can be produced by the same process as that used in <Step 2> of (Production process T) using the compound represented by formula (W-II).

### <Step 3>

A compound represented by formula (W-IV) can be produced by the same process as that used in <Step 2> of (Production process L) using the compound represented by formula (W-III).

### (Production process X)

### <In formula A-H, the case where j=0, L₁=CH₂, L₂=bond, W=CO>

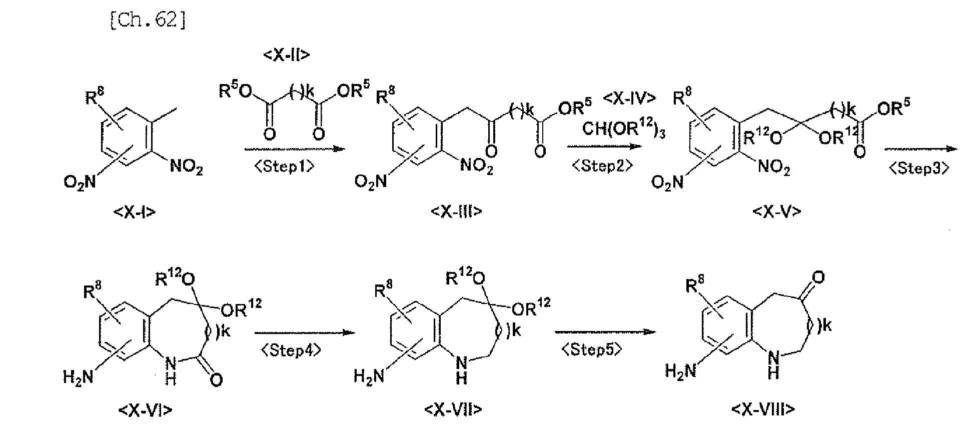

### <Step 1>

A compound represented by formula (X-III) can be produced by allowing a compound represented by formula (X-I) to react with a compound represented by formula (X-II) by a process similar to that described in published documents, for example, PCT WO 2005/044802 in the presence of a basic reagent such as sodium ethoxide, sodium methoxide, potassium t-butoxide, potassium carbonate, sodium carbonate, cesium carbonate, sodium hydroxide, potassium hydroxide, sodium hydride using a solvent which is inactive to the reaction, such as an alcoholic solvent, e.g., methanol, ethanol, or 2-propanol, an ethereal solvent, e.g., diethyl ether or tetrahydrofuran, 1,2-dimethoxyethane, or 1,4-dioxane, a polar solvent, e.g., N,N-dimethylformamide, dimethylsulfoxide, or a mixed solvent thereof at a temperature in the range of 0 °C to the solvent-reflux temperature.

### <Step 2>

A compound represented by formula (X-V) can be produced by allowing a compound represented by formula (X-III) to react with a compound represented by formula (X-IV) by a process similar to that described in published documents, for example, Synth Commun, 7, pp. 409, 1977, in the presence of a acid catalyst such as Trifluoroacetic acid, trifluoroborate-diethylether complex, Lanthanum(I II)chloride, p-toluenesulfonic acid, using a solvent such as an alcohol ic solvent, e.g., methanol, ethanol, or 2-propanol, an ethereal solvent , at a temperature in the range of 0 °C to the solvent-reflux temperatu re.

### <Step 3>

A compound represented by formula (X-VI) can be produced by the same process as that used in <Step 2> of (Production process L) using the compound represented by formula (X-V).

### <Step 4>

A compound represented by formula (X-VII) can be produced by conducting a reaction using the compound represented by formula (X-VI) by a process similar to that described in published documents, for example, Jikken Kagaku Koza (Experimental Chemistry Series), 4th edition, 26, Organic synthesis VIII, Asymmetric synthesis, reduction, sugar, and labeled compound, pp. 159-266, 1992, Maruzen Co., Ltd., in the presence of a reducing agent such as lithium aluminumhydride (LiAlH₄), borane-tetrahydrofurane complex (BH₃-THF), borane-dimethylsulfide complex (BH₃-Me₂S), sodium bis(2-methoxyethoxy)aluminumhydride, using a solvent such an ethereal solvent, e.g., diethyl ether or tetrahydrofuran, 1,2-dimethoxyethane, or 1,4-dioxane, or an aromatic hydrocarbon solvent, e.g., toluene or benzene, or a mixed solvent thereof at a temperature in the range of -78 °C to the solvent-reflux temperature.

### <Step 5>

A compound represented by formula (X-VIII) can be produced by the same process as that used in <Step 6> of (Production process G) using the compound represented by formula (X-VII).

### (Production process Y)

### <In formula A-H, the case where j=0, k=0, W=SO₂>

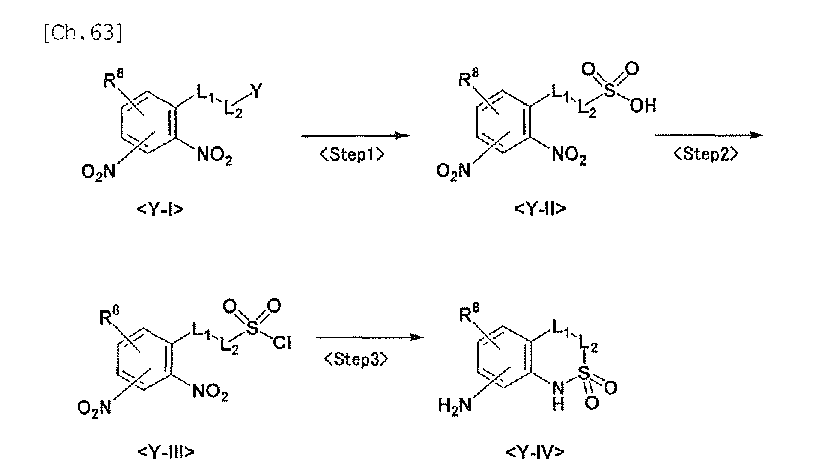

### <Step 1>

A compound represented by formula (Y-II) can be produced by conducting a reaction using the compound represented by formula (Y-I) by a process similar to that described in published documents, for example, Bioorganic and Medicinal Chemistry, 10(11), pp. 3529-3544, 2002, in the presence of sodium thiosulfate, or sodium sulfite using a solvent such as an alcoholic solvent, e.g., methanol, ethanol, or 2-propanol, at a temperature in the range of room temperature to the solvent-reflux temperature.

### <Step 2>

A compound represented by formula (Y-III) can be produced by conducting a reaction using the compound represented by formula (Y-II) by a process similar to that described in published documents, for example, Bioorganic and Medicinal Chemistry, 10(11), pp. 3529-3544, 2002, in the presence of phosphorous pentachloride, phosphoryl chloride, or chlorine gas using a solvent such as ethereal solvent, e.g., diethyl ether or tetrahydrofuran, 1,2-dimethoxyethane, or 1,4-dioxane, or a polar solvent, e.g., N,N-dimethylformamide, acetic acid, or a mixed solvent thereof at a temperature in the range of 0 °C to the solvent-reflux temperature.

### <Step 3>

A compound represented by formula (Y-IV) can be produced by the same process as that used in <Step 2> of (Production process L) using the compound represented by formula (Y-III).

When the compound synthesized by any of the above-described production processes has a reactive group such as a hydroxyl group, an amino group, or carboxyl group, as a substituent, the compound can be produced by appropriately protecting the reactive group with a protective group in the production processes and then removing the protective group in an appropriate stage. The processes of the introduction and the removal of such a protective group are appropriately selected according to the type of group to be protected or the type of protective group. The introduction and the removal of the protective group can be performed by a process described in published textbooks, for example, Greene et al., Protective Groups in Organic Synthesis, (the United States), 3rd edition, 1999.

The compound of the present invention can be used in combination with other drugs.

Examples of the drugs include acetaminophen and aspirin; opioid agonists, e.g., morphine; gabapentin; pregabalin; antidepressant drugs such as duloxetine and amitriptyline; antiepileptic drugs such as carbamazepine and phenytoin; antiarrhythmic drugs such as mexiletine, which are alternatively used and prescribed for neuropathic pain; NSAIDs such as diclofenac, indomethacin, ibuprofen, and naproxen; and anti-inflammatory drugs such as COX-2 inhibitors, e.g., Celebrex; NR2B antagonists; bradykinin antagonists; and anti-migraines. Among these, preferable examples of the drugs include morphine, gabapentin or Pregabalin, diclofenac, and Celebrex.

In addition to the use of the compound of the present invention in combination with other drugs, the compound of the present invention can be performed in combination with other treatments. Examples of the other treatments include acupuncture, laser therapy, and nerve block therapy.

For diseases or conditions in which TRPV1 is involved other than pain, the compound of the present invention can be used in combination with drugs used in the corresponding field. For example, for chronic rheumatic arthritis, the compound of the present invention can be used in combination with generally used NSAIDs, disease-modifying antirheumatic drugs (DMARDs), anti-TNF-α antibodies, soluble TNF-α receptors, steroids, immunosuppressants, or the like. For COPD or allergic diseases, the compound of the present invention can be used in combination with general therapeutic agents such as β2-receptor agonists or steroids. For an overactive bladder or urinary incontinence, the compound of the present invention can be used in combination with an anticholinergic drug.

When the compound of the present invention is used for treating the above diseases and conditions in combination with an existing drug, the dosage of the existing drug can be decreased, and thus, side effects of the existing drug can be reduced. The method of using the drugs in combinations is not limited to the above-mentioned diseases and conditions, and the drugs used in combinations are not limited to the above compounds listed as examples.

When the compound of the present invention is used in combination with another drug, the drugs may be prepared separately or as a medical mixture. In the case of separate dosing, both drugs may be administered at the same time. Alternatively, one drug may be administered in advance, and another drug may then be administered some time later.

### [Formulating for an agent for the prevention or the treatment of the present invention]

A medicine of the present invention is administered in the form of a pharmaceutical composition.

It is sufficient that the pharmaceutical composition of the present invention contains at least one compound represented by formula (I), (I-A), (I-B), (I-C), (I-D), (I'), (I''), (I'''), or (I""). The pharmaceutical composition of the present invention is prepared by being combined with pharmaceutically acceptable additives. In more detail, the compound of the present invention may be appropriately combined with the following additives to prepare various formulations. Examples of the additives include excipients (for example, lactose, sucrose, mannitel, crystalline cellulose, silicic acid, corn starch, and potato starch); binders (for example, celluloses (hydroxypropyl cellulose (HPC) and hydroxypropylmethyl cellulose (HPMC)), crystalline cellulose, sugars (lactose, mannitel, sucrose, sorbitol, erythritol, and xylitol), starches (corn starch and potato starch), α-starch, dextrine, polyvinylpyrrolidone (PVP), macrogol, and polyvinyl alcohol (PVA)); lubricants (for example, magnesium stearate, calcium stearate, talc, and carboxymethyl cellulose); disintegrants (for example, starches (corn starch and potato starch), sodium carboxymethyl starch, carmellose, carmellose calcium, crosscarmellose sodium, and crosspovidone); coating agents (for example, celluloses (hydroxypropyl cellulose (HPC) and hydroxypropylmethyl cellulose (HPMC)), aminoalkyl methacrylate copolymer E, and methacrylic acid copolymer LD); plasticizers (for example, triethyl citrate, and macrogol); masking agents (for example, titanium oxide); colorants; flavoring agents; antiseptics (benzalkonium chloride and parahydroxybenzoates); isotonic agents (for example, glycerol, sodium chloride, calcium chloride, mannitol, and glucose); pH adjusting agents (sodium hydroxide, potassium hydroxide, sodium carbonate, hydrochloric acid, sulfuric acid, and a buffer solution such as a phosphate buffer); stabilizers (for example, sugars, sugar alcohols, and xanthan gum); dispersion agents; antioxidants (for example, ascorbic acid, butylhydroxyanisole (BHA), propyl gallate, and d1-α-tocopherol); buffers; preservatives (for example, paraben, benzyl alcohol, and benzalkonium chloride); aromatics (for example, vanilin, 1-menthol, and rose oil); dissolution aids (for example, polyoxyethylene hardened castor oil, Polysorbate 80, polyethylene glycol, phospholipid cholesterol, and triethanolamine); absorption accelerators (for example, sodium glycolate, disodium edetate, sodium caprate, acylcarnitines, and limonene); gelation agents; suspending agents; emulsifying agents; and suitable additives and solvents which are normally used.

Such formulations include tablets, capsules, granules, powders, pills, aerosols, inhalants, ointments, plasters, suppositories, injections, troches, liquids, spirits, suspensions, extracts, and elixirs. These formulations may be administered to a patient by oral administration, subcutaneous administration, intramuscular administration, intranasal administration, percutaneous administration, intravenous administration, intraarterial administration, perineural administration, epidural administration, subdural administration, intraventricular administration, intrarectal administration, inhalation, or the like.

The dosage of the compound of the present invention is usually in the range of 0.005 mg to 3.0 g per day for an adult, preferably 0.05 mg to 2.5 g, and more preferably 0.1 mg to 1.5 g. The dosage may be appropriately increased or decreased in accordance with the progress of the disease and administration routes.

The entire quantity may be orally or parenterally given in one dose or given in two to six doses, or may be continuously administered by intravenous drip or the like.

### [Examples of pharmacological experiment]

The present invention will now be described more specifically using experimental examples. However, the present invention is not limited to these experimental examples.

### [Measurement of capsaicin-induced Ca influx in a transformed CHO cell line expressing human TRPV1]

### (1) Establishment of a transformed CHO cell line expressing human and rat TRPV1

Human and rat vanilloid receptor 1 (hTRPV1 and rTRPV1) cDNA was cloned from human brain and rat dorsal root ganglion, respectively. The cloned TRPV1 cDNA was incorporated in a pCAGGS vector. The vector was introduced to a CHO-K1 cell line, thus performing transformation. Clones obtained by limiting dilution were stimulated with capsaicin. Clones with a high responsiveness were selected using an increase in the Ca concentration as an indicator. The selected clones were used for the following experiment.

### (2) Measurement of Ca influx using FDSS-6000

The transformed CHO cells expressing human TRPV1 or rat were seeded in a 96-well plate (with black walls and transparent bottoms, manufactured by Greiner) at a density of 40,000 cells per well. The cells were cultured at 37°C in 5% CO₂ atmosphere for one night. A loading solution of FLIPR Calcium 3 assay kit (manufactured by Molecular Devices Corporation) containing 2.5 mmol/L of probenecid was then added to each of the wells in the same amount as the culture medium, and the cells were cultured at 37°C for 60 minutes. For three minutes after the cells were stimulated with capsaicin (1 nmol/L to 1 µmol/L), the change of the intracellular Ca concentration was measured using FDSS-6000 (λex: 480 nm, λem: 540 nm, manufactured by Hamamatsu Photonics K.K.). The integrated values of the increase rate of the intracellular Ca concentration were calculated for a group treated with the compounds of the present invention and a group treated with a vehicle, thus allowing capsaicin concentration-reaction curves to be obtained. A concentration (A2 value) of each of the compounds of the present invention, at which the capsaicin concentration-reaction curve obtained when the cells were treated with the vehicle was shifted two times rightward, was calculated. The inhibitory effects of the test compounds were compared using this value as an indicator.

In Table 1, compounds of the present invention having an A2 value of less than 100 nM are represented by A, and compounds having an A2 value of 100 nM or more are represented by B. When the A2 values of the compounds of the present invention were measured by the above-described method, the compounds have a potency of 1 µM or less.

### (3) Effects of compounds on CFA-induced rat inflammatory pain model

A CFA-induced rat inflammatory pain model is prepared by a general method, for example, the method used by Pomonis JD et al. (The Journal of Pharmacology and Experimental Therapeutics, Vol. 306, pp. 387-393). More specifically, 150 µL of CFA diluted to 50% with physiological saline is administered into the sole of a rat's paw, thus inducing inflammation.

A compound of the present invention is orally administered to rats one day or one week after the administration of CFA. Thereby, a decrease in the threshold of pain is suppressed, that is, the effectiveness as a therapeutic agent for inflammatory pain is verified.

### (4) Effects of compounds on a rat model of neuropathic pain

A compound of the present invention is orally administered to rats in a Chung's model, a Seltzer's model, or a STZ-induced diabetic pain model. Thereby, a decrease in the threshold of pain is suppressed, that is, the effectiveness as a therapeutic agent for neuropathic pain is verified.

### (5) Safety test

When a compound of the present invention is orally administered to rats at a single dosage of 30 mg/kg, no rat dies and a remarkable abnormal behaviour of the rat is not observed. Thus, the safety of the present invention is verified.

### (6) hERG inhibitory test by patch-clamp method

An effect on hERG (a human ether-a-go-go related gene) channel is measured with fully-automated patch-clamp system(PatchXpress 7000A; molecular device). To confirm the hERG I_{Kr} current in the cell, a depolarization pulse is applied while membrane potential is hold at -80 mV. After the generated current is stabilized, a test compound is added to a perfusate. The effect of the test compound on the hERG channel is confirmed on the basis of the change in tail current induced by applying depolarization pulses having a voltage of -50 mV for 0.2 seconds and +20 mV for 5 seconds and subsequent repolarization pulse having a voltage of -50 mV for 5 seconds. The stimulus is given once every 12 seconds. The measurement is performed at room temperature. The hERG channel inhibitory activity is calculated as the ratio of the tail current 5 minutes after adding the test compound to the maximum tail current before addition of the test compound. Calculation of this inhibitory activity enables to estimate the induction of QT prolongation and subsequent fatal adverse events (ventricular tachycardia and sudden death and like) by drugs.

### (7)Pharmacokinetics

For example, after a single oral administration of a compound of the present invention to 5- or 6-week-old male SD rats, time-course of plasma concentration is studied. Bioavailability is high and the maximum plasma concentration (Cmax) and the area under the plasma concentration-time curve (AUC) increase almost in proportion to the doses, and the linear relationship between the dose and the plasma concentration is verified. Inhibitory effects on human drug-metabolizing enzymes are measured and verified. Moreover, using liver microsomes of humans, monkeys, dogs, and rats, metabolic stability is examined. Therefore, it is clarified whether a compound receives first pass effect in the liver or not.

### (8) Effects on rectal temperature

A test compound was orally administered to rats at single doses of 3, 10 and 30 mg/kg. Then rectal temperature was measured 30, 60 and 120 minutes after administration.

Effects on rectal temperature in rats were shown in Table 1A.

Effects on rectal temperature can be observed using various animals as appropriate other than rats. The examples of various animals include Rodents (e.g., hamsters, mice, guinea pigs), Insectivores (e.g., house musk shrews), Duplicidentatas (e.g., rabbits), Carnivora (e.g., dogs, ferrets, minks, cats), Perissodactyls (e.g., horses), Artiodactyls (e.g., pigs, cattle, goats, sheep), Primates (e.g., various monkeys, chimpanzees). Further, effects on body temperature can be observed with humans.
Compound A: 4-(3-trifluoromethylpyridine-2-yl)-N-(5-trifuluoromethylpiridine-2-yl)-1-piperadinecarboxamide
Compound B: (E)-3-(4-t-butylphenyl)-N-(2,3-dihydrobenzo[b][1,4]dioxine-6-yl)acrylamide
Compound C : N-(4-[6-(4-trifluoromethyl-phenyl)-pyrimidin-4-yloxy]-benzothiazol-2-yl)-acetamide(*)
   (*) : NEUROSCIENCE 2007
   Program#/Poster# : 400.9/0022
   Title: The capsaicin receptor TRPV1: Is it a pain transducer or a regulator of body temperature?
   Location: San Diego Contention Center: Halls B-H
   Presentation Start/End Time : Monday, Nov 05, 2007, 8:00 AM - 9:00 AM Authors : N. R. GAVVA;
Compound D : : (E)-2-(8-trifluoromethyl-3,4-dihydrobenzo[b]oxepin-5(2H)-ylidene)-N-(3-hydroxy-1,2,3,4-tetrahydroquinolin-5-yl)acetamide (EXAMPLE 68 described in WO2007/010383)

**[table 1A]**

| Compoundcompound | Change in rectal temperature in rats |
|---|---|
| EXAMPLE 10 | + |
| EXAMPLE 13 | - |
| EXAMPLE 14 | - |
| EXAMPLE 15 | - |
| EXAMPLE 23 | - |
| EXAMPLE 26 | - |
| EXAMPLE 30 | - |
| EXAMPLE 35 | - |
| EXAMPLE 93 | - |
| EXAMPLE 109 | - |
| EXAMPLE 110 | - |
| EXAMPLE 151 | - |
| EXAMPLE 200 | - |
| EXAMPLE 213 | - |
| Compound C | ++ (increased) |
| Compound D | ++ (increased) |
| Compound A | increased¹⁾ |
| Compound B | Increased¹⁾ |

| | |
|---|---|
| ¹⁾from published information (Non-Patent Document4 or 5) | |

Differences of the mean value between a group treated with test subsutance and a vehicle-treated group were calculated at all measuring points and, based on the maximum absolute value of differences, changes in rectal temperature were divided into the following three categories:
-: the maximum value was less than 0.5 degree Celsius
+: the maximum value was more than 0.5 degree but less than 1.0 degree Celsius
++: the maximum value was more than 1.0 degree Celsius

The above results show that the compound of the present invention had an antagonism to the TRPV1 receptor. Furthermore, an analgetic effect is observed in the inflammatory pain model and the neuropathic pain model in vitro. In addition, no particular effect is observed in the safety test, which demonstrated the low toxicity of the present invention.

Furthermore, preferable compounds of the present invention have high metabolic stability and satisfactory pharmacokinetics. In addition, these compounds have advantage in solubility and do not cause the rise of body temperature (in particular, the change in the body temperature is very little) by the dose of pharmaceutical activity.

Accordingly, the compound of the present invention serves as a TRPV1 receptor modulator, in particular, a TRPV1 receptor antagonist and is expected as a preventive or therapeutic agent for preventing or treating pain, in particular, as a preventive or therapeutic agent for preventing or treating inflammatory pain or neuropathic pain.

It is expected that the compound of the present invention has a promising effect of preventing or treating the above various diseases and conditions. More specifically, the compound of the present invention can be used for treating acute pain; chronic pain; neuropathic pain; postherpetic neuralgia; trigeminal neuralgia; lower-back pain; pain after spinal cord injury; leg pain; causalgia; diabetic neuralgia; pain caused by edema, burns, sprains, bone fractures, and the like; pain after surgical operations; scapulohumeral periarthritis; osteoarthritis; arthritis; rheumatic arthritis pain; inflammatory pain; cancer pain; migraines; headaches; toothaches; neuralgia; muscle pain; hyperalgesia; pain caused by angina pectoris, menstruation, and the like; neuropathy; nerve damage; neurodegeneration; chronic obstructive pulmonary disease (COPD); asthma; airway hypersensitivity; stridor; cough; rhinitis; inflammation of mucosa such as eyes; nervous dermatitis; inflammatory skin complaint such as psoriasis and eczema; edema; allergic diseases; gastroduodenal ulcer; ulcerative colitis; irritable colon syndrome; Crohn disease; urinary incontinence; urge urinary incontinence; overactive bladder; cystitis; nephritis; pancreatitis; uveitis; splanchnopathy; ischemia; apoplexy; dystonia; obesity; sepsis; pruritus; and diabetes. In particular, a promising effect for neuropathic pain, inflammatory pain, and urinary incontinence can be expected.

### [Formulation examples]

Examples of pharmaceutical compositions of the present invention will be described below.

**[Table 2]**

| Formulation example 1 Tablet | |
|---|---|
| Compound of Example 1 | 100 g |
| Lactose | 137 g |
| Crystalline cellulose | 30 g |
| Hydroxypropyl cellulose | 15 g |
| Sodium carboxymethyl starch | 15 g |
| Magnesium stearate | 3 g |

The above ingredients are weighed and then mixed homogeneously. The resulting mixture is compressed to prepare a tablet having a weight of 150 mg.

**[Table 3]**

| Formulation example 2 Film coating | |
|---|---|
| Hydroxypropylmethyl cellulose | 9 g |
| Macrogol 6000 | 1 g |
| Titanium oxide | 2 g |

The above ingredients are weighed. Hydroxypropylmethyl cellulose and Macrogol 6000 are then dissolved in water, and titanium oxide is dispersed in the solution. The resulting liquid is coated on the surfaces of 300 g of the tablets prepared in Formulation example 1 to form a film. Thus, film-coated tablets are obtained.

**[Table 4]**

| Formulation example 3 Capsule | |
|---|---|
| Compound of Example 7 | 50 g |
| Lactose | 435 g |
| Magnesium stearate | 15 g |

The above ingredients are weighed and then mixed homogeneously. Subsequently, 300 mg of the resulting mixture is filled in an appropriate hard capsule with a capsule enclosing device, thus allowing a capsule to be prepared.

**[Table 5]**

| Formulation example 4 Capsule | |
|---|---|
| Compound of Example 16 | 100 g |
| Lactose | 63 g |
| Corn starch | 25 g |
| Hydroxypropyl cellulose | 10 g |
| Talc | 2 g |

The above ingredients are weighed. The compound of Example 16, lactose, and corn starch are then mixed homogeneously, and an aqueous solution of hydroxypropyl cellulose is added to the mixture. Granules are produced by a wet granulation method. Talc is then homogeneously mixed with the granules. Subsequently, 200 mg of the resulting mixture is filled in an appropriate hard capsule, thus allowing a capsule to be prepared.

**[Table 6]**

| Formulation example 5 Powder | |
|---|---|
| Compound of Example 25 | 200 g |
| Lactose | 790 g |
| Magnesium stearate | 10 g |

The above ingredients are weighed and then mixed homogeneously. Thus, 20% powder medicine is prepared.

**[Table 7]**

| Formulation example 6 Granules and fine granules | |
|---|---|
| Compound of Example 38 | 100 g |
| Lactose | 200 g |
| Crystalline cellulose | 100 g |
| Partially α-converted starch | 50 g |
| Hydroxypropyl cellulose | 50 g |

The above ingredients are weighed. The compound of Example 38, lactose, crystalline cellulose, and partially α-converted starch are then homogeneously mixed, and an aqueous solution of hydroxypropyl cellulose (HPC) is added to the mixture. Granules or fine granules are produced by a wet granulation method. The granules or fine granules are dried, thus allowing a granular medicine or a fine granular medicine to be prepared.

**[Table 8]**

| Formulation example 7 Cream | |
|---|---|
| Compound of Example 43 | 0.5 g |
| dl-α-Tocopherol acetate | 0.1 g |
| Stearyl glycyrrhetinate | 0.05 g |
| Stearic acid | 3 g |
| Higher alcohol | 1 g |
| Squalane | 10 g |
| Octyldodecyl myristate | 3 g |
| Trimethylglycine | 7 g |
| Antiseptic | Proper quantity |
| Saponifier | Proper quantity |

The above ingredients are weighed. The compound of Example 43 is then mixed with other ingredients and dissolved. A proper amount of purified water is added so that the total weight reaches 50 g, thus allowing a cream formulation to be prepared.

**[Table 9]**

| Formulation example 8 Suppository | |
|---|---|
| Compound of Example 50 | 100 g |
| Polyethylene glycol 1500 | 180 g |
| Polyethylene glycol 4000 | 720 g |

The compound of Example 50 is sufficiently ground with a mortar to prepare a fine powder. The powder is then formed into a suppository having a weight of 1 g by a fusion method.

### [EXAMPLES]

The present invention will now be described in more detail using examples, but the present invention is not limited to the examples.

The measurement of nuclear magnetic resonance (NMR) spectrum was performed using a JEOL JNM-LA300 FT-NMR (manufactured by JEOL Ltd.) or a JEOL JNM-EX270 FT-NMR (manufactured by JEOL Ltd.). Liquid chromatography-mass spectrometry (LC-MS) was performed using a Waters FractionLynx MS system (manufactured by Waters Corporation). A SunFire column (4.6 mm × 5 cm, 5 µm) (manufactured by Waters Corporation) was used. Acetonitrile and a 0.05% aqueous acetic acid solution were used as the mobile phase. The analysis was performed under the following gradient conditions: acetonitrile:0.05% aqueous acetic acid solution = 1:9 (0 minutes), 9:1 (5 minutes), and 9:1 (7 minutes).

### (EXAMPLE 1)

Synthesis of (E)-2-(8-trifluoromethyl-3,4-dihydrobenzo[b]oxepin-5(2H)-ylidene)-N-(2,2-dimethyl-4H-benzo [1,4]oxazin-3-on-6-yl)acetamide <Step 1> Synthesis of 2-iodo-5-trifluoromethylphenol

A toluene (200.0 mL) solution of 3-trifluoromethylphenol (16.6 g) was added dropwise to a toluene (300.0 mL) suspension of sodium hydride (7.1 g) under ice cooling. The reaction solution was stirred at the same temperature for 30 minutes, and iodine (26.0 g) was then added thereto. The solution was stirred at room temperature for 12 hours. Subsequently, 3 N hydrochloric acid was added to the solution so that the pH of the solution was adjusted to 2. The solution was extracted with ethyl acetate. The organic layer was sequentially washed with water and a saturated saline solution and then dried over anhydrous sodium sulfate. The solvent was distilled off under reduced pressure. The title crude compound (30.8 g) was obtained as pale yellow oil. <Step 2> Synthesis of 3-(5-methoxycarbonyl-4-penten)oxy-4-iodo-trifluoromethylbenzene

Potassium carbonate (52.8 mg), 6-bromo-2-hexenoic acid methyl ester (57.5 mg), and 18-crown ether-6 (a catalitic amount) were added to an N,N-dimethylformamide (10.0 mL) solution of the compound (100.0 mg) prepared in <Step 1> of Example 1. The reaction solution was stirred at room temperature for 12 hours. Water was added to the solution, and the solution was then extracted with ethyl acetate. The organic layer was sequentially washed with water and a saturated saline solution and then dried over anhydrous sodium sulfate. The solvent was distilled off under reduced pressure. The title crude compound (66.0 mg) was obtained as colorless oil.

### <Step 3> Synthesis of methyl (E)-(8-trifluoromethyl-3,4-dihydro-2H-benzo[b]oxepin-5-ylidene)acetate

Palladium acetate (3.7 mg), triphenylphosphine (8.6 mg), and silver carbonate (45.0 mg) were added to a tetrahydrofuran (1.0 mL) solution of the compound (65.0 mg) prepared in <Step 2> of Example 1. The reaction solution was refluxed under heating for eight hours in a nitrogen stream. The reaction solution was subjected to Celite filtration. Water was then added to the solution, and the solution was extracted with ethyl acetate. The organic layer was sequentially washed with water and a saturated saline solution and then dried over anhydrous sodium sulfate. The solvent was distilled off under reduced pressure. The title compound (47.0 mg) was obtained as colorless crystals.

### <Step 4> Synthesis of (E)-(8-trifluoromethyl-3,4-dihydro-2H-benzo[b]oxepin-5-ylidene)acetic acid

Water (1.0 mL) and lithium hydroxide (33.5 mg) were added to a tetrahydrofuran (5.0 mL) solution of the compound (160.0 mg) prepared in <Step 3> of Example 1, and the reaction solution was then refluxed under heating for six hours. The solvent was distilled off under reduced pressure. The reaction solution was then neutralized with 1 N hydrochloric acid and was extracted with ethyl acetate. The organic layer was washed with a saturated saline solution and then dried over anhydrous sodium sulfate. The solvent was then distilled off under reduced pressure. Ethyl acetate was added to the residue to solidify the resulting product. The title compound (120.0 mg) was obtained as colorless crystals.

### <Step 5> Synthesis of 2,2-dimethyl-6-nitro-4H-benzo[1,4]oxazin-3-one

Sodium carbonate (2.75 g) and chloroform (10.0 mL) solution of 2-bromoisobutyryl bromide (2.24 g) were added to a chloroform (40.0 mL) solution of 2-amino-4-nitrophenol (1.0 g) under ice cooling. The reaction solution was stirred at same temperature to room temperature overnight. The reaction mixture was filtered, and the solvent was then distilled off under reduced pressure. The residue was dissolved in N,N-dimethylformamide (50.0 mL), and sodium carbonate (1.03 g) was added to the solution, then stirred under heating at 80 °C for 2 hours. The mixture was left to cool, water was then added to the mixture, and the mixture was extracted with ethyl acetate. The organic layer was washed with saturated saline solution, and then dried over anhydrous sodium sulfate. The solvent was then distilled off under reduced pressure. The residue was purified by silica gel column chromatography (eluate; n-hexane:ethyl acetate = 100:0 to 70:30). The title compound (0.98 g) was obtained as a pale brown solid.

### <Step 6> Synthesis of 6-amino-2,2-dimethyl-4H-benzo[1,4]oxazin-3-one

10%Pd-C (100 mg) was added to tetrahydrofuran:methanol=1:1 (50 mL) solution of the compound (500.0 mg) prepared in <Step 5> of Example 1 was stirred under hydrogen atmosphere at room temperature overnight. The reaction mixture was subjected to Celite filtration. The solvent was then distilled off under reduced pressure. n-Hexane and diethyl ether were added to the residue to solidify the resulting product. The title compound (380.0 mg) was obtained as a pale brown solid.

### <Step 7>

Synthesis of (E)-2-(8-trifluoromethyl-3,4-dihydrobenzo[b]oxepin-5(2H)-ylidene)-N-(2,2-dimethyl-4H-benzo[1,4]oxazin-3-on-6-yl)acetamide

Oxalyl chloride (0.07 mL) and N,N-dimethylformamide (one drop) were added to a methylene chloride (5.0 mL) solution of the compound (110.0 mg) prepared in <step 4> of Example 1. The mixture was stirred at room temperature for 2 hours. The solvent was then distilled off under reduced pressure. A methylene chloride (5.0 mL) and pyridine (0.1 mL) solution of the compound prepared in <step 6> of Example 1 was added dropwise to the residue which was dissolved in methylene chloride (2.0 mL), and then stirred at room temperature for 2 hours. The reaction solution was neutralized with 1 N hydrochloric acid and was extracted with ethyl acetate. The organic layer was washed with a saturated saline solution and then dried over anhydrous sodium sulfate. The solvent was distilled off under reduced pressure. The residue was purified by silica gel column chromatography (eluate; n-hexane:ethyl acetate = 100:0 to 50:50). The title compound (100.0 mg) was obtained as a white solid.

### (EXAMPLE 2)

Synthesis of (E)-2-(8-trifluoromethyl-3,4-dihydrobenzo[b]oxepin-5(2H)-ylidene)-N-(2-methyl-4H-benzo [1,4]oxazin-3-on-6-yl)acetamide

### <Step 1> Synthesis of 2-methyl-6-nitro-4H-benzo [1,4]oxazin-3-one

The title compound (28.0 g) was obtained as a white solid from 2-amino-4-nitrophenol (20.0 g) and diethyl 2-bromo-2-methylmalonate (6.2 mL) by the same process as that used in <Step 5> of Example 1.

### <Step 2> Synthesis of 6-amino-2-methyl-4H-benzo[1,4]oxazin-3-one

The title compound (420.0 mg) was obtained as a pale brown solid from the compound (500.0 mg) prepared in <Step 1> of Example 2 by the same process as that used in <Step 6> of Example 1.

### <Step 3> Synthesis of (E)-2-(8-trifluoromethyl-3,4-dihydrobenzo[b]oxepin-5(2H)-ylidene)-N-(2-methyl-4H-benzo [1,4]oxazin-3-on-5-yl)acetamide

The title compound (140.8 mg) was obtained as a white solid from the compound (140.0 mg) prepared in <Step 2> of Example 2 by the same process as that used in <Step 7> of Example 1.

### (EXAMPLE 3)

Synthesis of (E)-2-(8-trifluoromethyl-3,4-dihydrobenzo[b]oxepin-5(2H)-ylidene)-N-(2-(2-hydroxyethyl)-4H-benzo[1,4]oxazin-3-on-6-yl)acetamide

### <Step 1> Synthesis of 2-(2-hydroxyethyl)-6-nitro-4H-benzo [1,4]oxazin-3-one

The title compound (18.0 g) was obtained as a pale brown solid from 2-amino-4-nitrophenol (20.0 g) and α-bromo-γ-butyrolactone (23.6 g) by the same process as that used in <Step 5> of Example 1.

### <Step 2> Synthesis of 6-amino-2-(2-hydroxyethyl)-4H-benzo[1,4]oxazin-3-one

The title compound (2.5 g) was obtained as a white solid from the compound (3.0 g) prepared in <Step 1> of Example 3 by the same process as that used in <Step 6> of Example 1.

### <Step 3> Synthesis of (E)-2-(8-trifluoromethyl-3,4-dihydrobenzo[b]oxepin-5(2H)-ylidene)-N-(2-(2-hydroxyethyl)-4H-benzo[1,4]oxazin-3-on-6-yl)acetamide

The title compound (13.0 mg) was obtained as a white solid from the compound (50.0 mg) prepared in <Step 2> of Example 3 by the same process as that used in <Step 7> of Example 1.

### (EXAMPLE 4)

Synthesis of (E)-2-(8-trifluoromethyl-3,4-dihydrobenzo[b]oxepin-5(2H)-ylidene)-N-(2H-benzo [1,4]thiazin-3(4H)-on-6-yl)acetamide <Step 1> Synthesis of (2,4-dinitrophenyl)thioacetic acid ethyl ester

Mercaptoacetic acid ethyl ester (5.0 g) and triethylamine (5.3 mL) were added to a tetrahydrofuran solution of 2,4-dinitrofluorobenzene (5.5 mL) and stirred at room temperature for 5 hours. Ice water was added to the reaction solution and extracted with ethyl acetate. The organic layer was sequentially washed with water and a saturated saline solution and then dried over anhydrous sodium sulfate. The solvent was distilled off under reduced pressure. The residue was purified by silica gel column chromatography (eluate; n-hexane:ethyl acetate = 100:0 to 50:50). The title compound (5.0 g) was obtained as a yellow solid.

### <Step 2> Synthesis of 6-amino-4-2H-benzo [1,4]thiazin-3(4H)-on

Ethyl acetate (20.0 mL) and acetic acid (20.0 mL) solution of the compound (5.0 g) prepared from <step 1> in example 4 was added to water (20.0 mL) and acetic acid (1.0 mL) suspension of iron powder (13.0 g) and then stirred under heating at 80 °C for 4 hours. The mixture was left to cool. The mixture was filtered and extracted with ethyl acetate. The organic layer was sequentially washed with water, aqueous sodium hydrogen carbonate solution and a saturated saline solution, and dried over anhydrous sodium sulfate. The solvent was distilled off under reduced pressure. Diethyl ether was added to the residue to solidify the resulting product. The title compound (2.1 g) was obtained as a pale brown solid.

### <Step 3>

Synthesis of (E)-2-(8-trifluoromethyl-3,4-dihydrobenzo[b]oxepin-5(2H)-ylidene)-N-(2H-benzo[b][1,4]thiazin-3(4H)-on-6-yl)acetamide

The title compound (440.0 mg) was obtained as a pale yellowish-white solid from the compound (300.0 mg) prepared in <Step 2> of Example 4 by the same process as that used in <Step 7> of Example 1.

### (EXAMPLE 5)

Synthesis of (E)-2-(8-trifluoromethyl-3,4-dihydrobenzo[b]oxepin-5(2H)-ylidene)-N-(1-oxo-2H-benzo[1,4]thiazin-3(4H)-on-6-yl)acetamide

m-Chloroperbenzoic acid (39.7 mg) was added to the methylene chloride (5.0 mL) solution of the compound (100.0 mg) prepared from <step 3> in example 4, and the mixture was stirred. After consumption of starting compound, aqueous sodium sulfite solution was added to the mixture and extracted with ethyl acetate. The organic layer was sequentially washed with aqueous sodium sulfite solution and saturated saline solution, and dried over anhydrous sodium sulfate. The solvent was distilled off under reduced pressure. Diethyl ether was added to the residue to solidify the resulting product. The title compound (42.0 mg) was obtained as a pale yellowish-white solid.

### (EXAMPLE 6)

Synthesis of (E)-2-(8-trifluoromethyl-3,4-dihydrobenzo[b]oxepin-5(2H)-ylidene)-N-(sulfazon-6-yl)acetamide

m-Chloroperbenzoic acid (127.1 mg) was added to the methylene chloride (5.0 mL) solution of the compound (100.0 mg) prepared from <step 3> in example 4, and the mixture was stirred at room temperature overnight. Aqueous sodium sulfite solution was added to the mixture, and the mixture was extracted with ethyl acetate. The organic layer was sequentially washed with aqueous sodium sulfite solution and saturated saline solution, and dried over anhydrous sodium sulfate. The solvent was distilled off under reduced pressure. Diethyl ether was added to the residue to solidify the resulting product. The title compound (42.0 mg) was obtained as a pale yellowish-white solid.

### (EXAMPLE 7)

Synthesis of (E)-2-(8-trifluoromethyl-3,4-dihydrobenzo[b]oxepin-5(2H)-ylidene)-N-(3,4-dihydro-2(1H)-quinoxalinon-7-yl)acetamide <Step 1 > Synthesis of 2,4-dinitroanilinoacetic acid ethyl ester

Sodium hydrogen carbonate (4.15 g) and glycine ethyl ester hydrochloride (3.79 g) were added to aqueous ethanol (100.0 mL) solution of 2,4-dinitrochlorobenzene (5.0 g), and refluxed for 4.5 hours. The mixture was left to cool. The solvents were distilled off under reduced pressure. The residue was extracted with ethyl acetate. The organic layer was sequentially washed with water and saturated saline solution, and dried over anhydrous sodium sulfate. The solvent was distilled off under reduced pressure. The residue was purified by silica gel column chromatography (eluate; n-hexane:ethyl acetate = 100:0 to 85:15). The title compound (3.2 g) was obtained as a yellow solid.

### <Step 2> Synthesis of 7-amino-3,4-dihydro-2(1H)-quinoxalinone hydrochloride

The title compound (260.0 mg) was obtained as a brown solid from the compound (300.0 mg) prepared in <Step 1> of Example 7 by a process similar to the process used in <Step 6> of Example 1.

### <Step 3> Synthesis of (E)-2-(8-trifluoromethyl-3,4-dihydrobenzo[b]oxepin-5(2H)-ylidene)-N-(3,4-dihydro-2(1H)-quinoxalinon-7-yl)acetamide

The title compound (25.0 mg) was obtained as a white solid from the compound (50.0 mg) prepared in <Step 2> of Example 7 by the same process as that used in <Step 7> of Example 1.

### (EXAMPLE 8)

Synthesis of (E)-2-(8-trifluoromethyl-3,4-dihydrobenzo[b]oxepin-5(2H)-ylidene)-N-(4-methyl-3,4-dihydro-2(1H)- quinoxalinon-7-yl)acetamide

Formalin (11.4 mg) was added to a water solution (0.5 mL) of sulfuric acid (0.18 g) under ice cooling. The compound (30.0 mg) prepared in <step 3> of Example 7 and tetrahydrofuran solution of sodium borohydride (13.6 mg) were added dropwise to the mixture at the same temperature and the mixture was stirred at same temperature for 5 minutes. Water was added to the mixture, the mixture was extracted with ethyl acetate. The organic layer was sequentially washed with water and saturated saline solution, and dried over anhydrous sodium sulfate. The solvent was distilled off under reduced pressure. Diethyl ether was added to the residue to solidify the resulting product. The title compound (21.0 mg) was obtained as a pale yellowish-white solid.

### (EXAMPLE 9)

Synthesis of (E)-2-(8-trifluoromethyl-3,4-dihydrobenzo[b]oxepin-5(2H)-ylidene)-N-(3-hydroxymethyl-3,4-dihydro-2(1H)-quinoxalinon-7-yl)acetamide

### <Step 1> Synthesis of 2,4-dinitroanilino-(2-hydroxymethyl)acetic acid methyl ester

The title compound (1.0 g) was obtained as a yellow solid from 2,4-dinitrofluorobenzene (1.0 g) and (DL)-serine methyl ester hydrochloride (0.84 g) by a process similar to the process used in <Step 1> of Example 7.

### <Step 2> Synthesis of 7-amino-3,4-dihydro-3-hydroxymethyl-2(1H)-quinoxalinon hydrochloride

The title compound (100.0 mg) was obtained as a black solid from the compound (200.0 mg) prepared in <Step 1> of Example 9 by a process similar to the process used in <Step 6> of Example 1.

### <Step 3> Synthesis of (E)-2-(8-trifluoromethyl-3,4-dihydrobenzo[b]oxepin-5(2H)-ylidene)-N-(3-hydroxymethyl-3,4-dihydro-2(1H)-quinoxalinon-7-yl)acetamide

The title compound (5.0 mg) was obtained as a pale brown solid from the compound (110.0 mg) prepared in <Step 2> of Example 9 by a process similar to the process used in <Step 7> of Example 1.

### (EXAMPLE 10)

Synthesis of (E)-2-(8-trifluoromethyl-3,4-dihydrobenzo[b]oxepin-5(2H)-ylidene)-N-(3,3-dimethyl-3,4-dihydro-2(1H)-quinoxalinon-7-yl)acetamide

### <Step 1> Synthesis of N-(2,4-dinitrophenyl)-2-methyl-alanine methyl ester

The title compound (1.37 g) was obtained as a yellow solid from 2,4-dinitrofluorobenzene (1.0 g) and 2-methyl-alanine methyl ester hydrochloride (0.83 g) by a process similar to the process used in <Step 1> of Example 7.

### <Step 2> Synthesis of 7-amino-3,4-dihydro-3,3-dimethyl-2(1H)-quinoxalinone

The title compound (470.0 mg) was obtained as a brown solid from the compound (500.0 mg) prepared in <Step 1> of Example 10 by the same process as that used in <Step 6> of Example 1.

### <Step 3> Synthesis of (E)-2-(8-trifluoromethyl-3,4-dihydrobenzo[b]oxepin-5(2H)-ylidene)-N-(3,3-dimethyl-3,4-dihydro-2(1H)-quinoxalinon-7-yl)acetamide

The title compound (120.0 mg) was obtained as a pale yellow solid from the compound (340.0 mg) prepared in <Step 2> of Example 10 by the same process as that used in <Step 7> of Example 1.

### (EXAMPLE 11)

Synthesis of (E)-2-(8-trifluoromethyl-3,4-dihydrobenzo[b]oxepin-5(2H)-ylidene)-N-(3,3-dimethyl-4-methyl-3,4-dihydro-2(1H)-quinoxalinon-7-yl)acetamide

The title compound (10.0 mg) was obtained as a pale yellowish-white solid from the compound (32.0 mg) prepared in <Step 3> of Example 10 by the same process as that used in Example 8.

### (EXAMPLE 12)

Synthesis of (E)-2-(8-trifluoromethyl-3,4-dihydrobenzo[b]oxepin-5(2H)-ylidene)-N-(1,4-dihydro-2H-3,1-benzoxazin-2-on-7-yl)acetamde <Step 1> Synthesis of 7-nitro-1,4-dihydro-2H-3,1-benzoxazin-2-one

Sodium hydride (0.9 g) and carbonyldiimidazole(1.8 g) were added to a tetrahydrofuran (50.0 mL) solution of 2-amino-4-nitrobenzyl alcohol under ice cooling, and refluxed for 6 hours. The mixture was left to cool. Aqueous saturated ammonium chloride solution was added to the mixture, and the mixture was extracted with ethyl acetate. The organic layer was sequentially washed with water and saturated saline solution, and dried over anhydrous sodium sulfate. The solvent was distilled off under reduced pressure. The residue was purified by silica gel column chromatography (eluate; n-hexane:ethyl acetate = 100:0 to 50:50). The title compound (1.2 g) was obtained as a white solid.

### <Step 2> Synthesis of 7-amino-1,4-dihydro-2H-3,1-benzoxadin-2-one

The title compound (39.3 mg) was obtained as a white solid from the compound (100.0 mg) prepared in <Step 1> of Example 12 by the same process as that used in <Step 6> of Example 1.

### <Step 3> Synthesis of (E)-2-(8-trifluoromethyl-3,4-dihydrobenzo[b]oxepin-5(2H)-ylidene)-N-(1,4-dihydro-2H-3,1-benzoxazin-2-on-7-yl)acetamide

The title compound (20.0 mg) was obtained as a white solid from the compound (28.0 mg) prepared in <Step 2> of Example 12 by the same process as that used in <Step 7> of Example 1.

### (EXAMPLE 13)

Synthesis of (E)-2-(8-trifluoromethyl-3,4-dihydrobenzo[b]oxepin-5(2H)-ylidene)-N-(3,4-dihydro-1H-quinazolin-2-on-7-yl)acetamide

### <Step 1> Synthesis of 2-amino-4-nitrobenzylamine

Borane-tetrahydrofuran complex (1.0 M solution of tetrahydrofuran) (2.2 mL)was added to a tetrahydrofuran (6.0 mL) solution of 2-amino-4-nitrobenzamide (100.0 mg) and refluxed for 2 hours. The mixture was left to cool. Methanol was then added to the mixture and neutralized with 10% hydrogen chloride in methanol. The solvents were distilled off under reduced pressure. A solution of 1 N aqueous sodium hydroxide solution was added to the residue and was extracted with methylene chloride. The organic layer was washed with saturated saline solution, and dried over anhydrous sodium sodium sulfate. The solvent was distilled off under reduced pressure. The title crude compound (92.1 mg) was obtained as an orange solid.

### <Step 2> Synthesis of 7-nitro-3,4-dihydro-1H-quinazolin-2-one

The title compound (75.4 mg) was obtained as a yellow solid from the compound (80.0 mg) prepared in <Step 1> of Example 13 by a process similar to the process used in <Step 1> of Example 12.

### <Step 3> Synthesis of 7-amino-3,4-dihydro-1H-quinazolin-2-one

The title compound (44.8 mg) was obtained as a pale brown solid from the compound (50.0 mg) prepared in <Step 2> of Example 13 by the same process as that used in <Step 6> of Example 1.

### <Step 4> Synthesis of (E)-2-(8-trifluoromethyl-3,4-dihydrobenzo[b]oxepin-5(2H)-ylidene)-N-(3,4-dihydro-1H-quinazolin-2-on-7-yl)acetamide

The title compound (56.2 mg) was obtained as a white solid from the compound (40.0 mg) prepared in <Step 3> of Example 13 by a process similar to the process used in <Step 7> of Example 1.

### (EXAMPLE 14)

### Synthesis of (E)-2-(8-trifluoromethyl-3,4-dihydrobenzo[b]oxepin-5(2H)-ylidene)-N-(3-methyl-3,4-dihydro-2(1H)-quinazolinon-7-yl) acetamide <Step 1> Synthesis of 2-amino-4-nitrobenzaldehyde

Manganese dioxide (1.0 g) was added to a methylene chloride (30.0 mL) solution of 2-amino-4-nitrobenzyl alcohol (500.0 mg), and was stirred at room temperature for 2 hours. The reaction mixture was subjected to Celite filtration. The solvent was then distilled off under reduced pressure. The title crude compound (456.0 mg) was obtained as a reddish-orange solid.

### <Step 2> Synthesis of 2-amino-4-nitro-N-methylbenzylamine

Methylamine (10 M solution of methanol) (0.6 mL) was added to a methanol (1.0 mL) solution of the compound (100.0 mg) prepared in <Step 1> of Example 14, and the reaction mixture was stirred at room temperature overnight. Sodium borohydride (22.7 mg) was added to the mixture under ice cooling, and the mixture was stirred at room temperature for 3 hours. The solvent was then distilled off under reduced pressure. 1 N aqueous sodium hydroxide solution was added to the mixture, the mixture was extracted with ethyl acetate. The Organic layer was washed with saturated saline solution and then dried over anhydrous sodium sulfate. The solvent was then distilled off under reduced pressure. The title compound (123.0 mg) was obtained as brown oil.

### <Step 3> Synthesis of 3-methyl-7-nitro-3,4-dihydro-2(1H)-quinazolinone

The title compound (40.0 mg) was obtained as a yellow solid from the compound (110.0 mg) prepared in <Step 2> of Example 14 by a process similar to the process used in <Step 1> of Example 12.

### <Step 4> Synthesis of 7-amino-3-methyl-3,4-dihydro-2(1H)-quinazolinone

The title compound (34.0 mg) was obtained as a white solid from the compound (50.0 mg) prepared in <Step 3> of Example 14 by the same process as that used in <Step 6> of Example 1.

### <Step 5> Synthesis of (E)-2-(8-trifluoromethyl-3,4-dihydrobenzo[b]oxepin-5(2H)-ylidene)-N-(3-methyl-3,4-dihydro-2(1H)-quinazolinon-7-yl)acetamide

The title compound (52.5 mg) was obtained as a white solid from the compound (30.0 mg) prepared in <Step 4> of Example 14 by a process similar to the process used in <Step 7> of Example 1.

### (EXAMPLE 15)

Synthesis of (E)-2-(8-trifluoromethyl-3,4-dihydrobenzo[b]oxepin-5(2H)-ylidene)-N-(3- (2-hydroxyehtyl)-3,4-dihydro-2(1H)-quinazolinon-7-yl)acetamide

### <Step 1> Synthesis of 2-amino-4-nitro-N-(2-hydroxyethyl)benzylamine

The title compound (112.0 mg) was obtained as a yellow solid from 2-hydroxyethylamine (72.1 µL) by the same process as that used in <Step 2> of Example 14.

### <Step 2> Synthesis of 2-amino-4-nitro-N-(2-tert-butyldimethylsiloxyethyl)benzylamine

tert-butyldimethylsilyl chloride (110.0 mg), imidazole (96.7 mg) and 4-dimethylaminopyridine (5.8 mg) were added to a N,N-dimethyformamide (5.0 mL) solution of the compound (100.0 mg) prepared in <Step 1> of Example 15, and the mixture was stirred at room temperature overnight. Water was added to the mixture and the mixture was extracted with ethyl acetate. The organic layer was washed with saturated saline solution and then dried over anhydrous sodium sulfate. The solvent was then distilled off under reduced pressure. The residue was purified by silica gel column chromatography (eluate; methylene chloride:methanol = 100:0 to 95:5). The title compound (145.0 mg) was obtained as yellow amorphous.

### <Step 3> Synthesis of 3-(2-tert-butyldimethylsiloxyethyl)-7-nitro-3,4-dihydro-2(1H)-quinazolinone

The title compound (252.0 mg) was obtained as a yellow solid from the compound (500.0 mg) prepared in <Step 2> of Example 15 by a process similar to the process used in <Step 1> of Example 12.

### <Step 4> Synthesis of 7-amino-3-(2-tert-butyldimethylsiloxyethyl)-3,4-dihydro-2(1H)-quinazolinone

The title compound (191.0 mg) was obtained as a white solid from the compound (190.0 mg) prepared in <Step 3> of Example 15 by the same process as that used in <Step 6> of Example 1.

### <Step 5> Synthesis of (E)-2-(8-trifluoromethyl-3,4-dihydrobenzo[b]oxepin-5(2H)-ylidene)-N-(3 -(2-tert-butyldimethylsiloxyehtyl)-3,4-dihydro-2(1H)-quinazolinon-7-yl)acetamide

The title compound (174.0 mg) was obtained as a white solid from the compound (180.0 mg) prepared in <Step 4> of Example 15 by a process similar to the process used in <Step 7> of Example 1.

### <Step 6> Synthesis of (E)-2-(8-trifluoromethyl-3,4-dihydrobenzo[b]oxepin-5(2H)-ylidene)-N-(3-(2-hydroxyehtyl)-3,4-dihydro-2(1H)-quinazolinon-7-yl)acetamide

The title compound (50.0 mg) was obtained as a white solid from deprotection of the compound (100.0 mg) prepared in <Step 5> of Example 15 by using acid catalyst.

### (EXAMPLE 16)

Synthesis of (E)-2-(8-trifluoromethyl-3,4-dihydrobenzo[b]oxepin-5(2H)-ylidene)-N-(3- (2-methoxyehtyl)-3,4-dihydro-2(1H)-quinazolinon-7-yl)acetamide

### <Step 1> Synthesis of 2-amino-4-nitro-N-(2-methoxyethyl)benzylamine

The title compound (391.0 mg) was obtained as a yellow oil from 2-methoxyethylamine (0.31 mL) by the same process as that used in <Step 2> of Example 14.

### <Step 2> Synthesis of 3-(2-methoxyethyl)-7-nitro-3,4-dihydro-2(1H)-quinazolinone

The title compound (105.0 mg) was obtained as a yellow solid from the compound (200.0 mg) prepared in <Step 1> of Example 16 by a process similar to the process used in <Step 1> of Example 12.

### <Step 3> Synthesis of 7-amino-3-(2-methoxyethyl)- 3,4-dihydro-2(1H)-quinazolinone

The title compound (63.0 mg) was obtained as a pale green solid from the compound (86.0 mg) prepared in <Step 2> of Example 16 by the same process as that used in <Step 6> of Example 1.

### <Step 4> Synthesis of (E)-2-(8-trifluoromethyl-3,4-dihydrobenzo[b]oxepin-5(2H)-ylidene)-N-(3- (2-methoxyehtyl)-3,4-dihydro-2(1H)-quinazolinon-7-yl)acetamide

The title compound (66.0 mg) was obtained as a pale yellow solid from the compound (56.0 mg) prepared in <Step 3> of Example 16 by a process similar to the process used in <Step 7> of Example 1.

### (EXAMPLE 17)

Synthesis of (E)-2-(8-trifluoromethyl-3,4-dihydrobenzo[b]oxepin-5(2H)-ylidene)-N-(3,4-dihydro-2,2-dioxo-1H-2,1,3-benzothiazin-7-yl)acetamide

### <Step 1> Synthesis of 7-nitro-3,4-dihydro-2,2-dioxo-1H-2,1,3-benzothiadiazin

Sulfamide (170.0 mg) was added to a pyridine (6.0 mL) solution of the compound (100.0 mg) prepared in <Step 1> of Example 13, and the mixture was refluxed for 6 hours. The mixture was left to cool. Water was then added to the mixture and the mixture was extracted with ethyl acetate. The organic layer was washed with saturated saline solution and then dried over anhydrous sodium sulfate. The solvent was then distilled off under reduced pressure. The title compound (120.0 mg) was obtained as brown solid.

### <Step 2> Synthesis of 7-amino-3,4-dihydro-2,2-dioxo-1H-2,1,3-benzothiadiazine

The title compound (57.4 mg) was obtained as a black solid from the compound (75.0 mg) prepared in <Step 1> of Example 17 by the same process as that used in <Step 6> of Example 1.

### <Step 3> Synthesis of (E)-2-(8-trifluoromethyl-3,4-dihydrobenzo[b]oxepin-5(2H)-ylidene)-N-(3,4-dihydro-2,2-dioxo-1H-2,1,3-benzothiadiazin-7-yl)acetamide

The title compound (61.0 mg) was obtained as a white solid from the compound (50.0 mg) prepared in <Step 2> of Example 17 by a process similar to the process used in <Step 7> of Example 1.

### (Example 18)

Synthesis of (E)-2-(8-trifluoromethyl-3,4-dihydrobenzo[b]oxepin-5(2H)-ylidene)-N-(3,4-dihydro-2(1H)-quinolinon-5-yl)acetamide

The title compound (53.8 mg) was obtained as a pale yellow amorphous from 5-amino-3,4-dihydro-2(1H)-quinolinone (60.0 mg) by a process similar to the process used in <Step 7> of Example 1.

### (EXAMPLE 19)

Synthesis of (E)-2-(8-trifluoromethyl-3,4-dihydrobenzo[b]oxepin-5(2H-ylidene)-N-(1-(2-hydroxyethyl)-3,4-dihydro-2(1H)-quinolinon-5-yl)acetamide

### <Step 1> Synthesis of 5-amino-1-(2-tert-butyldimethylsiloxyethyl)-3,4-dihydro-2(1H)-quinolinone

The title compound (24.0 mg) was obtained as pale yellow amorphous from 1-(2-tert-butyldimethylsiloxyethyl)-5-nitro-3,4-dihydro-2(1H)-quinolinone (40.0 mg) by the same process as that used in <Step 6> of Example 1.

### <Step 2> Synthesis of (E)-2-(8-trifluoromethyl-3,4-dihydrobenzo[b]oxepin-5(2H)-ylidene)-N-(1-(2-tert-butyldimethylsiloxyethyl)-3,4-dihydro-2(1H)-quinolinon-5-yl)acetamide

The title compound (27.0 mg) was obtained as a white amorphous from the crude compound (24.0 mg) prepared in <Step 1> of Example 19 by a process similar to the process used in <Step 7> of Example 1.

### <Step 3> Synthesis of (E)-2-(8-trifluoromethyl-3,4-dihydrobenzo[b]oxepin-5(2H)-ylidene)-N-(1-(2-hydroxyethyl)-3,4-dihydro-2(1H)-quinolinon-5-yl)acetamide

The title compound (10.0 mg) was obtained as a white amorphous from the crude compound (27.0 mg) prepared in <Step 2> of Example 19 by the same process as that used in <Step 6> of Example 15.

### (EXAMPLE 20)

Synthesis of (E)-2-(8-trifluoromethyl-3,4-dihydrobenzo[b]oxepin-5(2H)-ylidene)-N-(2H-1,4-benzoxazin-3(4H)-on-8-yl)acetamide

### <Step 1> Synthesis of 2,6-dinitrophenoxyacetic acid ethyl ester

The title compound (150.0 mg) was obtained as a yellow amorphous from 2,6-dinitrochlorobenzene (2.0 g) and glycolic acid ethyl ester (1.12 mL) by a process similar to the process used in <Step 1> of Example 7.

### <Step 2> Synthesis of 8-amino-2H-1,4-benzoxadin-3(4H)-on

The title compound (43.0 mg) was obtained as a yellow solid from the compound (150.0 mg) prepared in <Step 1> of Example 20 by the same process as that used in <Step 6> of Example 1.

### <Step 3> Synthesis of (E)-2-(8-trifluoromethyl-3,4-dihydrobenzo[b]oxepin-5(2H)-ylidene)-N-(2H-1,4-benzoxazin-3(4H)-on-8-yl)acetamide

The title compound (50.0 mg) was obtained as a pale yellow solid from the compound (43.0 mg) prepared in <Step 2> of Example 20 by a process similar to the process used in <Step 7> of Example 1.

### (EXAMPLE 21)

Synthesis of (E)-2-(8-trifluoromethyl-3,4-dihydrobenzo[b]oxepin-5(2H)-ylidene)-N-(3,4-dihydro-2 (1H)-quinoxalinon-5-yl)acetamide

### <Step 1> Synthesis of N-(2,6-dinitrophenyl)-glycine ethyl ester

The title compound (180.0 mg) was obtained as a yellow solid from 2,6-dinitrochlorobenzene (200.0 mg) and glycine ethyl ester hydrochloride (150.0 mg) by a process similar to the process used in <Step 1> of Example 7.

### <Step 2> Synthesis of 5-amino-3,4-dihydro-2 (1H)-quinoxalinone hydrochloride

The title compound (120.0 mg) was obtained as a brown solid from the compound (180.0 mg) prepared in <Step 1> of Example 21 by the same process as that used in <Step 6> of Example 1.

### <Step 3> Synthesis of (E)-2-(8-trifluoromethyl-3,4-dihydrobenzo[b]oxepin-5(2H)-ylidene)-N-(3,4-dihydro-2 (1H)-quinoxalinon-5-yl)acetamide

The title compound (89.0 mg) was obtained as a pale yellow solid from the compound (120.0 mg) prepared in <Step 2> of Example 21 by a process similar to the process used in <Step 7> of Example 1.

### (EXAMPLE 22)

Synthesis of (E)-2-(8-trifluoromethyl-3,4-dihydrobenzo[b]oxepin-5(2H)-ylidene)-N-(3,4-dihydro-4-methyl-2 (1H)-quinoxalinon-5-yl)acetamide

The title compound (19.0 mg) was obtained as a pale yellow solid from the compound (30.0 mg) prepared in <Step 3> of Example 21 by the same process as that used in Example 8.

### (EXAMPLE 23)

Synthesis of (E)-2-(7-trifluoromethylchroman-4-ylidene)-N-(3,4-dihydroquinolin-2(1H)-on-7-yl)acetamide
<Step 1> Synthesis of 3-(3-trifluoromethylphenoxy)propionic acid Sodium hydride (550.0 mg) was added to an N,N-dimethylformamide (20.0 mL) solution of 3-hydroxybenzotrifluoride (2.0 g), and the reaction solution was stirred at room temperature for one hour. β-Propiolactone (1.0 mL) was added thereto, and the solution was stirred at room temperature for 2.5 hours. Water was then added to the solution, and the pH was adjusted to 2 with 2 N hydrochloric acid. The solution was extracted with ethyl acetate. The organic layer was sequentially washed with water and a saturated saline solution and then dried over anhydrous sodium sulfate. The solvent was distilled off under reduced pressure, and n-hexane was then added to the residue to perform crystallization. The title compound (2.2 g) was obtained as colorless crystals.
<Step 2> Synthesis of 7-trifluoromethylchroman-4-one
   The compound (4.7 g) prepared in <Step 1> of Example 23 was dissolved in polyphosphoric acid (100 g), and the reaction solution was stirred at an outer temperature in the range of 100°C to 120°C for one hour. The reaction solution was poured into ice water and then extracted with ethyl acetate. The organic layer was washed with a saturated saline solution and then dried over anhydrous sodium sulfate. The solvent was distilled off under reduced pressure. The residue was purified by silica gel column chromatography (eluate; n-hexane:ethyl acetate = 10:1). The title compound (4.2 g) was obtained as colorless crystals.
<Step 3> Synthesis of ethyl (E)-2-(7-trifluoromethylchroman-4-ylidene)acetate
   A tetrahydrofuran (10 mL) solution of triethyl phosphonoacetate (8.5 mL) was added to a tetrahydrofuran (30.0 mL) suspension of 60% sodium hydride (1.7 g) at an inner temperature of 20°C or lower, and the reaction mixture was then stirred at room temperature for one hour. A tetrahydrofuran (10 mL) solution of the compound (4.2 g) prepared in <Step 2> of Example 23 was added to the mixture under ice cooling, and the mixture was then stirred overnight at room temperature. The solvent was then distilled off under reduced pressure. The residue was purified by silica gel column chromatography (eluate; n-hexane:ethyl acetate = 10:1). The title compound (1.4 g) was obtained as colorless crystals.
<Step 4> Synthesis of (E)-2-(7-trifluoromethylchroman-4-ylidene)acetic acid
   The title compound (0.35 g) was obtained as colorless crystals from the compound (1.0 g) prepared in <Step 3> of Example 23 by the same process as that used in <Step 4> of Example 1.
<Step 5> Synthesis of (E)-2-(7-trifluoromethylchroman-4-ylidene)-N-(3,4-dihydroquinolin-2(1H)-on-7-yl)acetamide
   The title compound (175.8 mg) was obtained as a pale yellowish-white solid from the compound (240.0 mg) prepared in <Step 4> of Example 23 by the same process as that used in <Step 7> of Example 1.

### (EXAMPLE 24)

Synthesis of (E)-2-(7-trifluoromethyl-2,2-dimethylchroman-4-ylidene)-N-(3,4-dihydroquinolin-2(1H)-on-7-yl)acetamide

### <Step 1> Synthesis of 2-hydroxy-4-trifluoromethylacetophenone

Methyllithium (1.0 M diethyl ether solution, 98.0 mL) was added to a tetrahydrofuran (60.0 mL) solution of 4-trifluoromethylsalicylic acid (6.0 g) under ice cooling, and the reaction solution was stirred at room temperature for two hours. Trimethylsilyl chloride (37.0 mL) and 1 N hydrochloric acid (100 mL) were added to the reaction solution under ice cooling. The reaction solution was extracted with ethyl acetate. The organic layer was sequentially washed with water and a saturated saline solution and then dried over anhydrous sodium sulfate. The solvent was distilled off under reduced pressure. The residue was purified by silica gel column chromatography (eluate; n-hexane:ethyl acetate = 100:0 to 95:5). The title compound (5.86 g) was obtained as pale yellow oil.

### <Step 2> Synthesis of 7-trifluoromethyl-2,2-dimethylchroman-4-one

Acetone (3.3 mL) and pyrrolidine (3.7 mL) were added to a methanol (140.0 mL) solution of the compound (5.71 g) prepared in <Step 1> of Example 24, and the reaction solution was stirred at room temperature for 12 hours. The solvent was distilled off under reduced pressure. A 10% aqueous citric acid solution (50.0 mL) and water (50.0 mL) were added to the residue, and the resulting solution was extracted with ethyl acetate. The organic layer was sequentially washed with water and a saturated saline solution and then dried over anhydrous sodium sulfate. The solvent was distilled off under reduced pressure. The title crude compound (6.27 g) was obtained as orange oil.

### <Step 3> Synthesis of 4-hydroxy-4-vinyl-7-trifluoromethyl-2,2-dimethylchroman

Vinyl magnesium chloride (38.0 mL) was added to a tetrahydrofuran (120.0 mL) solution of the crude compound (6.14 g) prepared in <Step 2> of Example 24 under ice cooling, and the reaction solution was stirred at room temperature for five hours. Water was added to the reaction solution, and the reaction solution was then extracted with ethyl acetate. The organic layer was sequentially washed with water and a saturated saline solution and then dried over anhydrous sodium sulfate. The solvent was distilled off under reduced pressure. The residue was purified by silica gel column chromatography (eluate; n-hexane:ethyl acetate = 100:0 to 90:10). The title compound (2.35 g) was obtained as a yellow oil.

### <Step 4> Synthesis of (E)-2-(7-trifluoromethyl-2,2-dimethylchroman-4-ylidene)acetaldehyde

Pyridinium dichromate (5.22 g) was added to a dichloromethane (35.0 mL) solution of the compound (1.89 g) prepared in <Step 3> of Example 24 and molecular sieves 4A (10.0 g) under ice cooling, and the reaction solution was stirred at room temperature for two hours. Diethyl ether was added to the reaction solution, and the reaction solution was subjected to Celite filtration. The solvent was distilled off under reduced pressure. The residue was purified by silica gel column chromatography (eluate; n-hexane:ethyl acetate = 100:0 to 90:10). The title compound (440 mg) was obtained as a yellow oil.

### <Step 5> Synthesis of (E)-2-(7-trifluoromethyl-2,2-dimethylchroman-4-ylidene)acetic acid

Sodium hydrogenphosphate (180 mg), 2-methyl-2-butene (0.63 mL), and water (2.0 mL) were added to a tert-butanol (8.0 mL) solution of the compound (400 mg) prepared in <Step 4> of Example 24. Sodium hypochlorite (400 mg) was added to the reaction solution under ice cooling, and the reaction solution was stirred at the same temperature for two hours. The reaction solution was neutralized with 1 N hydrochloric acid and then extracted with ethyl acetate. The organic layer was sequentially washed with water and a saturated saline solution and then dried over anhydrous sodium sulfate. The solvent was distilled off under reduced pressure. The title crude compound (477 mg) was obtained as colorless crystals.

### <Step 6> Synthesis of (E)-2-(7-trifluoromethyl-2,2-dimethylchroman-4-ylidene)-N-(3,4-dihydroquinolin-2(1H)-on-7-yl)acetamide

The title compound (138.6 mg) was obtained as a pale yellowish-white solid from the compound (260.0 mg) prepared in <Step 5> of Example 24 and 7-amino-3,4-dihydroquinolin-2(1H)-one (100.0 mg) by the same process as that used in <Step 7> of Example 1.

### (EXAMPLE 25)

Synthesis of (E)-2-(7-trifluoromethyl-2,2-dimethylchroman-4-ylidene)-N-(1-methyl-3,4-dihydro-2(1H)-quinolinon-7-yl)acetamide

The title compound (156.0 mg) was obtained as a pale yellow amorphous from the compound (100.0 mg) prepared in <Step 5> of Example 24 and 7-amino-1-methyl-3,4-dihydro-2(1H)-quinolinone hydrochloride(150.0 mg) by the same process as that used in <Step 7> of Example 1.

### (EXAMPLE 26)

Synthesis of (Z)-2-(6-trifluoromethyl-3,3-dimethyl-4-oxa-3,4-dihydroisoquinolin-1(2H)-ylidene)-N-(3,4-dihydroquinolin-2(1H)-on-7-yl)acetamide

### <Step 1> Synthesis of 6-trifluoromethyl-3,3-dimethyl-4-oxa-3,4-dihydroisoquinolin-1(2H)-one

2,2-Dimethoxypropane (24.0 mL) and concentrated sulfuric acid (2.0 mL) were added to a chloroform (200 mL) solution of 2-hydroxy-4-trifluoromethylbenzamide (10.0 g), and the reaction solution was refluxed under heating for 3 hours. The reaction solution was neutralized with a saturated aqueous sodium hydrogen carbonate solution and was then extracted with ethyl acetate. The organic layer was sequentially washed with water and a saturated saline solution and then dried over anhydrous sodium sulfate. The solvent was distilled off under reduced pressure. Diethyl ether was added to the residue and collected by filtration of the suspension. The title compound (9.52 g) was obtained as a white solid.

### <Step 2> Synthesis of 6-trifluoromethyl-3,3-dimethyl-4-oxa-3,4-dihydroisoquinolin-1(2H)-thione

The Lawesson's reagent (7.85 g) was added to a toluene (200 mL) solution of the compound (9.52 g) prepared in <Step 1> of Example 26, and the reaction solution was refluxed under heating for one hour. The reaction solution was left to cool and was then purified by silica gel column chromatography (eluate; n-hexane:ethyl acetate = 88:12 to 80:20). The title compound (9.72 g) was obtained as a yellow solid.

### <Step 3> Synthesis of 2-bromo-N-(3,4-dihydroquinolin-2(1H)-one-7-yl)acetamide

4-(4,6-Dimethoxy-1,3,5-triadine-2-yl)-4-methylmorpholinium chloride (7.88 g) was added to a methanol (190 mL) solution of 7-amino-3,4-dihydroquioline-2(1H)-one(3.08 g) and bromoacetic acid (3.17 g), and the mixture was stirred at room temperature for one hour. The solvent was distilled off under reduced pressure. Water was added to the residue. The precipitate was collected by filtration and washed with water. Ethanol was added to the mixture. After azeotropic removal water, ethyl acetate was added to the residue and collected by filtration of the suspension. The title compound (4.98 g) was obtained as a pale brown solid.

### <Step 4> Synthesis of 2-(6-trifluoromethyl-3,3-dimethyl-4-oxaisoquinolin-1-ylthio)-N-(3,4-dihydroquinolin-2(1H)-on-7-yl)acetamide

Potassium carbonate (0.39 g) was added to a N,N-dimethylformamide (20.0 mL) solution of the compound (1.00 g) prepared in <Step 2> of Example 26 and the compound (1.09 g) prepared in <Step 3> of Example 26, then stirred under heating at 80 °C for one hour. Water was added to the mixture, and the resulting solution was extracted with ethyl acetate. The organic layer was sequentially washed with water and a saturated saline solution, and then dried over anhydrous sodium sulfate. The solvent was then distilled off under reduced pressure. Diethyl ether was added to the residue and collected by filtration of the suspension. The title compound (1.58 g) was obtained as a pale off-white solid.

### <Step 5>

Synthesis of (Z)-2-(6-trifluoromethyl-3,3-dimethyl-4-oxa-3,4-dihydroisoquinolin-1(2H)-ylidene)-N-(3,4-dihydroquinolin-2 (1H)-on-7-yl)acetamide

N,N-Diisopropylethylamine (1.50 mL) and triphenylphosphine (1.36 g) were added to the compound (0.80 g) prepared in <Step 4> of Example 26, and the reaction mixture was subjected to microwave irradiation at 180 °C for one hour. The reaction mixture was purified by silica gel column chromatography (eluate; n-hexane:ethyl acetate = 50:50 to 0:100). The title compound (0.22 g) was obtained as a yellow amorphous.

### (EXAMPLE 27)

Synthesis of (Z)-2-(8-trifluoromethyl-3,4-dihydrobenzo[f][1,4]oxazepin-5(2H)-ylidene)-N-(3,4-dihydroquinolin-2(1H)-on-7-yl)acetamide

### <Step 1> Synthesis of 2-hydroxy-4-trifluoromethylbenzoic acid tert-butyl ester

A solution of tetrahydrofuran (50 mL) of N,N'-dicyclohexylcarbodiimide (11.0 g) was added dropwise to a tetrahydrofuran (50 mL) suspension of 2-hydroxy-4-trifluoromethylbenzoic acid (10.0 g), tert-butanol (92.8 mL) and 4-(N,N-dimethylamino)pyridine (0.24 g), and stirred at room temperature for 64 hours. The precipitate was filtered off, and the solvent was distilled off under reduced pressure. The residue was purified by silica gel column chromatography (eluate; n-hexane:ethyl acetate = 100: 0 to 95:5). The title compound (8.18 g) was obtained as colorless oil. <Step 2> Synthesis of tert-butyl 2-(2-(tert-butoxycarbonyl)-5-trifluoromethylphenoxy)ethylcarbamete

Cesium carbonate (11.4 g) was added to a N,N-dimethylformamide (50 mL) solution of the compound (4.58 g) prepared in <Step 1> of Example 27 and 2-(tert-butoxycarbonylamino)ethyl bromide (4.70 g), and the mixture was stirred under heating at 80 °C for one hour. Water was added to the mixture. The mixture was extracted with ethyl acetate. The organic layer was sequentially washed with water and a saturated saline solution and then dried over anhydrous sodium sulfate. The solvent was distilled off under reduced pressure. The title crude compound (7.78 g) was obtained as a colorless oil.

### <Step 3> Synthesis of 8-trifluoromethyl-3,4-dihydrobenzo[f][1,4]oxazepin-5(2H)-one

Trifluoroacetic acid (50 mL) was added to a mixture of the compound (3.54 g) prepared in <Step 2> of Example 27 and anisole (0.95 mL), and stirred at 50 °C for 30 minutes. Trifluoroacetic acid was distilled off under reduced pressure. The residue was dissolved in acetonitrile (175 mL). Benzotriazol-1-yloxy tris(dimethylamino)phosphonium hexaflurophosphate (7.72 g) and diisopropylethylamine (4.68 mL) were sequentially added to the mixture and stirred at room temperature for 3 hours. Water was added to the reaction mixture, and the mixture was extracted with ethyl acetate. The organic layer was sequentially washed with water and a saturated saline solution, and then dried over anhydrous sodium sulfate. The solvent was then distilled off under reduced pressure. The residue was purified by silica gel column chromatography (eluate; n-hexane:ethyl acetate = 67:33 to 0:100). The title compound (0.94 g) was obtained as colorless amorphous.

### <Step 4> Synthesis of 8-trifluoromethyl-3,4-dihydrobenzo[f][1,4]oxazepin-5(2H)-thione

The title compound (0.67 g) was obtained as a milky white solid from the compound (0.94 g) prepared in <Step 3> of Example 27 by the same process as that used in <Step 2> of Example 26.

### <Step 5> Synthesis of 2-(8-trifluoromethyl-3,4-dihydrobenzo[f] [1,4]oxazepin-5(2H)-ylthio)-N-(3,4-dihydroquinolin-2(1H)-on-7-yl)acetamide

The title compound (0.40 g) was obtained as an off-white solid from the compound (0.24 g) prepared in <Step4> of example 27 and the compound (0.29g) in <Step 3> of Example 26 by the same process as that used in <Step 4> of Example 26.

### <Step 6> Synthesis of (Z)-2-(8-trifluoromethyl-3,4-dihydrobenzo[f][1,4]oxazepin-5(2H)-y-lidene)-N-(3,4-dihydroquinolin-2(1H)-on-7-yl)acetamide

The title compound (80 mg) was obtained as a milky white solid from the compound (0.20 g) prepared in <Step 5> of Example 27 by the same process as that used in <Step 5> of Example 26.

### (EXAMPLE 28)

Synthesis of (E)-2-(8-trifluoromethyl-3,4-dihydrobenzo[b]oxepin-5(2H)-ylidene)-N-(3-hydroxymethyl-3,4-dihydro-2(1H)-quinolinon -7-yl)acetamide

### <Step 1> Synthesis of α-hydroxymethylbenzenepropanoic acid methyl ester

Lithium hexamethyldisilazide (1.0 M, tetrahydrofuran solution)(53.0 mL) was added dropwise to a tetrahydrofuran (100.0 mL) solution of β-hydroxypropanoic acid methyl ester (2.50 g) at -50 °C, and the reaction mixture was stirred at the same temperature for 30 minutes. Benzyl bromide (2.86 mL) was added to the mixture. The mixture was stirred at -20 °C for one hour. Aqueous saturated ammonium chloride solution was added to the mixture and extracted with ethyl acetate. The organic layer was washed with water and saturated saline solution, and dried over anhydrous sodium sulfate. The solvent was distilled off under reduced pressure. The residue was purified by silica gel column chromatography (eluate; n-hexane:ethyl acetate = 100:0 to 50:50). The title compound (2.0 g) was obtained as colorless oil.

### <Step 2> Synthesis of α-nitrooxymethyl-2,4-dinitrobenzenepropanoic acid methyl ester

A mixed acid of fuming nitric acid (0.5 mL) and concentrated sulfuric acid (1.0 mL) was added dropwise to a concentrated sulfuric acid (1.5 mL) solution of the compound (0.4 g) prepared in <Step1> of Example 28 under ice cooling, and the mixture was stirred at room temperature for one hour. Ice was added to the mixture and diluted with water. The mixture was extracted with diethyl ether. The organic layer was washed with water and saturated saline solution, and dried over anhydrous sodium sulfate. The solvent was distilled off under reduced pressure. The title crude compound (0.3 g) was obtained as colorless oil.

### <Step 3> Synthesis of 7-amino-3-hydroxymethyl-3,4-dihydro-2(1H)-quinolinon

The title compound (125.0 mg) was obtained as a pale brown solid from the compound (0.4 g) prepared in <Step 2> of Example 28 by the same process as that used in <Step 6> of Example 1.

### <Step 4> Synthesis of (E)-2-(8-trifluoromethyl-3,4-dihydrobenzo[b]oxepin-5(2H)-ylidene)-N-(3-hydroxymethyl-3,4-dihydro-2(1H)-quinolinon-7-yl)acetamide

The title compound (54.0 mg) was obtained as a pale yellowish-white solid from the compound (120.0 mg) prepared in <Step 3> of Example 28 by a process similar to the process used in <Step 7> of Example 1.

### (EXAMPLE 29)

Synthesis of (E)-2-(8-trifluoromethyl-3,4-dihydrobenzo[b]oxepin-5(2H)-ylidene)-N-(3,3-dimethyl-3,4-dihydro-2(1H)-quinolinon -7-yl)acetamide

### <Step 1> Synthesis of α,α-dimethyl-2,4-dinitrobenzenepropanoic acid

The title compound (350.0 mg) was obtained as a pale yellow solid from 2,2-dimethyl-3-phenylpropanoic acid (290.0 mg) by a process similar to the process used in <Step 2> of Example 28.

### <Step 2> Synthesis of α,α-dimethyl-2,4-dinitrobenzenepropanoic acid ethyl ester

Concentrated sulfuric acid (3.0 mL) was added dropwise to a ethanol (50.0 mL) solution of the compound (350.0 mg) prepared in <step2> of example 29 under ice cooling, and the mixture was refluxed for 18 hours. The mixture was left to cool. The solvent was distilled off under reduced pressure. Ice was added to the residue and diluted with water. The mixture was extracted with ethyl acetate. The organic layer was washed with water and saturated saline solution, and dried over anhydrous sodium sulfate. The solvent was distilled off under reduced pressure. The residue was purified by silica gel column chromatography (eluate; n-hexane:ethyl acetate = 100:0 to 90:10). The title compound (330.0 mg) was obtained as colorless oil.

### <Step 3> Synthesis of 7-amino-3,3-dimethyl-3,4-dihydro-2(1H)-quinolinone

The title compound (120.0 mg) was obtained as a pale yellow solid from the compound (330.0 mg) prepared in <Step 2> of Example 29 by a process similar to the process used in <Step 6> of Example 1.

### <Step 4> Synthesis of (E)-2-(8-trifluoromethyl-3,4-dihydrobenzo[b]oxepin-5(2H)-ylidene)-N-(3,3-dimethyl-3,4-dihydro-2(1H)-quinolinon -7-yl)acetamide

The title compound (65.0 mg) was obtained as a pale yellowish-white solid from the compound (41.9 mg) prepared in <Step 3> of Example 29 by a process similar to the process used in <Step 7> of Example 1.

### (EXAMPLE 30)

Synthesis of (E)-2-(8-trifruoromethyl-3,4-dihydrobenzo[b]oxepin-5(2H)-ylidene)-N-(3-(4-morpholinyl)-3,4-dihydro-2(1H)-quinolinon-7-yl)acetamide

### <Step 1> Synthesis of ethyl 3-(2,4-dinitrophenyl)-2-propenoate

Ethyl (triphenylphosphoranylidene)acetate (46.6 g) was added to a toluene (300.0 mL) solution of 2,4-dinitrobenzaldehyde (25.0 g), and the reaction mixture was refluxed under heating for two hours. The reaction mixture was cooled to room temperature, and the solvent was distilled off under reduced pressure. A diethyl ether was added to the residue, and then the formed triphenylphosphine oxide was filtered off. The filtrate was concentrated under reduced pressure. The residue was purified by silica gel column chromatography (eluate; n-hexane:ethyl acetate = 100:0 ∼ 80:20). The title compound (26.0 g) was obtained as a yellow solid.

### <Step 2> Synthesis of ethyl 3-(2,4-dinitrophenyl)-2-(4-morpholinyl)propanoate

A morpholine (1.0 g) and a lithium perchlorate (0.8 g) were added to a tetrahydrofuran (10.0 mL) solution of the compound (2.0 g) prepared in <Step 1> of Example 30, and the mixture was stirred at room temperature for two days. The solvent was distilled off under reduced pressure, and the residue was purified by silica gel column chromatography (eluate; n-hexane:ethyl acetate = 100:0 ∼ 80:20). The title compound (2.2 g) was obtained as a yellow oil.

### <Step 3> Synthesis of 7-amino-3-(4-morpholinyl)-3,4-dihydro-2(1H)-quinolinone

The title compound (800.0 mg) was obtained as a pale yellow-white solid from the compound (2.15 g) prepared in <Step 2> of Example 30 by the same process as that used in <Step 6> of Example 1.

### <step 4> synthesis of (E)-2-(8-trifluoromethyl-3,4-dihydrobenzo[b]oxepin-5(2H)-ylidene)-N-(3-(4-morpholinyl)-3,4-dihydro-2(1H)-quinolinon-7-yl)acetoamide

The title compound (190.0 mg) was obtained as a pale yellow-white solid from the compound (100.0 mg) prepared in <Step 3> of Example 30 by the same process as that used in <Step 7> of Example 1.

### (EXAMPLE 31)

Synthesis of (E)-2-(8-trifluoromethyl-3,4-dihydrobenzo[b]oxepin-5(2H)-ylidene)-N-(3-(1-piperidinyl)-3,4-dihydro-2(1H)-quinolinon-7-yl)acetamide

The title compound (116.0 mg) was obtained as a pale yellow-white solid from 7-amino-3-(1-piperidinyl)-3,4-dihydro-2(1H)-quinolinone (100.0 mg) prepared in the same process as that used in Example 30 by a process similar to the process used in <step 7> of Example 1.

### (EXAMPLE 32)

Synthesis of (E)-2-(7-trifluoromethyl-3,4-dihydrobenzo[b]oxepin-5(2H)-ylidene)-N-(3-(4-methyl-1-piperazinyl)-3,4-dihydro-2(1H)-quinolinon-7-yl)acetamide

The title compound (176.0 mg) was obtained as a pale yellow-white solid from 7-amino-3,4-dihydro-3-(4-methyl-1-piperazinyl)-2(1H)-quinolinone (140.0 mg) prepared in the same process as that used in Example 30 by a process similar to the process used in <step 7> of Example 1.

### (EXAMPLE 33)

Synthesis of (E)-2-(7-trifluoromethyl-2,2-dimethylchroman-4-ylidene)-N-(3-(4-morpholinyl)-3,4-dihydro-2(1H)-quinolinon-7-yl)acetamide

The title compound (46.0 mg) was obtained as a pale yellow-white solid from the compound (100.0 mg) prepared in <Step 3> of Example 30 by the same process as that used in <Step 7> of Example 1.

### (EXAMPLE 34,35)

Optical resolution of (E)-2-(8-trifluoromethyl-3,4-dihydrobenzo[b]oxepin-5(2H)-ylidene)-N-(3-(4-morpholinyl)-3,4-dihydro-2(1H)-quinolinon-7-yl)acetamide

Optical resolution of the compound (20 mg) obtained in Example 30 was performed by preparative chromatography (column; CHIRALPAK AS (2.0 cm x 25.0 cm) manufactured by Daicel Chemical Industries Ltd., eluate; n-hexane:ethanol = 50:50, flow rate; 15.0 mL/min, UV; 254 nm). Accordingly, enantiomers of the title compound were obtained as a first fraction (5.5 mg, white solid, 99.8%ee, retention time: 6.4 minutes; Example 34) and a second fraction (3.3 mg, white solid, 97.9%ee, retention time: 7.8 minutes; Example 35).

### (EXAMPLE 36)

Synthesis of (E)-2-(8-trifluoromethyl-3,4-dihydrobenzo[b]oxepin-5(2H)-yliden)-N-(3-oxo-1,2,3,4-tetrahydroquinolin-5-yl)acetamide

### <Step 1> Synthesis of ethyl 3-(2,6-dinitrophenyl)-2-oxopropanoate

A diethyl oxalate (48.2 g) and sodium ethoxide (11.2 g) were added to a ethanol (300.0 mL) solution of 2,6-dinitrotoluene (30.0 g), and the mixture was stirred at 40 °C for four hours. The reaction mixture was cooled to room temperature. A 1N hydrochloric acid was added to the mixture, and the solvent was distilled off under reduced pressure. The residual aqueous solution was extracted with ethyl acetate. The organic layer was washed with water, and dried over anhydrous sodium sulfate. The solvent was distilled off under reduced pressure, and the residue was purified by silica gel column chromatography (eluate; n-hexane:ethyl acetate = 80:20 ∼ 70:30). The title compound (31.1 g) was obtained as a pale red solid.

### <Step 2> Synthesis of ethyl 3-(2,6-dinitrophenyl)-2,2-diethoxypropanoate

A triethylorthoformate (9.6 mL) and trifluoroborane diethyl ether complex (2.4 mL) were added to an ethanol (16.2 mL) solution of the compound (5.4 g) prepared in <Step 1> of Example 36, and the mixture was refluxed under heating for three days. The reaction mixture was cooled to room temperature. Water was added to the reaction mixture, and extracted with ethyl acetate. The mixture was washed with a saturated saline solution, and dried over anhydrous sodium sulfate. The solvent was distilled off under reduced pressure, and the residue was purified by silica gel column chromatography (eluate; n-hexane:ethyl acetate = 100:0 ∼ 50:50). The title compound (1.45 g) was obtained as a yellow solid.

### <step 3> Synthesis of 5-amino-3,3-diethoxy-3,4-dihydro-2(1H)-quinolinone

The title compound (220.0 mg) was obtained as a yellow solid from the compound (1.4 g) prepared in <Step 2> of Example 36 by the same process as that used in <Step 6> of Example 1.

### <Step 4> Synthesis of 5-amino-3,3-diethoxy-1,2,3,4-tetrahydroquinoline

A lithium aluminium hydride (140.0 mg) was added to the tetrahydrofuran (4.0 mL) solution of the compound (180.0 mg) prepared in <Step 3> of Example 36, and the mixture was refluxed under heating for thirty minutes. The reaction mixture was cooled to room temperature. Water and 1N sodium hydroxide were added to the mixture, and diluted with tetrahydrofuran. The insoluble matter was filtered off using Celite, and the filtrate was concentrated under reduced pressure. The residue was purified by silica gel column chromatography (eluate; n-hexane:ethyl acetate = 100:0 - 70:30). The title compound (214.4 mg) was obtained as a pale brown solid.

### <step 5> Synthesis of (E)-2-(8-trifluoromethyl-3,4-dihydrobenzo[b]oxepin-5(2H)-ylidene)-N-(3,3-diethoxy-1,2,3,4-tetrahydroquinolin-5-yl) acetamide

The title compound (300.0 mg) was obtained as a white solid from the compound (150.0 mg) prepared in <Step 4> of Example 36 by the same process as that used in <Step 7> of Example 1.

### <Step 6> Synthesis of (E)-2-(8-trifluoromethyl-3,4-dihydrobenzo[b]oxepin-5(2H)-ylidene)-N-(3-oxo-1,2,3,4-tetrahydroquinolin-5-yl)acetamide

The title compound (24.0 mg) was obtained as a white solid from the compound (50.0 mg) prepared in <Step 5> of Example 36 by the same process as that used in <Step 6> of Example 15.

### (EXAMPLE 37)

Synthesis of (E)-2-(8-trifluoromethyl-3,4-dihydrobenzo[b]oxepin-5(2H)-ylidene)-N-(8-(3-hydroxypropoxy)-3,4-dihydro-2(1H)-quinolinon-5-yl)acetamide

### <step 1> Synthesis of 8-hydroxy-5-nitro-3,4-dihydro-2(1H)-quinolinone

The title compound (5.5 g) was obtained as an orange solid from 3,4-dihydro-8-hydroxy-2(1H)-quinolinone (5.0 g) prepared by a process similar to the process used in <step 2> of Example 28.

### <step 2> Synthesis of 8-(3-tert-butyldimethylsiloxypropoxy)-5-nitro-3,4-dihydro-2(1H)-quinolinone

A potassium carbonate (220.0 mg) and a 3-bromo-1-tert-butyldimethylsiloxypropane (350.0 mg) were added to a N,N-dimethylformamide (4.0 mL) solution of the compound (300.0 mg) prepared in <Step 1> of Example 37, and the mixture was stirred at 100 °C for one hour. The reaction mixture was cooled to room temperature. Water was added to the reaction mixture, and extracted with methylene chloride. The organic layer was washed with water, saturated sodium hydrogencarbonate and a saturated saline solution, sequentially. The organic layer was dried over anhydrous sodium sulfate, and the solvent was distilled off under reduced pressure. The residue was purified by silica gel column chromatography (eluate; n-hexane:ethyl acetate = 80:20 ∼ 70:30). The title compound (214.4 mg) was obtained as a brown solid.

### <step 3> Synthesis of 5-amino-8-(3-tert-butyldimethylsiloxypropoxy)-3,4-dihydro-2(1H)-quinolinone

The title compound (150.8 mg) was obtained as a brown oil from the compound (210.0 mg) prepared in <Step 2> of Example 37 by the same process as that used in <Step 6> of Example 1.

### <step 4> Synthesis of (E)-2-(8-trifluoromethyl-3,4-dihydrobenzo[b]oxepin-5(2H)-ylidene)-N-(8-(3-tert-butyldimethylsiloxypropoxy)-3,4-dihydro-2(1H)-quinolinon-5-yl)acetamide

The title compound (126.1 mg) was obtained as a brown oil from the compound (130.0 mg) prepared in <Step 3> of Example 37 by the same process as that used in <Step 7> of Example 1.

### <step 5> Synthesis of (E)-2-(8-trifluoromethyl-3,4-dihydrobenzo[b]oxepin-5(2H)-ylidene)-N-(8-(3-hydroxypropoxy)-3,4-dihydro-2(1H)-quinolinon-5-yl)acetamide

The title compound (86.8 mg) was obtained as a white solid from the compound (120.0 mg) prepared in <Step 4> of Example 37 by the same process as that used in <Step 6> of Example 15.

### (EXAMPLE 38)

Synthesis of (E)-2-(8-trifluoromethyl-3,4-dihydrobenzo[b]oxepin-5(2H)-ylidene)-N-(3-benzyl-3,4-dihydro-2(1H)-quinazolinon-7-yl)acetamide

### <step 1> Synthesis of 2-amino-4-nitro-N-(benzyl)-benzylamine

The title compound (2.2 g) was obtained as a yellow oil from the compound (1.5 g) prepared in <Step 1> of Example 14 and benzylamine (1.1 mL) by the same process as that used in <Step 2> of Example 14. <step 2> Synthesis of 3-benzyl-7-nitro-3,4-dihydro-2(1H)-quinazolinone

The title compound (711.0 mg) was obtained as a yellow solid from the compound (1.0 g) prepared in <step 1> of Example 38 by a process similar to the process used in <step 1> of Example 12.

### <step 3> Synthesis of 7-amino-3-benzyl-3,4-dihydro-2(1H)-quinazolinone

The title compound (57.3 mg) was obtained as a white solid from the compound (60.0 mg) prepared in <Step 2> of Example 38 by the same process as that used in <Step 6> of Example 1.

### <step 4> Synthesis of (E)-2-(8-trifluoromethyl-3,4-dihydrobenzo[b]oxepin-5(2H)-ylidene)-N-(3-benzyl-3,4-dihydro-2(1H)-quinazolinon-7-yl)acetamide

The title compound (67.0 mg) was obtained as a pale green solid from the compound (50.0 mg) prepared in <Step 3> of Example 38 by the same process as that used in <Step 7> of Example 1.

### (EXAMPLE 39)

Synthesis of (E)-2-(8-trifluoromethyl-3,4-dihydrobenzo[b]oxepin-5(2H)-ylidene)-N-(3-benzyl-1-methyl-3,4-dihydro-2(1H)-quinazolinon-7-yl)acetamide

### <Step 1> Synthesis of 3-benzyl-1-methyl-7-nitro-3,4-dihydro-2(1H)-quinazolinone

A sodium hydride (10.2 mg) and a methyl iodide (53.2 µL) were added to a N,N-dimethylformamide (2.0 mL) solution of the compound (100.0 mg) prepared in <Step 2> of Example 38, and the mixture was stirred at room temperature for two hours. Water was added to the reaction mixture, and extracted with ethyl acetate. The organic layer was washed with a saturated saline solution, and dried over anhydrous sodium sulfate. The solvent was distilled off under reduced pressure. The residue was purified by silica gel column chromatography (eluate; n-hexane:ethyl acetate = 100:0 ∼ 80:20). The title compound (42.0 mg) was obtained as a yellow amorphous.

### <step 2> Synthesis of 7-amino-3-benzyl-1-methyl-3,4-dihydro-2(1H)-quinazolinone

The title compound (37.0 mg) was obtained as a pale yellow amorphous from the compound (42.0 mg) prepared in <Step 1> of Example 39 by the same process as that used in <Step 6> of Example 1.

### <step 3> Synthesis of (E)-2-(8-trifluoromethyl-3,4-dihydrobenzo[b]oxepin-5(2H)-ylidene)-N-(3-benzyl-1-methyl-3,4-dihydro-2 (1H)-quinazolinon-7-yl) acetamide

The title compound (22.0 mg) was obtained as a pale yellow amorphous from the compound (38.0 mg) prepared in <Step 2> of Example 39 by the same process as that used in <Step 7> of Example 1.

### (EXAMPLE 40)

Synthesis of (E)-2-(8-trifluoromethyl-3,4-dihydrobenzo[b]oxepin-5(2H)-ylidene)-N-(3-hydroxymethyl-3-methyl-3,4-dihydro-2(1H)-quinoxalinon-7-yl)acetamide

### <step 1> Synthesis of N-(2,4-dinitrophenyl)-α-methyl-O-(tetrahydro-2H-pyran-2-yl)serine ethyl ester

The title compound (15.2 g) was obtained as a yellow oil from a 2,4-dinitrofluorobenzene (4.7 mL) and (DL)-O-(tetrahydro-2H-pyran-2-yl)serine ethyl ester by a process similar to the process used in <step 1> of Example 7.

### <Step 2> Synthesis of 7-amino-3-methyl-3-(tetrahydro-2H-pyran-2-yl)oxymethyl-3,4-dihydro-2(1H)-quinoxalinone

The title compound (300.0 mg) was obtained as a brown solid from the compound (1.0 g) prepared in <Step 1> of Example 40 by the same process as that used in <Step 6> of Example 1.

### <Step 3> Synthesis of (E)-2-(8-trifluoromethyl-3,4-dihydrobenzo[b]oxepin-5(2H)-ylidene)-N-(3-methyl-3-(tetrahydro-2H-pyran-2-yl)oxymethyl-3,4-dihydro-2(1H)-quinoxalinon-7-yl)acetamide

The title compound (320.0 mg) was obtained as a pale yellow solid from the compound (210.0 mg) prepared in <Step 2> of Example 40 by the same process as that used in <Step 7> of Example 1.

### <Step 4> Synthesis of (E)-2-(8-trifluoromethyl-3,4-dihydrobenzo[b]oxepin-5(2H)-ylidene)-N-(3-hydroxymethyl-3-methyl-3,4-dihydro-2(1H)-quinoxalinon-7-yl)acetamide

The title compound (260.0 mg) was obtained as a pale yellow-white solid from the compound (320.0 mg) prepared in <step 3> of Example 40 by a process similar to the process used in <Step 6> of Example 15.

### (EXAMPLE 41)

Synthesis of (E)-2-(8-trifluoromethyl-3,4-dihydrobenzo[b]oxepin-5(2H)-ylidene)-N-(3-hydroxymethyl-3,4-dimethyl-3,4-dihydro-2(1H)-quinoxalinon-7-yl)acetamide

The title compound (23.0 mg) was obtained as a pale yellow solid from the compound (50.0 mg) prepared in <Step 4> of Example 40 by a process similar to the process used in Example 8.

### (EXAMPLE 42)

Synthesis of (E)-2-(8-trifluoromethyl-3,4-dihydrobenzo[b]oxepin-5(2H)-ylidene)-N-(3-(N,N-dimethylamino)-3,4-dihydro-2(1H)-quinolinon-7-yl)acetamide

The title compound (79.5 mg) was obtained as a pale brown solid from a 7-amino-3-(N,N-dimethylamino)-3,4-dihydro-2(1H)-quinolinone (100.0 mg) prepared in the same process as that used in Example 30 by a process similar to the process used in <step 7> of Example 1.

### (EXAMPLE 43)

Synthesis of (E)-2-(8-trifluoromethyl-3,4-dihydrobenzo[b]oxepin-5(2H)-ylidene)-N-(3-(N,N-diethylamino)-3,4-dihydro-2(1H)-quinolinon-7-yl)acetamide

The title compound (10.5 mg) was obtained as a pale yellow solid from a 7-amino-3-(N,N-diethylamino)-3,4-dihydro-2(1H)-quinolinone (60.0 mg) prepared in the same process as that used in Example 30 by a process similar to the process used in <step 7> of Example 1.

### (EXAMPLE 44)

Synthesis of (E)-2-(8-trifluoromethyl-3,4-dihydrobenzo[b]oxepin-5-(2H)-ylidene)-N-(3-(N,N-bis(2-methoxyethyl)amino))-3,4-dihydro-2(1H)-quinolinon-7-yl)acetamide

The title compound (37.5 mg) was obtained as a yellow solid from a 7-amino-3-(N,N-bis(2-methoxyethyl)amino)-3,4-dihydro-2(1H)-quinolinone (100.0 mg) prepared in the same process as that used in Example 30 by a process similar to the process used in <step 7> of Example 1.

### (EXAMPLE 45)

Synthesis of (E)-2-(8-trifluoromethyl-3,4-dihydrobenzo[b]oxepin-5-(2H)-ylidene)-N-(3-(N-methyl-N-(2-methoxyethyl)amino)-3,4-dihydro-2(1H)-quinolinon-7-yl) acetamide

The title compound (48.4 mg) was obtained as a white solid from a 7-amino-3-(N-methyl-N-(2-methoxyethyl)amino)-3,4-dihydro-2(1H)-quinolinone (100.0 mg) prepared in the same process as that used in Example 30 by a process similar to the process used in <step 7> of Example 1.

### (EXAMPLE 46)

Synthesis of (E)-2-(8-trifluoromethyl-3,4-dihydrobenzo[b]oxepin-5-(2H)-ylidene)-N-(3-(pyrrolidin-1-yl)-3,4-dihydro-2(1H)-quinolinon-7-yl)acetamide

The title compound (66.7 mg) was obtained as a white solid from a 7-amino-3-(pyrrolidin-1-yl)-3,4-dihydro-2(1H)-quinolinone (70.0 mg) prepared in the same process as that used in Example 30 by a process similar to the process used in <step 7> of Example 1.

### (EXAMPLE 47)

Synthesis of (E)-2-(8-trifluoromethyl-3,4-dihydrobenzo[b]oxepin-5(2H)-ylidene)-N-(3-((3S)-fluoro pyrrolidin-1-yl)-3,4-dihydro-2(1H)-quinolinon-7-yl) acetamide

The title compound (56.1 mg) was obtained as a white solid from a 7-amino-3-((3S)-fluoropyrrolidin-1-yl)-3,4-dihydro-2(1H)-quinolinone (100.0 mg) prepared in the same process as that used in Example 30 by a process similar to the process used in <step 7> of Example 1.

### (EXAMPLE 48)

Synthesis of (E)-2-(8-trifluoromethyl-3,4-dihydrobenzo[b]oxepin-5(2H)-ylidene)-N-(3-((3S)-hydroxypyrrolidin-1-yl)-3,4-dihydro-2(1H)-quinolinon-7-yl)acetamide

The title compound (43.5 mg) was obtained as a yellow solid from a 7-amino-3-((3S)-tert-butyldimethylsiloxypyrrolidin-1-yl)-3,4-dihydro-2(1H)-quinolinone (180.0 mg) prepared in the same process as that used in Example 30 by a process similar to the process used in <step 7> of Example 1.

### (EXAMPLE 49)

Synthesis of (E)-2-(8-trifluoromethyl-3,4-dihydrobenzo[b]oxepin-5(2H)-ylidene)-N-(3-((2S)-hydroxymethylpyrrolidin-1-yl)-3,4-dihydro-2(1H)-quinolinon-7-yl) acetamide

The title compound (12.5 mg) was obtained as a white solid from a 7-amino-3-((2S)-tert-butyldimethylsiloxymethylpyrrolidin-1-yl)-3,4-dihydro-2(1H)-quinolinone (100.0 mg) prepared in the same process as that used in Example 30 by a process similar to the process used in <step 7> of Example 1.

### (EXAMPLE 50)

Synthesis of (E)-2-(8-trifluoromethyl-3,4-dihydrobenzo[b]oxepin-5(2H)-ylidene)-N-(3-((2S)-methoxymethylpyrrolidin-1-yl)-3,4-dihydro-2(1H)-quinolinon-7-yl)acetamide

The title compound (39.9 mg) was obtained as a pale yellow solid from a 7-amino-3-((2S)-methoxymethylpyrrolidin-1-yl)-3,4-dihydro-2(1H)-quinolinone (100.0 mg) prepared in the same process as that used in Example 30 by a process similar to the process used in <step 7> of Example 1.

### (EXAMPLE 51)

Synthesis of (E)-2-(8-trifluoromethyl-3,4-dihydrobenzo[b]oxepin-5(2H)-ylidene)-N-(3-(N-methyl-N-cyclohexylamino)-3,4-dihydro-2(1H)-quinolinon-7-yl)acetamide

The title compound (8.3 mg) was obtained as a pale yellow amorphous from a 7-amino-3-(N-methyl-N-cyclohexylamino)-3,4-dihydro-2(1H)-quinolinone (68.0 mg) prepared in the same process as that used in Example 30 by a process similar to the process used in <step 7> of Example 1.

### (EXAMPLE 52)

Synthesis of (E)-2-(8-trifluoromethyl-3,4-dihydrobenzo[b]oxepin-5(2H)-ylidene)-N-(3-(1-piperazinyl)-3,4-dihydro-2(1H)-quinolinon-7-yl)acetamide

The title compound (4.3 mg) was obtained as a pale yellow solid from a 7-amino-3-(1-ethoxycarbonyl-4-piperazinyl)-3,4-dihydro-2(1H)-quinolinone (120.0 mg) prepared in the same process as that used in Example 30 by a process similar to the process used in <step 7> of Example 1.

### (EXAMPLE 53)

Synthesis of (E)-2-(8-trifluoromethyl-3,4-dihydrobenzo[b]oxepin-5(2H)-ylidene)-N-(3-([1,4]oxazepan-4-yl)-3,4-dihydro-2(1H)-quinolinon-7-yl)acetamide

The title compound (62.9 mg) was obtained as a white solid from a 7-amino-3-([1,4]oxazepan-4-yl)-3,4-dihydro-2(1H)-quinolinone (100.0 mg) prepared in the same process as that used in Example 30 by a process similar to the process used in <step 7> of Example 1.

### (EXAMPLE 54)

Synthesis of (E)-2-(8-trifluoromethyl-3,4-dihydrobenzo[b]oxepin-5(2H-ylidene)-N-(3-(4-thiomorpholinyl)-3,4-dihydro-2(1H)-quinolinon-7-yl)acetamide

The title compound (260.4 mg) was obtained as a white solid from a 7-amino-3-(4-thiomorpholinyl)-3,4-dihydro-2-(1H)-quinolinone (200.0 mg) prepared in the same process as that used in Example 30 by a process similar to the process used in <step 7> of Example 1.

### (EXAMPLE 55)

Synthesis of (E)-2-(8-trifluoromethyl-3,4-dihydrobenzo[b]oxepin-5(2H)-ylidene)-N-(3-(4-methoxy-1-piperidinyl)-3,4-dihydro-2(1H)-quinolinon-7-yl)acetamide

The title compound (207.5 mg) was obtained as a white solid from a 7-amino-3-(4-methoxy-1-piperidinyl)-3,4-dihydro-2(1H)-quinolinone (150.0 mg) prepared in the same process as that used in Example 30 by a process similar to the process used in <step 7> of Example 1. (EXAMPLE 56)

Synthesis of (E)-2-(8-trifluoromethyl-3,4-dihydrobenzo[b]oxepin-5(2H)-ylidene)-N-(3-((3S)-methoxypyrrolidin-1-yl)-3,4-dihydro-2(1H)-quinolinon-7-yl)acetamide

The title compound (158.0 mg) was obtained as a white solid from a 7-amino-3-((3S)-methoxypyrrolidin-1-yl))-3,4-dihydro-2(1H)-quinolinone (130.0 mg) prepared in the same process as that used in Example 30 by a process similar to the process used in <step 7> of Example 1.

### (EXAMPLE 57)

Synthesis of (E)-2-((8-trifluoromethyl-3,4-dihydrobenzo[b]oxepin-5(2H)-ylidene)-N-(3-(N-methyl-N-(4-tetrahydropyranyl)amino)-3,4-dihydro-2(1H)-quinolinon-7-yl)acetamide

The title compound (17.5 mg) was obtained as a brown solid from a 7-amino-3-(4-(N-methyl-N-(4-tetrahydropyranyl)amino)-3,4-dihydro-2(1H)-quinolinone (14.5 mg) prepared in the same process as that used in Example 30 by a process similar to the process used in <step 7> of Example 1.

### (EXAMPLE 58)

Synthesis of (E)-2-(8-trifluoromethyl-3,4-dihydrobenzo[b]oxepin-5(2H)-ylidene)-N-(3-(4-morpholinyl)-2(1H)-quinolinon-7-yl)acetamide

### <Step 1> Synthesis of 7-amino-3-(4-morpholinyl)-2(1H)-quinolinone

2,3-dichloro-5,6-dicyano-p-benzoquinone (36.7 mg) was added to a acetonitrile (2.0 mL) solution of the compound (40.0 mg) prepared in <Step 3> of Example 30, and the mixture was refluxed for ten minutes. The reaction mixture was cooled to room temperature, and the solvent was distilled off under reduced pressure. The residue was purified by silica gel column chromatography (eluate; ethyl acetate:methanol = 100:0 ∼ 90:10). The title compound (6.0 mg) was obtained as a pale brown solid.

### <step 2> Synthesis of (E)-2-(8-trifluoromethyl-3,4-dihydrobenzo[b]oxepin-5(2H)-ylidene)-N-(3-(4-morpholinyl)-2(1H)-quinolinon-7-yl)acetamide

The title compound (2.6 mg) was obtained as a yellow amorphous from the compound (6.0 mg) prepared in <Step 1> of Example 58 by a process similar to the process used in <step 7> of Example 1.

### (EXAMPLE 59)

Synthesis of (E)-2-(8-trifluoromethyl-3,4-dihydrobenzo[b]oxepin-5(2H)-ylidene)-N-(2,2-dioxo-3,4-dihydro-(1H)-2,1-benzothiazin-7-yl)acetamide

### <step 1> Synthesis of 7-amino-2,2-dioxy-3,4-dihydro-(1H)-2,1-benzothiazine

The title compound (130.4 mg) was obtained as an orange solid from 2,4-dinitrobenzeneethansulfonyl chloride (510.0 mg) synthesized in accordance with the process described in PCT Publication No. 97/044345 pamphlet prepared in a process similar to the process used in <step 2> of Example 4.

### <step 2> Synthesis of (E)-2-(8-trifluoromethyl-3,4-dihydrobenzo[b]oxepin-5(2H)-ylidene)-N-(2,2-dioxy-3,4-dihydro-(1H)-2,1-benzothiazin-7-yl)acetamide

The title compound (36.4 mg) was obtained as a pale yellow amorphous from the compound (72.8 mg) prepared in <Step 1> of Example 59 by a process similar to the process used in <step 7> of Example 1.

### (EXAMPLE 60)

Synthesis of (E)-2-(2,2-diethyl-7-trifluoromethyl-chroman-4-ylidene)-N-(3,4-dihydro-2(1H)-quinolinon-7-yl)acetamide

### <Step 1> Synthesis of 2,2-diethyl-7-trifluoromethylchroman-4-one

The title compound (25.7 g) was obtained as yellow oil from the compound (44.5 g) prepared in <Step 1> of Example 24 and 3-pentanone (36.6 mL) by a similar to the process used in <Step 2> of Example 24. <Step 2> Synthesis of 2-(2,2-diethyl-4-hydroxy-7-trifluoromethylchroman-4-yl) acetic acid ethyl ester

n-Butyllithium (1.59 M, n-hexane solution, 128.0 mL) was added to a tetrahydrofuran (500.0 mL) solution of diisopropylamine (30.0 mL) at an outer temperature -78 °C, and the mixture was stirred at the same temperature for 30 minutes. Ethyl acetate (21.0 mL) was added to the mixture, and the mixture was stirred at the same temperature for 30 minutes. A tetrahydrofuran (500.0 mL) solution ot the compound (29.2 g) prepared in <Step 1> of Example 60 was added dropwise to the mixture at the same temperature, and the mixture was stirred at the same temperature for 20 minutes and room temperature for 90 minutes. Water was added to the mixture under ice cooling. The mixture was extracted with ethyl acetate. The organic layer was washed with water and saturated saline solution, and dried over anhydrous sodium sulfate. The solvent was distilled off under reduced pressure. The title crude compound (36.3 g) was obtained as a white solid.

### <Step 3> Synthesis of 2-(2,2-diethyl-4-hydroxy-7-trifluoromethylchroman-4-yl)acetic acid

The title compound (31.1 g) was obtained as yellow oil from the crude compound (36.0 g) prepared in <Step 2> of Example 60 by the same process as that used in <Step 4> of Example 1.

### <Step 4> Synthesis of (E)-2-(2,2-diethyl-7-trifluoromethylchromn-4-ylidene)acetic acid

Concentrated sulfuric acid (24.9 mL) was added to a toluene (1.5 L) solution of the compound (31.1 g) prepared in <Step 3> of Example 60, and the mixture was stirred at room temperature for 3 hours. Water was added to the mixture under ice cooling. The mixture was extracted with ethyl acetate. The organic layer was sequentially washed with water and saturated saline solution, and dried over anhydrous sodium sulfate. The solvent was distilled off under reduced pressure. The residue was purified by silica gel column chromatography (eluate; n-hexane:ethyl acetate = 100:0 to 25:75).The title compound (9.1 g) was obtained as a white solid.

### <Step 5> Synthesis of (E)-2-(2,2-diethyl-7-trifluoromethylchroman-4-ylidene)-N-(3,4-dihydro-2(1H)-quinolinon-7-yl)acetamide

The title compound (165 mg) was obtained as a white solid from the compound (100.0 mg) prepared in <Step 4> of Example 60 by a similar to the process used in <Step 7> of Example 1.

### (EXAMPLE 61)

Synthesis of (E)-2-(7-trifluoromethyl-spiro[chroman-2,1'-cyclobutane]-4-ylidene)-N-(3,4-dihydro-2(1H)-quinolinon-7-yl)acetamide

The title compound (38.0 mg) was obtained as a white solid from (E)-2-(7-trifluoromethyl-spiro[chroman-2,1'-cyclobutan]-4-ylidene)acetic acid (75.5 mg) prepared by the way described in PCT Publication No. 07/010383 pamphlet by a similar to the process used in <Step 7> of Example 1.

### (EXAMPLE 62)

Synthesis of (E)-2-(7-trifluoromethyl-2,2-bis(methoxyethyl)chroman-4-ylidene)-N-(3,4-dihydro-2(1H)-quinolinon-7-yl)acetamide

### <Step 1> Synthesis of 7-trifluoromethyl-2,2-bis(methoxymethyl)chroman-4-one

The title compound (2.2 g) was obtained as brown oil from the compound (1.5 g) prepared in <Step 1> of Example 24 and 1,3-dimethoxyacetone (950.0 mg) by a similar to the process used in <Step 2> of Example 24.

### <Step 2> Synthesis of 2-(2,2-bis(methoxymethyl)-4-hydroxy-7-trifluoromethylchroman-4-yl)acetic acid ethyl ester

The title compound (1.65 g) was obtained as brown oil from the compound (2.2 g) prepared in <Step 1> of Example 62 by the same process as that used in <Step 2> of Example 60.

### <Step 3> Synthesis of 2-(2,2-bis(methoxymethyl)-4-hydroxy-7-trifluoromethylchroman-4-yl)acetic acid

The title compound (1.28 g) was obtained as brown oil from the compound (1.5 g) prepared in <Step 2> of Example 62 by the same process as that used in <Step 4> of Example 1.

### <Step 4> Synthesis of (E)-2-(2,2-bis(methoxymethyl)-7-trifluoromethylchroman-4-ylidene)acetic acid

The title compound (365.0 mg) was obtained as a white solid from the compound (1.1 g) prepared in <Step 3> of Example 62 by the same process as that used in <Step 4> of Example 60.

### <Step 5> Synthesis of (E)-2-(7-trifluoromethyl-2,2-bis(methoxymethyl)chroman-4-ylidene)-N-(3,4-dihydro-2(1H)-quinolinon-7-yl)acetamide

The title compound (17.5 mg) was obtained as a pale yellow-white solid from the compound (60.0 mg) prepared in <Step 4> of Example 62 by a similar to the process used in <Step 7> of Example 1.

### (EXAMPLE 63)

Synthesis of (E)-2-(7-trifluoromethyl-spiro[chroman-2,3'-N-methylazetidine]-4-yliden)-N-(3,4-dihydro-2(1H)-quinolinon-5-yl)acetamide

### <Step 1> Synthesis of 1'-(tert-butoxycarbonyl)-7-trifluoromethyl-spiro[chroman-2,3'-azetidine]-4-one

The title compound (9.0 g) was obtained as a yellow solid from the compound (5.8 g) prepared in <Step 1> of Example 24 and tert-butoxycarbonyl-3-oxoazetidine (5.35 g)by a similar to the process used in <Step 2> of Example 24.

### <Step 2> Synthesis of (E)-2-(1'-(tert-butoxycarbonyl)-7-trifluoromethyl-spiro[chroman-2,3'-azetidine]-4-ylidene)aceticacid tert-butyl ester

The title compound (3.79 g) was obtained as yellow oil from the compound (14.8 g) prepared in <Step 1> of Example 63 by the same process as that used in <Step 3> of Example 23.

### <Step 3> Synthesis of (E)-2-(1'-(tert-butoxycarbonyl)-7-trifluoromethyl-spiro[chroman-2,3'-azetidine]-4-ylidene)acetic acid

The title compound (1.98 g) was obtained as a pale orange solid from the compound (4.1 g) prepared in <Step 2> of Example 63 by a similar to the process used in <Step 4> of Example 1.

### <Step 4> Synthesis of ((E)-2-(1'-(tert-butoxycarbonyl)-7-trifluoromethyl-spiro[chroman-2,3'-azetidine]-4-ylidene)-N-(3,4-dihydro-2(1H)-quinolinon-5-yl)acetamide

The title compound (19.3 mg) was obtained as yellow solid from the compound (50.0 mg) prepared in <Step 3> of Example 63 by the same process as that used in <Step 7> of Example 1.

### <Step 5> Synthesis of (E)-2-(1'-(tert-butoxycarbonyl)-7-trifluoromethyl-spiro[chroman-2,3'-azetidine]-4-ylidene)-N-(3,4-dihydro-2(1H)-quinolinon-5-yl)acetamide

4 N hydrogenchloride in 1,4-dioxane (5.0 mL) was added to a solution of 1,4-dioxane (5.0 mL) solution of the compound (270.0 mg) prepared in <Step 4> of Example 63, and the mixture was stirred at room temperature overnight. 4 N aqueous sodium hydroxide solution was added to the mixture. The mixture was extracted with ethyl acetate. The organic layer was washed with saturated saline solution and dried over sodium sulfate. The solvent was then distilled off under reduced pressure. Methanol was added to the residue to solidify the resulting product. The title compound (121.0 mg) was obtained as a yellow solid. <Step 6> Synthesis of (E)-2-(7-trifluoromethyl-spiro[chroman-2,3'-N-methylazetidine]-4-yliden)-N-(3,4-dihydro-2(1H)-quinolinon-5-yl)acetamide
36% Formalin solution (11.2 µL) and sodium triacetoxyborohydride (28.7 mg) were added to a mixture of 1,2-dichloroethane (10.0 mL) and N,N-dimethylformamide (10.0 mL) of the compound (40.0 mg) prepared in <Step 5> of Example 63, and the mixture was stirred at room temperature overnight. A saturated aqueous sodium hydrogen carbonate solution was added thereto, and the reaction solution was then extracted with ethyl acetate. The organic layer was sequentially washed with water and a saturated saline solution and then dried over anhydrous sodium sulfate. The solvent was distilled off under reduced pressure. n-Hexane and diethyl ether were added to the residue to solidify the resulting product. The title compound (33.1 mg) was obtained as a white solid.

### (EXAMPLE 64)

Synthesis of (E)-2-(7-trifluoromethyl-3,4-dihydro-2H-1-benzothiopyran-4-ylidene)-N-((3-(4-morpholinyl)-3,4-dihydro-2(1H)-quinolinon-7-yl)acetamide

### <Step 1> Synthesis of 2-(4-hydroxy-7-trifluoromethylthiochroman-4-yl)acetic acid ethyl ester

The title compound (150.0 mg) was obtained as dark yellow oil from 7-trifluoromethyl-thiochroman-4-on (250.0 mg) prepared by the way described in Experienta(30(5), 452-455, 1974.) by a similar to the process used in <Step 2> of Example 60.

### <Step 2> Synthesis of 2-(4-hydroxy-7-trifluoromethylthiochroman-4-yl)acetic acid

The title compound (139.0 mg) was obtained as an orange solid from the compound (150.0 mg) prepared in <Step 1> of Example 64 by the same process as that used in <Step 4> of Example 1.

### <Step 3> Synthesis of (E)-2-(7-trifluoromethylthiochroman-4-ylidene)acetic acid

The title compound (20.0 mg) was obtained as a white solid from the compound (139.0 mg) prepared in <Step 2> of Example 64 by the same process as that used in <Step 4> of Example 60.

### <Step 4> Synthesis of (E)-2-(7-trifluoromethyl-3,4-dihydro-2H-1-benzothiopyran-4-ylidene)-N-((3-(4-morpholinyl)-3,4-dihydro-2(1H)-quinolinon-7-yl)acetamide

The title compound (29.0 mg) was obtained as a pale orange solid from the compound (27.7 mg) prepared in <Step 3> of Example 64 by a similar to the process used in <Step 7> of Example 1.

### (EXAMPLE 65)

Synthesis of (Z)-2-(6-trifluoromethyl-3,3-dimethyl-4-oxa-3,4-dihydroisoqunolin-1(2H)-ylidene)-N-(3-(4-morpholinyl)-3,4-dihydro-2(1H)-quinolinon-7-yl)acetamide

The title compound (6.8 mg) was obtained as a pale yellow solid from the compound (0.15 g) prepared in <Step 3> of Example 30 by a similar to the process used in Example 26.

### (EXAMPLE 66)

Synthesis of (E)-2-(8-trifluoromethyl-3,4-dihydrobenzo[c]oxepin-5(1H)-ylidene)-N-(3,4-dihydro-2(1H)-quinazolinon-5-yl)acetamide

The title compound (99.8 mg) was obtained as a pale yellow solid from (E)-(8-trifluoromethyl-3,4-dihydrobenzoo[c]oxepin-5(1H)-ylidene)acetic acid (117 mg) prepared by the way described in PCT Publication No. 07/010383 pamphlet by a similar to the process used in <Step 7> of Example 1.

The compounds described blow were prepared from a known arylamine represented by formula (IX) described above and a carboxylic acid [formula (VIII) described above] used in Examples described above by a similar to the process used in <Step 7> of Example 1.
(EXAMPLE 67) (E)-2-(7-trifluoromethyl-2,2-diethylchroman-4-ylidene)-N-(3,4-dihydro-1-methyl-2(1H)quinolinon-7-yl)acetamide
(EXAMPLE 68) (E)-2-(8-trifluoromethyl-3,4-dihydrobenzo[b]oxepin-5(2H)-ylidene)-N-(1,4-dihydro-3(2H)-isoquinolinon-6-yl)acetamide
(EXAMPLE 69) (E)-2-(7-trifluoromethyl-2,2-dimethylchroman-4-ylidene)-N-(1,4-dihydro-3(2H)-isoquinolinon-6-yl)acetamide
(EXAMPLE 70) (E)-2-(7-trifluoromethyl-2,2-dimethylchroman-4-ylidene)-N-(3,4-dihydro-2(1H)-quinolinon-5-yl)acetamide
(EXAMPLE 71) (E)-2-(7-trifluoromethyl-spiro[chroman-2,1'-cyclobutane]-4-ylidene)-N-(3,4-dihydro-2(1H)-quinolinon-5-yl)acetamide
(EXAMPLE 72) (E)-2-(7-trifluoromethyl-2,2-bis(methoxymethyl)chroman-4-ylidene)-N-(3,4-dihydro-2(1H)-quinolinon-5-yl)acetamide
(EXAMPLE 73) (E)-2-(7-trifluoromethyl-2,2-dimethylch-romn-4-ylidene)-N-(2-methyl-4H-benzo[1,4]oxazin-3-on-6-yl)acetamide
(EXAMPLE 74) (E)-2-(7-trifluoromethyl-2,2-dimethylchroman-4-ylidene)-N-(2-(2-hydroxyethyl)-4H-benzo[1,4]oxazin-3-on-6-yl)acetamide
(EXAMPLE 75) (E)-2-(7-trifluoromethyl-spiro[chroman-2,1'-cyclobutane]-4-ylidene)-N-(2,2-dimethyl-4H-benzo[1,4]oxazin-3-on-6-yl)acetamide
(EXAMPLE 76) (E)-2-(7-trifluoromethyl-spiro[chroman-2,1'-cyclobutane]-4-ylidene)-N-(2-methyl-4H-benzo[1,4]oxazin-3-on-6-yl)acetamide
(EXAMPLE 77) (E)-2-(7-trifluoromethyl-spiro[chroman-2,1'-cyclobutane]-4-ylidene)-N-(2-(2-hydroxyethyl)-4H-benzo[1,4]oxazin-3-on-6-yl)acetamide
(EXAMPLE 78) (E)-2-(7-trifluoromethyl-2,2-diethylchroman-4-ylidene)-N-(4H-benzo[1,4]oxazin-3-on-6-yl)acetamide
(EXAMPLE 79) (E)-2-(7-trifluoromethyl-2,2-diethylchroman-4-ylidene)-N-(2-methyl-4H-benzo[1,4]oxazin-3-on-6-yl)acetamide
(EXAMPLE 80) (E)-2-(7-trifluoromethyl-2,2-diethylchroman-4-ylidene)-N-(4-methyl-4H-benzo[1,4]oxazin-3-on-6-yl)acetamide
(EXAMPLE 81) (E)-2-(7-trifluoromethyl-2,2-diethylchroman-4-ylidene)-N-(2,4-dimethyl-4H-benzo[1,4]oxazin-3-on-6-yl)acetamide
(EXAMPLE 82) (E)-2-(7-trifluoromethyl-2,2-diethylchroman-4-ylidene)-N-(2,2,4-trimethyl-4H-benzo[1,4]oxazin-3-on-6-yl)acetamide
(EXAMPLE 83) (E)-2-(7-trifluoromethyl-2,2-diethylchroman-4-ylidene)-N-(2-(2-hydroxyethyl)-4H-benzo[1,4]oxazin-3-on-6-yl)acetamide
(EXAMPLE 84) (E)-2-(7-trifluoromethyl-spiro[chroman-2,1'-cyclobutane]-4-ylidene)-N-(2H-benzo[1,4]oxazin-3(4H)-on-8-yl)acetamide
(EXAMPLE 85) (E)-2-(7-trifluoromethyl-2,2-diethylchroman-4-ylidene)-N-(2H-benzo[1,4]oxazin-3(4H)-on-8-yl)acetamide
(EXAMPLE 86) (E)-2-(7-trifluoromethyl-spiro[chromn-2,1'-cyclobutane]-4-ylidene)-N-(4-(2-hydroxyethyl)-3,4-dihydro-2H-benzo[1,4]oxazin-6-yl)acetamide
(EXAMPLE 87) (E)-2-(7-trifluoromethyl-spiro[chroman-2,1'-cyclobutane]-4-ylidene)-N-(3,4-dihydro-4-methyl-2H-benzo[1,4]oxazin-6-yl)acetamide
(EXAMPLE 88) (E)-2-(7-trifluoromethyl-2,2-diethylchroman-4-ylidene)-N-(3,4-dihydro-4-methyl-2H-benzo[1,4]oxazin-6-yl)acetamide
   The compounds described blow were prepared from a arylamine represented by formula (IX) described above prepared by the same process as that used in Example 30 and a carboxylic acid [formula (VIII) described above] used in Examples described above by a similar to the process used in <Step 7> of Example 1.
(EXAMPLE 89) (E)-2-(8-trifluoromethyl-3,4-dihydrobenzo[b]oxepin-5(2H)-ylidene)-N-(3,4-dihydro-3-(N-methyl-N-(2-hydroxyethyl)amino)-2(1H)-quinolinon-7-yl)acetamide
(EXAMPLE 90) (E)-2-(8-trifluoromethyl-3,4-dihydrobenzo[b]oxepin-5(2H)-ylidene)-N-(3-(cis-2,6-dimethylmorpholin-4-yl)-3,4-dihydro-2(1H)-quinolinon-7-yl) acetamide
(EXAMPLE 91) (E)-2-(8-trifluoromethyl-3,4-dihydrobenzo[b]oxepin-5(2H)-ylidene)-N-(3-(4,4-difluoropiperidin-1-yl)-3,4-dihydro-2(1H)-quinolinon-7-yl)acetamide
(EXAMPLE 92) (E)-2-(8-trifluoromethyl-3,4-dihydrobenzo[b]oxepin-5(2H)-ylidene)-N-(3-((S)-3-fluoropyrrolidin-1-yl)-3,4-dihydro-2(1H)-quinolinon-7-yl)acetamide
(EXAMPLE 93)

Synthesis of (E)-2-(8-trifluoromethyl-3,4-dihydrobenzo[b]oxepin-5(2H)-ylidene)-N-(3-hydroxymethyl-2(1H)-quinolinon-7-yl)acetamide

### <Step 1> Synthesis of 3-hydroxymethyl-7-nitro-2(1H)-quinolinone

1 N tetrahydrofuran (63.40 mL) solution of lithium hexamethyldisilazide was added to a tetrahydrofuran (60.0 mL) solution of 3-hydroxypropanoic acid methyl ester (3.00 g) at -50 °C, and the reaction mixture was stirred at -20 °C for 30 minutes. The mixture was cooled to -50 °C, and then a tetrahydrofuran (6.00 mL) solution of 2,4-dinitrobenzaldehyde (5.65 g) was added dropwise thereto. The mixture was stirred at room temperature for one hour. Water was added to the mixture. The mixture was extracted with ethyl acetate. The organic layer was sequentially washed with water and saturated saline solution, and dried over anhydrous sodium sulfate. The solvent was distilled off under reduced pressure. The residue was purified by silica gel column chromatography (eluate; n-hexane:ethyl acetate = 90:10, 50:50 to 0:100).he title compound (1.26 g) was obtained as pale orange oil.

### <Step 2> Synthesis of 7-amino-3-hydroxymethyl-2(1H)-quinolinone

The title compound (0.23 g) was obtained as a brown solid from the compound (3.00 g) prepared in <Step 1> of Example 93 by a similar to the process used in <Step 6> of Example 1.

### <Step 3> Synthesis of (E)-2-(8-trifluoromethyl-3,4-dihydrobenzo[b]oxepin-5(2H)-ylidene)-N-(3-hydroxymethyl-2(1H)-quinolinon-7-yl)acetamide

The title compound (42.8 mg) was obtained as a pale yellow solid from the compound (50.0 mg) prepared in <Step 2> of Example 93 and the compound (71.56 mg) prepared in <Step 4> of Example 1 by a similar to the process used in <Step 7> of Example 1.

The compounds described blow were prepared from the compound in <Step 2> of Example 93 and a carboxylic acid [formula (VIII) described above] used in Examples described above by a similar to the process used in <Step 7> of Example 1.
(EXAMPLE 94) (E)-2-(7-trifluoromethyl-2,2-dimethylchroman-4-ylidene)-N-(3-hydroxymethyl-2(1H)-quinolinon-7-yl)acetamide
(EXAMPLE 95) (E)-2-(7-trifluoromethyl-2,2-diethylchroman-4-ylidene)-N-(3-hydroxymethyl-2(1H)-quinolinon-7-yl)acetamide
(EXAMPLE 96) (E)-2-(7-trifluoromethyl-spiro[chroman-2,1'-cyclobutane]-4-ylidene)-N-(3-hydroxymethyl-2(1H)-quinolinon-7-yl)acetamide
(EXAMPLE 97) Synthesis of (E)-2-(8-trifluoromethyl-3,4-dihydrobenzo[b]oxepin-5(2H)-ylidene)-N-(3-(N-methyl-N-ethyl)amino-2(1H)-quinolinon-7-yl)acetamide

### <Step 1> Synthesis of 3-bromo-1,2-dihydro-7-nitro-2-oxoquinoline

Bromine (1.75 mL) was added to a pyridine (44.0 mL) solution of 1,2-dihydro-7-nitro-2-oxo-3-quinolinecarboxylic acid (4.00 g) at 0 °C, and the mixture was stirred in the range of 100 °C to 120 °C for 1.5 hours. The mixture was left to cool. Subsequently, 1 N hydrochloric acid was added thereto so that the solution became acidic. The precipitate was collected by filtration and washed with water. The title compound (2.78 g) was obtained as a brown solid.

### <Step 2> Synthesis of 3-(N-ethyl-N-methyl)amino-1,2-dihydro-7-amino-2-oxoquinoline

Ethylmethylamine (0.16 mL)was added to a N,N'-dimethylimidazolidinone (1.0 mL) solution of the compound (50.0 mg) prepared in <Step 1> of Example 97, and the mixture was heated at 120 °C for 18 hours in sealed tube. The mixure was left to cool. Water was added to the mixture. The resulting precipitate was collected by filtration. An acetic acid (1.0 mL) and ethyl acetate (1.0 mL) mixed solution of the collected solid was added to an acetic acid (1.0 mL) suspension of iron powder (0.10 g) at 80 °C. The mixture was refluxed for one hour. After cooling, the mixture was subjected to Celite filtration. The filtrate was neutralized with aqueous saturated sodium hydrogen carbonate solution and then extracted with ethyl acetate. The organic layer was washed with saturated saline solution and then dried over anhydrous sodium sulfate. The solvent was distilled off under reduced pressure. The title crude compound (33.0 mg) was obtained.

### <Step 3> Synthesis of (E)-2-(8-trifluoromethyl-3,4-dihydrobenzo[b]oxepin-5(2H)-ylidene)-N-(3-(N-methyl-N-ethyl)amino-2(1H)-quinolinon-7-yl)acetamide

The title compound (34.3 mg) was obtained as a pale yellow solid from the compound (40.00 mg) prepared in <Step 2> of Example 97 and the compound (48.17 mg) prepared in <Step 4> of Example 1 by a similar to the process used in <Step 7> of Example 1.

The compounds described blow were prepared from a arylamine represented by formula (IX) described above prepared by a similar to the process used in Example 58 and a carboxylic acid [formula (VIII) described above] used in Examples described above by a similar to the process used in <Step 7> of Example 1.
(EXAMPLE 98) (E)-2-(8-trifluoromethyl-3,4-dihydrobenzo[b]oxepin-5(2H)-ylidene)-N-(3-(4,4-difluoropiperidin-1-yl)-2(1H)-quinolinon-7-yl)acetamide
(EXAMPLE 99) (E)-2-(8-trifluoromethyl-3,4-dihydrobenzo[b]oxepin-5(2H)-ylidene)-N-(3-(4,4-difluoropiperidin-l-yl)-1-methyl-2(1H)-quinolinon-7-yl)acetamide
(EXAMPLE 100) (E)-2-(7-trifluoromethyl-2,2-dimethylchroman-4-ylidene)-N-(3-(4,4-difluoropiperidin-1-yl)-1-methyl-2(1H)-quinolinon-7-yl)acetamide
(EXAMPLE 101) (E)-2-(7-trifluoromethyl-spiro[chroman-2,1'-cyclobutane]-4-ylidene)-N-(3-(4,4-difluoropiperidin-1-yl)-1-methyl-2(1H)-quinolinon-7-yl)acetamide
(EXAMPLE 102) (E)-2-(8-trifluoromethyl-3,4-dihydrobenzo[b]oxepin-5(2H)-ylidene)-N-(3-(4-thiomorpholinyl)-2(1H)-quinolinon-7-yl)acetamide
(EXAMPLE 103) (E)-2-(8-trifluoromethyl-3,4-dihydrobenzo[b]oxepin-5(2H)-ylidene)-N-(3-(cis-2,6-dimethylmorpholin-4-yl)-2(1H)-quinolinon-7-yl)acetamide

The compounds described blow were prepared from 6-amino-4-(2-tert-butyldimethylsiloxyethyl)-4H-benzo[1,4]oxazin-3-one prepared from 6-nitro-4H-benzo[1,4]-oxazin-3-one by a similar to the process used in Example 39 and a carboxylic acid [formula (VIII) described above] used in Examples described above by a similar to the process used in <Step 7> of Example 1.
(EXAMPLE 104) (E)-2-(7-trifluoromethyl-2,2-dimethylchroman-4-ylidene)-N-(4-(2-hydroxyethyl)-4H-benzo[1,4]oxazin-3-on-6-yl)acetamide
(EXAMPLE 105) (E)-2-(7-trifluoromethyl-2,2-diethylchroman-4-ylidene)-N-(4-(2-hydroxyethyl)-4H-benzo[1,4]oxazin-3-on-6-yl)acetamide
(EXAMPLE 106) (E)-2-(7-trifluoromethyl-spiro[chroman-2,1'-cyclobutane]-4-ylidene)-N-(4-(2-hydroxyethyl)-4H-benzo[1,4]oxazin-3-on-6-yl)acetamide

The compounds described blow were prepared from 6-amino-2-(2-tert-butyldimethylsiloxyethyl)-4-methyl-4H-benzo[1,4]oxazin-3-one prepared from 2-(2-hydroxyethyl)-6-nitro-4H-benzo[1,4]-oxazin-3-oneby a similar to the process used in <Step2> of Example 15 and Example 39 and a carboxylic acid [formula (VIII) described above] used in Examples described above by a similar to the process used in <Step 7> of Example 1.
(EXAMPLE 107) (E)-2-(7-trifluoromethyl-2,2-diethylchroman-4-ylidene)-N-(2-(2-hydroxyethyl)-4-methyl-4H-benzo[1,4]oxazin-3-on-6-yl)acetamide
(EXAMPLE 108) (E)-2-(7-trifluoromethyl-spiro[chroman-2,1'-cyclobutane]-4-ylidene)-N-(2-(2-hydroxyethyl)-4-methyl-4H-benzo[1,4]oxazain-3-on-6-yl)acetamide
(EXAMPLE 109) Synthesis of (E)-2-(7-trifluoromethyl-2,2-dimethylchroman-4-ylidene)-N-(3,3-dimethyl-2(1H)-indolinon-6-yl)acetamide

### <Step 1> 2,4-dinitrophenylacetic acid methyl ester

10% hydrogen chloride in methanol (50.0 mL) was added to a methanol (150 mL) solution of 2,4-dinitrophenylacetic acid (25.0 g), the resulting mixture was refluxed for 5 hours. The mixture was left to cool. The solvent was distilled off under reduced pressure. The residue was extracted with ethyl acetate. The organic layer was sequentially washed with aqueous saturated sodium hydrogencarobonate solution and a saturated saline solution, and then dried over anhydrous sodium sulfate. The solvent was distilled off under reduced pressure. The title crude compound (28.6 g) was obtained as pale orange oil.

### <Step 2> Synthesis of 2-methyl-2-(2,4-dinitrophenyl)propanoic acid methyl ester

A tetrahydrofuran (150.0 mL) solution of the compound (27.10 g) prepared in <Step 1> at Example 109 was added dropwise to a tetrahydrofuran (150.0 mL) suspension of sodium hydride (13.54 g) and iodomethane (35.12 mL) over a period of 30 minutes at 0 °C. The mixture was stirred at room temperature for 2 hours and refluxed for 6 hours. The mixture was left to cool. Aqueous saturated ammonium chloride solution was added to the mixture, and then was extracted with ethyl acetate. The organic layer was washed with saturated saline solution, and then dried over anhydrous sodium sulfate. The solvent was distilled off under reduced pressure. The residue was purified by silica gel column chromatography (eluate; n-hexane:ethyl acetate = 100:0, 95:5 to 90:10).The title compound (6.3 g) was obtained as pale orange oil.

### <Step 3> Synthesis of 6-amino-3,3-dimethylindolin-2-one

The title compound (1.3 g) was obtained as a brown solid from the compound (6.30 g) prepared in <Step 2> of Example 109 by a similar to the process used in <Step 6> of Example 1.

### <Step 4> Synthesis of (E)-2-(7-trifluoromethyl-2,2-dimethylchroman-4-ylidene)-N-(3,3-dimethyl-2(1H)-indolinon-6-yl)acetamide

The title compound (91.9 mg) was obtained as pale white solid from the compound (50.0 mg) prepared in <Step 3> of Example 109 and the compound (0.12 g) prepared in <Step 5> of Example 24 by the same process as that used in <Step 7> of Example 1.

The compounds described blow were prepared from the compound prepared in <Step 3> of Example 109 and a carboxylic acid [formula (VIII) described above] used in Examples described above by a similar to the process used in <Step 7> of Example 1.
(EXAMPLE 110) (E)-2-(7-trifluoromethyl-spiro[chroman-2,1'-cyclobutane]-4-ylidene)-N-(3,3-dimethyl-2(1H)-indolinon-6-yl)acetamide
(EXAMPLE 111) (E)-2-(7-trifluoromethyl-2,2-bis(methoxymethyl)chroman-4-ylidene)-N-(3,3-dimethyl-2(1H)-indolinon-6-yl)acetamide

The compounds described blow were prepared from arylamine represented by formula (IX) described above prepared by a similar to the process used in Example 14 and a carboxylic acid [formula (VIII) described above] used in Examples described above by a similar to the process used in <Step 7> of Example 1.
(EXAMPLE 112) (E)-2-(8-trifluoromethyl-3,4-dihydrobenzo[b]oxepin-5(2H)-ylidene)-N-(3-ethyl-3,4-dihydro-2(1H)-quinazolinon-7-yl)acetamide
(EXAMPLE 113) (E)-2-(7-trifluoromethyl-2,2-dimethylchroman-4-ylidene)-N-(3-ethyl-3,4-dihydro-2(1H)-quinazolinon-7-yl)acetamide
(EXAMPLE 114) (E)-2-(7-trifluoromethyl-2,2-diethylchroman-4-ylidene)-N-(3-ethyl-3,4-dihydro-2(1H)-quinazolinon-7-yl)acetamide
(EXAMPLE 115) (E)-2-(7-trifluoromethyl-spiro[chroman-2,1'-cyclobutane]-4-ylidene)-N-(3-ethyl-3,4-dihydro-2(1H)-quinazolinon-7-yl)acetamide
(EXAMPLE 116) (E)-2-(8-trifluoromethyl-3,4-dihydrobenzo[b]oxepin-5(2H)-ylidene)-N-(3,4-dihydro-3-(1-methylethyl)- 2(1H)-quinazolinon-7-yl)acetamide
(EXAMPLE 117) (E)-2-(7-trifluoromethyl-2,2-dimethylchroman-4-ylidene)-N-(3,4-dihydro-3-(1-methylethyl)-2(1H)-quinazolinon-7-yl)acetamide
(EXAMPLE 118) (E)-2-(7-trifluoromethyl-2,2-diethylchroman-4-ylidene)-N-(3,4-dihydro-3-(1-methylethyl)-2(1H)-quinazolinon-7-yl)acetamide
(EXAMPLE 119) (E)-2-(7-trifluoromethyl-spiro[chroman-2,1'-cyclobutane]-4-ylidene)-N-(3,4-dihydro-3-(1-methylethyl)-2(1H)-quinazolinon-7-yl)acetamide
(EXAMPLE 120) (E)-2-(7-trifluoromethyl-2,2-dimethylchroman-4-ylidene)-N-(3,4-di-hydro-3-(2-hydroxyethyl)- 2(1H)-quinazolinon-7-yl)acetamide
(EXAMPLE 121) (E)-2-(7-trifluoromethyl-2,2-diethylchroman-4-ylidene)-N-(3,4-dihydro-3-(2-hydroxyethyl)-2(1H)-quinazolinon-7-yl)acetamide
(EXAMPLE 122) (E)-2-(8-trifluoromethyl-3,4-dihydrobenzo[b]oxepin-5(2H)-ylidene)-N-(3,4-dihydro-3-(4-hydroxybutyl)- 2(1H)-quinazolinon-7-yl)acetamide
(EXAMPLE 123) (E)-2-(7-trifluoromethyl-2,2-dimethylchroman-4-ylidene)-N-(3,4-dihydro-3-(4-hydroxybutyl)- 2(1H)-quinazolinon-7-yl)acetamide
(EXAMPLE 124) (E)-2-(7-trifluoromethyl-2,2-diethylchroman-4-ylidene)-N-(3,4-dihydro-3-(4-hydroxybutyl)- 2(1H)-quinazolinon-7-yl)acetamide
(EXAMPLE 125) (E)-2-(7-trifluoromethyl-spiro[chroman-2,1'-cyclobutane]-4-ylidene)-N-(3,4-dihydro-3-(4-hydroxybutyl)-2(1H)-quinazolinon-7-yl)acetamide
(EXAMPLE 126) (E)-2-(8-trifluoromethyl-3,4-dihydrobenzo[b]oxepin-5(2H)-ylidene)-N-(3,4-dihydro-3-((S)-2-hydroxypropyl)-2(1H)-quinazolinon-7-yl)acetamide
(EXAMPLE 127) (E)-2-(7-trifluoromethyl-2,2-dimethylchroman-4-ylidene)-N-(3,4-dihydro-3-((S)-2-hydroxypropyl)-2(1H)-quinazolinon-7-yl)acetamide
(EXAMPLE 128) (E)-2-(7-trifluoromethyl-2,2-diethylchroman-4-ylidene)-N-(3,4-dihydro-3-((S)-2-hydroxypropyl)-2(1H)-quinazolinon-7-yl)acetamide
(EXAMPLE 129) (E)-2-(7-trifluoromethyl-spiro[chroman-2,1'-cyclobutane]-4-ylidene)-N-(3,4-dihydro-3-((S)-2-hydroxypropyl)-2(1H)-quinazolinon-7-yl) acetamide
(EXAMPLE 130) (E)-2-(8-trifluoromethyl-3,4-dihydrobenzo[b]oxepin-5(2H)-ylidene)-N-(3,4-dihydro-3-((R)-2-hydroxypropyl)-2(1H)-quinazolinon-7-yl)acetamide
(EXAMPLE 131) (E)-2-(7-trifluoromethyl-2,2-dimethylchroman-4-ylidene)-N-(3,4-dihydro-3-((R)-2-hydroxypropyl)-2(1H)-quinazolinon-7-yl)acetamide
(EXAMPLE 132) (E)-2-(7-trifluoromethyl-2,2-diethylchroman-4-ylidene)-N-(3,4-dihydro-3-((R)-2-hydroxypropyl)-2(1H)-quinazolinon-7-yl)acetamide
(EXAMPLE 133) (E)-2-(7-trifluo-romethyl-spiro[chroman-2,1'-cyclobutane]-4-ylidene)-N-(3,4-dihydro-3-((R)-2-hydroxpropyl)-2(1H)-quinazolinon-7-yl) acetamide
(EXAMPLE 134) (E)-2-(8-trifluoromethyl-3,4-dihydrobenzo[b]oxepin-5(2H)-ylidene)-N-(3,4-dihydro-3-(2-hydroxy-(S)-1-methylethyl)-2(1H)-quinazolinon-7-yl)acetamide
(EXAMPLE 135) (E)-2-(7-trifluoromethyl-2,2-dimethylchroman-4-ylidene)-N-(3,4-dihydro-3-(2-hydroxy-(S)-1-methylethyl-2(1H)-quinazolinon-7-yl)acetamide
(EXAMPLE 136) (E)-2-(7-trifluoromethyl-2,2-diethylchroman-4-ylidene)-N-(3,4-dihydro-3-(2-hydroxy-(S)-1-methylethyl)-2(1H)-quinazolinon-7-yl)acetamide
(EXAMPLE 137) (E)-2-(7-trifluoromethyl-spiro[chroman-2,1'-cyclobutane]-4-ylidene)-N-(3,4-dihydro-3-(2-hydroxy-(S)-1-methylethyl)-2(1H)-quinazolinon-7-yl)acetamide
(EXAMPLE 138) (E)-2-(7-trifluoromethyl-2,2-dimethylchroman-4-ylidene)-N-(3,4-dihydro-3-(2-hydroxy-(R)-1-methylethyl)-2(1H)-quinazolinon-7-yl)acetamide
(EXAMPLE 139) (E)-2-(7-trifluoromethyl-2,2-diethylchroman-4-ylidene)-N-(3,4-dihydro-3-(2-hydroxy-(R)-1-methylethyl)-2(1H)-quinazolinon-7-yl)acetamide
(EXAMPLE 140) (E)-2-(7-trifluoromethyl-spiro[chroman-2,1'-cyclobutane]-4-ylidene)-N-(3,4-dihydro-3-(2-hydroxy-(R)-1-methylethyl)-2(1H)-quinazolinon-7-yl)acetamide
(EXAMPLE 141) (E)-2-(7-trifluoromethyl-2,2-dimethylchroman-4-ylidene)-N-(3,4-dihydro-3-(2-hydroxy-1-(hydroxymethyl)ethyl)-2(1H)-quinazolinon-7-yl)acetamide
(EXAMPLE 142) (E)-2-(8-trifluoromethyl-3,4-dihydrobenzo[b]oxepin-5(2H)-ylidene)-N-(3,4-dihydro-3-(2-hydroxy-2-methylpropyl)-2(1H)-quinazolinon-7-yl)acetamide
(EXAMPLE 143) (E)-2-(7-trifluoromethyl-2,2-dimethylchroman-4-ylidene)-N-(3,4-dihydro-3-(2-hydroxy-2-methylpropyl)-2(1H)-quinazolinon-7-yl)acetamide
(EXAMPLE 144) (E)-2-(7-trifluoromethyl-spiro[chromn-2,1'-cyclobutane]-4-ylidene)-N-(3,4-dihydro-3-(2-hydroxy-2-methylpropyl)-2(1H)-quinazolinon-7-yl)acetamide
(EXAMPLE 145) (E)-2-(7-trifluoromethyl-2,2-diethylchroman-4-ylidene)-N-(3,4-dihydro-3-(2-hydroxy-2-methylpropyl)-2(1H)-quinazolinon-7-yl)acetamide
(EXAMPLE 146) (E)-2-(8-trifluoromethyl-3,4-dihydrobenzo[b]oxepin-5(2H)-ylidene)-N-(3,4-dihydro-3-(tetrahydrofuran-2-ylmethyl)-2(1H)-quinazolinon-7-yl)acetamide
(EXAMPLE 147) (E)-2-(7-trifluoromethyl-2,2-dimethylchroman-4-ylidene)-N-(3,4-dihydro-3-(tetrahydrofuran-2-ylmethyl)-2(1H)-quinazolinon-7-yl)acetamide
(EXAMPLE 148) (E)-2-(7-trifluoromethyl-spiro[chroman-2,1'-cyclobutane]-4-ylidene)-N-(3,4-dihydro-3-(tetrahydrofuran-2-ylmethyl)-2(1H)-quinazolinon-7-yl)acetamide
(EXAMPLE 149) (E)-2-(7-trifluoromethyl-2,2-diethylchroman-4-ylidene)-N-(3,4-dihydro-3-(tetrahydrofuran-2-ylmethyl)-2(1H)-quinazolinon-7-yl)acetamide

The compounds described blow were prepared from arylamine, represented by formula (IX) described above prepared by a similar to the process used in Example 38 and Example 39, and a carboxylic acid [formula (VIII) described above] used in Examples described above by a similar to the process used in <Step 7> of Example 1.
(EXAMPLE 150) (E)-2-(8-trifluoromethyl-3,4-dihydrobenzo[b]oxepin-5 (2H)-ylidene)-N-(3,4-dihydro-1-methyl-2(1H)-quinazolinon-7-yl)acetamide
(EXAMPLE 151) (E)-2-(7-trifluoromethyl-chroman-4-yliden)-N-(3,4-dihydro-1-methyl-2(1H)-quinazolinon-7-yl)acetamide
(EXAMPLE 152) (E)-2-(7-trifluoromethyl-2,2-dimethylchromn-4-ylidene)-N-(3,4-dihydro-1-methyl-2(1H)-quinazolinon-7-yl)acetamide
(EXAMPLE 153) (E)-2-(7-trifluoromethyl-spiro[chroman-2,1'-cyclobutane]-4-ylidene)-N-(3,4-dihydro-1-methyl-2(1H)-quinazolinon-7-yl)acetamide
(EXAMPLE 154) (E)-2-(7-trifluoromethyl-2,2-diethylchroman-4-ylidene)-N-(1-methyl-3,4-dihydro-2(1H)-quinazolinon-7-yl)acetamide
(EXAMPLE 155) (E)-2-(8-trifluoromethyl-3,4-dihydrobenzo[b]oxepin-5(2H)-ylidene)-N-(3,4-dihydro-1-ethyl- 2(1H)-quinazolinon-7-yl)acetamide
(EXAMPLE 156) (E)-2-(7-trifluoromethyl-2,2-dimethylchroman-4-ylidene)-N-(3,4-dihydro-1-ethyl-2(1H)-quinazolinon-7-yl)acetamide
(EXAMPLE 157) (E)-2-(7-trifluoromethyl-spiro[chroman-2,1'-cyclobutane]-4-ylidene)-N-(3,4-dihydro-1-ethyl-2(1H)-quinazolinon-7-yl)acetamide
(EXAMPLE 158) (E)-2-(8-trifluoromethyl-3,4-dihydrobenzo[b]oxepin-5(2H)-ylidene)-N-(3,4-dihydro-3-ethyl-1-methyl-2(1H)-quinazolinon-7-yl)acetamide
(EXAMPLE 159) (E)-2-(7-trifluoromethyl-2,2-dimethylchroman-4-ylidene)-N-(3,4-dihydro-3-ethyl-1-methyl-2(1H)-quinazolinon-7-yl)acetamide
(EXAMPLE 160) (E)-2-(7-trifluoromethyl-2,2-diethylchroman-4-ylidene)-N-(3,4-dihydro-3-ethyl-1-methyl-2(1H)-quinazolinon-7-yl)acetamide
(EXAMPLE 161) (E)-2-(7-trifluoromethyl-spiro[chroman-2,1'-cyclobutane]-4-ylidene)-N-(3-ethyl-3,4-dihydro-1-methyl-2(1H)-quinazolinon-7-yl)acetamide
(EXAMPLE 162) (E)-2-(8-trifluoromethyl-3,4-dihydrobenzo[b]oxepin-5(2H)-ylidene)-N-(3,4-dihydro-1-methyl-3-(1-methylethyl)-2(1H)-quinazolinon-7-yl)acetamide
(EXAMPLE 163) (E)-2-(7-trifluoromethyl-2,2-dimethylchroman-4-ylidene)-N-(3,4-dihydro-3-(1-methylethyl)-1-methyl-2(1H)-quinazolinon-7-yl)acetamide
(EXAMPLE 164) (E)-2-(7-trifluoromethyl-2,2-diethylchroman-4-ylidene)-N-(3,4-dihydro-3-(1-methylethyl)-1-methyl-2(1H)-quinazolinon-7-yl)acetamide
(EXAMPLE 165) (E)-2-(7-trifluoromethyl-2,2-dimethylchroman-4-ylidene)-N-(3,4-dihydro-3-(2-hydroxyethyl)-1-methyl-2(1H)-quinazolinon-7-yl)acetamide
(EXAMPLE 166) (E)-2-(7-trifluoromethyl-2,2-diethylchroman-4-ylidene)-N-(3,4-dihydro-3-(2-hydroxyethyl)-1-methyl-2(1H)-quinazolinon-7-yl)acetamide
(EXAMPLE 167) (E)-2-(7-trifluoromethyl-spiro[chroman-2,1'-cyclobutane]-4-ylidene)-N-(3,4-dihydro-3-(2-hydroxyethyl)-1-methyl-2(1H)-quinazolinon-7-yl)acetamide
(EXAMPLE 168) (E)-2-(7-trifluoromethyl-2,2-dimethylchroman-4-ylidene)-N-(3,4-dihydro-3-(3-hydroxypropyl)-1-methyl-2(1H)-quinazolinon-7-yl)acetamide
(EXAMPLE 169) (E)-2-(7-trifluoromethyl-2,2-diethylchroman-4-ylidene)-N-(3,4-dihydro-3-(3-hydroxypropyl)-1-methyl-2(1H)-quinazolinon-7-yl)acetamide
(EXAMPLE 170) (E)-2-(7-trifluoromethyl-spiro[chroman-2,1'-cyclobutan]-4-yliden)-N-(3,4-dihydro-3-(3-hydroxypropyl)-1-methyl- 2(1H)-quinazolinon-7-yl)acetamide
(EXAMPLE 171) (E)-2-(8-trifluoromethyl-3,4-dihydrobenzo[b]oxepin-5(2H)-ylidene)-N-(3,4-dihydro-3-(2-hydroxypropyl)-1-methyl-2(1H)-quinazolinon-7-yl)acetamide
(EXAMPLE 172) (E)-2-(7-trifluoromethyl-2,2-dimethylchroman-4-ylidene)-N-(3,4-dihydro-3-(2-hydroxy-2-methylpropyl)-1-methyl-2(1H)-quinazolinon-7-yl)acetamide
(EXAMPLE 173) (E)-2-(7-trifluoromethyl-spiro[chroman-2,1'-cyclobutane]-4-ylidene)-N-(3,4-dihydro-3-(2-hydroxy-2-methylpropyl)-1-methyl-2(1H)-quinazolinon-7-yl)acetamide
(EXAMPLE 174) (E)-2-(7-trifluoromethyl-2,2-diethylchroman-4-ylidene)-N-(3,4-dihydro-3-(2-hydroxy-2-methylpropyl)-1-methyl-2(1H)-quinazolinon-7-yl)acetamide
(EXAMPLE 175) (E)-2-(8-trifluoromethyl-3,4-dihydrobenzo[b]oxepin-5(2H)-ylidene)-N-(3,4-dihydro-3-(2-hydroxy-1-(hydroxymethyl)ethyl)-1-methyl-2(1H)-quinazolinon-7-yl)acetamide
(EXAMPLE 176) (E)-2-(7-trifluoromethyl-2,2-dimethylchroman-4-ylidene)-N-(3,4-dihydro-3-(2-hydroxy-1-hydroxymethylethyl)-1-methyl-2(1H)-quinazolinon-7-yl)acetamide
(EXAMPLE 177) (E)-2-(7-trifluoromethyl-2,2-diethylchroman-4-ylidene)-N-(3,4-dihydro-3-(tetrahydropyran-4-yl)-1-methyl-2(1H)-quinazolinon-7-yl)acetamide
(EXAMPLE 178) (E)-2-(7-trifluoromethyl-spiro[chroman-2,1'-cyclobutane]-4-ylidene)-N-(3,4-dihydro-3-(tetrahydropyran-4-yl)-1-methyl-2(1H)-quinazolinon-7-yl)acetamide
(EXAMPLE 179) (E)-2-(7-trifluoromethyl-2,2-dimethylchroman-4-ylidene)-N-(3,4-dihydro-3-(tetrahydrofuran-2-ylmethyl)-1-methyl-2(1H)-quinazolinon-7-yl)acetamide

The compounds described blow were prepared from 5-amino-3,4-dihydro-1H-quinazolin-2-one prepared by a similar to the process used in Example 13 and a carboxylic acid [formula (VIII) described above] used in Examples described above by a similar to the process used in <Step 7> of Example 1.
(EXAMPLE 180) (E)-2-(8-trifluoromethyl-3,4-dihydrobenzo[b]oxepin-5(2H)-ylidene)-N-(3,4-dihydro-2(1H)-quinazolinon-5-yl)acetamide
(EXAMPLE 181) (E)-2-(7-trifluoromethyl-2,2-dimethylchroman-4-ylidene)-N-(3,4-dihydro-2(1H)-quinazolinon-5-yl)acetamide
(EXAMPLE 182) (E)-2-(7-trifluoromethyl-2,2-diethylchroman-4-ylidene)-N-(3,4-dihydro-2(1H)-quinazolinon-5-yl)acetamide
(EXAMPLE 183) (E)-2-(7-trifluoromethyl-spiro[chroman-2,1'-cyclobutane]-4-ylidene)-N-(3,4-dihydro-2(1H)-quinazolinon-5-yl)acetamide

The compounds described blow were prepared from 5-amino-3,4-dihydro-2,2-dioxo-1H-2,1,3-benzothiadiazine prepared by a similar to the process used in Example 17 and a carboxylic acid [formula (VIII) described above] used in Examples described above by a similar to the process used in <Step 7> of Example 1.
(EXAMPLE 184) (E)-2-(7-trifluoromethyl-2,2-dimethylchroman-4-ylidene)-N-(3,4-dihydro-2,2-dioxo-1H-2,1,3-benzothiadiazin-5-yl)acetamide
(EXAMPLE 185) (E)-2-(7-trifluoromethyl-2,2-diethylchroman-4-ylidene)-N-(3,4-dihydro-2,2-dioxo-1H- 2,1,3-benzothiadiazin-5-yl)acetamide
(EXAMPLE 186) (E)-2-(7-trifluoromethyl-spiro[chroman-2,1'-cyclobutane]-4-ylidene)-N-(3,4-dihydro-2,2-dioxo-1H- 2,1,3-benzothiadiazin-5-yl)acetamide

The compounds described blow were prepared from arylamine represented by formula (IX) described above prepared by a similar process used in <Step 1> of Example 38 and Example 17, and the compound prepared in <Step 5> of Example 24 by a similar to the process used in <Step 7> of Example 1.
(EXAMPLE 187) (E)-2-(7-trifluoromethyl-2,2-dimethylchroman-4-ylidene)-N-(3,4-dihydro-2,2-dioxo-3-methyl-1H- 2,1,3-benzothiadiazin-7-yl)acetamide
(EXAMPLE 188) (E)-2-(7-trifluoromethyl-2,2-dimethylchroman-4-ylidene)-N-(3,4-dihydro-2,2-dioxo-3-(2-hydroxyethyl)-1H- 2,1,3-benzothiadiazin-7-yl)acetamide

The compounds described blow were prepared from the compound prepared in <Step 1> of Example 59 and a carboxylic acid [formula (VIII) described above] used in Examples described above by a similar to the process used in <Step 7> of Example 1.
(EXAMPLE 189) (E)-2-(7-trifluoromethyl-2,2-dimethylchromn-4-ylidene)-N-(2,2-dioxo-3,4-dihydro-1H-2,1-benzothiazin-7-yl)acetamide
(EXAMPLE 190) (E)-2-(7-trifluoromethyl-2,2-diethylchronan-4-ylidene)-N-(3,4-dihydro-2,2-dioxo-1H- 2,1-benzothiazin-7-yl)acetamide
(EXAMPLE 191) (E)-2-(7-trifluoromethyl-spiro[chroman-2,1'-cyclobutane]-4-ylidene)-N-(2,2-dioxo-3,4-dihydro-1H-2,1-benzothiazin-7-yl)acetamide
(EXAMPLE 192) (E)-2-(7-trifluoromethyl-2,2-bis(methoxymethyl)chroman-4-ylidene)-N-(2,2-dioxo-3,4-dihydro-1H-2,1-benzothiazin-7-yl)acetamide

The compounds described blow were prepared from the compound prepared in <Step 1> of Example 59 by a similar to the process used in Example 26.
(EXAMPLE 193) (Z)-2-(6-trifluoromethyl-3,3-dimethyl-4-oxa-3,4-dihydroisoqunolin-1(2H)-ylidene)-N-(2,2-dioxo-3,4-dihydro-1H-2,1-benzothiazin-7-yl)acetamide

The compounds described blow were prepared from 5-amino-2,2-dioxy-3,4-dihydro(1H)-2,1-benzothiazine prepared by a similar to the process used in Example 59 and a carboxylic acid [formula (VIII) described above] used in Examples described above by a similar to the process used in <Step 7> of Example 1.
(EXAMPLE 194) (E)-2-(8-trifluoromethyl-3,4-dihydrobenzo[b]oxepin-5(2H)-ylidene)-N-(3,4-dihydro-2,2-dioxo-1H- 2,1-benzothiazin-5-yl)acetamide
(EXAMPLE 195) (E)-2-(7-trifluoromethyl-2,2-dimethylchroman-4-ylidene)-N-(3,4-dihydro-2,2-dioxo-1H- 2,1-benzothiazin-5-yl)acetamide
(EXAMPLE 196) (E)-2-(7-trifluoromethyl-2,2-diethylchroman-4-ylidene)-N-(2,2-dioxo-3,4-dihydro-1H-2,1-benzothiazin-5-yl)acetamide
(EXAMPLE 197) (E)-2-(7-trifluoromethyl-spiro[chroman-2,1'-cyclobutane]-4-ylidene)-N-(3,4-dihydro-2,2-dioxo-1H- 2,1-benzothiazin-5-yl)acetamide
(EXAMPLE 198) Synthesis of (E)-2-(7-trifluoromethyl-2,2-dimethylchroman-4-ylidene)-N-(3,4-dihydro-2,2-dioxo-1-methyl-1H-2,1-benzothiazin-7-yl)acetamide

Potassium carbonate (1.63 mg) and iodomethane (3.04 mg) were added sequentially to a N,N-dimethylformamide (1.00 mL) solution of the compound (5.00 mg) prepared in example 189, and the mixture was stirred at room temperature for three hours. Water was added to the reaction mixture, and extracted with ethyl acetate. The organic layer was washed with saturated saline solution, dried over anhydrous sodium sulfate, and the solvent was distilled off under reduced pressure. The residue was purified by preparative thin layer chromatography (developing solvent; n-hexane:ethyl acetate = 50:50) to give title compound (4.6 mg) as a white solid.

The compounds described below were prepared from the compound prepared in Example 190 or the compound prepared in Example 191 by a similar to the process used in Example 198.
(EXAMPLE 199) (E)-2-(7-trifluoromethyl-2,2-diethylchroman-4-ylidene)-N-(3,4-dihydro-1-methyl-2,2-dioxo-1H-2,1-benzothiazin-7-yl)acetamide
(EXAMPLE 200) (E)-2-(7-trifluoromethyl-spiro[chroman-2,1'-cyclobutane]-4-ylidene)-N-(3,4-dihydro-2,2-dioxo-1-methyl-1H- 2,1-benzothiazin-7-yl)acetamide
(EXAMPLE 201) Synthesis of (E)-2-(7-trifluoromethyl-spiro[chroman-2,1'-cyclobutane]-4-ylidene)-N-(6-chloro-2H-1,4-benzoxazin-3(4H)-on-8-yl) acetamide

### <step 1> Synthesis of 8-amino-6-chloro-4H-benzo[1,4]oxazin-3-one

A tetrahydrofuran (14.0 mL)-ethanol (7.0 mL) solution of 6-chloro-8-nitro-4H-benzo[1,4]oxazin-3-one (0.40 g) was added dropwise to a water (21.0 mL) solution of sodium hydrosulfite (4.90 g) at 0 °C. The mixture was stirred at same temperature for one hour, and stirred at room temperature overnight. A saturated sodium hydrogen carbonate aqueous solution was added to the reaction mixture, and extracted with dichloromethane. The organic layer was washed with saturated saline solution, and dried over anhydrous sodium sulfate. The solvent distilled off under reduced pressure to give the title compound (0.24 g) as a pale brown solid.

### <step 2> Synthesis of (E)-2-(7-trifluoromethyl-spiro[chroman-2,1'-cyclobutane]-4-ylidene)-N-(6-chloro-2H-1,4-benzoxazin-3(4H)-on-8-yl)acetamide

The title compound (81.0 mg) was obtained as a yellow solid from the compound (50.0 mg) prepared in <Step 1> of Example 201 and the (E)-2-(7-trifluoromethyl-spiro[chroman-2,1'cyclobutane]-4-ylidene)acetic acid (79.12 mg) by the same process as that used in <Step 7> of Example 1.

The compounds described blow were prepared from arylamine represented by formula (IX) described above prepared from 6-chloro-8-nitro-4H-benzo[1,4]oxazin-3-one by a similar to the process used in Example 39 and a carboxylic acid [formula (VIII) described above] used in Examples described above by a similar to the process used in <Step 7> of Example 1.
(EXAMPLE 202) (E)-2-(7-trifluoromethyl-2,2-diethylchroman-4-ylidene)-N-(4-methyl-2H-benzo[1,4]oxazin-3(4H)-on-8-yl)acetamide
(EXAMPLE 203) (E)-2-(7-trifluoromethyl-spiro[chroman-2,1'-cyclobutane]-4-ylidene)-N-(4-methyl-2H-benzo[1,4]oxazin-3(4H)-on-8-yl)acetamide
(EXAMPLE 204) (E)-2-(8-trifluoromethyl-3,4-dihydrobenzo[b]oxepin-5(2H)-ylidene)-N-(4-ethyl-2H-benzo[1,4]oxazin-3(4H)-on-8-yl)acetamide
(EXAMPLE 205) (E)-2-(7-trifluoromethyl-2,2-dimethylchroman-4-ylidene)-N-(4-ethyl-2H-benzo[1,4]oxazin-3(4H)-on-8-yl) acetamide
(EXAMPLE 206) (E)-2-(7-trifluoromethyl-2,2-diethylchroman-4-ylidene)-N-(4-ethyl-2H-benzo [1,4]oxazin-3(4H)-on-8-yl)acetamide
(EXAMPLE 207) (E)-2-(7-trifluoromethyl-spiro[chromn-2,1'-cyclobutane]-4-ylidene)-N-(4-ethyl-2H-benzo[1,4]oxazin-3(4H)-on-8-yl)acetamide
(EXAMPLE 208) (E)-2-(7-trifluoromethyl-2,2-diethylchroman-4-ylidene)-N-(4-(3-hydroxypropyl)- 2H-benzo[1,4]oxazin-3(4H)-on-8-yl)acetamide
(EXAMPLE 209) (E)-2-(7-trifluoromethyl-spiro[chroman-2,1'-cyclobutane]-4-ylidene)-N-(4-(3-hydroxypropyl)- 2H-benzo[1,4]oxazin-3(4H)-on-8-yl)acetamide
(EXAMPLE 210) Synthesis of (E)-2-(8-trifluoromethyl-3,4-dihydrobenzo[b]oxepin-5(2H)-ylidene)-N-(3-(4-morpholinyl)-3,4-dihydro-2(1H)-quinolinon-7-yl)acetamide hydrochloride

10% hydrochloric acid methanol solution (112 µL) was added to a dichloromethane (6.0 mL)-methanol (6.0 mL) solution of the compound (0.14 g) prepared in <step 4> of example 30, and the mixture was stirred at room temperature for three hours. The solvent was distilled off under reduced pressure. Ether was added to the residue, and solidified to give title compound (131 mg) as a pale yellow solid.
(EXAMPLE 211) Synthesis of (E)-2-(8-trifluoromethyl-3,4-dihydrobenzo[b]oxepin-5(2H)-ylidene)-N-(3-(4-morpholinyl)-3,4-dihydro-2(1H)-quinolinon-7-yl)acetamide methanesulfonate

The title compound (141 mg) was obtained as a pale yellow solid from the compound (0.14g) prepared in <Step 4> of Example 30 and the methanesulfonic acid (310 µL) by a similar to the process used in Example 210.
(EXAMPLE 212,213) Resolution of optically active isomers of (E)-2-(7-trifluoromethyl-2,2-dimethylchroman-4-ylidene)-N-(3-(4-morpholinyl)-3,4-dihydro-2(1H)-quinolinon-7-yl)acetamide

The title optically active compounds were obtained from the enantiomers (each enantiomer; 1.20 g) prepared by chiral resolution of the compound prepared in <step 3> of Example 30 and the compound (1.39 g) prepared in <Step 5> of Example 24 by a similar to the process used in Example 34 and 35.

The compound of Example 212 (1.95 g, pale yellow powder, 100%ee, retention time 9.4 minutes)

The compound of Example 213 (2.02 g, pale yellow powder, 99.6%ee, retention time 15.6 minutes)

The optical purities were determined by HPLC analysis using chiral column chromatography (column: CHIRALCEL OJ-H (0.46 x 25.0 cm) manufactured by Daicel Chemical Industries, Ltd., solvent: n-hexane:ethanol = 50:50, flow rate: 1.0 mL/min, UV: 254 nm). (EXAMPLE 214) Synthesis of (E)-2-(7-trifluoromethyl-2,2-diethylchromn-4-ylidene)-N-(2,3-dihydro-4-methyl-4H-benzo[1,4]oxazin-8-yl)acetamide <step 1> Synthesis of 8-amino-2,3-dihydro-4-methyl-4H-benzo[1,4]oxazine

The title compound (94.0 mg) was obtained as an orange solid from the 8-amino-4-methyl-2H-1,4-benzoxazin-3(4H)-one (0.18 g) by the same process as that used in <Step 1> of Example 13. <step 2> Synthesis of (E)-2-(7-trifluoromethyl-2,2-diethylchroman-4-ylidene)-N-(2,3-dihydro-4-methyl-4H-benzo[1,4]oxazin-8-yl)acetamide

The title compound (24.7 mg) was obtained as a pale yellow solid from the compound (30.0 mg) prepared in <Step 1> of Example 214 and the compound (57.4 mg) prepared in <Step 4> of Example 60 by the same process as that used in <Step 7> of Example 1.

The compound described blow was prepared from arylamine represented by formula (IX) described above prepared by a similar to the process used in Example 214 and a carboxylic acid [formula (VIII) described above] used in Examples described above by a similar to the process used in <Step 7> of Example 1.
(EXAMPLE 215) (E)-2-(7-trifluoromethyl-spiro[chroman-2,1'-cyclobutane]-4-ylidene)-N-(2,3-dihydro-4-methyl-4H-benzo[1,4]oxazin-8-yl) acetamide
(EXAMPLE 216) (E)-2-(7-trifluoromethyl-2,2-bis (methoxymethyl) chroman-4-ylidene)-N-(2,3-dihydro-4-methyl-4H-benzo[1,4]oxazin-8-yl)acetamide
(EXAMPLE 217) (E)-2-(7-trifluoromethyl-2,2-diethylchroman-4-ylidene)-N-(2,3-dihydro-4-ethyl-4H-benzo[1,4]oxazin-8-yl)acetamide
(EXAMPLE 218) (E)-2-(7-trifluoromethyl-2,2-bis(methoxymethyl)chroman-4-ylidene)-N-(2,3-dihydro-4-ethyl-4H-benzo[1,4]oxazin-8-yl)acetamide
(EXAMPLE 219) (E)-2-(7-trifluoromethyl-2,2-diethylchroman-4-ylidene)-N-(2,3-dihydro-4-(3-hydroxypropyl)-4H-benzo[1,4]oxazin-8-yl)acetamide
(EXAMPLE 220) (E)-2-(7-trifluoromethyl-spiro[chroman-2,1'-cyclobutane]-4-ylidene)-N-(2,3-dihydro-4-(3-hydroxypropyl)-4H-benzo[1,4]oxazin-8-yl)acetamide
(EXAMPLE 221) (E)-2-(7-trifluoromethyl-2,2-bis(methoxymethyl)chroman-4-ylidene)-N-(2,3-dihydro-4-(3-hydroxypropyl)-4H-benzo[1,4]oxazin-8-yl)acetamide

The compounds described below were prepared from known arylamine represented by formula (IX) described above and a carboxylic acid [formula (VIII) described above] used in Examples described above by a similar to the process used in <step 7> of Example 1.
(EXAMPLE 222) (E)-2-(7-trifluoromethyl-2,2-diethylchroman-4-ylidene)-N-(3,4-dihydro-1-methyl-2(1H)-quinolinon-5-yl)acetamide
(EXAMPLE 223) (E)-2-(7-trifluoromethyl-spiro[chroman-2,1'-cyclobutane]-4-ylidene)-N-(3,4-dihydro-1-methyl-2(1H)-quinolinon-5-yl)acetamide
(EXAMPLE 224) (E)-2-(7-trifluoromethyl-2,2-bis(methoxymethyl)chroman-4-ylidene)-N-(3,4-dihydro-1-methyl-2(1H)-quinolinon-7-yl)acetamide
(EXAMPLE 225) (E)-2-(7-trifluoromethyl-2,2-bis(methoxymethyl)chroman-4-ylidene)-N-(3,4-dihydro-1-methyl-2(1H)-quinolinon-5-yl)acetamide
(EXAMPLE 226) (E)-2-(7-trifluoromethyl-spiro[chroman-2,3'-N-methylazetidine]-4-ylidene)-N-(3,4-dihydro-2(1H)-quinolinon-7-yl)acetamide

The compounds described below were prepared from the compound prepared in <step 4> of Example 62 and an arylamine [formula (IX) described above] used in Examples described above by a similar to the process used in <step 7> of Example 1.
(EXAMPLE 227) (E)-2-(2,2-bis(methoxymethyl)-7-trifluoromethylchroman-4-ylidene)-N-(4-(2-hydroxyethyl)-4H-benzo[1,4]oxazin-3-on-6-yl)acetamide
(EXAMPLE 228) (E)-2-(7-trifluoromethyl-2,2-bis(methoxymethyl)chroman-4-ylidene)-N-(3,4-dihydro-3-(2-hydroxyethyl)-2(1H)-quinazolinon-7-yl)acetamide
(EXAMPLE 229) (E)-2-(7-trifluoromethyl-2,2-bis(methoxymethyl)chroman-4-ylidene)-N-(3,4-dihydro-3-(2-hydroxyethyl)-1-methyl-2(1H)-quinazolinon-7-yl)acetamide
(EXAMPLE 230) (E)-2-(7-trifluoromethyl-2,2-bis(methoxymethyl)chroman-4-ylidene)-N-(3,4-dihydro-3-(2-hydroxy-2-methylpropyl)-1-methyl-2(1H)-quinazolinon-7-yl)acetamide
(EXAMPLE 231) (E)-2-(7-trifluoromethyl-2,2-bis(methoxymethyl)chroman-4-ylidene)-N-(2,3-dihydro-2-(2-hydroxyethyl)-4-methyl-4H-benzo[1,4]oxazin-3-on-6-yl)acetamide
(EXAMPLE 232) (E)-2-(7-trifluoromethyl-2,2-bis(methoxymethyl)chroman-4-ylidene)-N-(3,4-dihydro-2(1H)-quinazolinon-5-yl)acetamide
(EXAMPLE 233) (E)-2-(7-trifluoromethyl-2,2-bis(methoxymethyl)chroman-4-ylidene)-N-(2,2-dioxo-3,4-dihydro-1H-benzo[2,1]thiazin-5-yl)acetamide

The compounds described below were prepared from the compound prepared in <step 3> of Example 63 and an arylamine [formula (IX) described above] used in Examples described above by a similar to the process used in <step 4>, <step 5> and <step 6> of Example 63.
(EXAMPLE 234) (E)-2-(7-trifluoromethyl-spiro[chroman-2,3'-N-methylazetidine]-4-ylidene)-N-(3,3-dimethyl-2(1H)-indolinon-6-yl)acetamide
(EXAMPLE 235) (E)-2-(7-trifluoromethyl-spiro[chroman-2,3'-N-methylazetidine]-4-ylidene)-N-(2,3-dihydro-4H-benzo[1,4]oxazin-3-on-8-yl)acetamide
(EXAMPLE 236) (E)-2-(7-trifluoromethyl-spiro[chroman-2,3-N-methylazetidine]-4-ylidene)-N-(3,4-dihydro-2(1H)-quinazolinon-5-yl)acetamide
(EXAMPLE 237) (E)-2-(7-trifluoromethyl-spiro[chroman-2,3'-N-methylazetidine]-4-ylidene)-N-(2,2-dioxo-3,4-dihydro-1H-benzo[2,1]thiazin-7-yl)acetamide
(EXAMPLE 238) (E)-2-(7-trifluoromethyl-spiro[chroman-2,3'-N-methylazetidine]-4-ylidene)-N-(2,2-dioxo-3,4-dihydro-1H-benzo[2,1]thiazin-5-yl)acetamide

The compounds described below were prepared from the compounds prepared in Example 194, Example 195, Example 196, Example 197 and Example 233 by a similar to the process used in Example 198.
(EXAMPLE 239) (E)-2-(8-trifluoromethyl-3,4-dihydrobenzo[b]oxepin-5(2H)-ylidene)-N-(2,2-dioxo-3,4-dihydro-1-methyl-1H-benzo[2,1]thiazin-5-yl)acetamide
(EXAMPLE 240) (E)-2-(7-trifluoromethyl-2,2-dimethylchroman-4-ylidene)-N-(2,2-dioxo-3,4-dihydro-1-methyl-1H-benzo[2,1]thiazin-5-yl)acetamide
(EXAMPLE 241) (E)-2-(7-trifluoromethyl-2,2-diethylchroman-4-ylidene)-N-(2,2-dioxo-3,4-dihydro-1-methyl-1H-benzo[2,1]thiazin-5-yl)acetamide
(EXAMPLE 242) (E)-2-(7-trifluoromethyl-spiro[chroman-2,1'-cyclobutane]-4-ylidene)-N-(2,2-dioxo-3,4-dihydro-1-methyl-1H-benzo[2,1]thiazin-5-yl)acetamide
(EXAMPLE 243) (E)-2-(7-trifluoromethyl-2,2-bis(methoxymethyl)chroman-4-ylidene)-N-(2,2-dioxo-3,4-dihydro-1-methyl-1H-benzo[2,1]thiazin-5-yl) acetamide
(EXAMPLE 244) (E)-2-(7-trifluoromethyl-2,2-diethylchromn-4-ylidene)-N-(2,2-dioxo-3,4-dihydro-1-ethyl-1H-benzo[2,1]thiazin-5-yl)acetamide
(EXAMPLE 245) (E)-2-(7-trifluoromethyl-2,2-bis(methoxymethyl)chroman-4-ylidene)-N-(2,2-dioxo-3,4-dihydro-1-ethyl-1H-benzo[2,1]thiazin-5-yl)acetamide
(EXAMPLE 246)

Synthesis of (E)-2-(7-trifluoromethyl-2,2-diethylchroman-4-ylidene)-N-(2,2-dioxo-3,4-dihydro-1-(2-hydroxyethyl)-1H-benzo[2,1]thiazin-5-yl)acetamide

### <step 1> Synthesis of (E)-2-(2,2-diethyl-7-trifluoromethyl-chroman-4-ylidene)-N-(3,4-dihydro-2,2-dioxo-1-(2-(t-butyldimethylsiloxy)ethyl)-1H-2,1-benzothiazin-5-yl)acetamide

Diethyl azodicarboxylate (40% in toluene solution) (72.7 µL)was added to a tetrahydrofuran (3.0 mL) solution of the compound (40.0 mg) prepared in Example 196, 2-(t-butyldimethylsiloxy)ethyl alcohol (28.5 mg) and triphenylphosphine (42.4 mg), and the mixture was stirred at room temperature for three hours. The solvent was distilled off under reduced pressure, and the residue was purified by silica gel column chromatography (eluate; n-hexane:ethyl acetate = 5:1) to give the title compound (32.4 mg) as a colorless amorphous.

### <step 2> Synthesis of (E)-2-(2,2-diethyl-7-trifluoromethyl-chroman-4-ylidene)-N-(3,4-dihydro-2,2-dioxo-1-(2-hydroxyethyl)-1H-benzo[2,1]thiazin-5-yl)acetamide

The title compound (20.5 mg) was prepared as a white solid from a compound (31.4 mg) prepared in <step 1> of Example 246 by a similar to the process used in <step 6> of Example 15.

The compounds described below were prepared from the compounds prepared in Example 196 by a similar to the process used in Example 246.
(EXAMPLE 247) (E)-2-(7-trifluoromethyl-2,2-diethylchroman-4-ylidene)-N-(2,2-dioxo-3,4-dihydro-1-(3-hydroxypropyl)-1H-benzo[2,1]thiazin-5-yl)acetamide
(EXAMPLE 248) (E)-2-(7-trifluoromethyl-2,2-diethylchroman-4-ylidene)-N-(2,2-dioxo-3,4-dihydro-1-(2-methoxyethyl)-1H-benzo[2,1]thiazin-5-yl)acetamide
(EXAMPLE 249) (E)-2-(7-trifluoromethyl-2,2-diethylchroman-4-ylidene)-N-(2,2-dioxo-3,4-dihydro-1-(oxiran-2-yl)methyl-1H-benzo[2,1]thiazin-5-yl)acetamide

The compounds described below were prepared from the compounds prepared in Example 233 by a similar to the process used in Example 246.
(EXAMPLE 250) (E)-2-(7-trifluoromethyl-2,2-bis(methoxymethyl)chroman-4-ylidene)-N-(2,2-dioxo-3,4-dihydro-1-(2-hydroxyethyl)-1H-benzo[2,1]thiazin-5-yl)acetamide
(EXAMPLE 251) (E)-2-(7-trifluoromethyl-2,2-bis(methoxymethyl)chroman-4-ylidene)-N-(2,2-dioxo-3,4-dihydro-1-(3-hydroxypropyl)-1H-benzo[2,1]thiazin-5-yl)acetamide
(EXAMPLE 252) (E)-2-(7-trifluoromethyl-2,2-bis(methoxymethyl)chroman-4-ylidene)-N-(2,2-dioxo-3,4-dihydro-1-(2-methoxyethyl)-1H-benzo[2,1]thiazin-5-yl)acetamide
(EXAMPLE 253) (E)-2-(7-trifluoromethyl-2,2-bis(methoxymethyl)chroman-4-ylidene)-N-(2,2-dioxo-3,4-dihydro-1-(oxiran-2-yl)methyl-1H-benzo[2,1]thiazin-5-yl)acetamide

The compounds described below were prepared from 5-nitro-3,4-dihydro-2(1H)-quinolinone by a similar to the process used in Example 39.
(EXAMPLE 254) (E)-2-(7-trifluoromethyl-2,2-diethylchroman-4-ylidene)-N-(3,4-dihydro-1-ethyl-2(1H)-quinolinon-5-yl)acetamide
(EXAMPLE 255) (E)-2-(7-trifluoromethyl-2,2-bis(methoxymethyl)chroman-4-ylidene)-N-(3,4-dihydro-1-ethyl-2(1H)-quinolinon-5-yl)acetamide

The compound described below was prepared from 6-nitro-3,4-dihydro-2H-benzo[1,4]oxazine by a similar to the process used in Example 8 and <step 6> of Example 1.
(EXAMPLE 256) (E)-2-(7-trifluoromethyl-2,2-bis(methoxymethyl)chroman-4-ylidene)-N-(2,3-dihydro-4-ethyl-4H-benzo[1,4]oxazin-6-yl)acetamide

The compounds described below were prepared from an arylamine [formula (IX) described above] synthesized from 2-(2-hydroxyethyl)-6-nitro-2H-benzo[1,4]oxazin-3(4H)-one in a similar to the process used in <step 2> of Example 15 and a carboxylic acid [formula (VIII) described above] used in Examples described above by a similar to the process used in <step 7> of Example 1.
(EXAMPLE 257) (E)-2-(7-trifluoromethyl-2,2-diethylchroman-4-ylidene)-N-(2,3-dihydro-2-(2-hydroxyethyl)-4-ethyl-4H-benzo[1,4]oxazin-3-on-6-yl)acetamide
(EXAMPLE 258) (E)-2-(7-trifluoromethyl-spiro[chromn-2,1'-cyclobutane]-4-ylidene)-N-(2,3-dihydro-2-(2-hydroxyethyl)-4-ethyl-4H-benzo[1,4]oxazin-3-on-6-yl)acetamide
(EXAMPLE 259) (E)-2-(7-trifluoromethyl-2,2-bis(methoxymethyl)chroman-4-ylidene)-N-(2,3-dihydro-2-(2-hydroxyethyl)-4-ethyl-4H-benzo[1,4]oxazin-3-on-6-yl)acetamide
(EXAMPLE 260) (E)-2-(7-trifluoromethyl-2,2-diethylchroman-4-ylidene)-N-(2,3-dihydro-2,4-bis(2-hydroxyethyl)-4H-benzo[1,4]oxazin-3-on-6-yl)acetamide
(EXAMPLE 261) (E)-2-(7-trifluoromethyl-spiro[chroman-2,1'-cyclobutane]-4-ylidene)-N-(2,3-dihydro-2,4-bis(2-hydroxyethyl)-4H-benzo[1,4]oxazin-3-on-6-yl)acetamide
(EXAMPLE 262) (E)-2-(7-trifluoromethyl-2,2-bis(methoxymethyl)chroman-4 ylidene)-N-(2,3-dihydro-2,4-bis(2-hydroxyethyl)-4H-benzo[1,4]oxazin-3-on-6-yl)acetamide

The compounds described below were prepared from 8-nitro -2H-benzo[1,4]oxazin-3(4H)-one by a similar to the process used in Example 39.
(EXAMPLE 263) (E)-2-(7-trifluoromethyl-2,2-diethylchroman-4-ylidene)-N-(2,3-dihydro-4-(2-hydroxyethyl)-4H-benzo[1,4]oxazin-3-on-8-yl)acetamide
(EXAMPLE 264) (E)-2-(7-trifluoromethyl-spiro[chroman-2,1'-cyclobutane]-4-ylidene)-N-(2,3-dihydro-4-(2-hydroxyethyl)-4H-benzo[1,4]oxazin-3-on-8-yl)acetamide
(EXAMPLE 265) (E)-2-(7-trifluoromethyl-2,2-bis(methoxymethyl)chroman-4-ylidene)-N-(2,3-dihydro-4-(2-hydroxyethyl)-4H-benzo[1,4]oxazin-3-on-8-yl)acetamide

The compounds described below were prepared from 8-amino4-(2-hydroxyethyl)-2H-benzo[1,4]oxazin-3(4H)-one by a similar to the process used in Example 214.
(EXAMPLE 266) (E)-2-(7-trifluoromethyl-2,2-diethylchroman-4-ylidene)-N-(2,3-dihydro-4-(2-hydroxyethyl)-4H-benzo[1,4]oxazin-8-yl)acetamide
(EXAMPLE 267) (E)-2-(7-trifluoromethyl-spiro[chroman-2,1'-cyclobutane]-4-ylidene)-N-(2,3-dihydro-4-(2-hydroxyethyl)-4H-benzo[1,4]oxazin-8-yl)acetamide
(EXAMPLE 268) (E)-2-(7-trifluoromethyl-2,2-bis(methoxymethyl)chroman-4-ylidene)-N-(2,3-dihydro-4-(2-hydroxyethyl)-4H-benzo[1,4]oxazin-8-yl)acetamide
(EXAMPLE 269)

Synthesis of (E)-2-(7-trifluoromethyl-spiro[chroman-2,4'-tetrahydropyran]-4-ylidene)-N-(3,4-dihydro-2(1H)-quinolinon-7-yl)acetamide

### <step 1> Synthesis of 7-trifluoromethyl-spiro[chroman-2,4'-tetrahydropyran]-4-one

The title compound (2.98 g) was prepared as a pale orange solid from a compound (6.0 g) prepared in <step 1> of Example 24 and tetrahydropyran-4-one (3.24 g) by a similar to the process used in <step 2> of Example 24.

### <step 2> Synthesis of 2-(4-hydroxy-7-trifluoromethyl-spiro[chroman-2,4'-tetrahydropyran]-4-yl) acetic acid ethyl ester

The title compound (2.40 g) was prepared as a yellow oil from a compound (1.60 g) prepared in <step 1> of Example 269 by a similar to the process used in <step 2> of Example 60.

### <step 3> Synthesis of 2-(4-hydroxy-7-trifluoromethyl-spiro[chroman-2,4'-tetrahydropyran]-4-yl)acetic acid

The title compound (1.48 g) was prepared as a pale yellow solid from a compound (2.09 g) prepared in <step 2> of Example 269 by a similar to the process used in <step 4> of Example 1.

### <step 4> Synthesis of (E)-2-(7-trifluoromethyl-spiro[chroman-2,4'-tetrahydropyran]-4-ylidene)acetic acid

The title compound (1.22 g) was prepared as a white solid from a compound (1.48 g) prepared in <step 3> of Example 269 by a similar to the process used in <step 4> of Example 60.

### <step 5> Synthesis of (E)-2-(7-trifluoromethyl-spiro[chroman-2,4'-tetrahydropyran]-4-ylidene)-N-(3,4-dihydro-2(1H)-quinolinon-7-yl)acetamide

The title compound (31.0mg) was prepared as a white solid from a compound (78.9mg) prepared in <step 4> of Example 269 by a similar to the process used in <step 7> of Example 1.

The compounds described below were prepared from known arylamine represented by formula (IX) described above and a compound prepared in <Step 4> of Example 269 by a similar to the process used in <step 7> of Example 1.
(EXAMPLE 270) (E)-2-(7-trifluoromethyl-spiro[chroman-2,4-tetrahydropyran]-4-ylidene)-N-(3,4-dihydro-1-methyl-2(1H)-quinolinon-7-yl)acetamide
(EXAMPLE 271) (E)-2-(7-trifluoromethyl-spiro[chroman-2,4'-tetrahydropyran]-4-ylidene)-N-(3,4-dihydro-2(1H)-quinolinon-5-yl)acetamide
(EXAMPLE 272) (E)-2-(7-trifluoromethyl-spiro[chroman-2,4'-tetrahydropyran]-4-ylidene)-N-(2,3-dihydro-2-methyl-4H-benzo[1,4]oxazin-3-on-6-yl)acetamide

The compounds described below were prepared from arylamine [formula (IX) described above] used in Examples described above and a compound prepared in <step 4> of Example 269 by a similar to the process used in <step 7> of Example 1.
(EXAMPLE 273) (E)-2-(7-trifluoromethyl-spiro[chroman-2,4'-tetrahydropyran]-4-ylidene)-N-(2,3-dihydro-4H-benzo[1,4]oxazin-3-on-8-yl)acetamide
(EXAMPLE 274) (E)-2-(7-trifluoromethyl-spiro[chroman-2,4'-tetrahydropyran]-4-ylidene)-N-(3,3-dimethyl-2(1H)-indolinon-6-yl)acetamide
(EXAMPLE 275) (E)-2-(7-trifluoromethyl-spiro[chroman-2,4'-tetrahydropyran]-4-ylidene)-N-(2,3-dihydro-4-ethyl-4H-benzo[1,4]oxazin-6-yl)acetamide
(EXAMPLE 276) (E)-2-(7-trifluoromethyl-spiro[chroman-2,4'-tetrahydropyran]-4-ylidene)-N-(2,3-dihydro-4-methyl-4H-benzo[1,4]oxazin-8-yl)acetamide
(EXAMPLE 277) (E)-2-(7-trifluoromethyl-spiro[chroman-2,41-tetrahydropyran]-4-ylidene)-N-(3,4-dihydro-3-(2-hydroxyethyl)-2(1H)-quinazolinon-7-yl)acetamide
(EXAMPLE 278) (E)-2-(7-trifluoromethyl-spiro[chroman-2,4'-tetrahydropyran]-4-ylidene)-N-(3,4-dihydro-3-(2-hydroxyethyl)-1-methyl-2(1H)-quinazolinon-7-yl)acetamide
(EXAMPLE 279) (E)-2-(7-trifluoromethyl-spiro[chromn-2,4'-tetrahydropyran]-4-ylidene)-N-(3,4-dihydro-2(1H)-quinazolinon-5-yl)acetamide
(EXAMPLE 280) (E)-2-(7-trifluoromethyl-spiro[chroman-2,4'-tetrahydropyran]-4-ylidene)-N-(2,2-dioxo-3,4-dihydro-1H-benzo[2,1]thiazin-7-yl)acetamide
(EXAMPLE 281) (E)-2-(7-trifluoromethyl-spiro[chroman-2,4'-tetrahydropyran]-4-ylidene)-N-(2,2-dioxo-3,4-dihydro-1H-benzo[2,1]thiazin-5-yl)acetamide

The compound described below was prepared from 5-amino-2,2-dioxo-3,4-dihydro-1H-2,1-benzothiazine prepared in a similar to the process used in Example 59 and (E)-(8-trifluoromethyl-3,4-dihydrobenzo[c]oxepin-5(1H)-ylidene)acetic acid (117 mg) prepared by the way described in PCT Publication No. 07/010383 pamphlet by a similar to the process used in <step 7> of Example 1.
(EXAMPLE 282) (E)-2-(8-trifluoromethyl-3,4-dihydrobenzo[c]oxepin-5(1H)-ylidene)-N-(2,2-dioxo-3,4-dihydro-1H-benzo[2,1]thiazin-5-yl)acetamide

The compounds described below were prepared from the compounds prepared in Example 281, Example 238 and Example 282 by a similar to the process used in <step 1> of Example 39.
(EXAMPLE 283) (E)-2-(7-trifluoromethyl-splro[chroman-2,4'-tetrahydropyran]-4-ylidene)-N-(2,2-dioxo-3,4-dihydro-1-methyl-1H-benzo[2,1]thiazin-5-yl)acetamide
(EXAMPLE 284) (E)-2-(8-trifluoromethyl-3,4-dihydrobenzo[c]oxepin-5(1H)-ylidene)-N-(2,2-dioxo-3,4-dihydro-1-methyl-1H-benzo[2,1]thiazin-5-yl)acetamide
(EXAMPLE 285) Synthesis of (E)-2-(7-trifluoromethyl-2,2-diethylchroman-4-ylidene)-N-(3,4-dihydro-3-ethyl-2(1H)-quinazolinon-5-yl)acetamide

### <step 1> Synthesis of N-(2-amino-6-nitrobenzyl)-2-nitrobenzensulphonamide

2-nitrobenzenesulphonyl chloride (0.70 g) and triethylamine (0.63 mL) were added sequentially to a dichloromethane (50 mL) solution of 2-amino-6-nitrobenzylamine (0.50 g) at ice-cooled, and the mixture was stirred at room temperature for three hours. Aqueous saturated sodium hydrogen carbonate solution was added to the reaction mixture, and the mixture was extracted with dichloromethane. The organic layer was washed with water and saturated saline solution sequentially, and dried over anhydrous sodium sulfate. The solvent was distilled off under reduced pressure. The residue was solidified with n-hexane/diethyl ether to give the title compound (840 mg) as a yellow solid.

### <step 2> Synthesis of N-(2-amino-6-nitrobenzyl)-N-ethyl-2-nitrobenzenesulphonamide

The title compound (0.66 g) was prepared as a yellow solid from the compound (0.84 g) prepared in <step 1> of Example 285 by the same process as that used in <step 1> of Example 198.

### <step 3> Synthesis of N-(2-amino-6-nitrobenzyl)-N-ethylamine

Lithium hydroxide monohydrate (0.29 g) and thioglycolic acid (0.24 mL) were added sequentially to a N,N-dimethylformamide (5 mL) solution of a compound (0.66 g) prepared in <step 2> of Example 285. The reaction mixture was stirred at room temperature for one hour. A 1 N aqueous sodium hydroxide solution was added to the mixture, and extracted with ethyl acetate. The organic layer was washed with 1N sodium hydroxide, water and saturated saline solution sequentially, and dried over anhydrous sodium sulfate. The solvent was distilled off under reduced pressure to give the title compound (0.31 g) as a yellow solid.

### <step 4> Synthesis of 3-ethyl-5-nitro-3,4-dihydro-2(1H)quinazolinone

The title compound (0.20 g) was prepared as a yellow solid from the compound (0.29 g) prepared in <step 3> of Example 285 by a similar to the process used in <step 1> of Example 12.

### <step 5> Synthesis of 5-amino-3-ethyl-3,4-dihydro-2(1H)quinqzolinone

The title compound (0.16 g) was prepared as a brown solid from the compound (0.20 g) prepared in <step 4> of Example 285 by a similar to the process used in <step 6> of Example 1.

### <step 6> Synthesis of (E)-2-(7-trifluoromethyl-2,2-diethylchroman-4-ylidene)-N-(3,4-dihydro-3-ethyl-2(1H)-quinazolinon-5-yl)acetamide

The title compound (54.1 mg) was prepared as a white solid from the compound (30 mg) prepared in <step 5> of Example 285 by a similar to the process used in <step 7> of Example 1.

The compounds described below were prepared from the compound prepared in <step 5> of Example 285 and a carboxylic acid [formula (VIII) described above] used in Examples described above by a similar to the process used in <step 7> of Example 1.
(EXAMPLE 286) (E)-2-(7-trifluoromethyl-spiro[chroman-2,1'-cyclobutane]-4-ylidene)-N-(3,4-dihydro-3-ethyl-2(1H)-quinazolinon-5-yl)acetamide
(EXAMPLE 287) (E)-2-(7-trifluoromethyl-2,2-bis(methoxymethyl)chroman-4-ylidene)-N-(3,4-dihydro-3-ethyl-2(1H)-quinazolinon-5-yl)acetamide
(EXAMPLE 288) Synthesis of (E)-2-(7-trifluoromethyl-2,2-diethylchroman-4-ylidene)-N-(3,4-dihydro-1-methyl-2(1H)-quinazolinon-5-yl)acetamide

### <step 1> Synthesis of N-(2-amino-6-nitrobenzyl)-N-(3,4-dimethoxybenzyl)-2-nitrobenzenesulphonamide

The title compound (2.63 g) was prepared as a yellow solid from the compound (2.00 g) prepared in <step 1> of Example 285 and veratryl alcohol (1.43 g) by a similar to the process used in <step 1> of Example 246.

### <step 2> Synthesis of N-(2-amino-6-nitrobenzyl)-N-(3,4-dimethoxybenzyl) amine

The title compound (0.66 g) was prepared as a yellow solid from the compound (1.04 g) prepared in <step 1> of Example 288 by a similar to the process used in <step 3> of Example 285.

### <step 3> Synthesis of 3-(3,4-dimethoxybenzyl)-5-nitro-3,4-dihydro-2(1H)-quinqzolinone

The title compound (0.62g) was prepared as pale red solid from the compound (0.95g) prepared in <step 2> of Example 288 by a similar to the process used in <step 1> of Example 12.

### <Step 4> Synthesis of 5-amino-1-methyl-3,4-dihydro-2(1H)-quinazolinone

Potassium carbonate (0.36 g) and methyl iodide (0.16 mL) were added to N,N-dimethylformamide (8.0 mL) solution of the compound (0.30 g) prepared in <Step 3> of Example 288, and the mixture was stirred at 40 °C for three hours. Then, potassium carbonate (0.36 g) and methyl iodide (0.16 mL) were added to the solution, and the mixture was stirred at 40 °C for three hours. Water was added to the reaction mixture, and extracted with ethyl acetate. The organic layer was washed with a saturated saline solution, and dried over anhydrous sodium sulfate. The solvent was distilled off under reduced pressure. Trifluoroacetic acid (4.0 mL) was added to the residue, and the mixture was stirred at room temperature for four and a half hours. 1 N sodium hydroxide solution was added to the reaction mixture, and extracted with ethyl acetate. The organic layer was washed with a saturated saline solution, and dried over anhydrous sodium sulfate. The solvent was distilled off under reduced pressure. 10%Pd-C (30 mg) was added to methanol (8.0 mL) solution of the residue was stirred under hydrogen atmosphere at room temperature for one hour. The reaction mixture was subjected to Celite filtration. The solvent was then distilled off under reduced pressure. The residue was purified by silica gel column chromatography (eluate; dichloromethane:methanol = 9:1).The title compound (60.0 mg) was obtained as a white solid.

### <step 5> Synthesis of (E)-2-(7-trifluoromethyl-2,2-diethylchroman-4-ylidene)-N-(3,4-dihydro-1-methyl-2(1H)-quinazolinon-5-yl)acetamide

The title compound (3.9mg) was prepared as a white solid from the compound (17.0mg) prepared in <step 4> of Example 288 by a similar to the process used in <step 7> of Example 1.

The compounds described below were prepared from the compound prepared in <step 6> of Example 288 and a carboxylic acid [formula (VIII) described above] used in Examples described above by a similar to the process used in <step 7> of Example 1.
(EXAMPLE 289) (E)-2-(7-trifluoromethyl-spiro[chroman-2,1'-cyclobutane]-4-ylidene)-N-(3,4-dihydro-1-methyl-2(1H)-quinazolinon-5-yl)acetamide
(EXAMPLE 290) (E)-2-(7-trifluoromethyl-2,2-bis(methoxymethyl)chroman-4-ylidene)-N-(3,4-dihydro-1-methyl-2(1H)-quinazolinon-5-yl)acetamide
(EXAMPLE 291) (E)-2-(7-trifluoromethyl-spiro[chroman-2,4'-tetrahydropyran]-4-ylidene)-N-(3,4-dihydro-1-methyl-2(1H)-quinazolinon-5-yl)acetamide
(EXAMPLE 292) Synthesis of (E)-2-(8-trifluoromethyl-3,4-dihydrobenzo[b]oxepin-5(2H)-ylidene)-N-(4-(4-morpholinyl)-2(1H)-quinolinone-7-yl)acetamide

### <Step 1>

### Synthesis of 2-(acetylamino)-4-nitrobenzoic acid methyl ester

Acetic anhydride (6.2 mL) was added to 2-amino-4-nitrobenzoic acid methyl ester (2.84 g). The reaction solution was stirred at 90 °C for three hours. The mixture was left to cool. The appeared solid was filtered and washed with diethyl ether. The title compound (2.03 g) was obtained as a pale yellow solid.

### <Step 2>

### Synthesis of 4-hydroxy-7-nitro-2(1H)-quinolinone

Potassium hexamethyldisilazane (0.5M, toluene solution, 88.2 mL) was added dropwise to a tetrahydrofuran (126.0 mL) solution of the compound (3.0 g) prepared in <step 1> of Example 292 under ice cooling. The reaction solution was stirred at room temperature for three hours. 1 N aqueous hydrochloric acid was added to the mixture, the mixture was extracted with ethyl acetate. The organic layer was sequentially washed with water and saturated saline solution, and dried over anhydrous sodium sulfate. The solvent was distilled off under reduced pressure. The residue was washed by mixed solvents (dichloromethane:methanol = 90:10). The title compound (0.96 g) was obtained as a brown solid.

### <Step 3>

### Synthesis of 4-chloro-7-nitro-2(1H)-quinolinone

Phosphoryl chloride (1.3 mL) was added to the compound (0.95 g) prepared in <step 2> of Example 292. The reaction solution was refluxed for 30 minutes. The mixture was left to cool. 1 N aqueous sodium hydroxide was added to the mixture, the mixture was extracted with dichloromethane. The organic layer was sequentially washed with water, saturated aqueous sodium hydrogen carbonate solution and saturated saline solution, and dried over anhydrous sodium sulfate. The solvent was distilled off under reduced pressure. Concentrated hydrochloric acid (15.0 mL) was added to the residue. The reaction solution was refluxed for four hours. The mixture was left to cool. Water was added to the mixture, the appeared solid was filtered. The title compound (437.0 mg) was obtained as a pale yellow solid.

### <Step 4>

### Synthesis of 4-(4-morpholinyl)-7-nitro-2(1H)-quinolinone

Morpholine (0.4 mL) was added to a N,N-dimethylformamide (4.5 mL) solution of the compound (0.1 g) prepared in <step 3> of Example 292. The reaction solution was stirred at 100 °C for one hour. The mixture was left to cool. Saturated aqueous ammonium chloride solution was added to the mixture, the mixture was extracted with ethyl acetate. The organic layer was sequentially washed with water and saturated saline solution, and dried over anhydrous sodium sulfate. The solvent was distilled off under reduced pressure. Dichloromethane was added to the residue to solidify the resulting product. The title compound (78.2 mg) was obtained as a pale yellow solid.

### <Step 5>

### Synthesis of 7-amino-4-(4-morpholinyl)-2(1H)-quinolinone

The title compound (45.0 mg) was obtained as a pale yellow solid from the compound (60.0 mg) prepared in <Step 4> of Example 292 by the same process as that used in <Step 6> of Example 1.

### <Step 6>

### Synthesis of (E)-2-(8-trifluoromethyl-3,4-dihydrobenzo[b]oxepin-5(2H)-ylidene)-N-(4-(4-morpholinyl)-2(1H)-quinolinone-7-yl)acetamide

The title compound (65.0 mg) was obtained as a pale yellow solid from the compound (50.0 mg) prepared in <Step 5> of Example 292 by the same process as that used in <Step 7> of Example 1.

### (EXAMPLE 293) Synthesis of (E)-2-(7-trifluoromethyl-spiro[chroman-2,3'-oxetan]-4-ylidene)-N-(3,4-dihydro-2(1H)-quinolinon-7-yl)acetamide

### <step 1> Synthesis of 7-trifluoromethyl-spiro[chroman-2,3'-oxetan]-4-one

The title compound (2.08 g) was prepared as a red-brown oil from the compound (6.09 g) prepared in <step 1> of Example 24 and oxetan-3-one (2.15 g) by a similar to the process used in <step 2> of Example 24.

### <step 2> Synthesis of tert-butyl (E)-2-(7-trifluoromethyl-spiro[chroman-2,3'-oxetan]-4-ylidene)acetate

The title compound (0.15g) was prepared as a pale yellow oil from the compound (0.50g) prepared in <step 1> of Example 293 by a similar to the process as that used in <step 3> of Example 23.

### <step 3> Synthesis of (E)-2-(7-trifluoromethyl-spiro[chroman-2,3'-oxetan]-4-ylidene)acetic acid

The title compound (0.10g) was prepared as a yellow solid from the compound (0.14g) prepared in <step 2> of Example 293 by a similar to the process used in <step 6> of Example 15.

### <step 4> Synthesis of (E)-2-(7-trifluoromethyl-spiro[chroman-2,3'-oxetan]-4-ylidene)-N-(3,4-dihydro-2(1H)-quinolinon-7-yl)acetamide

The title compound (8.0mg) was prepared as a white solid from the compound (20.0mg) prepared in <step 3> of Example 293 by a similar to the process used in <step 7> of Example 1.

The compounds described below were prepared from known arylamine represented by formula (IX) described above and a compound prepared in <Step 3> of Example 293 by a similar to the process used in <step 7> of Example 1.
(EXAMPLE 294) (E)-2-(7-trifluoromethyl-spiro[chroman-2,3'-oxetane]-4-ylidene)-N-(2,2-dioxo-3,4-dihydro-1H-benzo[2,1]thiazin-5-yl)acetamide
(EXAMPLE 293) (E)-2-(7-trifluoromethyl-spiro[chroman-2,3'-oxetane]-4-ylidene)-N-(3,4-dihydro-1-methyl-2(1H)-quinolinon-7-yl)acetamide
(EXAMPLE 296) (E)-2-(7-trifluoromethyl-spiro[chroman-2,3'-oxetane]-4-ylidene)-N-(3,4-dihydro-2(1H)-quinolinon-5-yl)acetamide

The compounds described below were prepared from arylamine [formula (IX) described above] and a compound prepared in <step 3> of Example 293 by a similar to the process used in <step 7> of Example 1.
(EXAMPLE 297) (E)-2-(7-trifluoromethyl-spiro[chroman-2,3'-oxetane]-4-ylidene)-N-(2,3-dihydro-4H-benzo[1,4]oxazin-3-on-8-yl)acetamide
(EXAMPLE 298) (E)-2-(7-trifluoromethyl-spiro[chroman-2,3'-oxetane]-4-ylidene)-N-(3,3-dimethyl-2(1H)-indolinon-6-yl)acetamide
(EXAMPLE 299) (E)-2-(7-trifluoromethyl-spiro[chroman-2,3'-oxetane]-4-ylidene)-N-(3,4-dihydro-2(1H)-quinazolinon-5-yl)acetamide
(EXAMPLE 300) (E)-2-(7-trifluoromethyl-spiro[chroman-2,3'-oxetane]-4-ylidene)-N-(2,2-dioxo-3,4-dihydro-1H-benzo[2,1]thiazin-7-yl)acetamide

The compounds described below were prepared from 5-amino-1-ethyl-3,4-dihydro-2(1H)-quinazolinone prepared in a similar to the process used in Example 288 and a carboxylic acid [formula (VIII) described above] used in Examples described above by a similar to the process used in <step 7> of Example 1.
(EXAMPLE 301) (E)-2-(7-trifluoromethyl-spiro[chroman-2,1'-cyclobutane]-4-ylidene)-N-(3,4-dihydro-1-ethyl-2(1H)-quinazolinon-5-yl)acetamide

The structures of the compound synthesized in Examples 1 to 301 are shown in [Ch.64] - [Ch.83]. The data of liquid chromatography-mass spectrometry (LC-MS) of these examples are shown in [Table 11] - [Table 13]. The NMR data of typical compounds are shown in [Table 14] -[Table 16] (300 MHz: no mark, 270 MHz: marked with *). The structures of the intermediate compounds are shown [Ch.84] - [Ch.85]. The NMR data of these intermediate compounds are shown in [Table 17] - [Table 18] (300 MHz: no mark, 270 MHz: marked with *). The "A" described in [Ch.84] - [Ch.85] correspond to the amine parts of each Example.
[Ch. * *] mean a figure included in the general formulae, the reaction scheme or the structures of Example in the specification. And
[Table * *] mean a table that the pharmacological data, spectral data or combination of chemical structures are shown in the specification.

The "* *" mean a Serial number that was sequentially fixed from page 1 of the specification.

**[Table 1]**

| Example | A2 value | Example | A2 value | Example | A2 value | Example | A2 value |
|---|---|---|---|---|---|---|---|
| 1 | A | 16 | A | 31 | A | 46 | A |
| 2 | A | 17 | A | 32 | B | 47 | A |
| 3 | A | 18 | A | 33 | A | 48 | A |
| 4 | A | 19 | A | 34 | A | 49 | A |
| 5 | B | 20 | A | 35 | A | 50 | A |
| 6 | A | 21 | A | 36 | A | 51 | B |
| 7 | A | 22 | A | 37 | A | 52 | B |
| 8 | A | 23 | A | 38 | A | 53 | A |
| 9 | A | 24 | A | 39 | A | 54 | A |
| 10 | A | 25 | A | 40 | A | 55 | A |
| 11 | A | 26 | A | 41 | A | 56 | A |
| 12 | A | 27 | A | 42 | A | 57 | B |
| 13 | A | 28 | A | 43 | A | 58 | A |
| 14 | A | 29 | A | 44 | A | 59 | A |
| 15 | A | 30 | A | 45 | A | | |

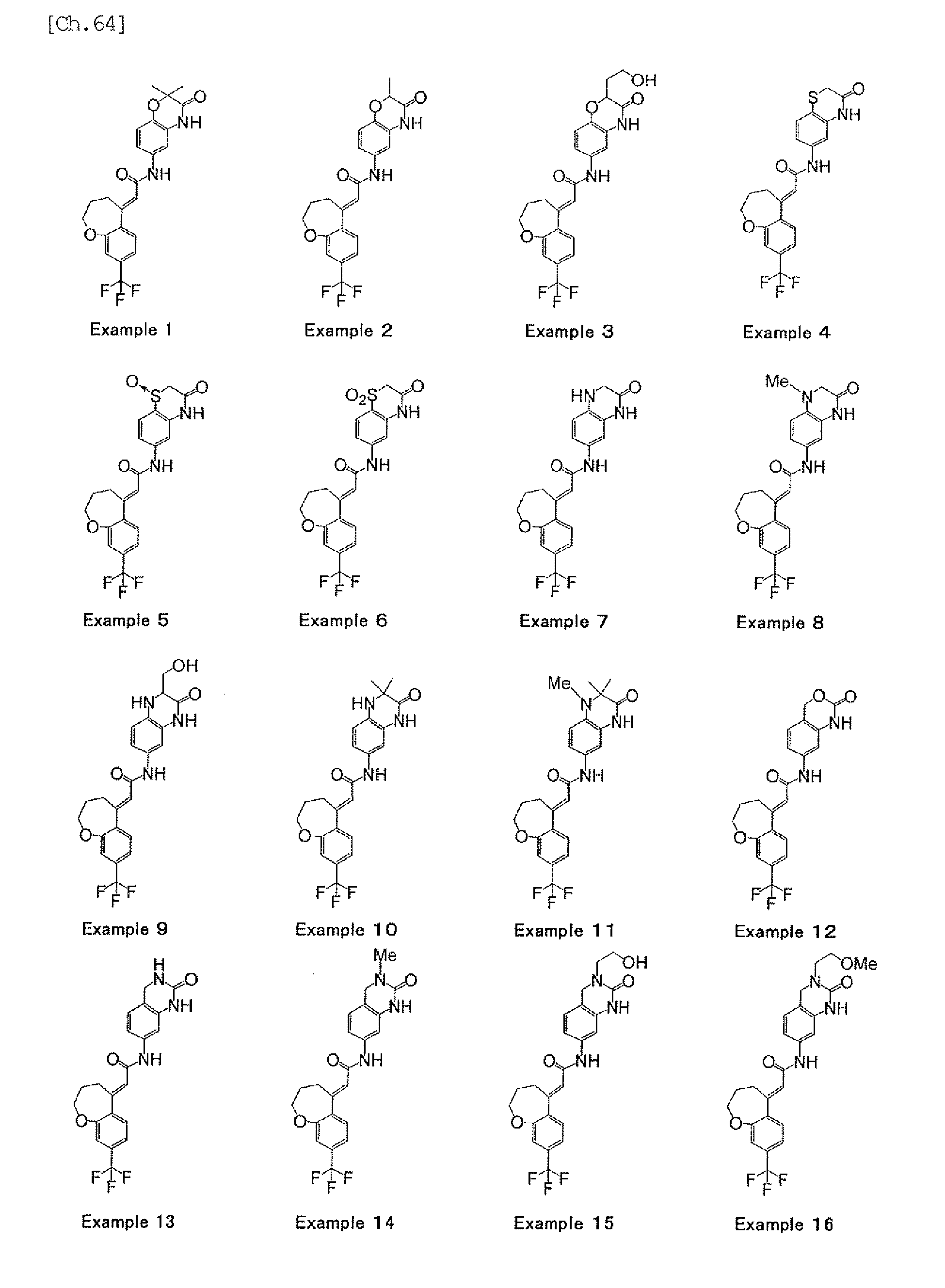

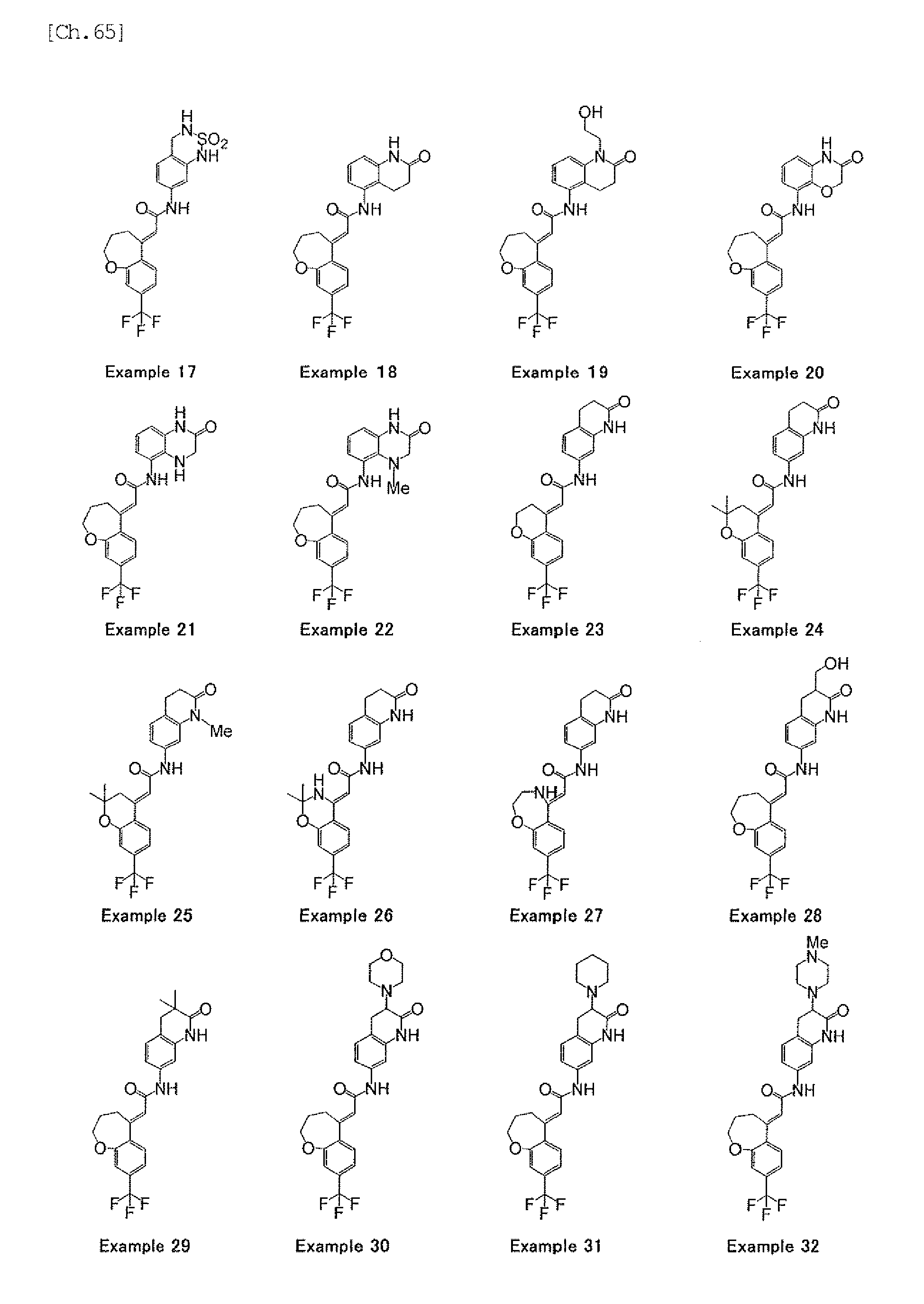

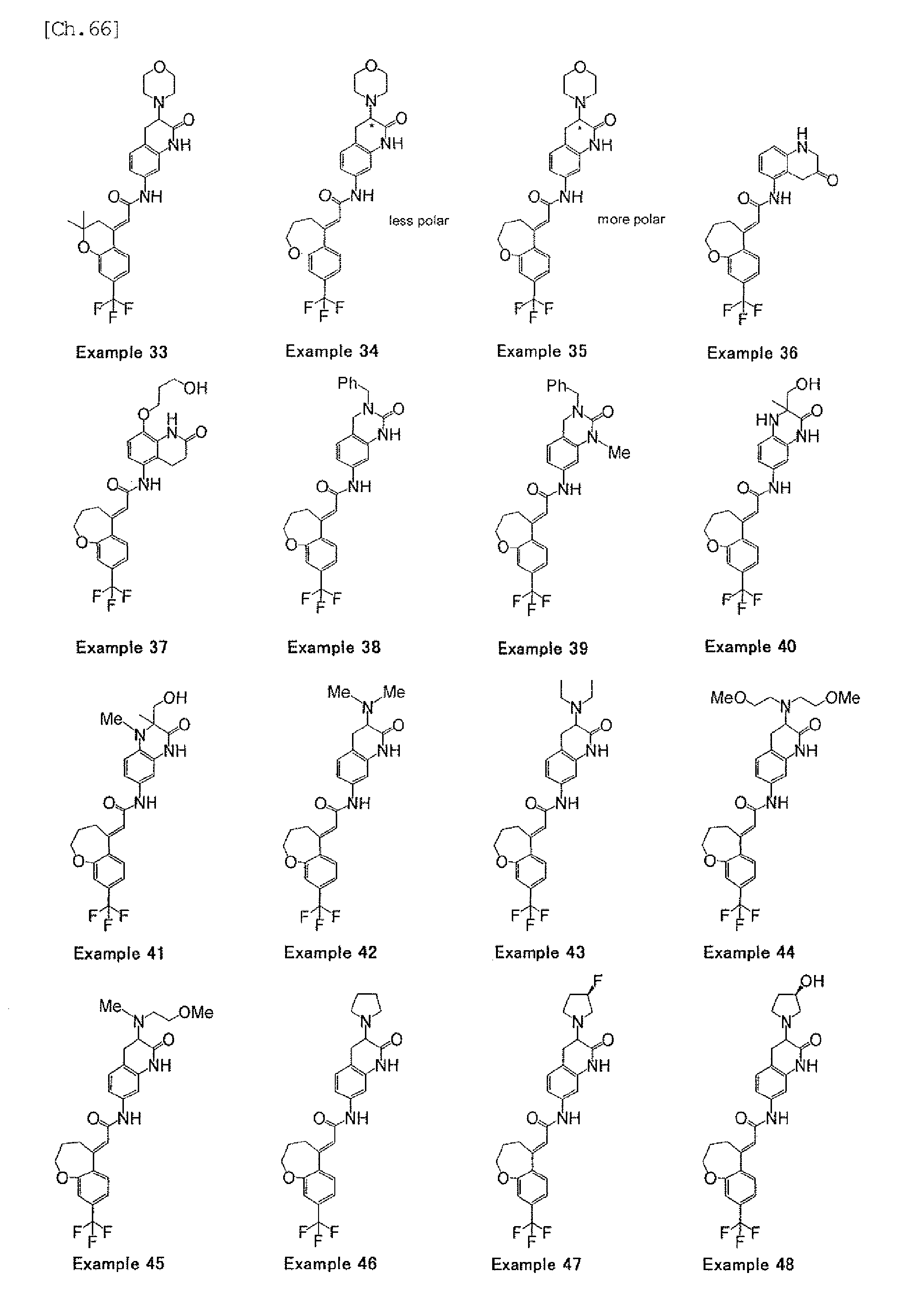

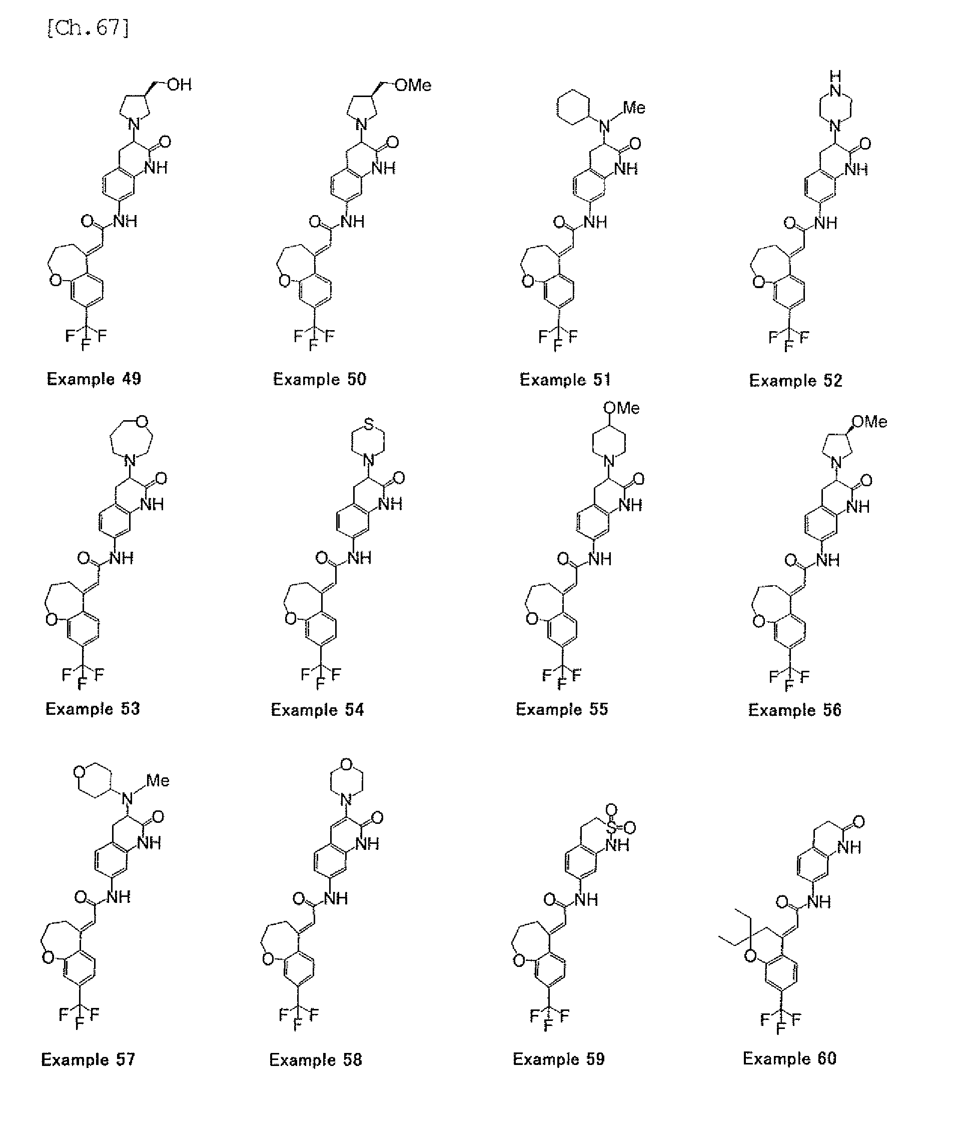

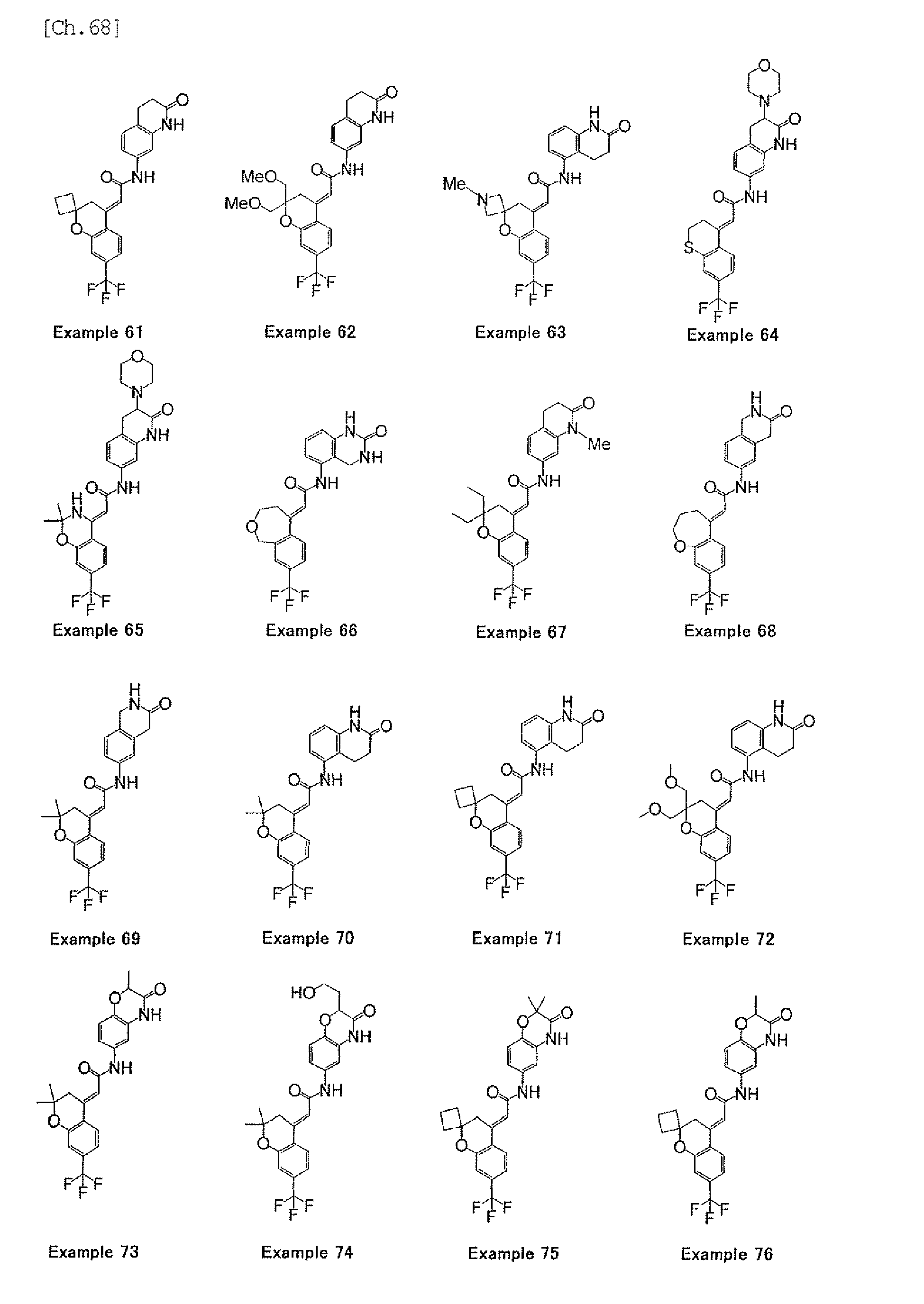

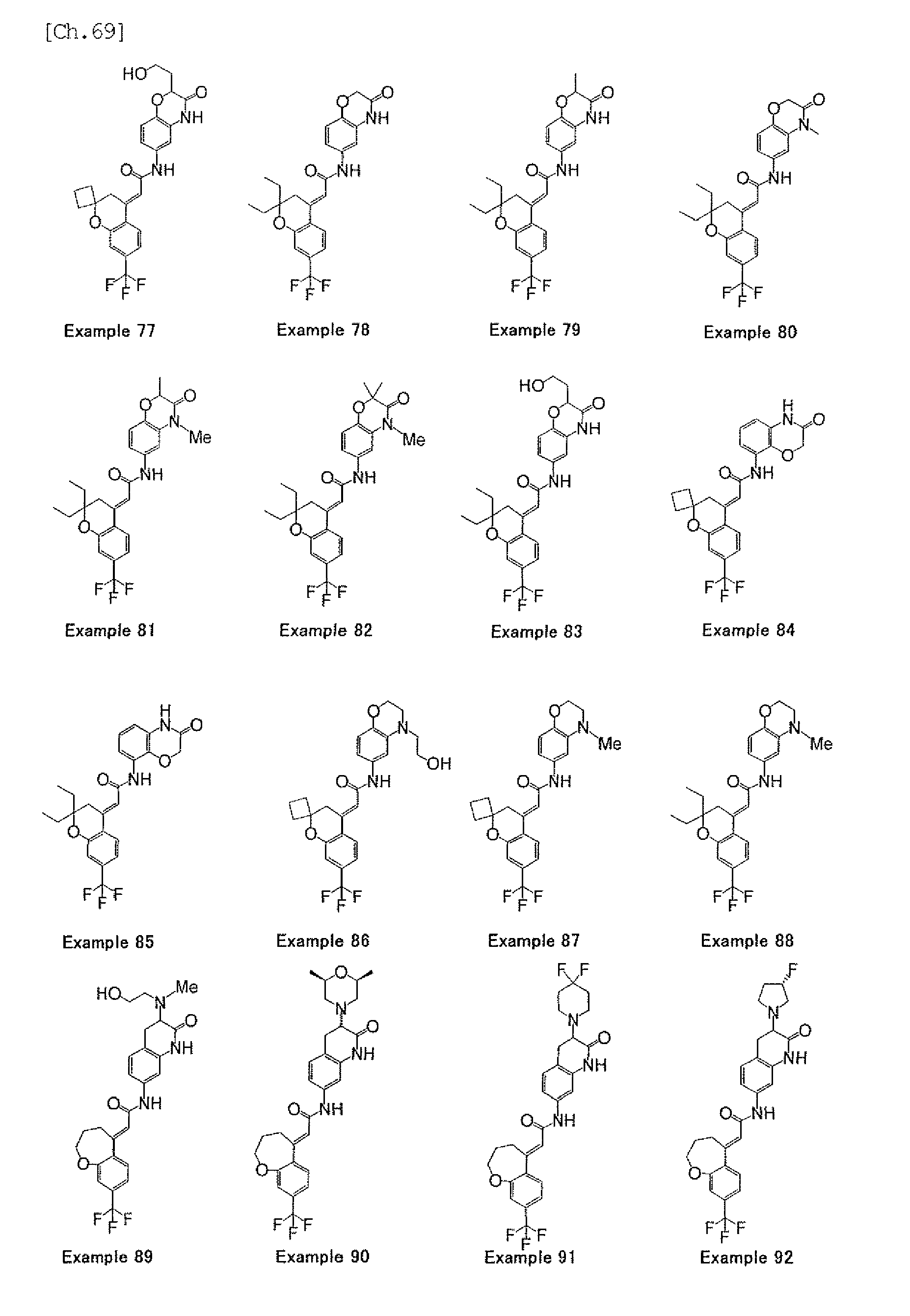

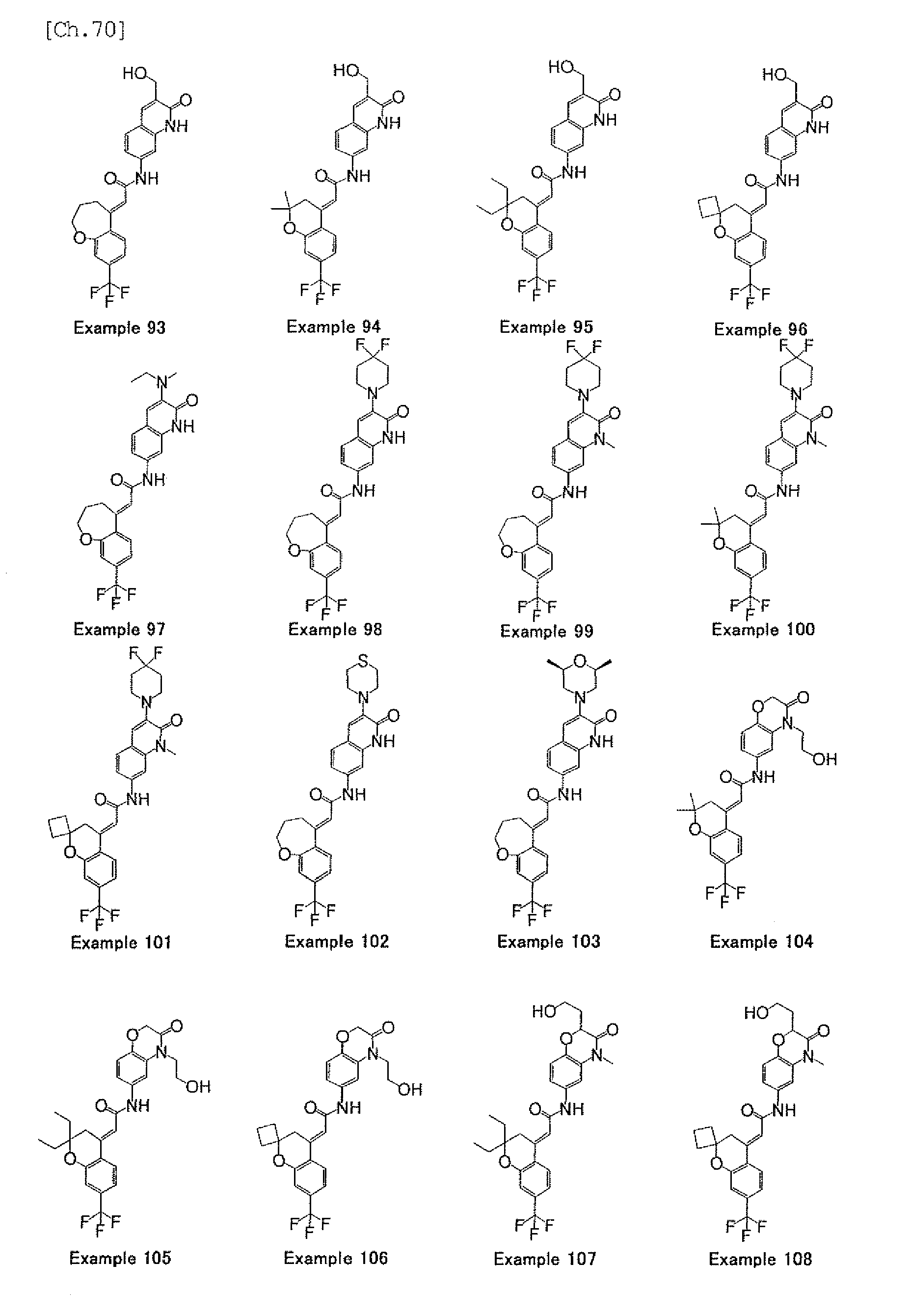

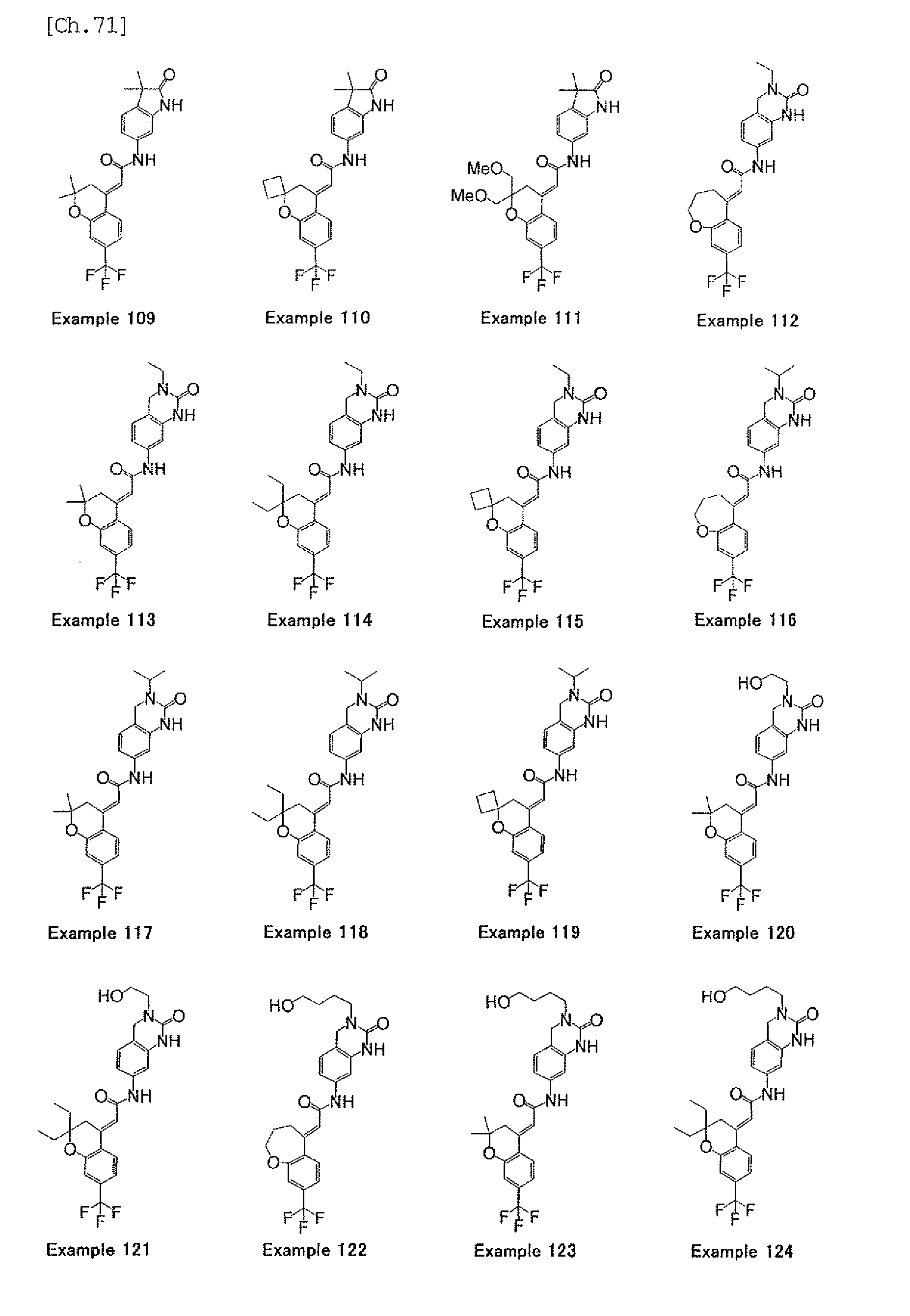

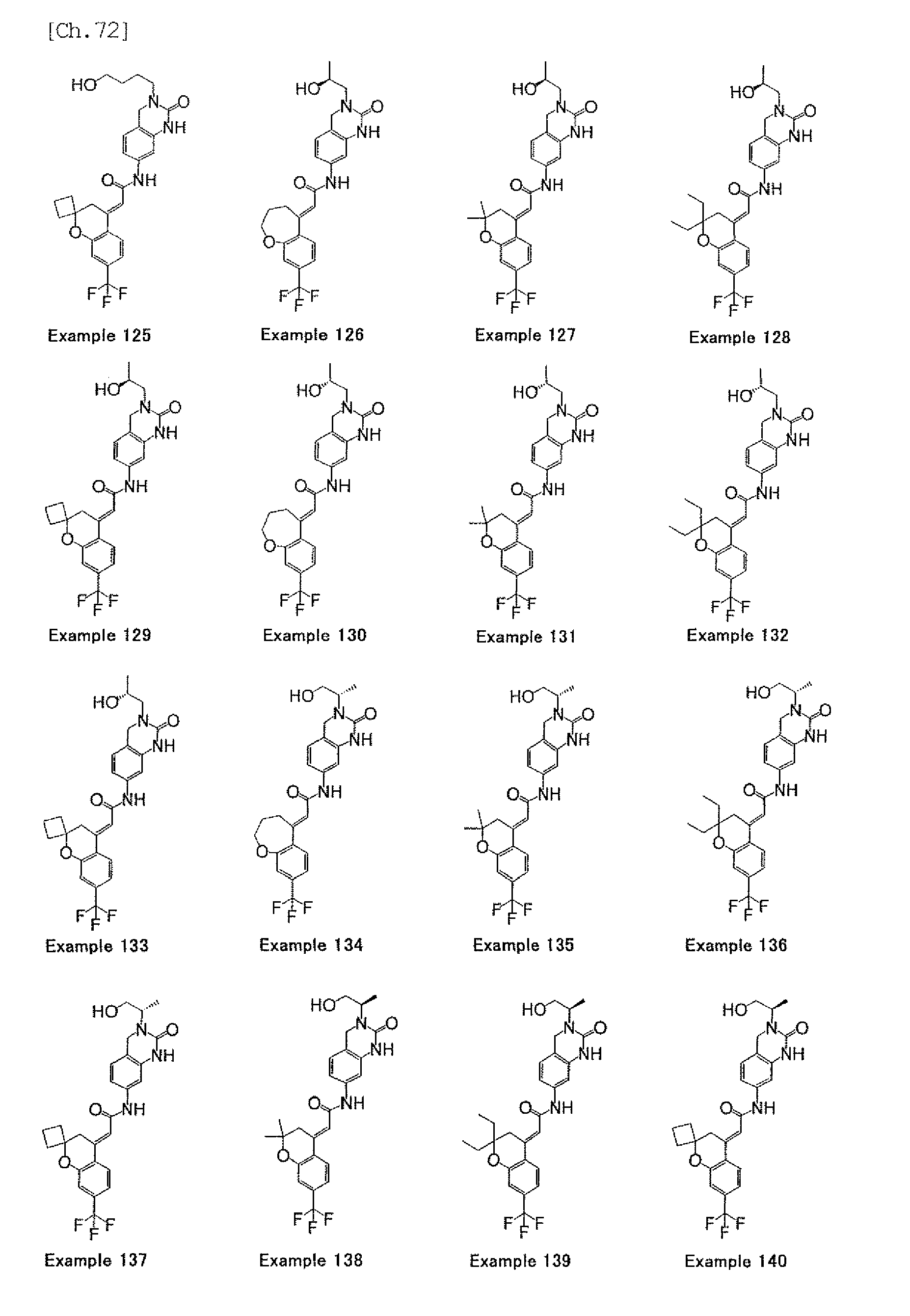

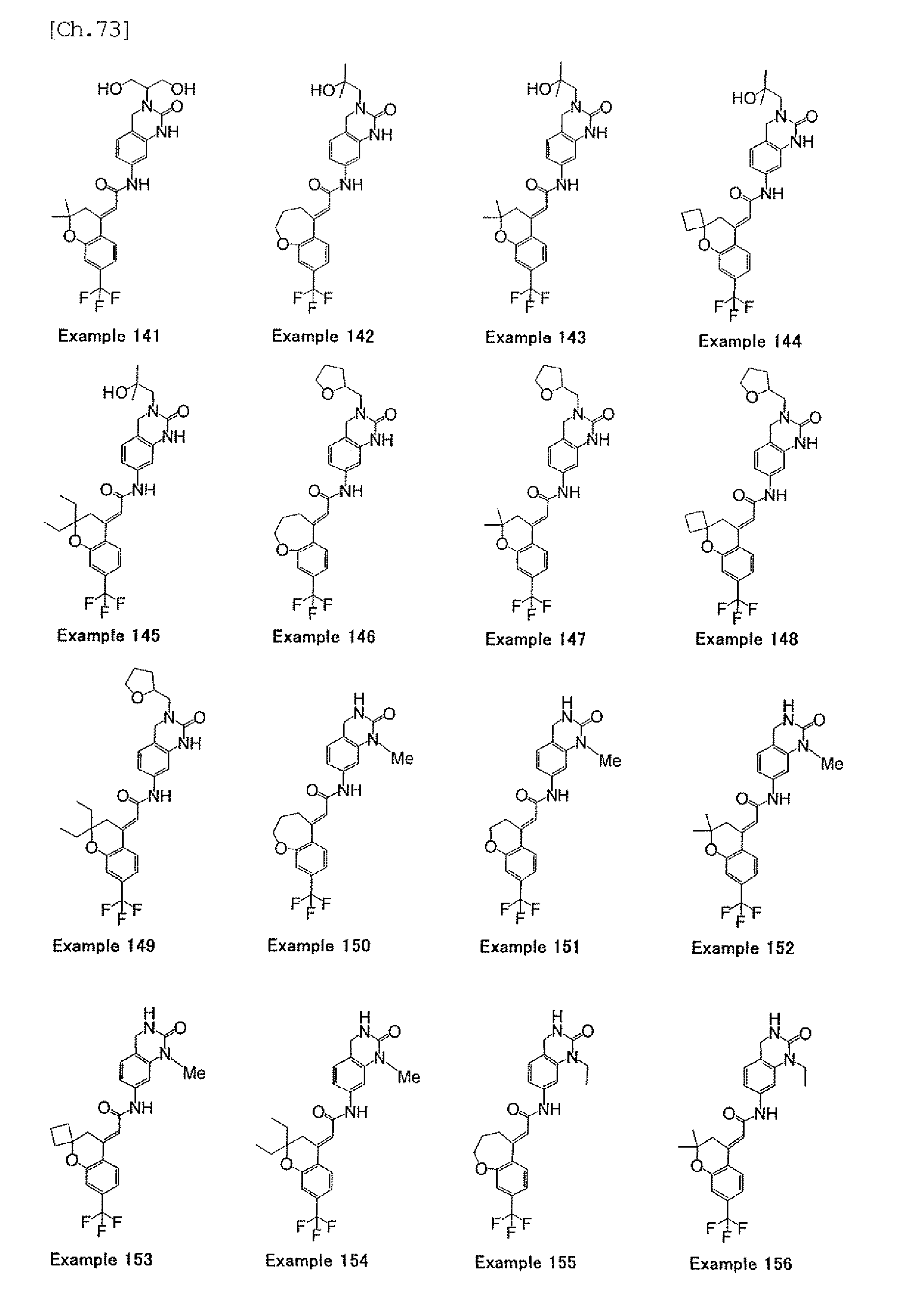

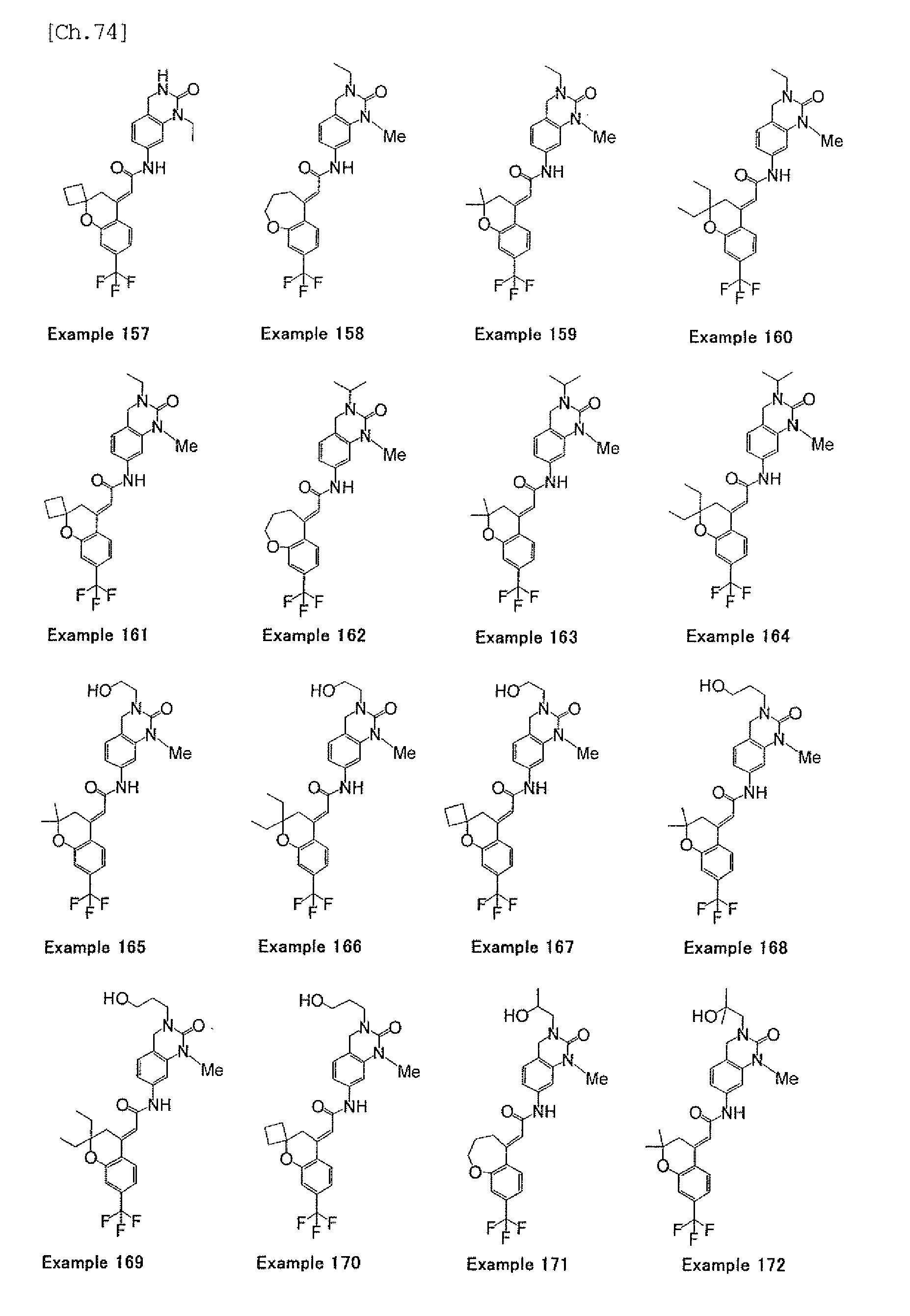

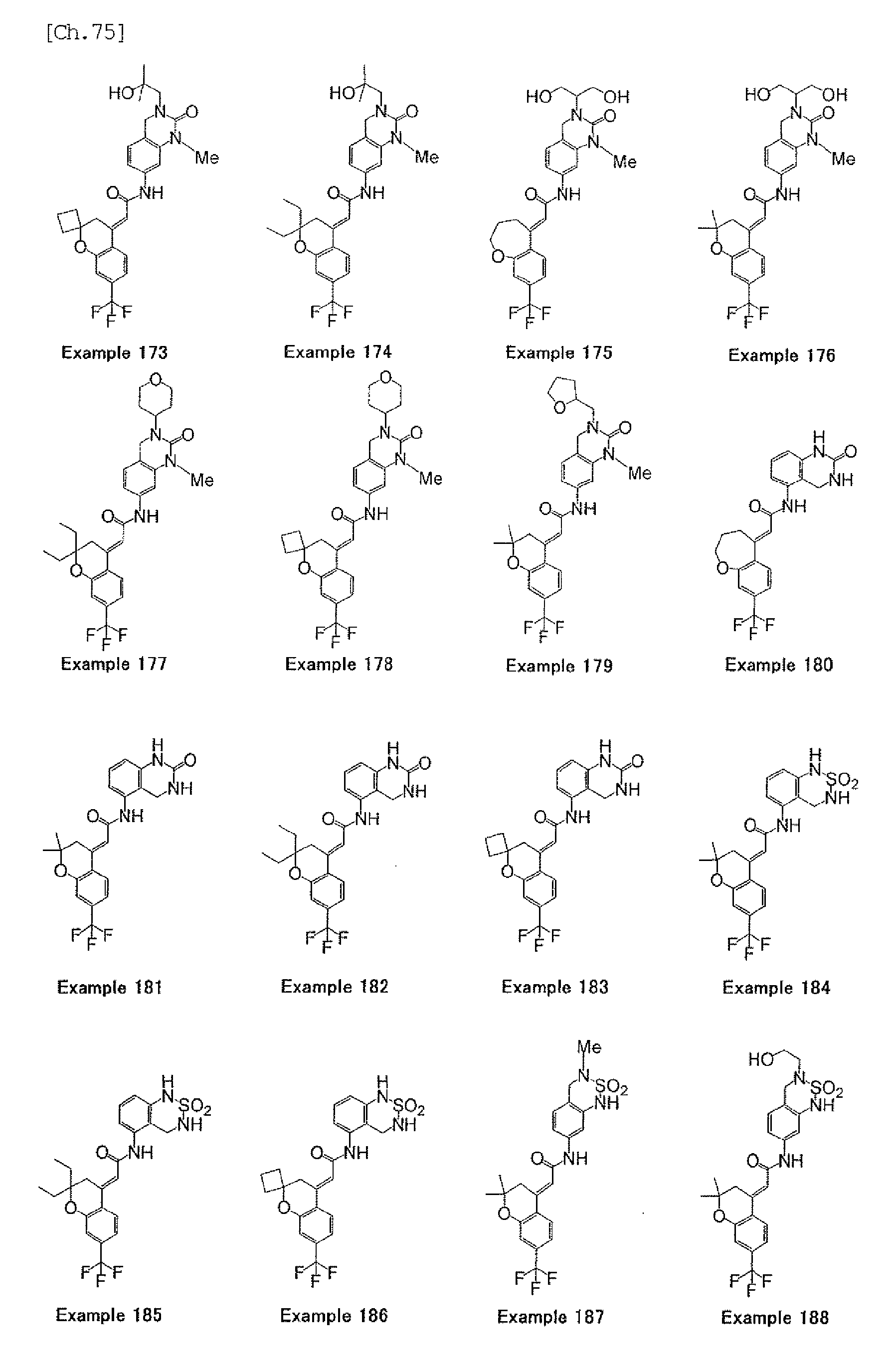

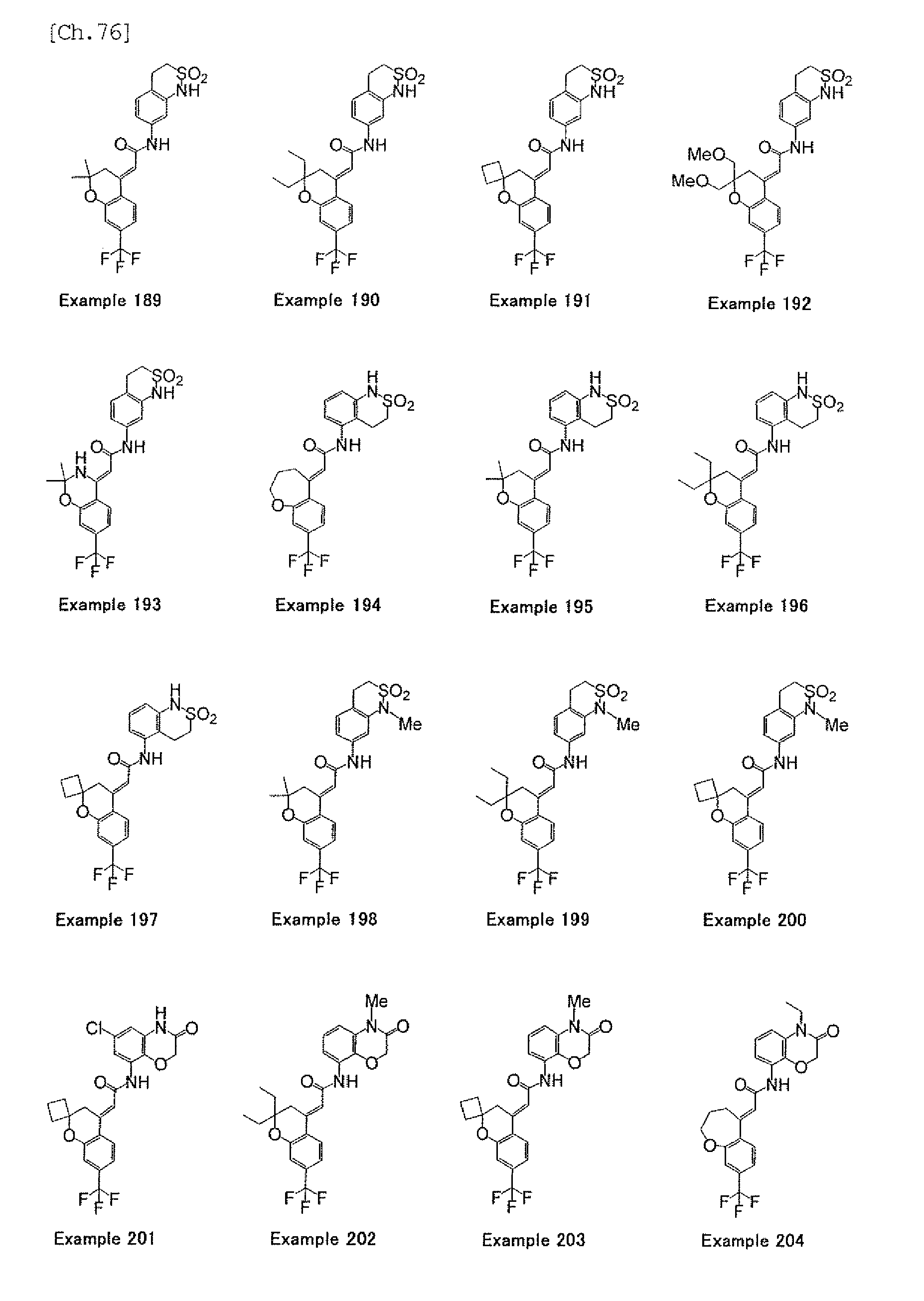

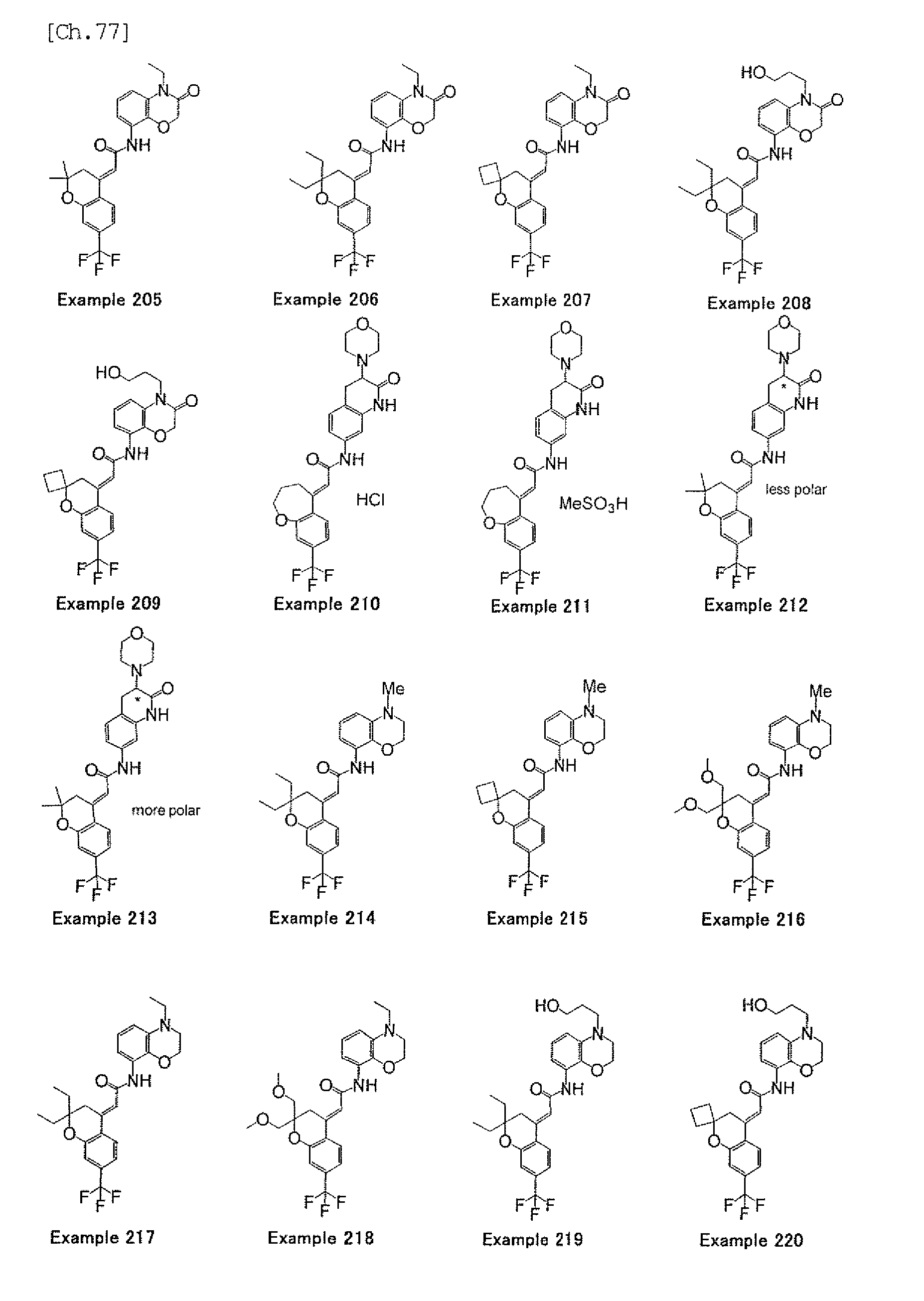

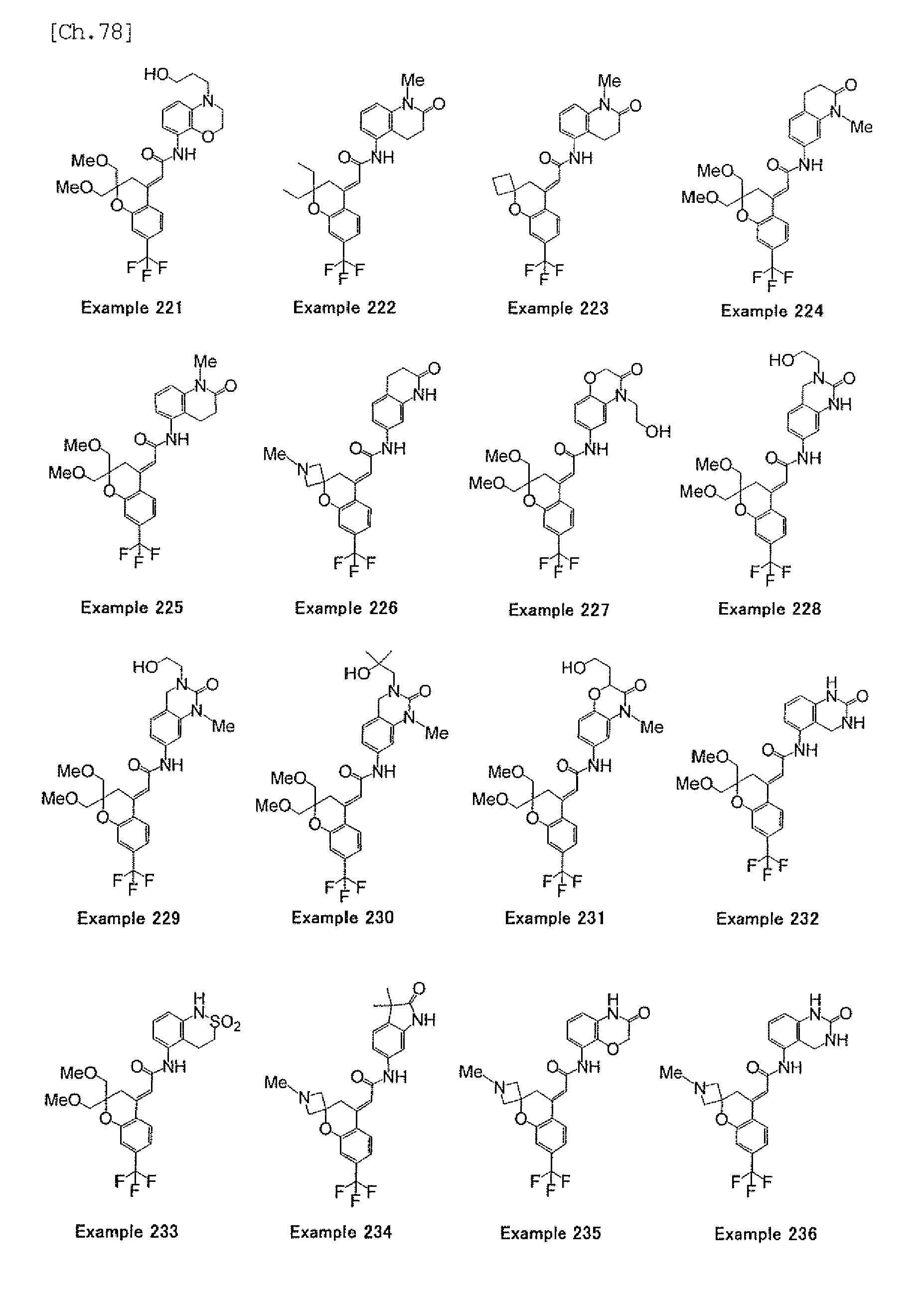

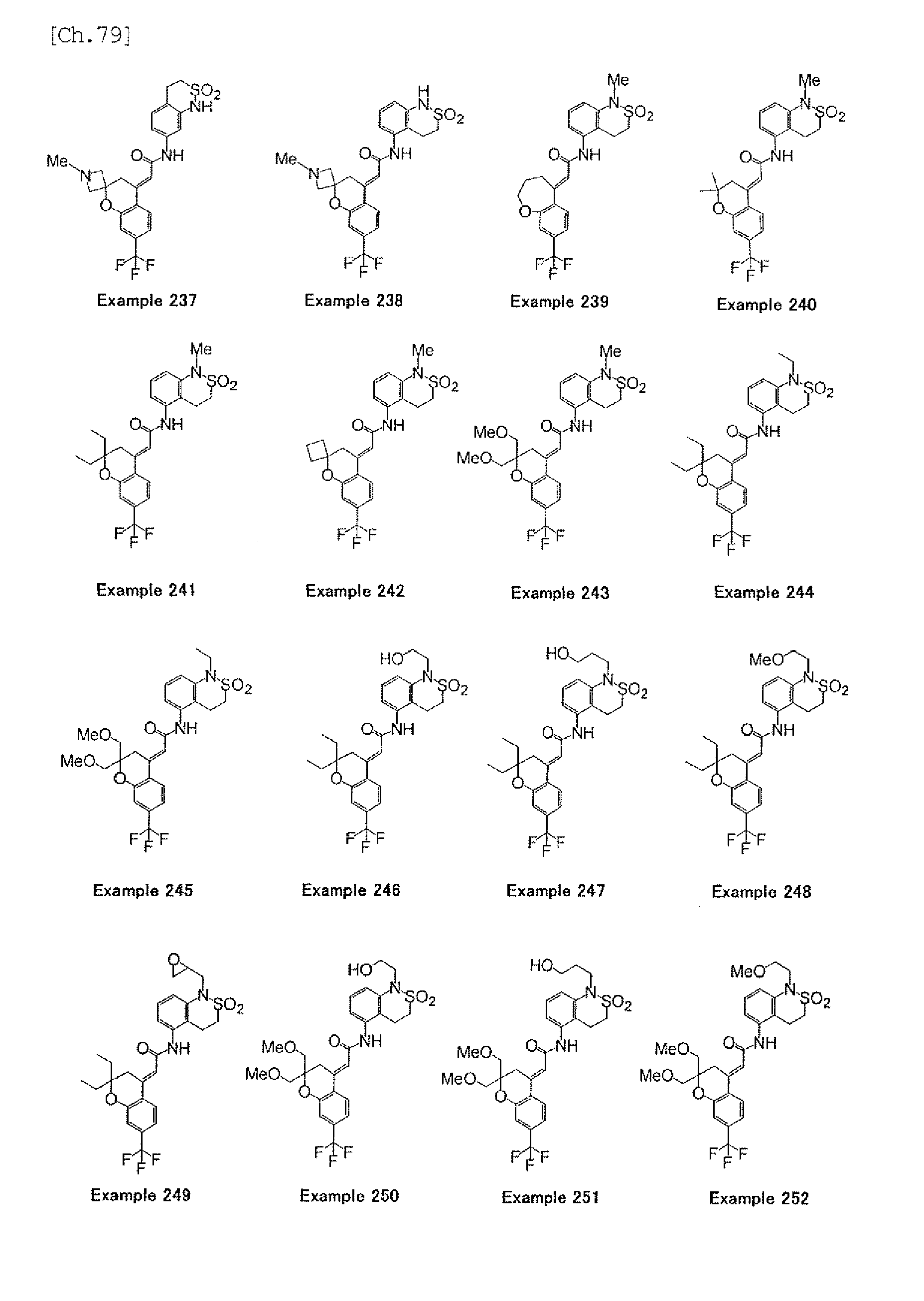

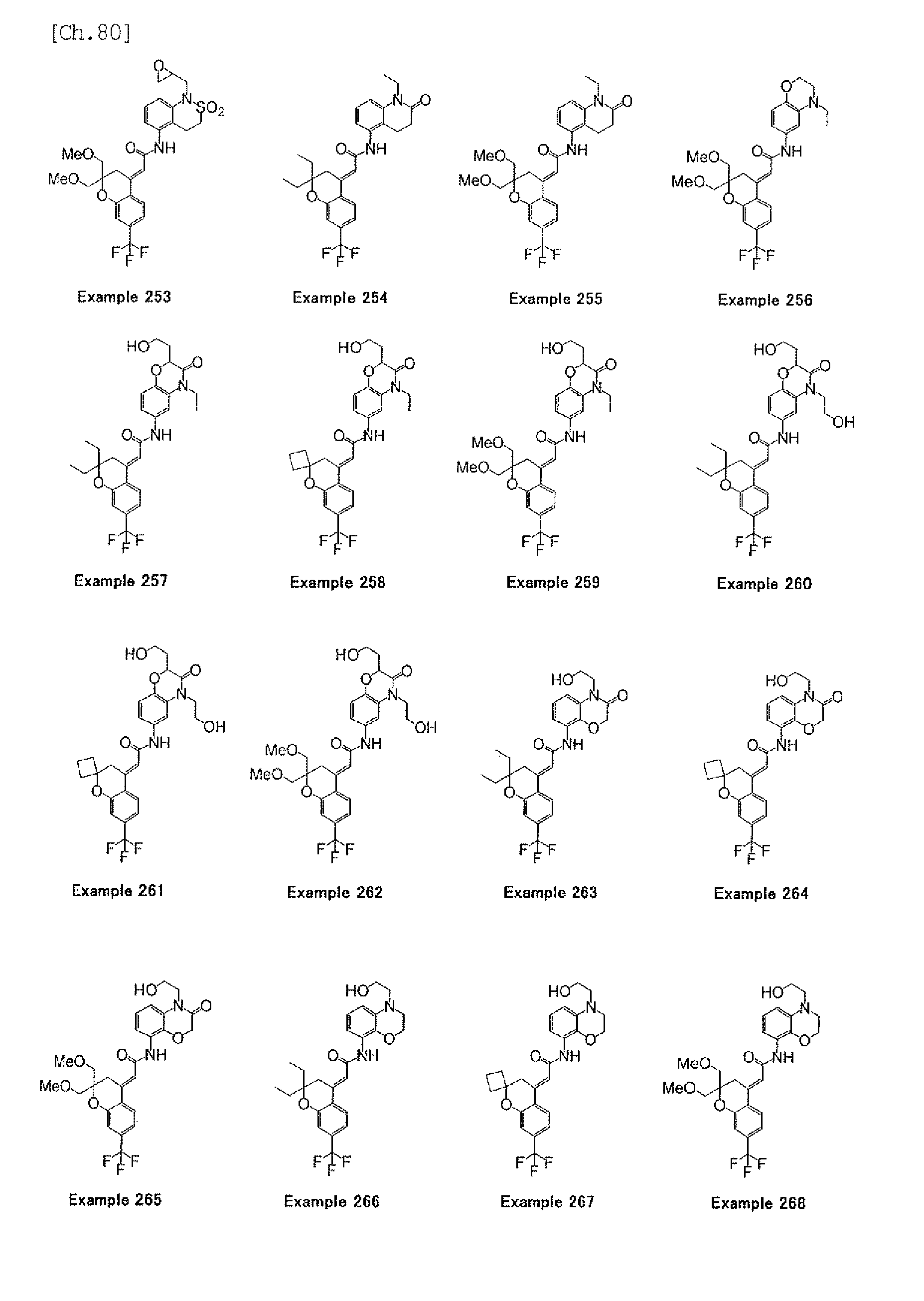

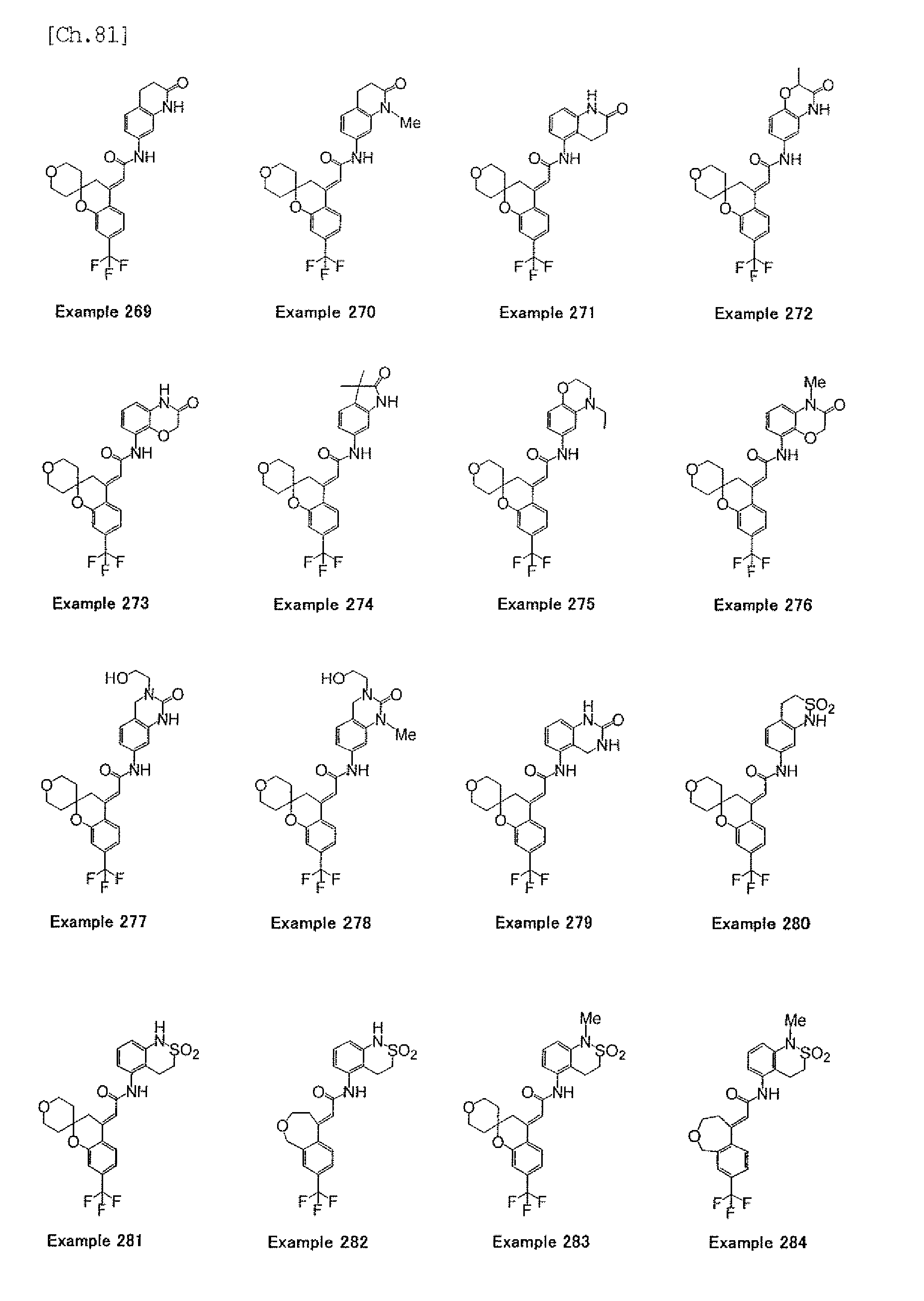

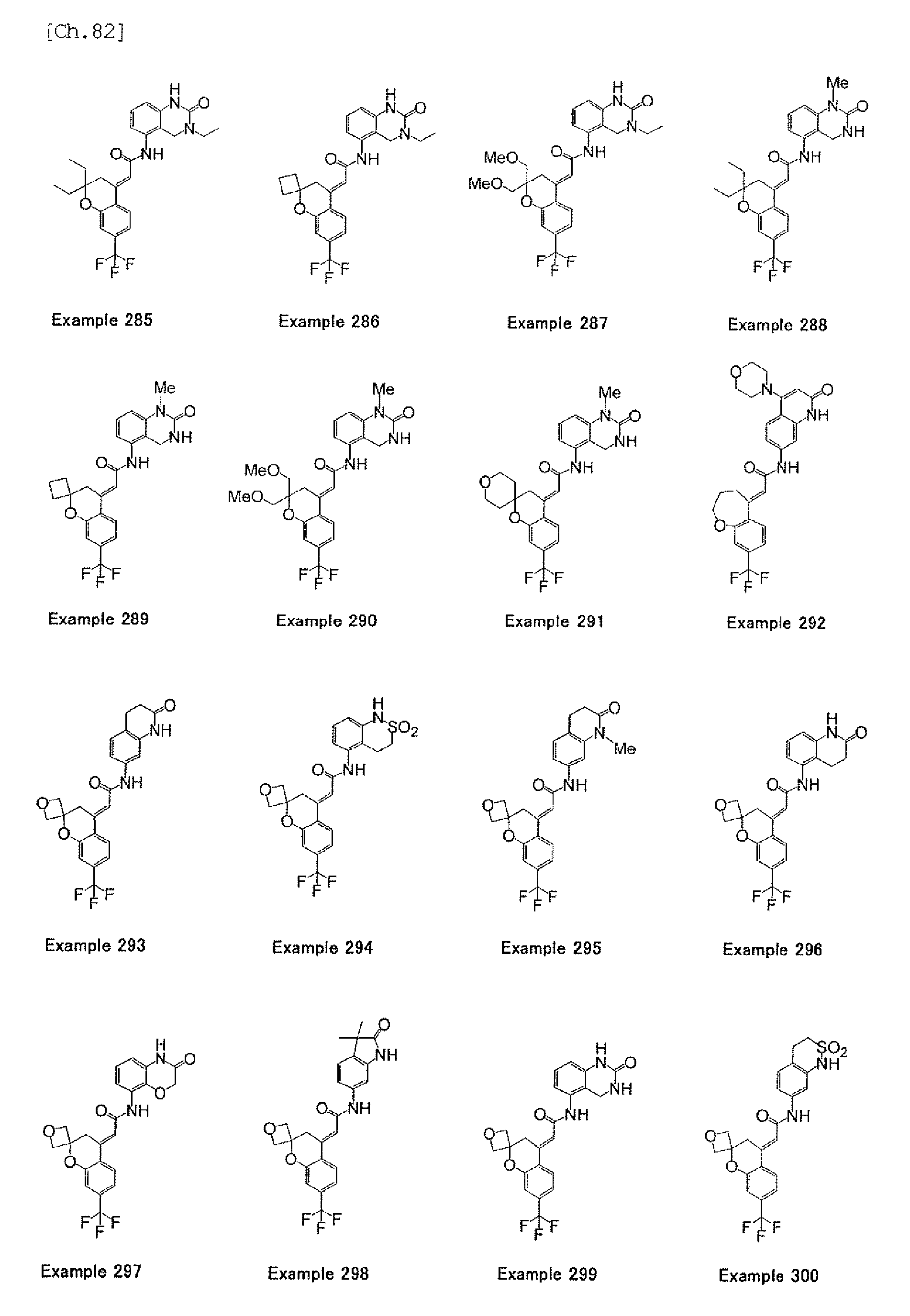

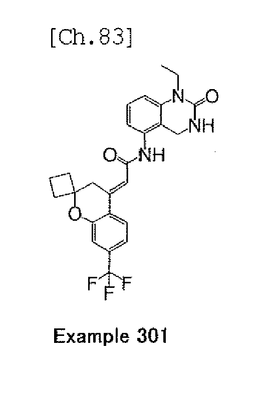

**[Table 11]**

| Example | LC Mass | Retention time (min) | Example | LC Mass | Retention time (min) | Example | LC Mass | Retention time (min) |
|---|---|---|---|---|---|---|---|---|
| | (M+1)⁺ | | | (M+1)⁺ | | | (M+1)⁺ | |
| 1 | 447 | 5.09 | 41 | 476 | 4.4 | 81 | 489 | 6.13 |
| 2 | 433 | 4.89 | 42 | 488 | 2.93 | 82 | 503 | 6.32 |
| 3 | 463 | 4.38 | 43 | 548 | 3.55 | 83 | 505 | 5.13 |
| 4 | 435 | 4.93 | 44 | 460 | 2.8 | 84 | 445 | 5.33 |
| 5 | 451 | 4.16 | 45 | 509 | 2.95 | 85 | 461 | 5.72 |
| 6 | 467 | 4.79 | 46 | 486 | 2.87 | 86 | 475 | 5.28 |
| 7 | 418 | 4.28 | 47 | 504 | 3.47 | 87 | 445 | 5.83 |
| 8 | 432 | 4.62 | 48 | 502 | 2.87 | 88 | 461 | 6.18 |
| 9 | 448 | 4.06 | 49 | 516 | 2.93 | 89 | 490 | 2.95 |
| 10 | 446 | 4.6 | 50 | 530 | 3.05 | 90 | 530 | 4.05 |
| 11 | 460 | 5.03 | 51 | 526 | 3.15 | 91 | 536 | 4.92 |
| 12 | 419 | 4.62 | 52 | 501 | 2.89 | 92 | 504 | 3.38 |
| 13 | 418 | 4.35 | 53 | 516 | 3.03 | 93 | 445 | 4.30 |
| 14 | 430 * | 4.59 | 54 | 519 | 4.04 | 94 | 459 | 4.57 |
| 15 | 462 | 4.26 | 55 | 530 | 3.09 | 95 | 487 | 5.08 |
| 16 | 476 | 4.72 | 56 | 516 | 3.03 | 96 | 471 | 4.72 |
| 17 | 452 * | 4.74 | 57 | 530 | 2.97 | 97 | 472 | 4.85 |
| 18 | 417 | 4.48 | 58 | 500 | 4.75 | 98 | 534 | 5.33 |
| 19 | 461 | 4.52 | 59 | 453 | 4.81 | 99 | 548 | 5.80 |
| 20 | 419 | 4.85 | 60 | 459 | 5.55 | 100 | 562 | 6.05 |
| 21 | 418 | 4.34 | 61 | 443 | 5.17 | 101 | 574 | 6.22 |
| 22 | 432 | 4.95 | 62 | 491 | 4.55 | 102 | 516 | 5.20 |
| 23 | 403 | 4.48 | 63 | 458 | 2.40 | 103 | 528 | 5.15 |
| 24 | 431 | 4.93 | 64 | 504 | 3.80 | 104 | 477 | 4.92 |
| 25 | 445 | 5.35 | 65 | 517 | 3.87 | 105 | 505 | 5.40 |
| 26 | 432 | 4.84 | 66 | 418 | 3.78 | 106 | 489 | 5.10 |
| 27 | 418 | 4.28 | 67 | 473 | 5.95 | 107 | 519 | 5.48 |
| 28 | 447 | 4.32 | 68 | 417 | 4.42 | 108 | 503 | 5.10 |
| 29 | 445 | 5.15 | 69 | 431 | 4.72 | 109 | 445 | 5.28 |
| 30 | 503 | 3.78 | 70 | 431 | 4.82 | 110 | 457 | 5.45 |
| 31 | 501 | 2.97 | 71 | 443 | 4.92 | 111 | 505 | 4.82 |
| 32 | 516 | 2.87 | 72 | 491 | 4.33 | 112 | 446 | 4.77 |
| 33 | 517 | 4.06 | 73 | 447 | 5.23 | 113 | 460 | 5.03 |
| 34 | 502 | 3.68 | 74 | 477 | 4.70 | 114 | 488 | 5.58 |
| 35 | 502 | 3.74 | 75 | 473 | 5.60 | 115 | 472 | 5.22 |
| 36 | 417 | 4.75 | 76 | 459 | 5.40 | 116 | 460 | 4.97 |
| 37 | 491 | 4.22 | 77 | 489 | 4.83 | 117 | 474 | 5.25 |
| 38 | 508 | 5.35 | 78 | 461 | 5.50 | 118 | 502 | 5.78 |
| 39 | 522 | 5.83 | 79 | 475 | 5.72 | 119 | 486 | 5.42 |
| 40 | 462 | 4.18 | 80 | 475 | 5.90 | 120 | 476 | 4.57 |

| | | | | | | | | |
|---|---|---|---|---|---|---|---|---|
| * (M-1)⁻ | | | | | | | | |

**[Table 12]**

| Example | LC Mass | Retention time (min) | Example | LC Mass | Retention time (min) | Example | LC Mass | Retention time (min) |
|---|---|---|---|---|---|---|---|---|
| | (M+1)⁺ | | | (M+1)⁺ | | | (M+1)⁺ | |
| 121 | 504 | 5.08 | 161 | 486 | 5.68 | 201 | 479 | 5.90 |
| 122 | 490 | 4.43 | 162 | 474 | 5.42 | 202 | 475 | 6.10 |
| 123 | 504 | 4.70 | 163 | 488 | 5.70 | 203 | 459 | 5.73 |
| 124 | 532 | 5.20 | 164 | 516 | 6.25 | 204 | 447 | 5.43 |
| 125 | 516 | 4.83 | 165 | 490 | 4.85 | 205 | 461 | 5.73 |
| 126 | 476 | 4.42 | 166 | 518 | 5.42 | 206 | 489 | 6.32 |
| 127 | 490 | 4.68 | 167 | 502 | 5.03 | 207 | 473 | 5.95 |
| 128 | 518 | 5.20 | 168 | 504 | 4.98 | 208 | 519 | 5.67 |
| 129 | 502 | 4.85 | 169 | 532 | 5.55 | 209 | 503 | 5.27 |
| 130 | 476 | 4.40 | 170 | 516 | 5.17 | 210 | 502 ^{**} | 3.77 |
| 131 | 490 | 4.67 | 171 | 490 | 4.72 | 211 | 502 ^{**} | 3.75 |
| 132 | 518 | 5.20 | 172 | 518 | 5.22 | 212 | 516 | 4.05 |
| 133 | 502 | 4.83 | 173 | 530 | 5.40 | 213 | 516 | 4.05 |
| 134 | 476 | 4.35 | 174 | 546 | 5.78 | 214 | 461 | 6.48 |
| 135 | 490 | 4.70 | 175 | 506 | 4.18 | 215 | 445 | 6.20 |
| 136 | 518 | 5.13 | 176 | 520 | 4.45 | 216 | 493 | 5.48 |
| 137 | 502 | 4.85 | 177 | 558 | 5.97 | 217 | 475 | 6.67 |
| 138 | 490 | 4.70 | 178 | 542 | 5.58 | 218 | 507 | 5.72 |
| 139 | 518 | 5.13 | 179 | 530 | 5.50 | 219 | 505 | 5.90 |
| 140 | 502 | 4.85 | 180 | 418 | 4.25 | 220 | 489 | 5.60 |
| 141 | 506 | 4.23 | 181 | 432 | 4.47 | 221 | 537 | 4.97 |
| 142 | 490 | 4.55 | 182 | 460 | 4.98 | 222 | 473 | 5.62 |
| 143 | 504 | 4.83 | 183 | 444 | 4.63 | 223 | 457 | 5.33 |
| 144 | 516 | 5.00 | 184 | 468 | 4.90 | 224 | 505 | 4.95 |
| 145 | 532 | 5.37 | 185 | 496 | 5.38 | 225 | 505 | 4.62 |
| 146 | 502 | 4.87 | 186 | 480 | 5.05 | 226 | 458 | 2.58 |
| 147 | 516 | 5.13 | 187 | 482 | 5.33 | 227 | 537 | 4.45 |
| 148 | 528 | 5.32 | 188 | 512 | 4.88 | 228 | 536 | 4.13 |
| 149 | 544 | 5.68 | 189 | 467 | 5.13 | 229 | 550 | 4.47 |
| 150 | 432 | 4.67 | 190 | 495 | 5.63 | 230 | 578 | 4.70 |
| 151 | 418 | 4.50 | 191 | 479 | 5.30 | 231 | 551 | 4.67 |
| 152 | 446 | 4.97 | 192 | 527 | 4.78 | 232 | 492 | 4.10 |
| 153 | 458 | 5.15 | 193 | 468 | 5.05 | 233 | 527 | 4.67 |
| 154 | 474 | 5.53 | 194 | 453 | 4.65 | 234 | 472 | 2.73 |
| 155 | 446 | 4.88 | 195 | 467 | 4.93 | 235 | 460 | 2.48 |
| 156 | 460 | 5.17 | 196 | 495 | 5.47 | 236 | 459 | 2.32 |
| 157 | 472 | 5.35 | 197 | 479 | 5.10 | 237 | 494 | 2.63 |
| 158 | 460 | 5.18 | 198 | 481 | 5.50 | 238 | 494 | 2.33 |
| 159 | 474 | 5.48 | 199 | 509 | 6.00 | 239 | 467 | 5.10 |
| 160 | 502 | 6.07 | 200 | 493 | 5.67 | 240 | 481 | 5.37 |

| | | | | | | | | |
|---|---|---|---|---|---|---|---|---|
| ** free form | | | | | | | | |

**[Table 13]**

| Example | LC Mass | Retention time (min) | Example | LC Mass | Retention time (min) |
|---|---|---|---|---|---|
| | (M+1)⁺ | | | (M+1)⁺ | |
| 241 | 509 | 5.80 | 281 | 509 | 4.65 |
| 242 | 493 | 5.55 | 282 | 453 | 4.32 |
| 243 | 541 | 4.97 | 283 | 523 | 4.87 |
| 244 | 523 | 5.93 | 284 | 467 | 4.55 |
| 245 | 555 | 5.12 | 285 | 488 | 5.47 |
| 246 | 539 | 5.45 | 286 | 472 | 2.73 |
| 247 | 553 | 5.35 | 287 | 520 | 4.52 |
| 248 | 553 | 5.35 | 288 | 474 | 5.40 |
| 249 | 551 | 5.80 | 289 | 458 | 5.03 |
| 250 | 571 | 4.55 | 290 | 506 | 4.35 |
| 251 | 585 | 4.47 | 291 | 488 | 4.43 |
| 252 | 585 | 4.47 | 292 | 500 | 4.55 |
| 253 | 583 | 4.90 | 293 | 445 | 4.28 |
| 254 | 487 | 5.82 | 294 | 481 | 4.43 |
| 255 | 519 | 4.83 | 295 | 459 | 4.70 |
| 256 | 507 | 5.43 | 296 | 445 | 4.20 |
| 257 | 533 | 5.78 | 297 | 447 | 4.43 |
| 258 | 517 | 5.38 | 298 | 459 | 4.57 |
| 259 | 565 | 4.72 | 299 | 446 | 3.88 |
| 260 | 549 | 5.13 | 300 | 481 | 4.53 |
| 261 | 533 | 5.78 | 301 | 472 | 5.22 |
| 262 | 581 | 4.18 | | | |
| 263 | 505 | 5.53 | | | |
| 264 | 489 | 5.13 | | | |
| 265 | 537 | 4.50 | | | |
| 266 | 491 | 5.83 | | | |
| 267 | 475 | 5.55 | | | |
| 268 | 523 | 4.88 | | | |
| 269 | 473 | 4.60 | | | |
| 270 | 487 | 4.93 | | | |
| 271 | 473 | 4.33 | | | |
| 272 | 489 | 4.83 | | | |
| 273 | 475 | 4.68 | | | |
| 274 | 487 | 4.90 | | | |
| 275 | 489 | 5.55 | | | |
| 276 | 489 | 5.13 | | | |
| 277 | 518 | 4.22 | | | |
| 278 | 532 | 4.45 | | | |
| 279 | 474 | 4.12 | | | |
| 280 | 509 | 4.68 | | | |

**[Table 14]**

| Example | NMR data (δ : ppm)<* : 270MHz> |
|---|---|
| 1 | (*DMSO-d₆*) 10.63 (1H, s), 10.16 (1H, s), 7.56 (1H, d, J=8Hz), 7.47 (1H, d, J=2Hz), 7.42 (1H, dd, J=1,8Hz), 7.27 (1H, d, J = 1Hz) ,7.11(1H,dd,J=2,9Hz),6.88(1H,d,J=9Hz),6.43(1H,s),4.22(2H,t,J=6Hz),3.17(2H,t,J= 6Hz), 2.18-2.04 (2H, m), 1.38 (6H, s) |
| 2 | (*DMSO-d₆*) 10.68 (1H, s), 10.17 (1H, s), 7.56 (1H, d, J = 8Hz), 7.48 (1H, d, J = 2Hz), 7.42 (1H, d, J = 8Hz), 727 (1H, s) ,7.11(1H,dd,J=2.9Hz),6.91(1H,d,J=9Hz),6.43(1H,s),4.61(1H,q,J=7Hz),4.22(2H,t,J=6Hz),3.17 (2H,t,J=6Hz),2.18-2.04(2H,m),1.41(3H,d,J=7Hz) |
| 3 | (*DMSO-d₆*) 10.70 (1H, s), 10.17 (1H, s), 7.55 (1H, d, J=8Hz), 7.47 (1H, d, J=2Hz), 7.44-7.39 (1H, m), 7.27 (1H, d, J=1Hz), 7.12 (1H, dd, J=1.9Hz), 6.91 (1H, d, J=9Hz), 6.42 (1H, s), 4.69-4.58 (2H, m), 4.22 (2H, t, J=6Hz), 3.64-3.52 (2H, m), 3.17 (2H, t, J=6Hz), 2.17-2.05 (2H, m), 1.98-1.88 (1H, m), 1.86-1.72 (1H, m) |
| 4 | (*DMSO-d₆*) 10.59 (1H, s), 10.27 (1H, s), 7.58-7.37 (3H, m), 7.28-7.18 (3H, m), 6.44 (1H, s), 421 (2H, t, J=6Hz), 3.43 (2H, s), 3.15 (2H, t, J=7Hz), 2.18-2.05 (2H, m) |
| 6* | (*DMSO-d₆*) 11.27 (1H, s), 10.69 (1H, s), 7.83-7.70 (2H, m), 7.62-7.38 (3H, m), 7.29 (1H, s), 6.50 (1H, s), 4.68 (2H, s), 4.23 (2H, t, J=6Hz), 3.18 (2H, t, J=6Hz), 2.25-2.05 (2H, m) |
| 7 | (*DMSO-d₆*) 10.27 (1H, s), 9.95 (1H, s), 7.54 (1H, d, J = 8Hz), 7.44-7.38 (1H, m), 7.28-7.23 (2H, m), 7.02 (1H, dd, J = 2.8Hz),6.60(1H,d,J=8Hz),6.41(1H,s),5.84(1H,s),4.21(2H,t,J=6Hz),3.68(2H,s),3.17(2H,t,J=7Hz),2.18-2.04 (2H,m) |
| 8* | (*DMSO-d₆*) 10.46 (1H, s), 10.03 (1H, s), 7.55 (1H, d, J=8Hz), 7.41(1H, d, J = 8Hz), 7.33 (1H, d, J = 2Hz), 7.26 (1H, s),7.16(1H,dd,J=2.8Hz),6.66(1H,d,J=8Hz),6.42(1H,s),4.22(2H,t,J=6Hz),3.60(2H,s),3.17(2H,t,J=7Hz), 2.18-2.02(2H,m) |
| 9* | (*DMSO-d₆*) 10.29(1H,s),9.92(1H,s),7.54(1H,d,J=8Hz),7.41(1H,d,J=8Hz),7.26(1H,s),7.22(1H,s),7.00 (1H,d,J=9Hz),6.69(1H,d,J=9Hz),6.41(1H,s),5.86(1H,s),4.90(1H,t,J=5Hz),421(2H,t,J=6Hz),3.80-3.45 (3H,m),3.17(2H,t,J=6Hz),2.74(3H,s),2.20-2.00(2H,m) |
| 10* | (*DMSO-d₆*) 10.18(1H,s),9.93(1H,s),753(1H,d,J=8Hz),7.40(1H,d,J=8Hz),7.25-7.20(2H,m),7.01(1H,dd,J =2,8Hz),6.60(1H,d,J=8Hz),6.40(1H,s),5.84(1H,s),4.20(2H,t,J=6Hz),3.16(2H,t,J=7Hz),2.18-2.00(2H,m), 1.19(6H,s) |
| 11* | (*DMSO-d₆*) 10.43(1H,s),10.03(1H,s),7.55(1H,d,J=8Hz),7.41(1H,d,J=8Hz),7.32(1H,d,J=2Hz),726(1H, s),7.18(1H,dd,J=2.9Hz),6.64(1H,d,J=9Hz),6.43(1H,s),4.22(2H,t,J=6Hz),3.18(2H,t,J=6Hz),2.73(3H,s), 2.20-2.05(2H,m),1.22(6H,s) |
| 12 | (*DMSO-d₆*) 10.28(1H,s),10.18(1H,s),7.56(1H,d,J=8Hz),7.44(1H,d,J=2Hz),7.41(1H,d,J=1Hz),7.27(1H, d,J=1Hz),7.22(1H,dd,J=2.8Hz),7.13(1H,d,J=8Hz),6.45(1H,s),5.23(2H,s),4.22(2H,t,J=6Hz),3.17(2H,t,J= 7Hz),2.18-2.07(2H,m) |
| 13* | (*DMSO-d₆*) 10.14(1H,s), 9.07(1H,s),7.56(1H,d,J=8Hz), 7.42 (1H,d,J=8Hz) ,7.30-7.19 (2H,m) ,7.14 (1H,d,J=8Hz),7.00(1H,d,J=8Hz),6.79 (1H,s) ,6.46(1H,s),4.26(2H,s),4.22 (2H,t,J=6Hz) ,3.17 (2H,t,J =7Hz) ,2.20-2.02 (2H,m) |
| 14* | (*DMSO-d₆*) 10.15(1H,s),9.25(1H,s),7.56(1H,d,J=8Hz),7.42 (1H,d,J=8Hz) ,7.27 (1H,s) ,7.23(1H,s), 7.16(1H,d,J=8Hz),7.01(1H,d,J=8Hz),6.45(1H,s),4.34(2H,s),4.22 (2H,t,J=6Hz) ,3.16 (2H,t,J=6Hz) , 2.85(3H,s), 2.19-2.04 (2H,m) |
| 15* | (*DMSO-d₆*) 10.15(1H,s),9.20(1H,s),7.55(1H,d,J=8Hz),7.42 (1H,d,J=8Hz) ,7.27 (1H,s) ,7.23(1H,s), 7.14(1H,d,J=8Hz),7.00(1H,d,J=8Hz),6.45 (1H,s) ,4.74(1H,t,J=5Hz),4.45(2H,s),4.22 (2H,t,J=6Hz) , 3.56 (2H,dt,J=5,6Hz) ,3.47-3.28(2H,m),3.22-3.10(2H,m),2.18-2.02 (2H,m) |
| 16* | (*DMSO-d₆*) 10.15(1H,s),9.23(1H,s),7.56(1H,d,J=8Hz),7.42 (1H,d,J=8Hz) ,727 (1H,s) ,723(1H,d, J=2Hz),7.15(1H,dd,J=2.8Hz),7.01(1H,d,J=8Hz),6.45 (1H,s) ,4.43(2H,s), 4.22 (2H,t,J=6Hz) ,3.56-3.40(4H,m),3.26(3H,s),3.16(2H,t,J=6Hz),2.19-2.03 (2H,m) |
| 17* | (*DMSO-d₆*) 10.25-10.14(2H,m),7.56(1H,d,J=8Hz),7.42 (1H,d,J=8Hz) ,7.34-7.20 (3H,m) ,7.16(1H, dd,J=2.8Hz),7.06(1H,d,J=8Hz),6.44 (1H,s) ,4.36(2H,d,J=8Hz),4.22 (2H,t,J=6Hz) ,3.17 (2H,t,J= 7Hz) ,2.19-2.04 (2H,m) |
| 18 | (*DMSO-d₆*) 10.12(1H,s), 9.68(1H,s),7.59(1H,d,J =8Hz), 7.43 (1H,d,J =8Hz) ,7.27(1H,s), 7.20-7.08 (2H,m) ,6.72(1H,d,J=6Hz),6.57 (1H,s) ,4.22 (2H,t,J=6Hz) ,3.14 (2H,t,J=6Hz) ,2.80 (2H,t,J= 6Hz) ,2.42 (2H,t,J=6Hz) ,2.17-2.02 (2H,m) |
| 19 | (*DMSO-d₆*) 9.75 (1H,s) ,7.57 (1H,d,J=8Hz) ,7.41 (1H,d,J=8Hz) ,7.28-7.15(3H,m),7.10(1H,d,J= 7Hz),6.54 (1H,s) ,4.84 (1H,t,J=6Hz) , 4.20 (2H,t,J=6Hz) ,3.93 (2H,t,J=6Hz) ,3.60-3.49(2H,m), 3.12(2H,t,J=6Hz),2.81-2.68(2H,m),2.56-2.41(2H,m),2.13-2.01(2H,m) |

**[Table 15]**

| Example | NMR data (δ : ppm)<* : 270MHz> |
|---|---|
| 20 | (*DMSO-d₆*) 10.76(1H,s),9.63 (1H,s) ,7.73 (1H,d,J=8Hz) ,7.65(1H,d,J=8Hz),7.42(1H,d,J=8Hz), 7.26 (1H,s) ,6.91 (1H,d,J=8Hz) , 6.75 (1H,s) ,6.67 (1H,d,J=8Hz) ,4.63(2H,s),421 (2H,t,J= 6Hz) ,3.13(2H,t,J=6Hz),2.18-2.05(2H,m) |
| 21 | (*DMSO-d₆*) 10.33(1H,s),9.47 (1H,s) ,7.57 (1H,d,J=8Hz) ,7.44(1H,d,J=9Hz),7.27(1H,s),7.02 (1H, dd,J=3,6Hz) ,6.66-6.57 (2H,m) ,6.50 (1H,s) ,5.42 (1H,s) ,4.22 (2H,t,J=6Hz) ,3.76 (2H,d,J= 2Hz),3.15(2H,t,J=6Hz),2.15-2.04(2H,m) |
| 22 | (*DMSO-d₆*) 10.48(1H,s),9.41 (1H,s) ,7.86 (1H,d,J=8Hz) ,7.72(1H,d,J=8Hz),7.41(1H,d,J=8Hz), 7.26 (1H,s) ,7.03 (1H,t,J=8Hz) ,6.88 (1H,s) ,6.65 (1H,d,J=8Hz) ,4.22 (2H,t,J=6Hz) ,3.56(2H, s), 3.18-3.09(2H,m),2.56(3H,s),2.18-2.07(2H,m) |
| 23 | (*DMSO-d₆*) 10.19(1H,s),10.15 (1H,s) , 7.84 (1H,d,J=8Hz) , 7.35(1H,dd,J=8.2Hz),7.28(1H,d,J= 2Hz),7.23 (1H,d,J=1Hz) ,7.20 (1H,dd,J=8,2Hz) ,7.10 (1H,d,J=8Hz) ,6.79 (1H,s) ,4.28 (2H,t,J =6Hz) ,3.47-3.26(2H,m),2.82(2H,t,J=7Hz),2.43(2H,t,J=7Hz) |
| 24 | (*DMSO-d₆*) 10.22(1H,s),10.13 (1H,s) ,7.82 (1H,d,J=8Hz) ,7.35-7.28(2H,m),7.20(1H,dd,J=8.2Hz), 7.16 (1H,d,J=2Hz) ,7.10 (1H,d,J=8Hz) ,6.85 (1H,s) ,3.45-3.18(2H,m),2.82(2H,t,J=8Hz),2.43(2H,t, J=8Hz),1.33(6H,s) |
| 25* | (*DMSO-d₆*) 1028(1H,s),7.82 (1H,d,J=9Hz)7.53 (1H,s) ,7.35-7.08 (4H, m) , 6.84 (1H,s) ,323 (3H,s), 2.86-2.73(2H,m),2.58-2.40(4H,m),1.32(6H,s) |
| 26 | (*CDCl₃*) 9.60(1H,s),7.65(1H,d,J=8Hz),7.51(1H,s),727-721(2H,m),7.19(1H,s), 7.08(1H,d,J=8Hz),6-93 (1H,s),6.86(1H,dd,J=8,2Hz),5.18 (1H,s) ,2.93 (2H,t,J=7Hz) ,2.67-2.59 (2H,m) ,1.62 (6H,s) |
| 27 | (*DMSO-d₆*) 10.10(1H,s),9.47(1H,s),9.33(1H,t,J=6Hz),7.69(1H,d,J=8Hz), 7.61 (1H,d,J=8Hz) ,7.43 (1H,s),7.18(1H,s),7.14(1H,d,J=8Hz),7.02(1H,d,J=8Hz),4.90 (1H,s) ,429 (2H,t,J=5Hz) ,3.49-3.35 (2H,m) ,2.79 (2H,t,J=7Hz) ,242 (2H,t,J=7Hz) |
| 28* | (*DMSO-d₆*) 10.16(1H,s),10.14(1H,s),7.56(1H,d,J=8Hz),7.42(1H,d,J=8Hz),7.32(1H,s),727(1H,s),7.18 (1H,d,J=8Hz), 7.11(1H,d,J=8Hz),6.46(1H,s),4.70(1H,t,J=5Hz),4.22(2H,t,J=6Hz),3.75-3.65(1H,m),3.58-3.45(1H,m),3.17(2H,t,J=7Hz),2.98-2.70(2H,m),2.60-2.45(1H,m),2.18-2.03(2H,m) |
| 29* | (*DMSO-d₆*) 10.16(1H,s),10.05(1H,s),7.56(1H,d,J=8Hz),7.42(1H,d,J=8Hz),7.32(1H,d,J=2Hz),7.27(1H, s),7.18(1H,dd,J=2.8Hz),7.08(1H,d,J=8Hz),6.46(1H,s),4.22(2H,t,J=6Hz),3.17(2H,t,J=7Hz),2.69(2H,s). 2.18-2.05(2H,m),1.05(6H,s) |
| 30 | (*DMSO-d₆*) 10.18(1H,s),10.16 (1H,s) ,7.56 (1H,d,J=8Hz) , ,7.42(1H,d,J=8Hz),7.31(1H,s),726(1H, m),7.18-7.07 (2H,m) ,6.45 (1H,s) ,422 (2H,t,J=6Hz) , 3,54-3.46(4H,m),3.25-3.12(3H,m),3.08-2.84 (2H,m),2.70-2.56(4H,m),2.17-2.05(2H,m) |
| 31* | (*DMSO-d₆*) 10.14(1H,s),10.10 (1H,s) ,7.54 (1H,d,J=8Hz) ,7.41(1H,d,J=8Hz),7.28(1H,s),7.25(1H, s),7.17-7.04 (2H,m) ,6.43 (1H,s) ,421 (2H,t,J=6Hz) ,3,50-3.10(3H,m),3.05-2.80(2H,m),2.66-2.53 (4H,m),2.17-2.03(2H,m),1.50-1.28(6H,m) |
| 32 | (*DMSO-d₆*) 1016(1H,s),10.15 (1H,s) ,7.56 (1H,d,J=8Hz) ,7.42(1H,d,J=8Hz),7.30(1H,s),727(1H, s),7.18-7.07(2H,m),6.45 (1H,s) ,422 (2H,t,J=6Hz) ,324-3.12(2H,m),3.07-2.80(2H,m),2.68-2.57(2H,m), 2.34-2.04(6H,m),2.11(6H,s) |
| 33 | (*DMSO-d₆*) 10.21(1H,s),10.20 (1H,s) ,781 (1H,d,J=8Hz) , 7.36-7.27(2H,m),7.22-7.08(3H,m),6.84 (1H,s) , 3.51 (4H,t,J=4Hz) ,3.38-3.28(2H,m),3.24-3.18(1H,m),3.09-2.83(1H,m),2.70-2.56(4H,m),1.33 (6H,s) |
| 34 | (*DMSO-d₆*) 10.17(2H,s),7.56 (1H,d,J=8Hz) ,7.42(1H,d,J=8Hz),7.34-7.23(2H,m),7.18-7.07 (2H,m) , 6.45 (1H,s) ,421 (2H,t,J=6Hz) ,3.54-3.46(4H,m),3.25-3.12(3H,m),3.08-2.84(2H,m),2.70-2.56(4H,m), 2.17-2.05(2H,m) |
| 35 | (*DMSO-d₆*) 10.17(2H,s),7.56 (1H,d,J=8Hz) ,7.42(1H,d,J=8Hz),7.34-7.23(2H,m),7.18-7.07 (2H,m) , 6.45 (1H,s) ,421 (2H,t,J=6Hz) , 3.54-3.46(4H,m),3.25-3.12(3H,m),3.08-2.84(2H,m),2.70-2.56(4H,m), 2.17-2.05(2H,m) |
| 36 | (*DMSO-d₆*) 9.52 (1H,s) ,7.60 (1H,d,J=8Hz) , 7.42 (1H,d,J=8Hz),727 (1H,s) .7.07-6.92 (2H, m) , 6.66 (1H,d,J=8Hz) ,6.56 (1H,s) ,6.02(1H,s),422 (2H,t,J=5Hz) ,3.63(2H,s),3.46(2H,s),3.22-3.07(2H,m),2.18-2.01(2H,m) |
| 37 | (*DMSO-d₆*) 9.61 (1H,s) ,9.05 (1H,s) ,7.57 (1H,d,J=8Hz) ,7.42(1H,d,J=8Hz),7.27 (1H,s) ,7.05 (1H,d,J=9Hz),6.88(1H,d,J=9Hz).6.52 (1H,s) ,4.57 (1H,t,J=5Hz) ,422(2H,t,J=6Hz),4.06 (2H,t,J= 6Hz) ,3.64-3.54(2H,m),3.14(2H,t,J=7Hz),2.84-2.74(2H,m),2.47-2.37(2H,m),2.15-2.02(2H,m),1.95-1.85(2H, m) |

**[Table 16]**

| Example | NMR data(δ : ppm) <* : 270MHz> |
|---|---|
| 38* | (*DMSO-d₆*) 10.15(1Hs),9.38(1H,s),7.54(1H,d,J=8Hz),7.45-7.21 (8H,m) ,7.11(1H,dd,J=2,8Hz),6.96 (1H,d,J=8Hz),6.44(1H,s),4.53(2H,s),425(2H,s),4.20 (2H,t,J=6Hz) ,3.15 (2H,t,J=6Hz) , 2.17-2.03 (2H,m) |
| 39* | (*DMSO-d₆*) 10.25(1H,s),7.57(1H,d,J=8Hz),7.48-7.16 (9H,m) , 7.06(1H,d,J=8Hz),6.44(1H,s),4.58(2H, s),427(2H,s),422 (2H,t,J=6Hz) ,324(3H,s),3.17 (2H,t,J=6Hz) ,2.20-2.03 (2H,m) |
| 40* | (*DMSO-d₆*) 10.20(1H,s),9.90(1H,s),7.54(1H,d,J=8Hz),7.41(1H,d,J=8Hz),7.25(1H,s),7.21 (1H,d,J=2Hz), 6.99 (1H,dd,J=2.8Hz),6.65(1H,d,J=8Hz),6.41(1H,s),5.69(1H,s),4.97-4.92(1H,m),4.21(2H,t,J=6Hz),3.49 (1H,dd,J=6,11Hz),3.35(1H,dd,J=5.11Hz),3.17(2H,t,J=7Hz),2.18-2.02(2H,m),1.18(3H,s) |
| 41* | (*DMSO-d₆*) 10.39(1H,s),9.95(1H,s),7.54(1H,d,J=8Hz),7.40(1H,d,J=8Hz),7.24(1H,s),7.22(1H,d,J=2Hz), 7.13(1H,dd,J=2.9Hz),6.58(1H,d,J=9Hz),6.41(1H,s),4.82-4.75(1H,m),4.21(2H,t,J=6Hz),3.71(1H,dd,J= 6,11Hz),3.49(1H,dd,J=5,11Hz),3.16(2H,t,J=6Hz),2.77(3H,s),2.18-2.00(2H,m),1.17(3H,s) |
| 58 | (*DMSO-d₆*) 11.77(1H,s),10.38(1H,s),7.83(1H,s),7.57(1H,d,J=8Hz),7,50(1H,d,J=9Hz),7.43(1H,d,J= 8Hz),7.34(1H,d,J=8Hz),7.28(1H,s),7.07(1H,s),6.49(1H,s),4.23(2H,t,J=6Hz),3.82-3.68(4H,m),3.54-3.00(6H, m),2.20-2.03 (2H,m) |
| 59 | (*DMSO-d₆*) 10.20(1H,s),10.16(1H,s),7.56(1H,d,J=8Hz),7.42(1H,d,J=8Hz),7.30-7.17(3Hi,m),7.12(1H,d,J =8Hz),6.44(1H,s),4.22(2H,t,J=6Hz),3.45-3.11(6H,m),2.18-2.06(2H,m) |
| 61 | (*DMSO-d₆*) 10.23(1H,s),10.93(1H,s),7.79(1H,d,J=8Hz),7.38-7.29(2H,m),7.25-7.16(2H,m),7.10(1H,d,J= 8Hz),6.86(1H,s),3.49(2H,s),2.82(2H,t,J=7Hz),2.43(2H,t,J=7Hz),2.29-2.93(2H,m),2.11-1.99(2H,m),1.93-1.77 (1H,m),1.74-1.59(1H,m) |
| 62 | (*DMSO-d₆*) 10.19(1H,s),10.11(1H,s),7.79(1H,d,J=8Hz),7.37-7.28(1H,m),7.32(1H,s),724-7.01(3H,m), 6.82(1H,s),3.55-3.18(6H,m),3.26(6H,s),2.79(2H,t,J=9Hz),243(2H,t,J=9Hz) |
| 63 | (*DMSO-d₆*) 10.13(1H,s),9.71(1H,s),7.83(1H,d,J=8Hz),7.37(1H,d,J=8Hz),727(1H,s),723(1H,d,J=8Hz), 7.13(1H,t,J=8Hz),7.00(1H,s),6.73(1H,d,J=8Hz),3.53(2H,s),3.39-3.27(2H,m),3.05(2H,d,J=8Hz),2.83(2H,t, J=8Hz),2.43(2H,t,J=8Hz),229(3H,s) |
| 66 | (*DMSO-d₆*) 9.64(1H,s),9.06(1H,s),7.76-7.58(3H,m),7.14-7.98(2H,m),6.86(1H,s),6.63(1H,d,J=8Hz),6.44 (1H,s),4.81(2H,s),423(2H,s),3.94(2H,t,J=5Hz),3.48-3.22(2H,m) |
| 81* | (*DMSO-d₆*) 10.28(1H,s),7.80(1H,d,J=8Hz),7.59(1H,d,J=2Hz),7.30(1H,d,J=2Hz),7.22(1H,dd,J=9.2Hz), 7.18(1H,s),6.98(1H,d,J=9Hz),6.82(1H,s),468(1H,q,J=7Hz),3.35(2H,s),327(3H,s),1.71-1.53(4H,m),1.43 (3H,d,J=7Hz),0.88(6H,t,J=7Hz) |
| 88* | (*DMSO-d₆*) 9.95(1H,s),7.78(1H,d,J=8Hz),729(1H,d,J=9Hz),7.16(1H,br),7.07(1H,d,J=2Hz),6.86(1H,dd, J=9,2Hz),6.80(1H,m,6.60(1H,d,J=9Hz), 4.23-4.15(2H,m),3.39-3.30(2H,m),3.27-3.19(2H,m),2.82(3H,s), 1.60-1.53(4H,m),0.88(6H,t,J=7Hz) |
| 107* | (*DMSO-d₆*) 10.29(1H,s),7.80(1H,d,J=8Hz),7.58(1H,d,J=2Hz),7.31(1H,d,J=8Hz),723(1H,dd,J=9,2Hz), 7.18(1H,brt),6.98(1H,d,J=9Hz),6.82(1H,s),4.72-4.62(2H,m),3.64-3.52(2H,m),3.41-3.29(2H,m),327(2H,s), 2.03-1.73(2H,m),1.70-1.54(4H,m),0.88(6H,t,J=7Hz) |
| 109 | (*DMSO-d₆*) 10.36(1H,s),10.27(1H,s),7.83(1H,d,J=8Hz),7.46(1H,d,J=2Hz),7.32(1H,dd,J=8.2Hz),7.20 (1H,d,J=8Hz),7.16(1H,d,J=1Hz),7.12(1H,dd,J=8.1Hz),6.85(1H,s),3.33-3.20(2H,m),1.33(6H,s),123(6H,s) |
| 154 | (*DMSO-d₆*) 9.91(1H,s),7.73(1H,d,J=8Hz),7.30-7.18(3H,m),7.11(1H,s),7.04(1H,d,J=8Hz),6.81 (1H,s),6.67(1H,br),4.27-4.21(2H,m),3.35-3.28(2H,m),3.16(3H,s),1.72-1.57(4H,m),0.89(6H,t,J=8Hz) |
| 182 | (*DMSO-d₆*) 9.61(1H,5),9.06(1H,s),7.81(1H,d,J=9Hz),7.29(1H,d,J=8Hz),7.17(1H,s),7.14-8.98(2H,m), 6.93-6.85(2H,m),6.63(1H,d,J=8Hz),423(2H,s),3.47-3.25(2H,m),1.69-1.52(4H,m),0.87(6H,t,J=7Hz) |
| 183 | (*DMSO-d₆*) 9.71(1H,s),9.14(1H,s),7.89(1H,d,J=9Hz),7.40(1H,d,J=8Hz),7.29(1H,s),7.22-7.10(2H,m), 7.03-6.92(2H,m),6.70(1H,d,J=7Hz),4.32(2H,s),3.62-3.30(2H,m),2.37-2.02(4H,m),1.97-1.64(2H,m) |
| 186 | (*DMSO-d₆*) 10.28(1H,br),9.75(1H,s),7.82(1H,d,J=8Hz),7.34(1H,d,J=8Hz),7.28-7.00(1H,m),7.22(1H,5), 7.18(1H,d,J=8Hz),7.06(1H,d,J=8Hz),6.92(1H,s),6.60(1H,d,J=8Hz),4.34(2H,d,J=7Hz),345(2H,s),229-1.99(4H,m),1.92-1.75(1H,m),1.73-1.57(1H,m) |
| 194 | (*DMSO-d₆*) 10.20(1H,br),9.63(1H,s),7.58(1H,d,J=8Hz),7.43(1H,d,J=8Hz),7.27(1H,s),7.20-7.07(2H,m), 6.62(1H,d,J=9Hz),6.54(1H,s),4.22(2H,t,J=6Hz),3.25-3.08(4H,m),2.64-2.38(2H,m),2.16-2.03(2H,m) |
| 196 | (*DMSO-d₆*) 10.18(1H,br),9.65(1H,s),7.82(1H,d,J=9Hz),7.30(1H,d,J=9Hz),7.21-7.08(3H,m),6.93(1H,s), 6.62(1H,d,J=9Hz),3.48-3.26(4H,m),3.20(2H,t,J=7Hz),1.70-1.53(4H,m),0.88(6H,t,J=7Hz) |
| 269* | (DMSO-d₆)) 1021(1H,s),10.12(1H,s),781(1H,d,J=8Hz),7.34(1H,d,J=8Hz),7.31(1H,d,J=2Hz),727(1H, s),7.20(1H,dd,J=2,8Hz),7.10(1H,d,J=8Hz),6.87(1H,s),3.78-3.59(4H,m),3.42(2H,s),282(2H,t,J=7Hz),243 (2H,t,J=7Hz),1.78-1.59(4H,m) |
| 293* | (DMSO-d₆)) 10.25(1H,s),10.13(1H,s),7.78(1H,d,J=8Hz),7.39(1H,d,J=8Hz),7.31(1H,s)7.31(1H,s),721 (1H,d,J=8Hz),7.10(1H,d,J=8Hz),6.87(1H,s),4.62(2H,d,J=7Hz),4.48(2H,d,J=7Hz),3.74(2H,s),2.90-2.70 (2H,m),2.57-2.36(2H,m) |

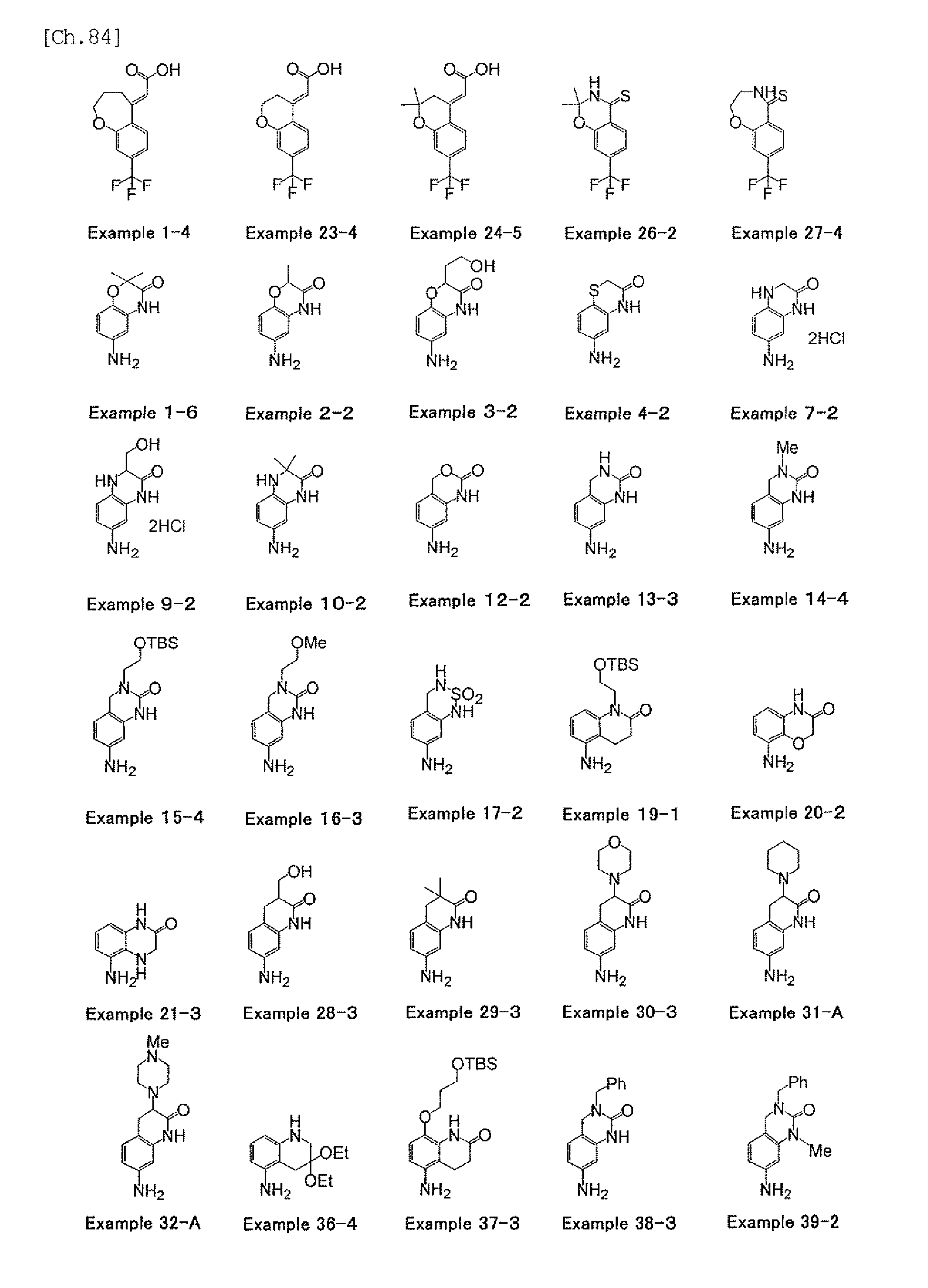

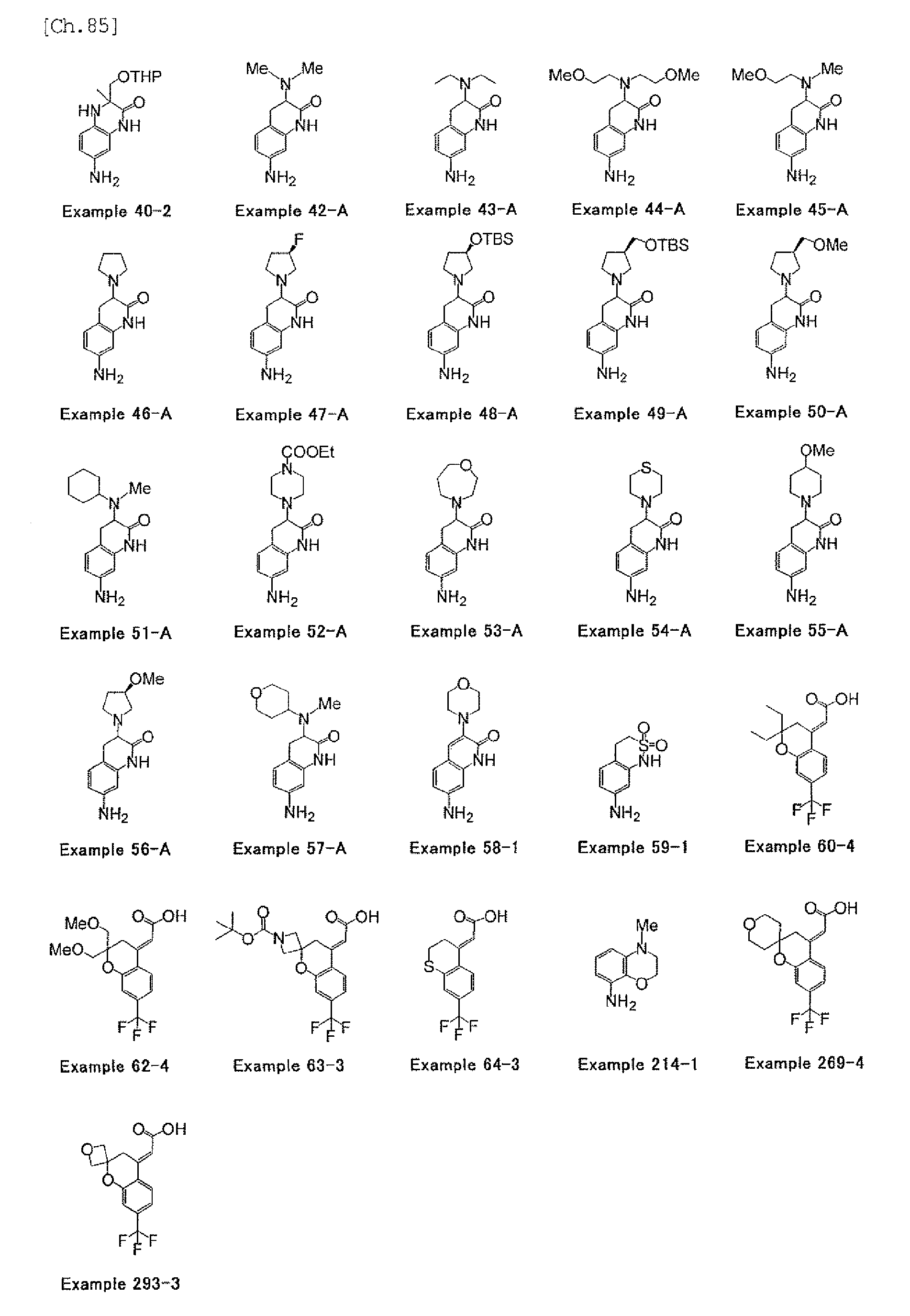

**[Table 17]**

| Example | NMR data (δ : ppm)<* : 270MHz> |
|---|---|
| 1-4* | (*CDCl₃*) 7.42 (1H,d,J=8Hz) ,728-7.18 (2H,m) ,6.19 (1H,s) ,423 (2H, t, J=6Hz) ,322 (2H, t, J=6Hz) .230-216 (2H, m) |
| 23-4* | (*DMSO-d₆*) 8.01(1H,d,J=8Hz) ,7.27-7.20 (2H,m) ,6.52 (1H,s) ,427 (2H,t,J=6Hz) ,3.28 (2H,d,J=6Hz) |
| 24-5* | (*CDCl₃*) 7.68(1H,d,J=9Hz),7.19-7.07(2H,m),6.47(1H,s),3.28(2H,s),1.39(6H,s) |
| 26-2 | (*CDCl₃*) 8.43(1H,d,J=8Hz),8.28(1H,bs),7.30 (1H,dd,J=1,8Hz) ,7.18(1H,d,J=1Hz),1.68(6H,s) |
| 27-4* | (*CDCl₃*) 8.59(1H,bs),8.13(1H,d,J=8Hz),7.47-7.38 (1H,m) ,7.30-7.27(1H,m),4.43(2H,d,J=6Hz),3.58 (2H,d,J=6,6Hz) |
| 1-6 | (*DMSO-d₆*) 10.34(1H,s),6.59(1H,d,J=8Hz),6.14(1H,d,J=3Hz),6.11(1H,dd,J=3,8Hz),4.79 (2H,bs) , 1.32(6H,s) |
| 2-2 | (*DMSO-d₆*) 10.39(1H,s),6.62(1H,d,J=8Hz),6.15(1H,d,J=3Hz),6.11(1H,dd,J=3.8Hz),481 (2H,bs) , 4.42(1H,q,J=7Hz),1.35(3H,d,J=7Hz) |
| 3-2 | (*DMSO-d₆*) 10.41(1H,s),6.61(1H,d,J=8Hz),6.15(1H,d,J=3Hz),6.11(1H,dd,J=3,8Hz),4.82 (2H,bs) , 4.60(1H,t,J=5Hz),4.42(1H,dd,J=4,9Hz),3.57-3.50(2H,m),1.95-1.68(2H,m) |
| 4-1* | (*CDCl₃*) 6.78(1H,d,J=9Hz),6.46(1H,d,J=2Hz),6.31(1H,dd,J=9,2Hz),4.50(2H,s),4.03-3.94(2H,m). 3.91-3.83 (2H,m) , 3.51(2H,bs),0.86 (9H,s) ,0.01(6H,s) |
| 5-2* | (*DMSO-d₆*) 10.28(1H,s),6.90(1H,d,J=8Hz),6.25-6.17(2H,m),5.20(2H,s),3.33(2H,s) |
| 7-2 | (*DMSO-d₆*) 10.51(1H,s),10.02(2H,bs),6.81=6.67(3H,m),4.89 (3H,bs) ,3.75(2H,s) |
| 9-2* | (*DMSO-d₆*) 10.49(1H,s),9.88(2H,s),6.75-6.68(3H,m),3.85-3.50(3H,m) |
| 10-2* | (*CDCl₃*) 6.55(1H,d,J=8Hz),6.30(1H,dd,J=2,8Hz),6.11(1H,d,J=2Hz),1.37(6H,s) |
| 12-2 | (*DMSO-d₆*) 9.86(1H,s),6.79 (1H,d,J=8Hz) ,6.18(1H,dd,J=2,8Hz),6.11(1H,d,J=2Hz),5.21(2H,bs), 5.06(2H,s) |
| 13-3* | (*DMSO-d₆*) 8.74(1H,s),6.67 (1H,d,J=8Hz) ,6.62(1H,s),6.07(1H,dd,J=2,8Hz),6.00(1H,d,J=2Hz), 4.95(2H,s),4.12(2H,s) |
| 14-4* | (*DMSO-d₆*) 8.93(1H,s),6.69 (1H,d,J=8Hz) ,6.08(1H,dd,J=2,8Hz),5.99(1H,d,J=2Hz),4.98(2H,s), 4.19(2H,s),2.81(3H,s) |
| 15-4* | (*DMSO-d₆*) 8.89(1H,s),6.65 (1H,d,J=8Hz) ,6.07(1H,dd,J=2,8Hz),5.99(1H,d,J=2Hz),4.97(2H,s), 4.32(2H,s),3.72(2H,t,J=6Hz),4.40-4.26(2H,m),0.84(9H,s),0.01(6H,s) |
| 16-3* | (*DMSO-d₆*) 8.91(1H,s),6,68 (1H,d,J=8Hz) ,6.08(1H,dd,J=2,8Hz),6.00(1H,d,J=2Hz),4.98(2H,s), 4.28(2H,s),3.51-3.38(4H,m),3.25(3H,s) |
| 17-2* | (*DMSO-d₆*) 9.71(1H,bs),7.09 (1H,t,J=7Hz) ,6.72(1H,d,J=8Hz),6.14(1H,dd,J=2,8Hz),5.93(1H,d,J =2Hz),5.06(2H,s),4.21(2H,d,J=7Hz) |
| 19-1 | (*CDCl₃*)7.08(1H,J=8Hz),6.80(1H,d,J=8Hz),6.50(1H,d,J=8Hz),4.07(2H,t,J=6Hz),3.90(2H,t,J=6Hz), 3.69(2H,s),2.78-2.65(4H,m),0.91(9H,s),0.07(6H,s) |
| 20-2* | (*DMSO-d₆*) 10.46(1H,s),6.63 (1H,t,J=8Hz) .6.31 (1H,d,J=8Hz) ,6.12(1H,d,J=8Hz),4.85(2H,s), 4.49(2H,s) |
| 21-2* | (*DMSO-d₆*) 10.52(1H,s),6.86(1H,d,J=8Hz),6.81-6.65 (2H,m) ,3.97 (2H,s) |
| 28-3* | (*CDCl₃*) 7.46(1H,s)6.95(1H,d,J=8Hz),6.35(1H,dd,J=2,8Hz),6.08(1H,d,J=2Hz),3.95-3.77(2H,m), 3.66(2H,s),3.36-3.28(1H,m),2.80-2.68(3H,m) |
| 29-3* | (*CDCl₃*) 7.32(1H,s),6.81(1H,d,J=8Hz),6.32(1H,dd,J=2,8Hz),6.05(1H,d,J=2Hz),3.63(2H,s),2.68(2H, s),1.90(6H,s) |
| 30-3 | (*DMSO-d₆*) 9.89 (1H,s) ,6.78(1H,d,J=8Hz),6.11(1H,t,J=8Hz),6.07(1H,s),4.95(2H,br),3.51(4H,t,J= 4Hz),3.18-3.10(1H,m),2.93-2.80(1H,m),2.78-2.67(1H,m),2.67-2.55(4H,m) |
| 31-A | (*DMSO-d₆*) 9.81 (1H,s) ,6.77(1H,d,J=8Hz),6.10(1H,dd,J=8.2Hz),6.06(1H,d,J=2Hz),4.93(2H,b), 3.16(1H,dd,J=10,6Hz),2.83(1H,dd,15,10Hz),2.72(1H,dd,J=15,6Hz),2.61-2.53(4H,m),1.50-1.32(6H,m) |
| 32-A | (*DMSO-d₆*) 9.85 (1H,s) ,6.77(1H,d,J=8Hz),6.11(1H,d,J=8Hz),6.07(1H,s),4.94(2H,br),3.17-3.08(1H, m), 2.90-2.68 (2H,m),2.67-2.54(4H,m),2.33-2.17(4H,m),211(3H,s) |
| 36-4* | (*CDCl₃*)6.84(1H,t,J=8Hz),6.11(1H,d,J=8Hz),6.07(1H,d,J=8Hz),3.79(1H,s),3.69-3.55(4H,m),3.48(2H, s),3.27(2H,s),2.70(2H,s),1.20(6H,t,J=7Hz) |
| 37-3 | (*CDCl₃*).7.77(1H,s),6.65(1H,d,J=9Hz,6.36(1H,d,J=9Hz),4.03(2H,t,J=6Hz),3.77(2H,t,J=6Hz),3.41(2H, bs),2.84-2.74(2H,m),2.68-2.59(2H,m),2.00-1.89(2H,m),0.88(9H,s),0.04(6H,s) |
| 38-3* | (*DMSO-d₆*) 9.05(1H,s),7.38-7.20 (5H,m) ,6.63(1H,d,J=8Hz),6.10-5.98(2H,m),4.99(2H,s),4.49(2H, s),4.11(2H,s) |

**[Table 18]**

| Example | NMR data (δ : ppm) <* : 270MHz> |
|---|---|
| 39-2* | (*DMSO-d₆*) 7.37-7.18 (5H, m) , 6.70 (1H, d, J = 8Hz), 6.19-6.10 (2H, m), 5.08 (2H, s), 4.52 (2H, s), 4.10 (2H, s), 3.15(3H, s) |
| 40-2* | (*DMSO*-*d₆*) 10.02 (1H, s), 6.50-6.40 (1H, m), 6.10-6.00 (2H, m), 5.07 (1H, s), 4.55-4.35 (3H, m), 3.70-3.20 (4H, m), 1.75-1.20 (6H, m), 1.20-1.15 (3H, m) |
| 42-A | (*DMSO*-*d₆*) 9.82 (1H, s), 6.77 (1H, d, J = 8Hz), 6.15-6.03 (2H, m), 4.94 (2H, s), 3.05 (1H, dd, J = 6,10Hz), 2.81 (1H, dd, J = 10,15Hz), 2.90 (1H, dd, J = 6,15Hz), 2.29 (6H, s) |
| 43-A | (*DMSO*-*d₆*) 9.79 (1H, s), 6.78 (1H, d, J = 8Hz), 6.13-6.04 (2H, m), 4.94 (2H, s), 3.43-3.31 (1H, m), 2.86-2.48 (6H, m), 0.99-0.92 (2H, m) |
| 44-A | (*DMSO*-*d₆*) 9.78 (1H, s), 6.78 (1H, d, J = 8Hz), 6.10 (1H, dd,J = 3,8Hz), 4.95 (2H, bs), 4.02 (2H, q, J = 7Hz), 3.37-3.15 (4H, m), 321 (6H, s), 2.98-2.57 (6H, m) |
| 45-A | (*DMSO*-*d₆*) 9.80 (1H, s), 6.77 (1H, d, J = 8Hz), 6.10 (1H, d, J = 8Hz), 6.06 (1H, s), 4.95 (1H, bs), 3.42-3.27 (3H, m), 321(3H, s), 2.88-2.60 (4H, m), 236(3H, s) |
| 46-A | (*DMSO*-*d₆*) 9.85 (1H, s) ,6.77 (1H, d, J = 8Hz), 6.10 (1H, d, J = 8Hz), 6.06 (1H, s), 4.93 (2H, br), 2.95 (1H, t, J = 6Hz), 2.79 (2H, d, J = 6Hz), 2.62-2.52 (4H, m), 1.65-1.55 (4H, m) |
| 47-A | (*DMSO*-*d₆*) 9.90 (1H, s), 6.79 (1H, d, J = 8Hz), 6.11 (1H, d, J = 3,8Hz), 6.07 (1H, s), 5.14-5.01 (1H, m), 4.95 (2H, s), 3.10-2.16 (7H, m), 2.09-2.83 (2H, m) |
| 48-A | (*DMSO*-*d₆*) 9.86 (1H, s), 6.81-6.73 (1H, m), 6.14-6.03 (2H, m), 4.93 (2H, bs), 4.34-4.23 (1H, m), 3.14-2.82 (2H, m), 2.81-2.58 (4H, m), 2.48-2.37 (1H, m), 2.00-1.85 (1H, m),1.56-1.44 (1H, m), 0.83 (9H, s), 0.01 (3H, s), 0.00 (3H, s) |
| 49-A | (*DMSO*-*d₆*) 9.82 (1H, s) , 6.80-6.72 (1H, m), 6.13-6.05 (1H, m), 4.94 (2H, br), 3.72-3.62 (1H, m), 3.48-3.10 (2H, m), 3.02-2.63 (5H, m), 1.92-1.42 (5H, m), 0.82 (9H, s) ,0.00 (6H, s) |
| 50-A | (*DMSO*-*d₆*) 9.40 (1H, s), 6.76 (1H, d, J = 8Hz), 6.18-6.10 (2H, m), 4.60 (2H, bs), 3.61 (1H, dd, J = 11, 7Hz), 3.52-3.40 (1H, m), 3.30-2.96 (5H, m), 2.87-2.63 (4H, m), 1.94-1.89 (1H, m), 1.88-1.52 (2H, m), 1.51-1.39 (1H, m) |
| 51-A | (*DMSO*-*d₆*) 9.75 (1H, s), 6.77 (1H, d, J = 8Hz), 6.10 (1H, d, J = 8Hz), 6.06 (1H, s), 4.92 (2H, bs), 3.40 (2H, s), 2.90-2.78 (1H, m), 2.70-2.42 (2H, m), 2.24 (3H, s), 1.82-1.67 (4H, m), 1.26-1.06 (6H, m) |
| 52-A | (*DMSO*-*d₆*) 9.88 (1H, s), 6.78 (1H, d, J = 8Hz), 6.15-6.03 (2H, m), 4.96 (2H, bs), 4.02 (2H, q, J = 7Hz), 3.40-3.20 (5H, m), 2.92-2.55 (6H, m), 1.22-1.10 (3H, m) |
| 53-A | (*DMSO*-*d₆*) 9.79 (1H, s), 6.78 (1H, d, J = 8Hz) , 6.10 (1H, dd, J = 2.8Hz), 6.07 (1H, d, J = 2Hz), 4.95 (2H, s), 3.73-3.40 (2H, m), 3.01-2.65 (2H, m), 1.84-1.64 (2H, m) |
| 54-A | (*DMSO*-*d₆*) 9.85 (1H, s), 6.78 (1H, d, J = 8Hz), 6.11 (1H, dd, J = 2.8Hz), 6.06 (1H, d, J = 2Hz), 4.96 (2H, s), 3.37-3.26 (1H, m), 3.06-2.79 (5H, m), 2.69 (1H, dd, J = 6,15Hz), 2.63-2.44 (4H, m) |
| 55-A | (*DMSO*-*d₆*) 9.83 (1H, s), 6.77 (1H, d, J = 8Hz), 6.10 (1H, d, J = 8Hz), 6.06 (1H, s), 4.94 (2H, s), 3.27-3.05 (5H, m), 2.90-2.65 (4H, m), 2.62-2.49 (2H, m), 1.87-1.69 (2H, m), 1.43-1.24 (2H, m) |
| 56-A | (*DMSO*-*d₆*) 9.87 (1H, s), 6.77 (1H, d, J = 8Hz), 6.11 (1H, dd, J = 2,8Hz), 6.07 (1H, d, J = 2Hz), 4.94 (2H, s), 3.87-3.75 (1H, m), 3.13 (3H, s), 2.98 (1H, t, J = 6Hz), 2.86-2.55 (6H, m), 1.98-1.82 (1H, m), 1.65-1.50 (1H, m) |
| 57-A | (*CDCl₃*) 8.08 (1H, s), 6.93 (1H, d, J = 8Hz), 6.31 (1H, dd, J = 2,8Hz), 6.11 (1H, d, J = 2Hz), 4.04-3.96 (2H, m), 3.76 (1H, dd, J = 6, 12Hz), 3.66 (2H, bs), 3.45-3.33 (2H, m), 3.12-2.87 (2H, m), 2.80 (1H, dd, J = 6, 15Hz), 2.45 (3H, s), 1.89-1.78 (2H, m), 1.68-1.50 (2H, m) |
| 58-1 | (*DMSO*-*d₆*) 11.39 (1H, s), 7.19 (1H, d, J = 9Hz), 6.93 (1H, s), 6.41 (1H, dd, J = 2,8Hz), 6.36 (1H, d, J=8Hz), 5.47 (2H, bs), 3.76-3.68 (4H, m), 3.08-2.98 (4H, m) |
| 59-1 | (*DMSO*-*d₆*) 9.74 (1H, s), 6.79 (1H, d, J = 8Hz), 6.19 (1H, d, J = 8Hz), 5.97 (1H, s), 5.04 (2H, s), 3.26-3.16 (2H, m), 3.14-3.05 (2H, m) |
| 60-4 | (CDCl3) 7.65 (1H, d, J = 8Hz), 7.18-7.07 (2H, m), 6.46 (1H, s), 3.28 (2H, s), 1.77-1.58 (4H, m), 0.93 (6H, t, J = 8Hz) |
| 62-4 | (DMSO-d6) 7.94 (1H, d, J = 8Hz), 7.30-7.13 (2H, m), 6.55 (1H, s), 3.50-3.20 (12H, m) |
| 63-3 | (CDCl3)) 7.70 (1H, d, J = 9Hz), 7.35-7.10 (2H, m), 6.52 (1H, s), 4.02 (2H, d, J = 10Hz), 3.93 (2H, d, J = 10Hz), 3.58 (2H, s), 1.45 (9H, s) |
| 64-3 | (DMSO-d6)) 7.82 (1H, d, J = 9Hz), 7.56 (1H, s), 7.42 (1H, d, J = 9Hz), 3.43-3.25 (2H, m), 3.16-3.08 (2H, m) |
| 214-1 | (CDCl3)) 6.66 (1H, t, J = 8Hz), 6.19 (1H, d, J = 8Hz), 6.18 (1H, d, J = 8Hz), 4.33 (2H, t, J = 3Hz), 3.66 (2H, br), 3.25 (2H, t, J = 3Hz), 2.86 (3H, s) |
| 269-4 | (DMSO-d6) 7.97 (1H, d, J = 8Hz), 7.28-7.20 (2H, m), 6.59 (1H, s), 3.78-3.56 (4H, m), 3.46-3.20 (2H, m), 1.80-1.56 (4H, m) |
| 293-3 | (CDCl3)) 7.68 (1H, d, J = 8Hz), 7.28-7.13 (2H, m), 6.52 (1H, s), 4.80 (2H, d, J = 7Hz), 4.62 (2H, d, J = 7Hz), 3.73 (2H, s) |

In the compound represented by formula (I) shown below, the compoun ds (Compound No. 1-2538 in Table ; Compound No. 1-2538) by combined with th e each groups shown by a group(a group:a1-a141) and b group(b group:b1-b18) can be synthesized as well as the above example. The compound represented by formula (I) can be synthesized by combing arbitrarily the groups selected from the below R¹, R², X¹, X² etc.., and the compounds of the combination shown by below table are preferred. The specific example of a group (a1-a141) and b group(b1-b18) in the table are shown in the below chemical formulae, a1-a141, b1-b18.

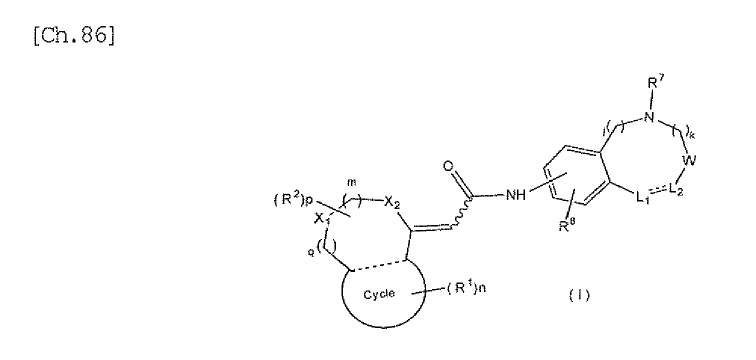

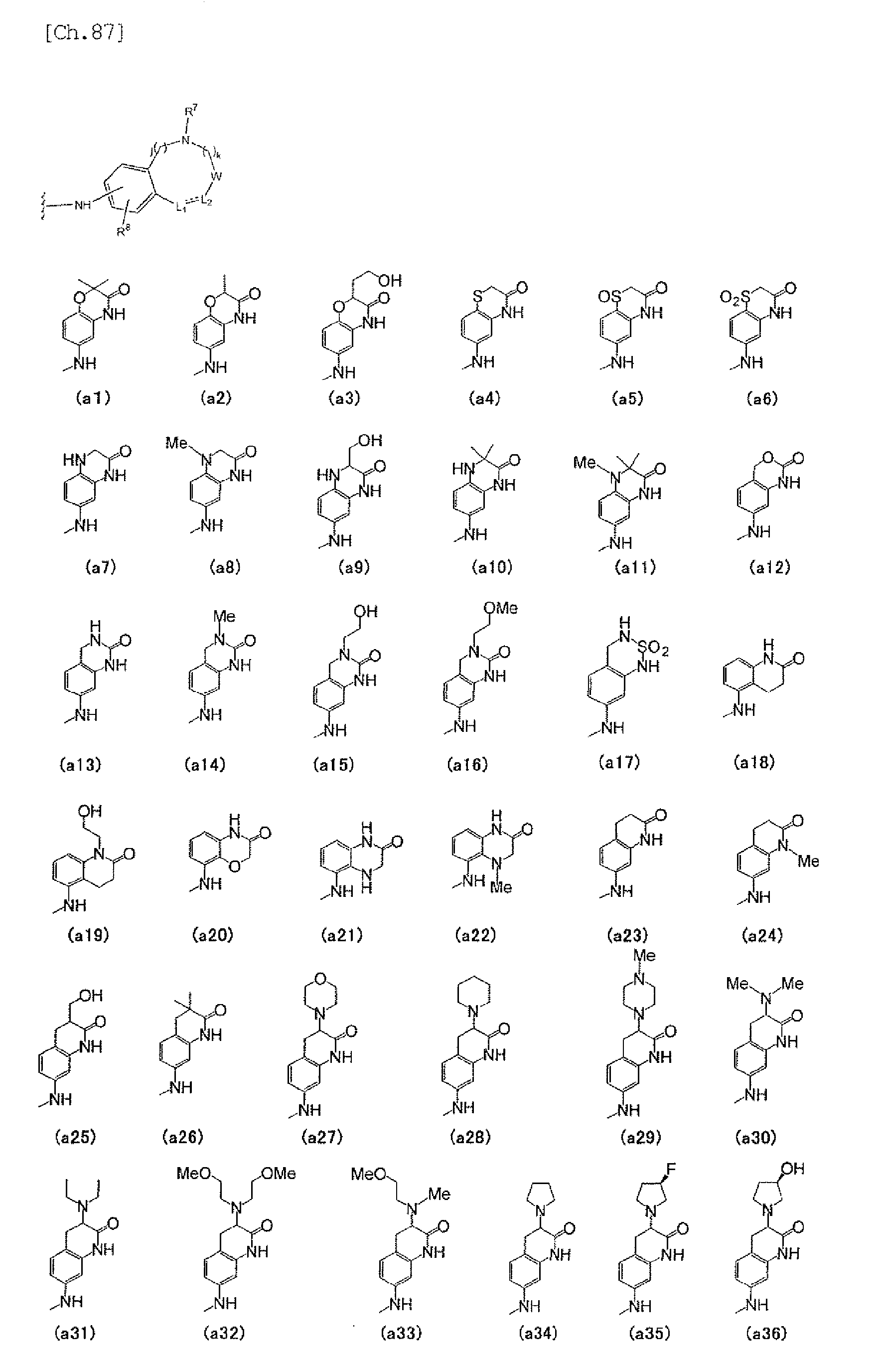

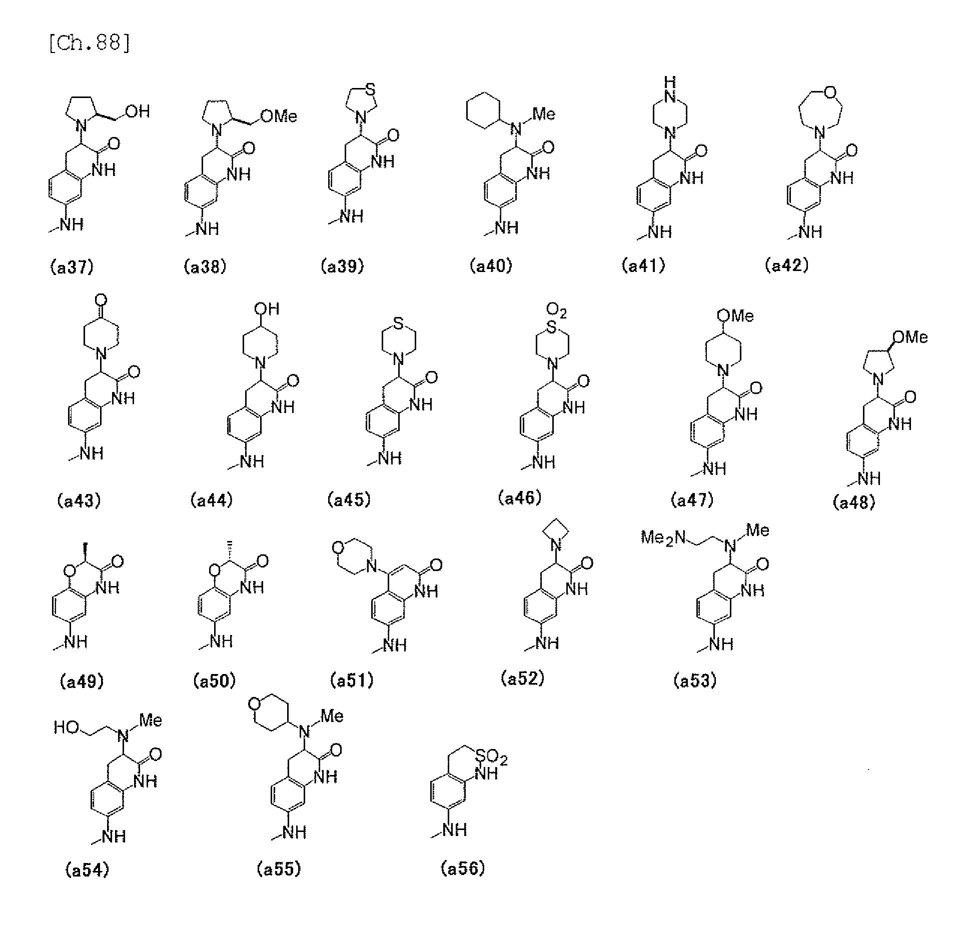

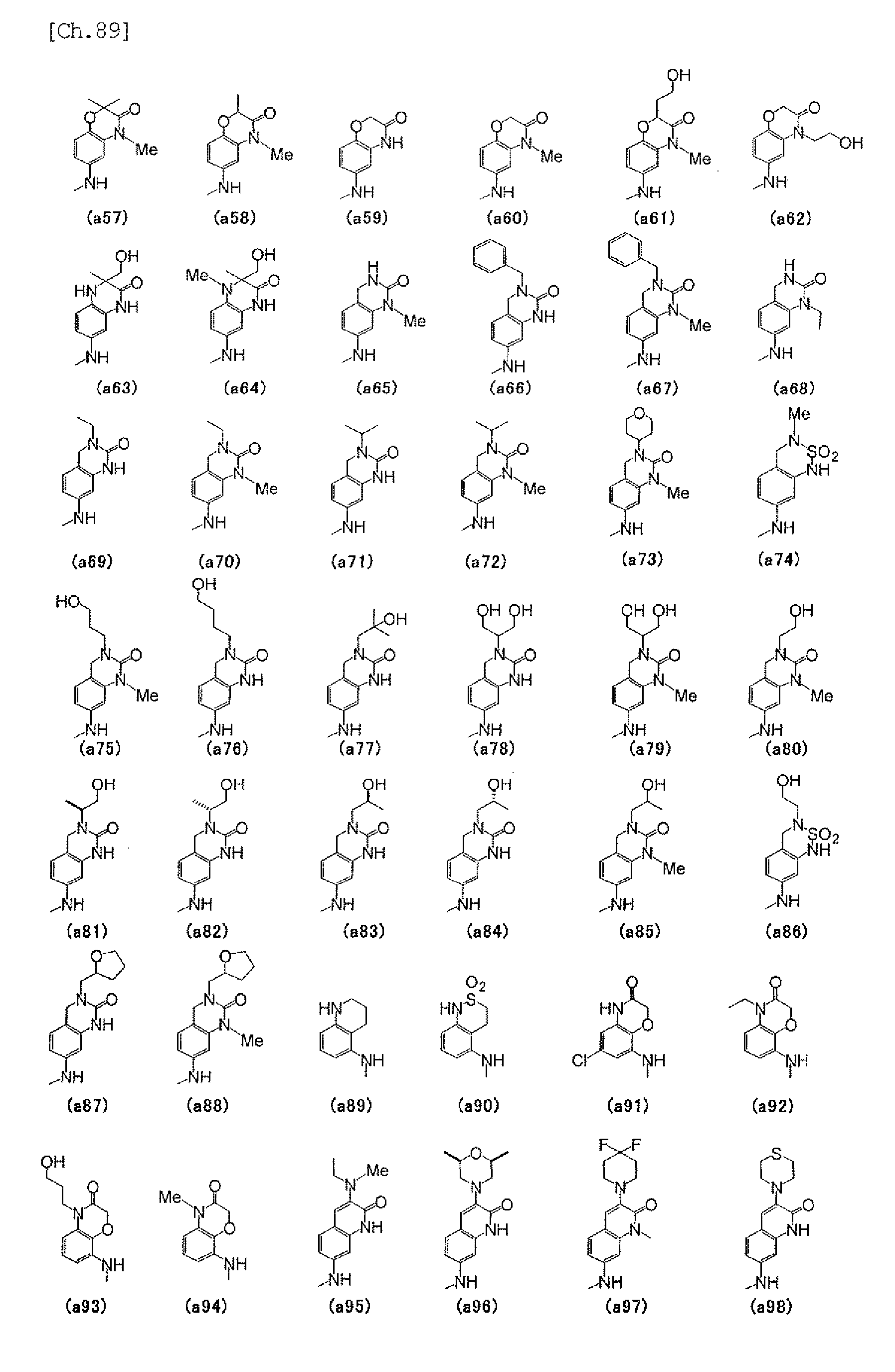

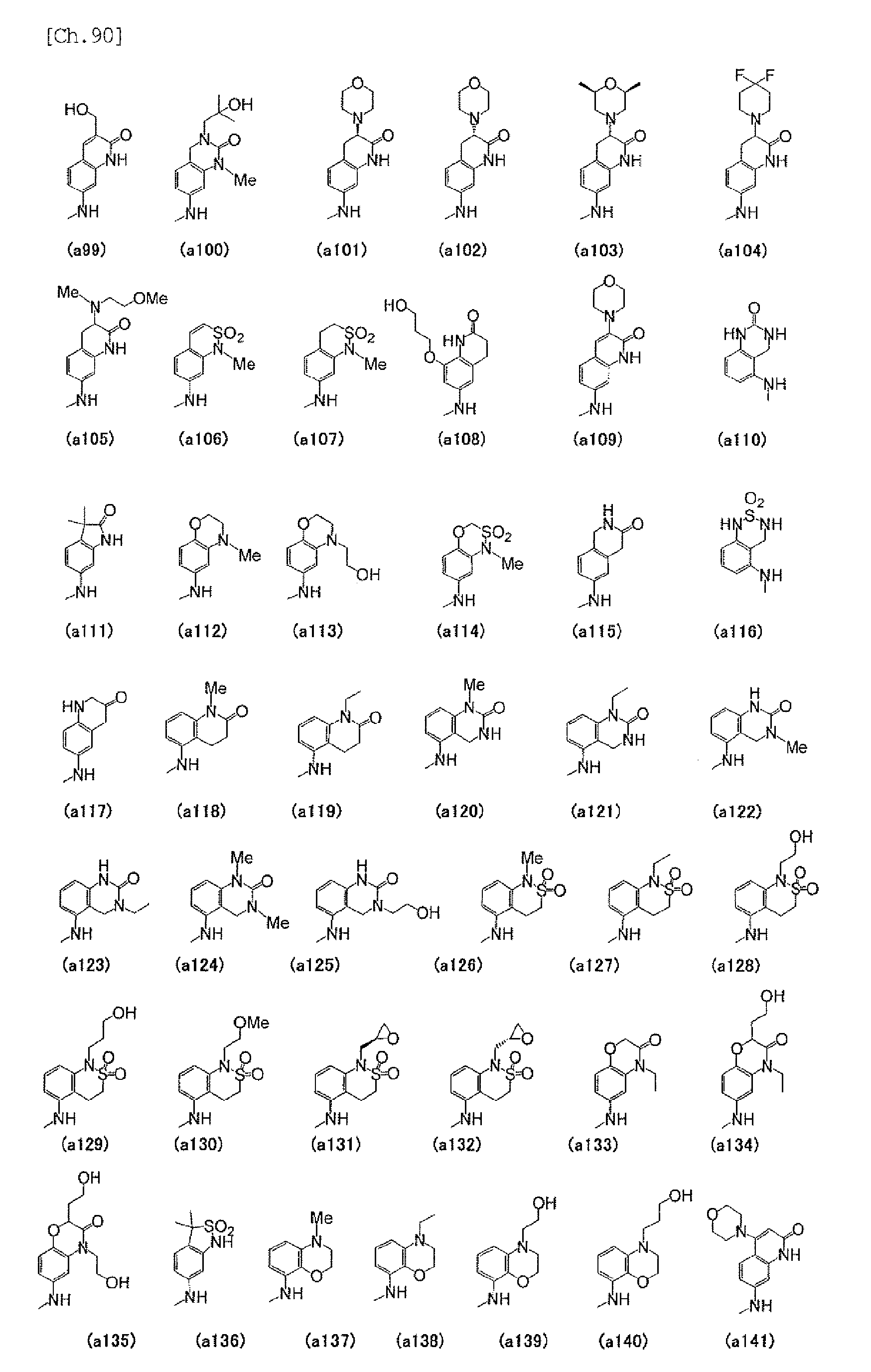

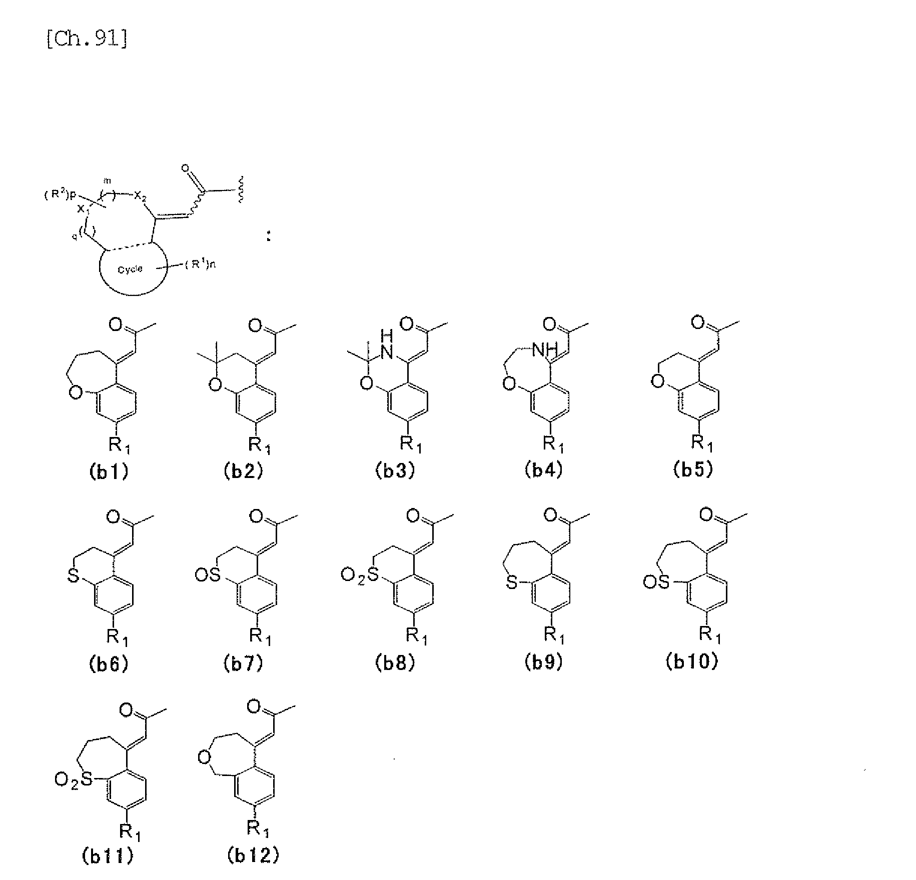

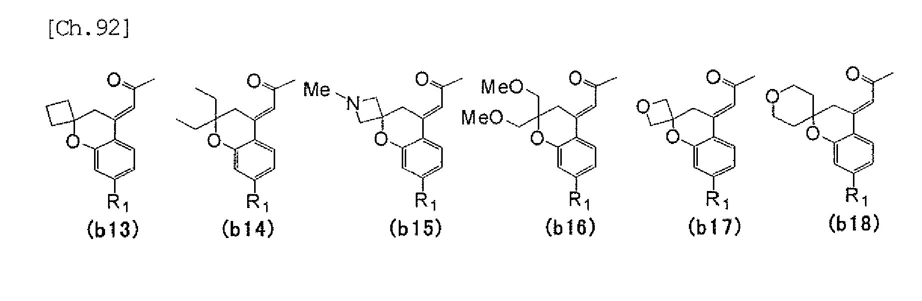

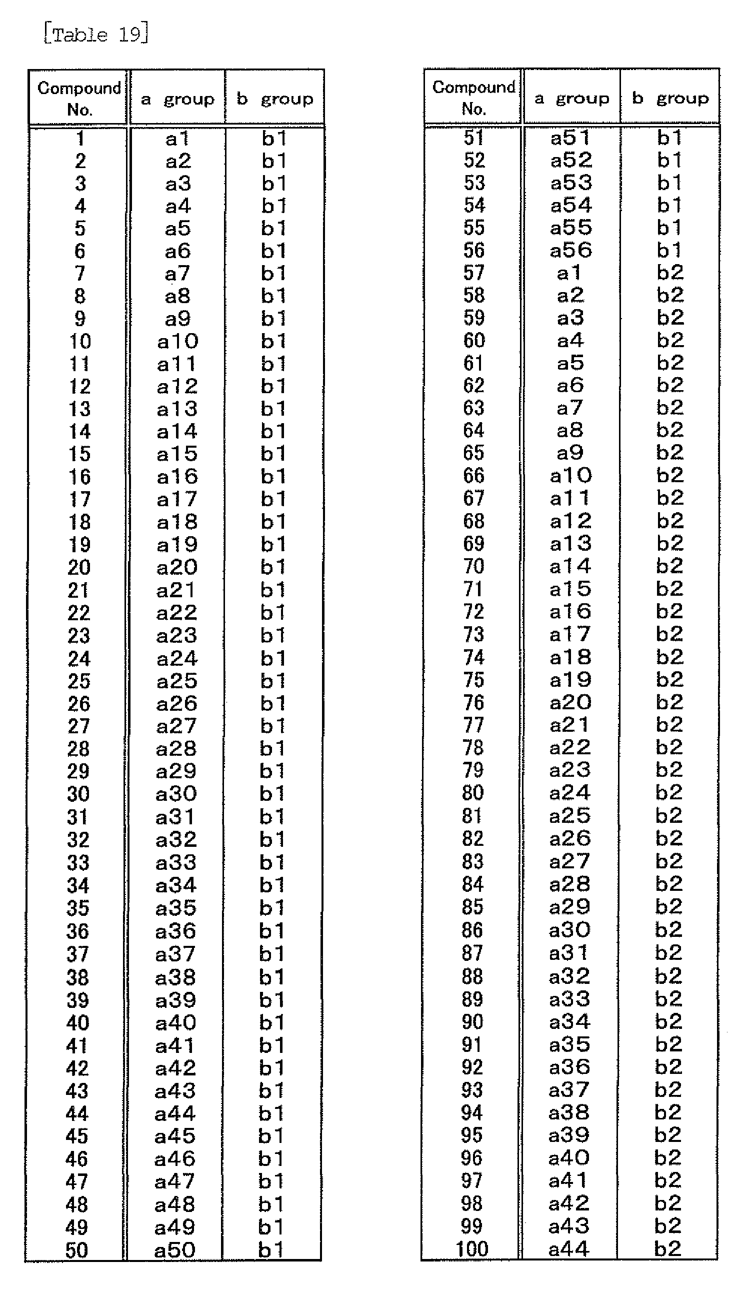

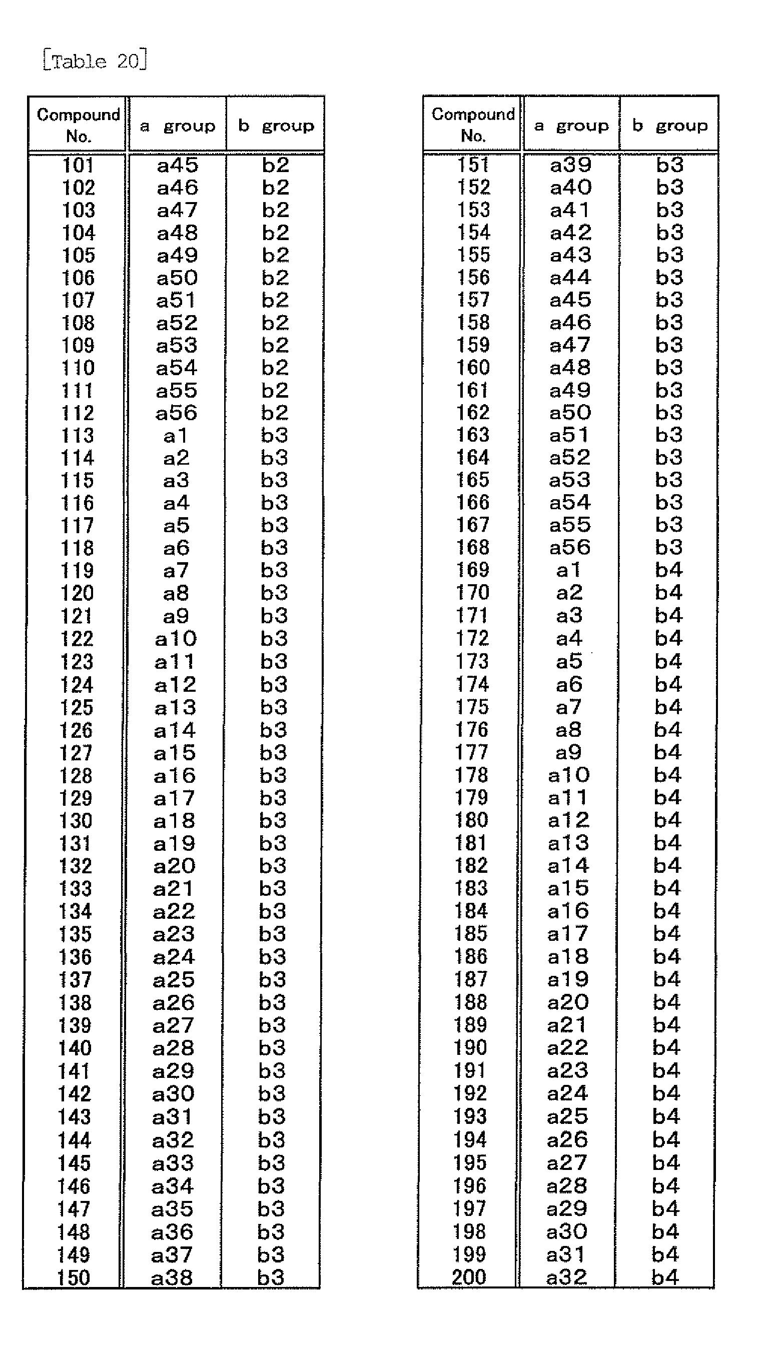

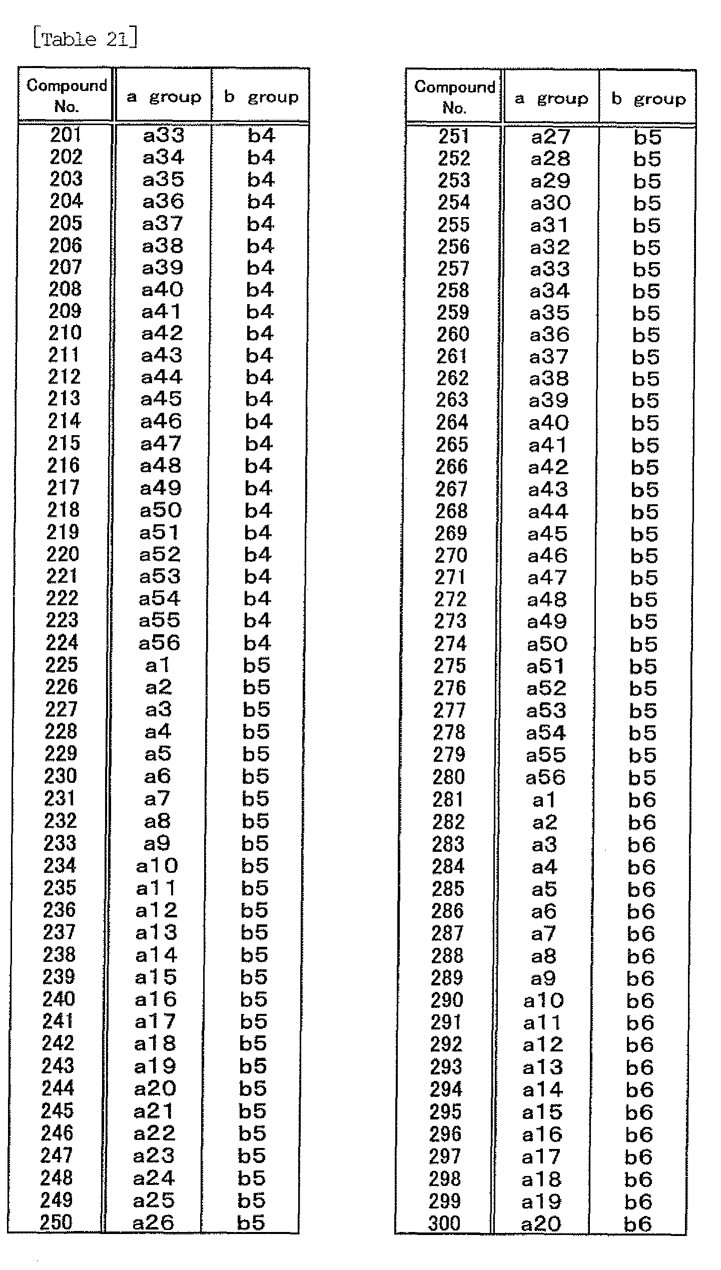

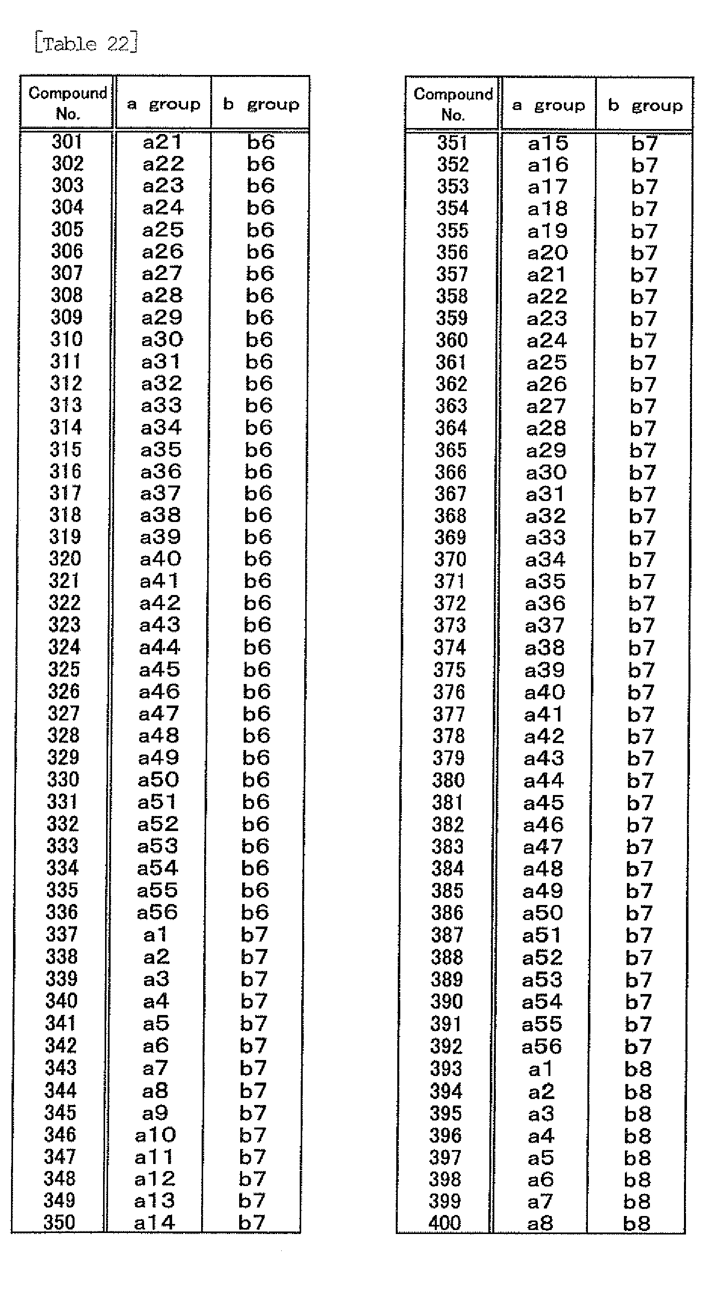

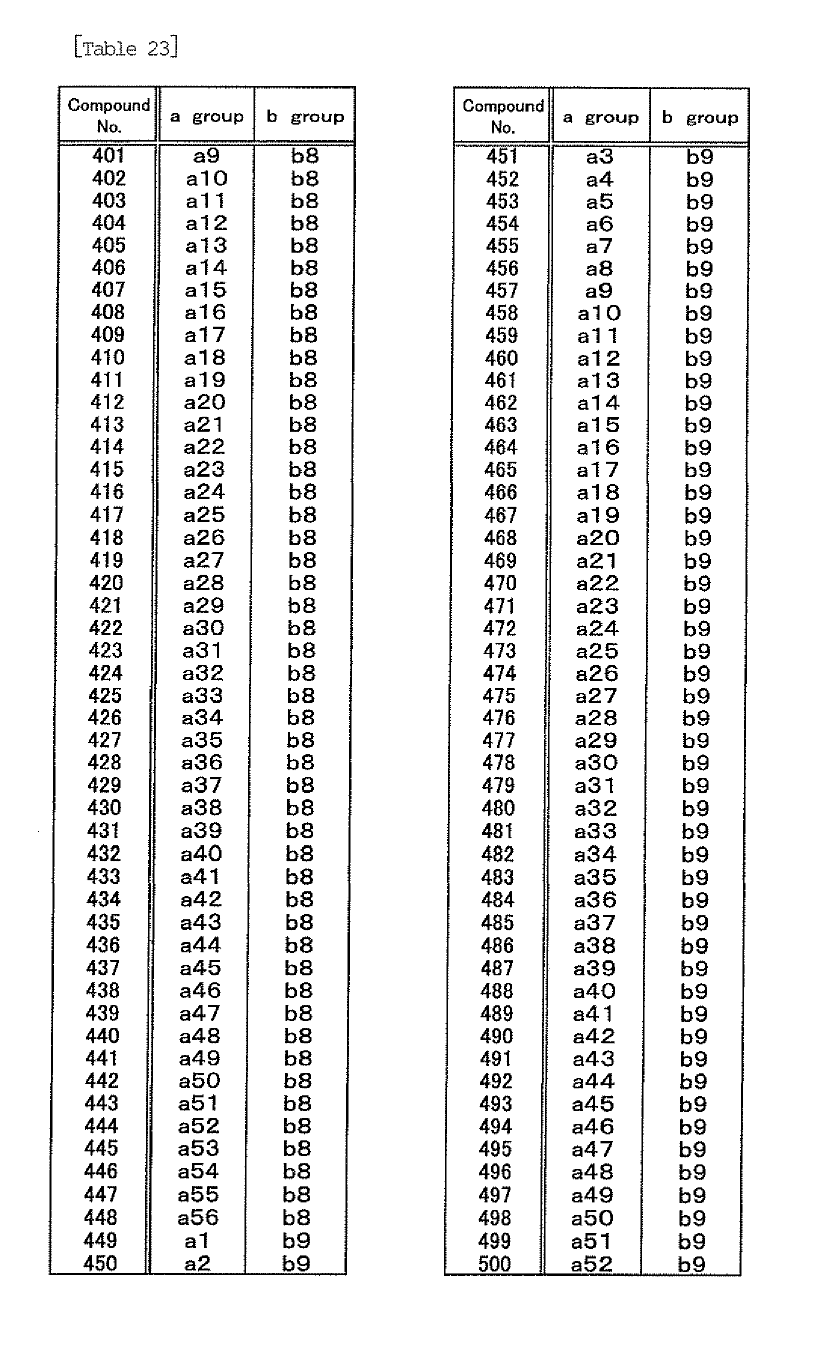

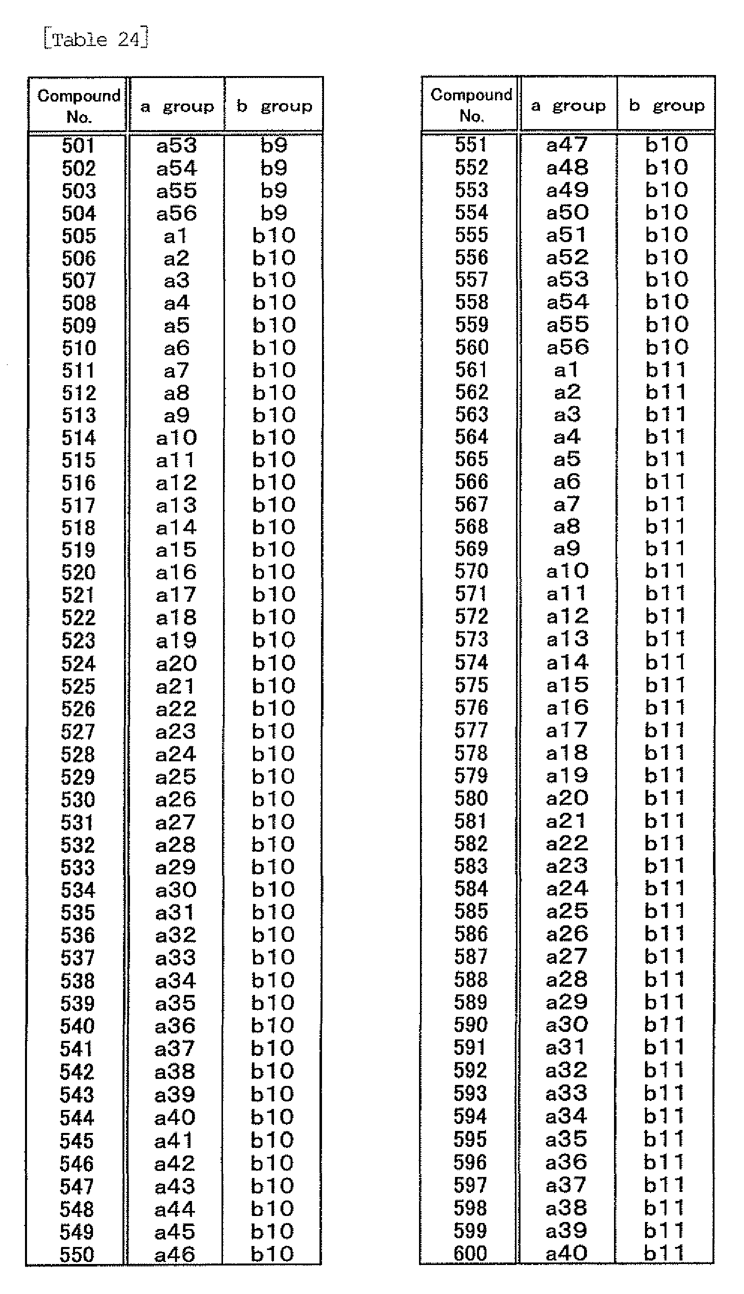

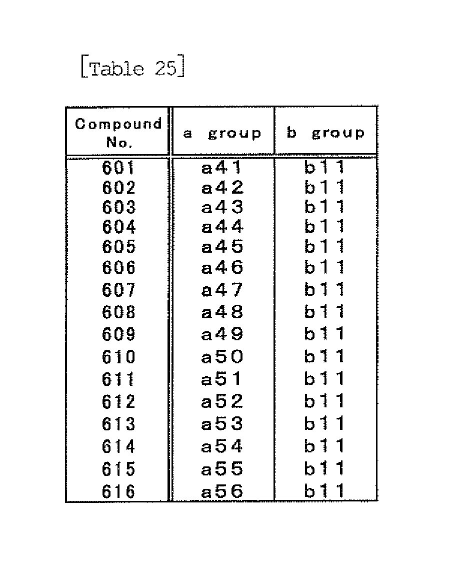

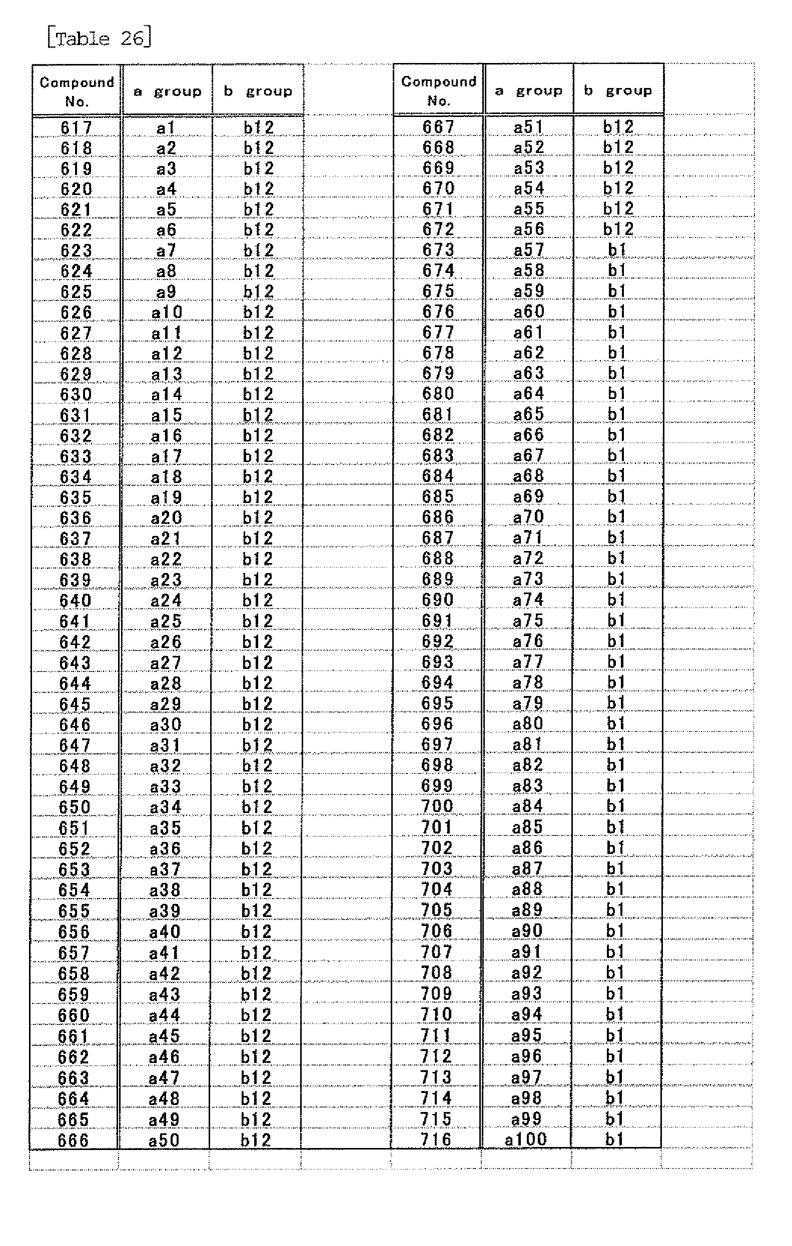

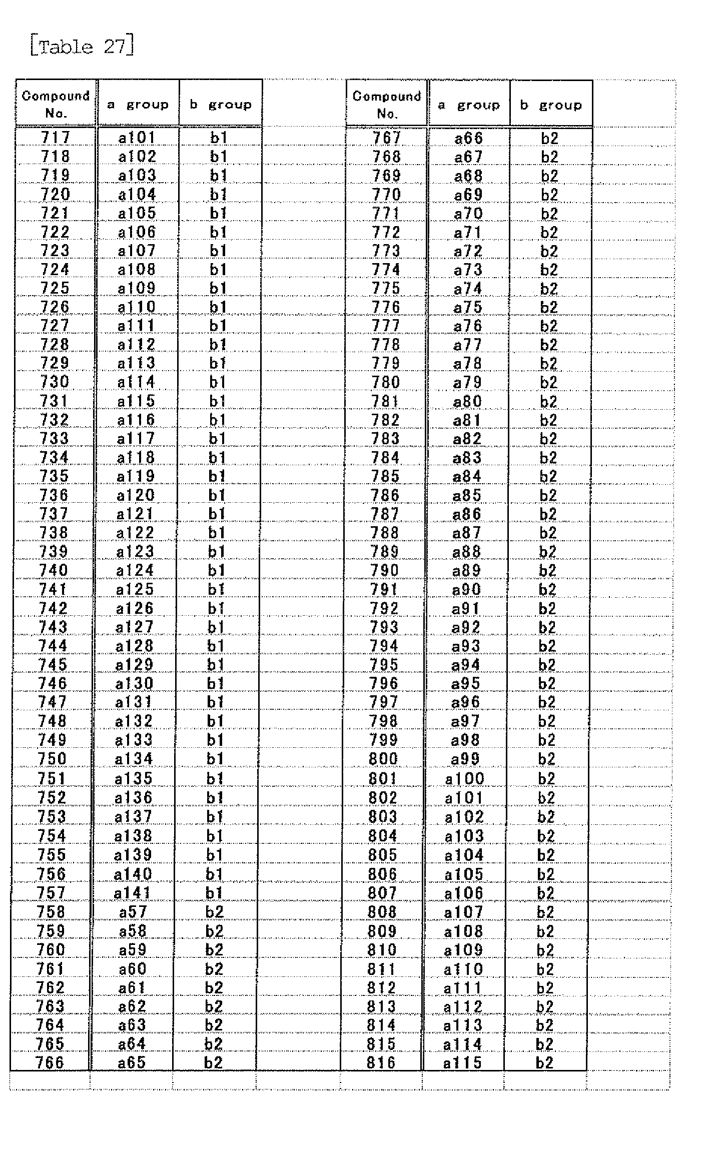

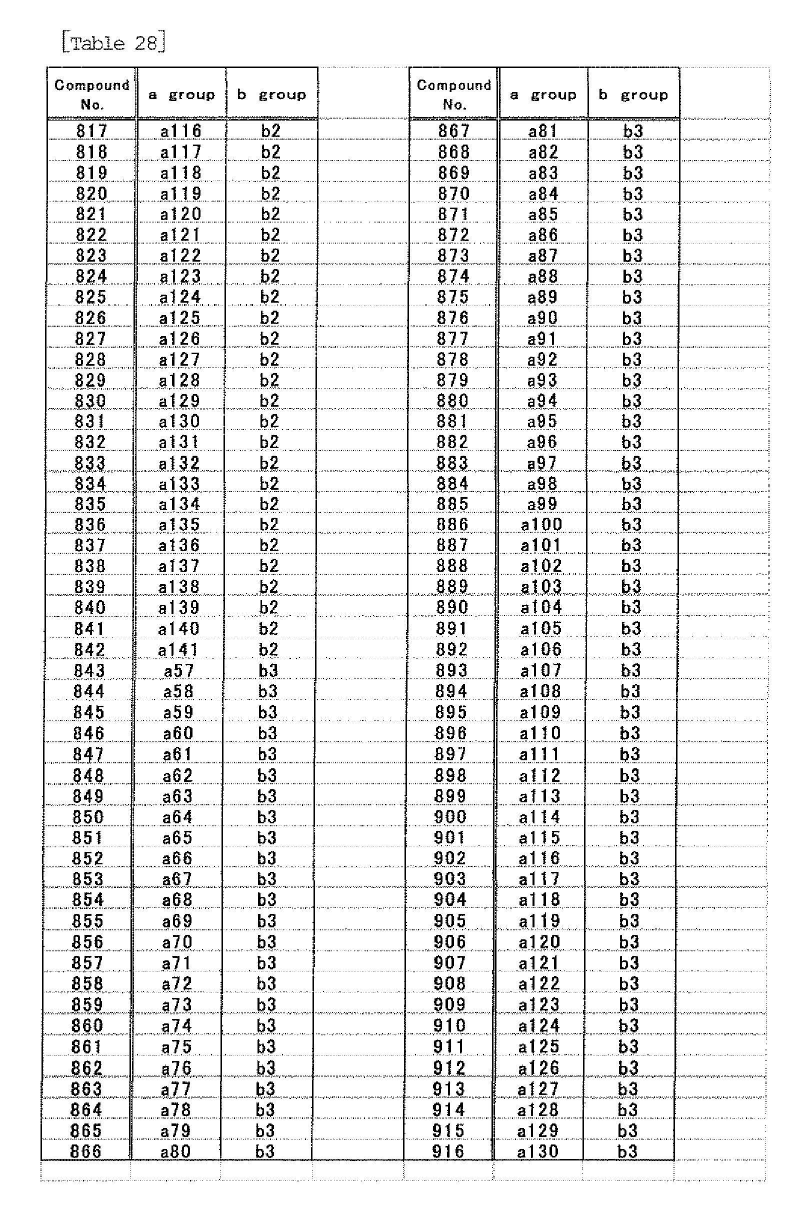

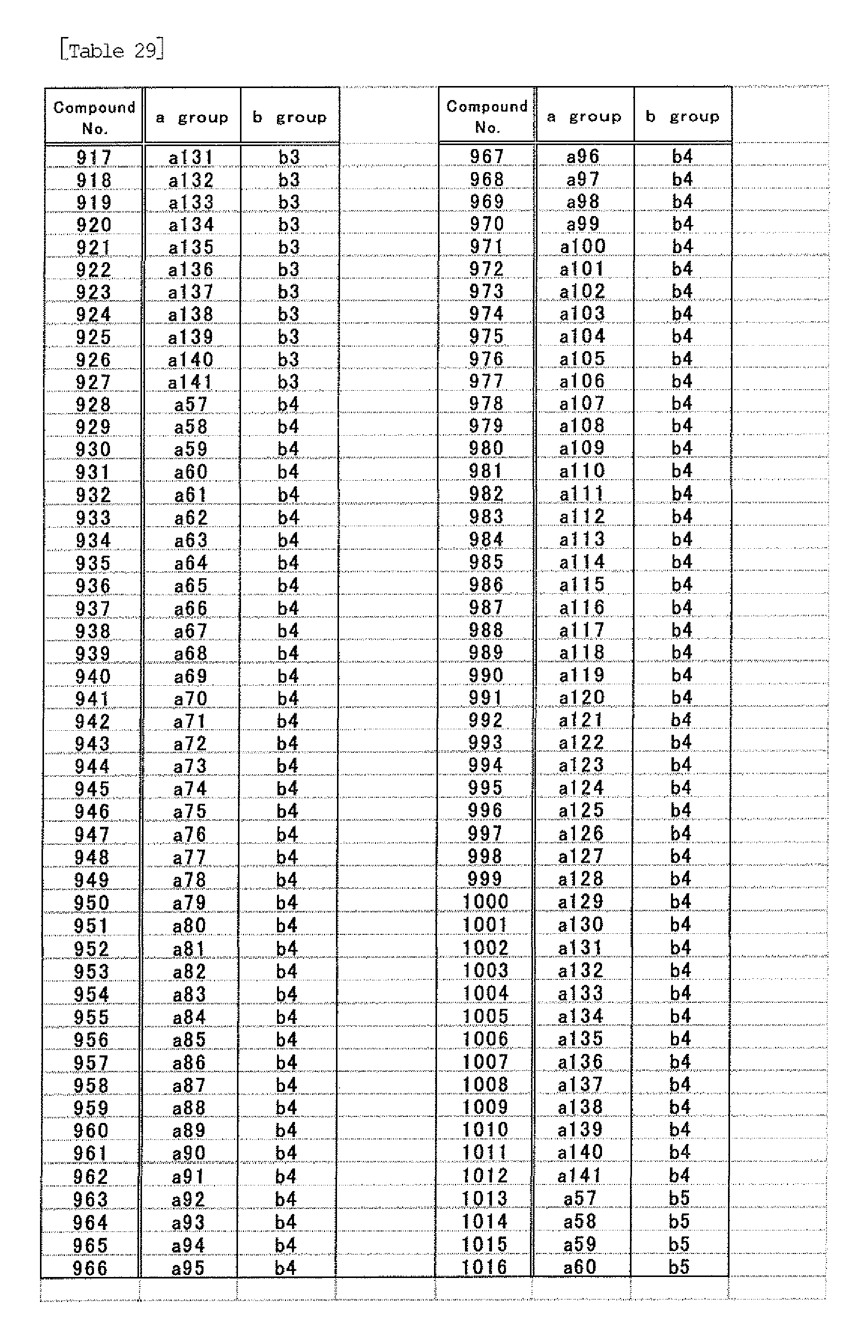

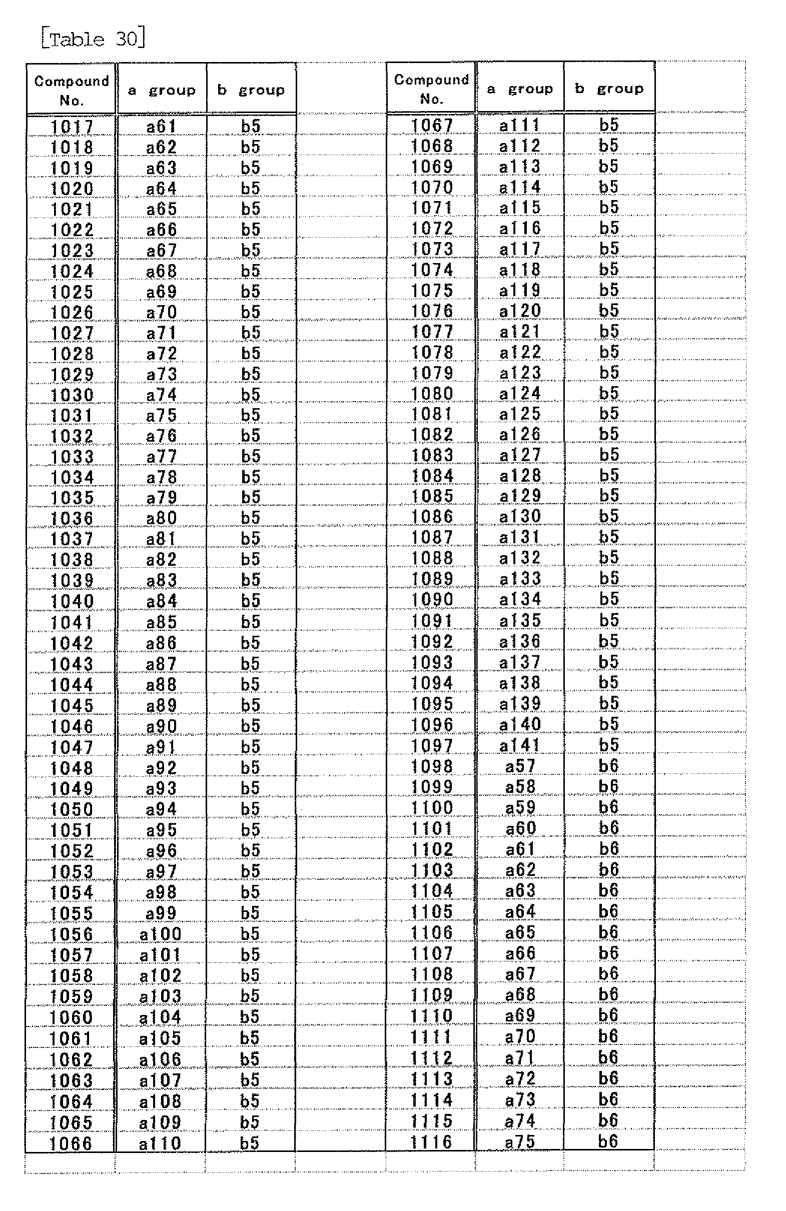

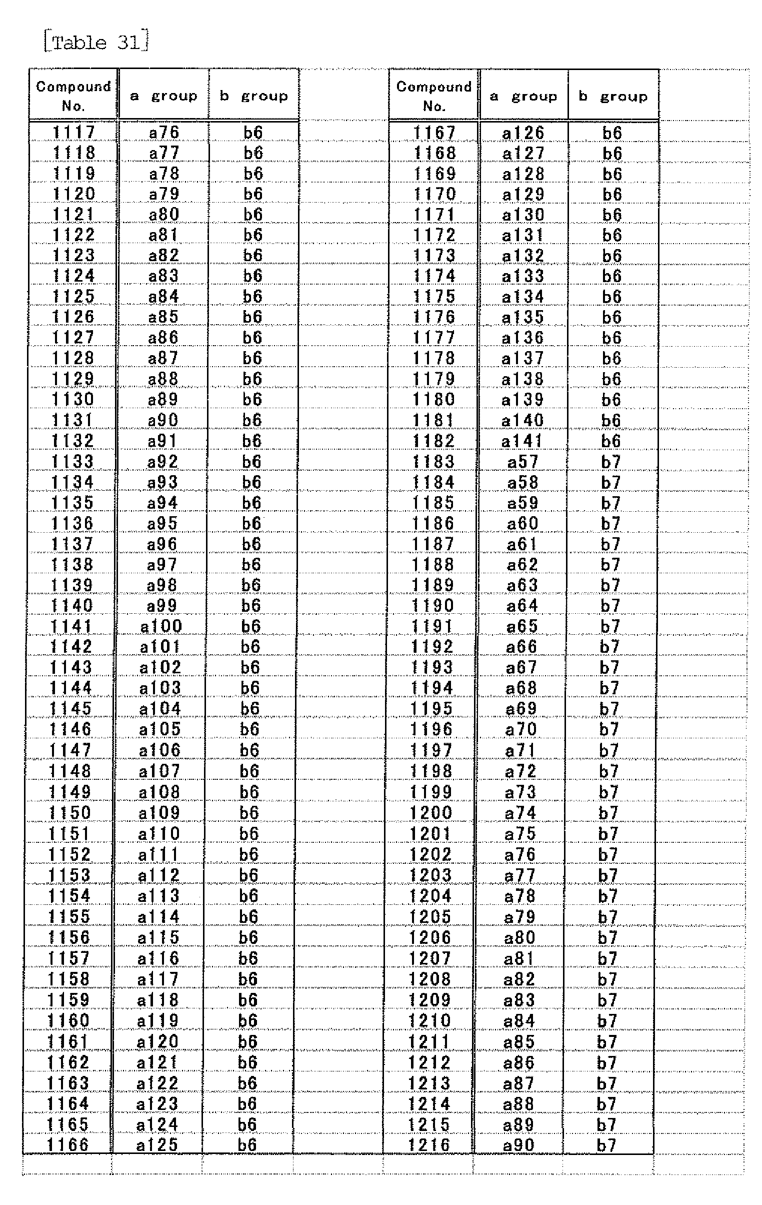

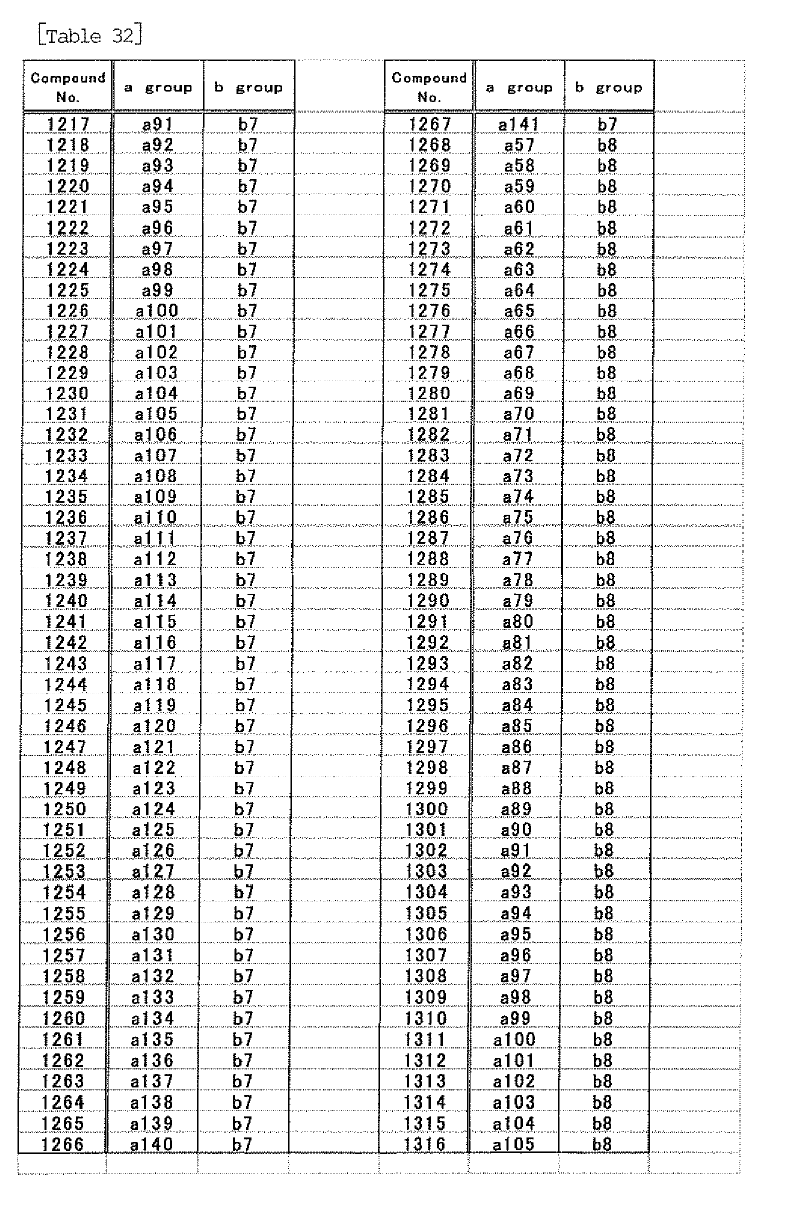

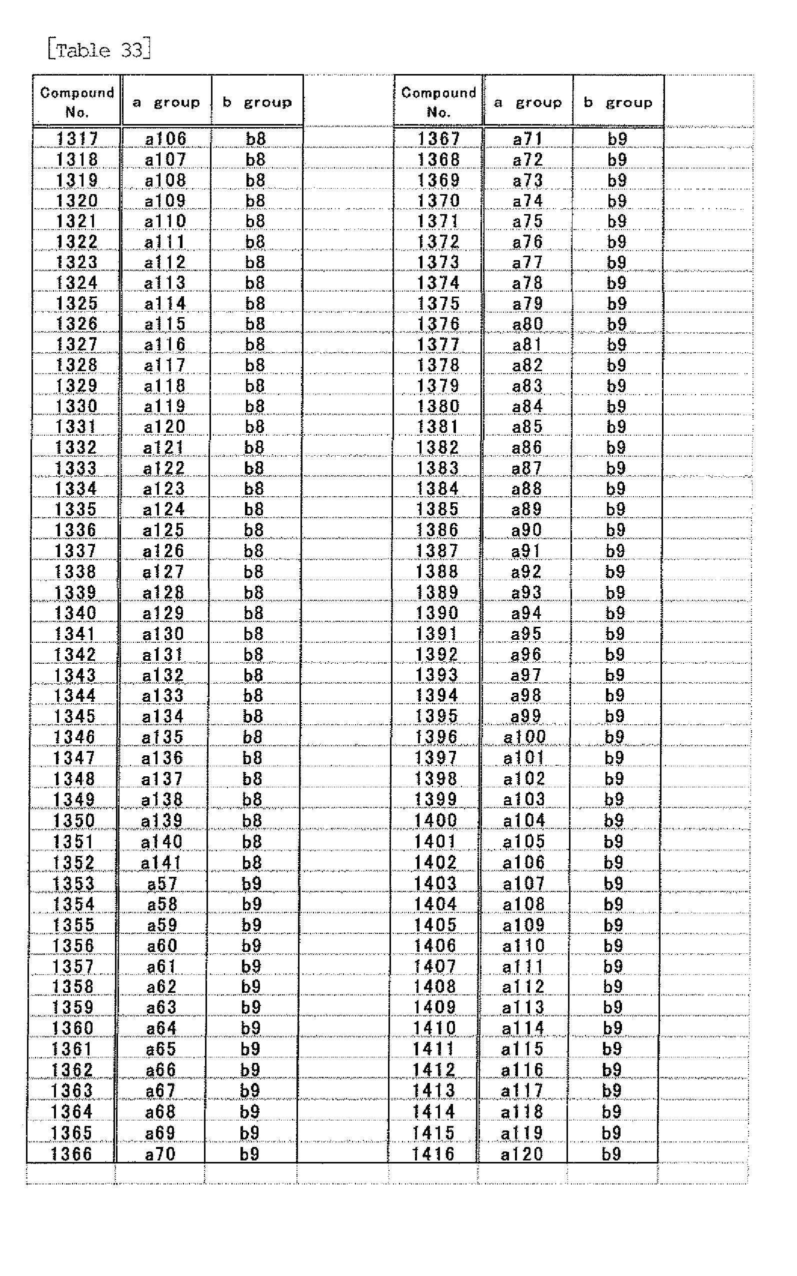

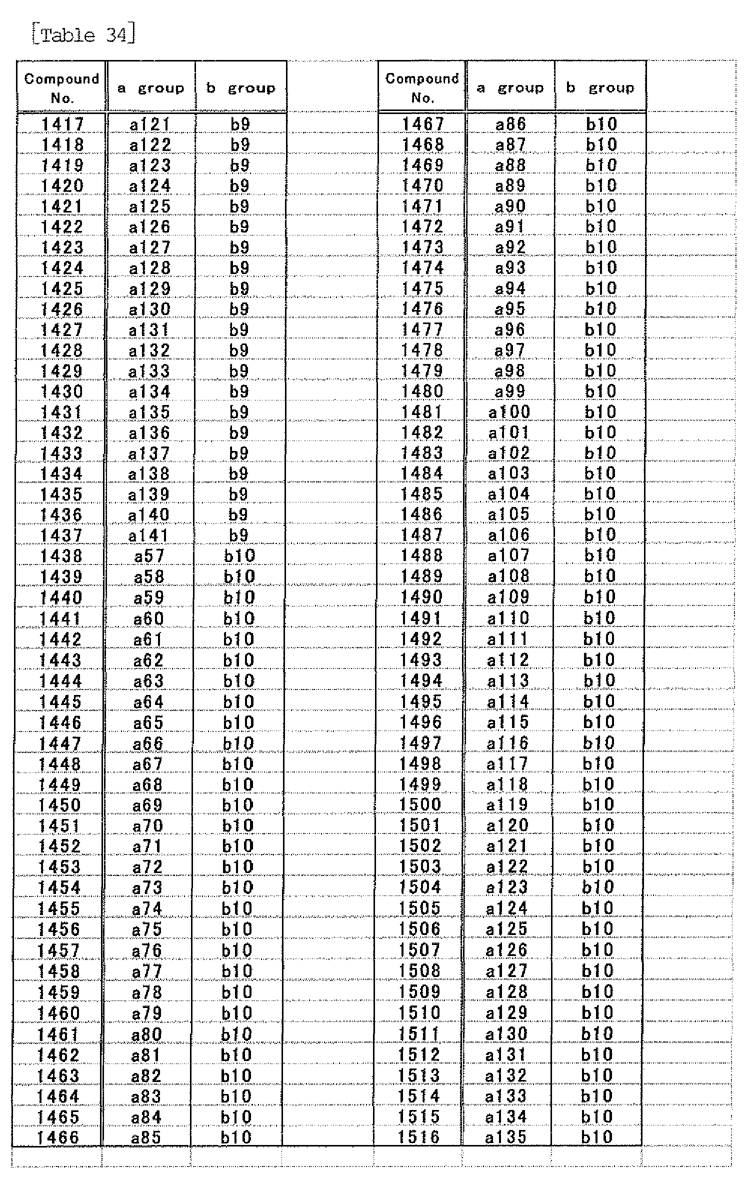

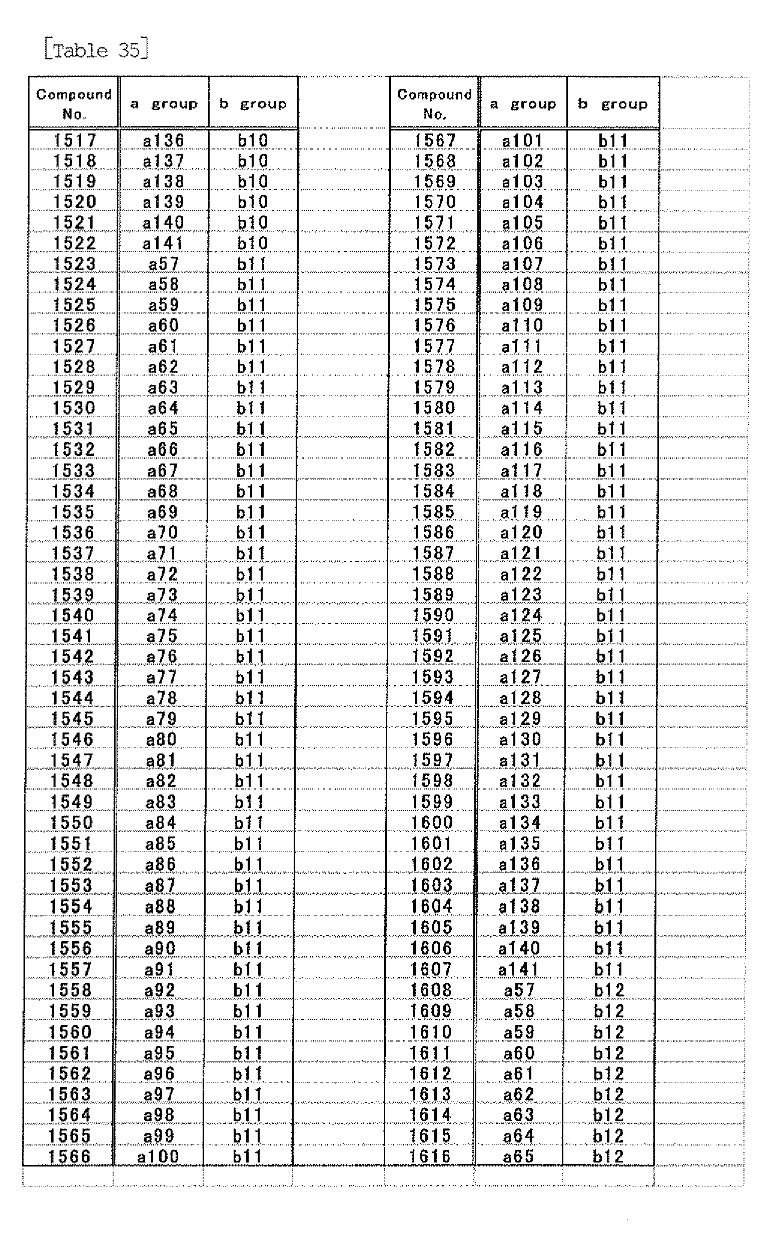

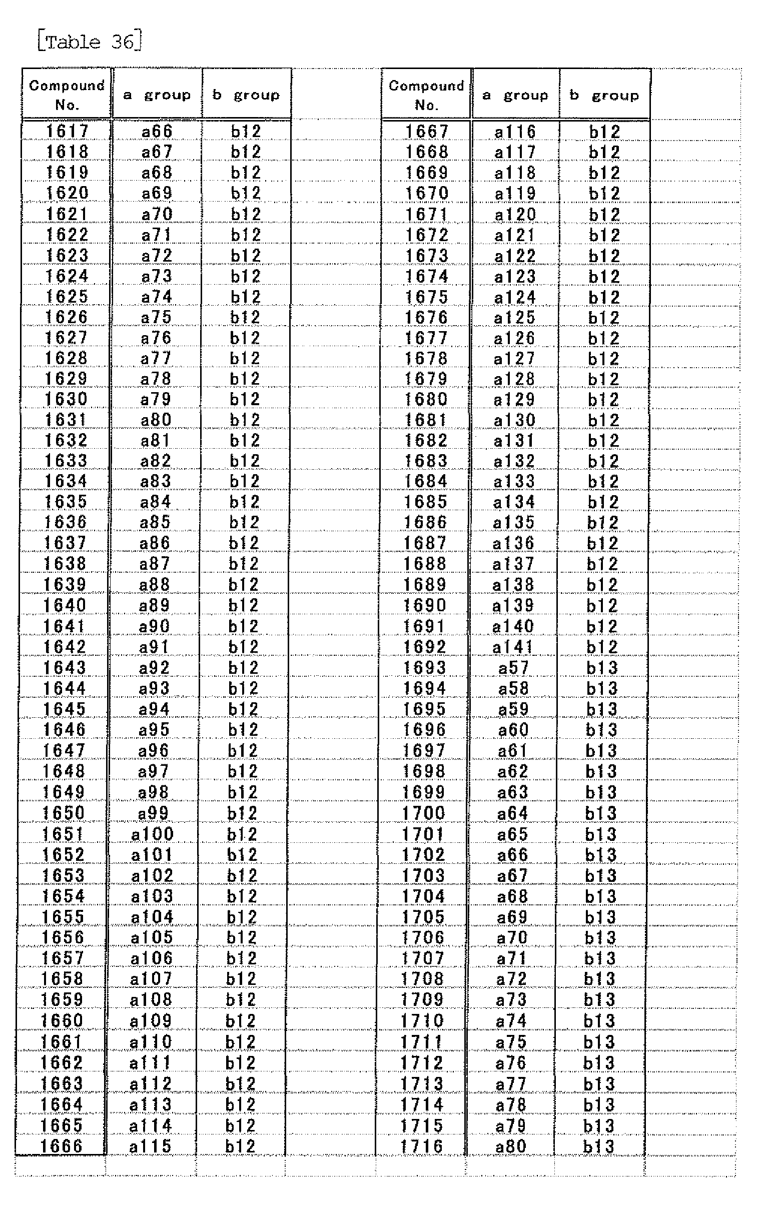

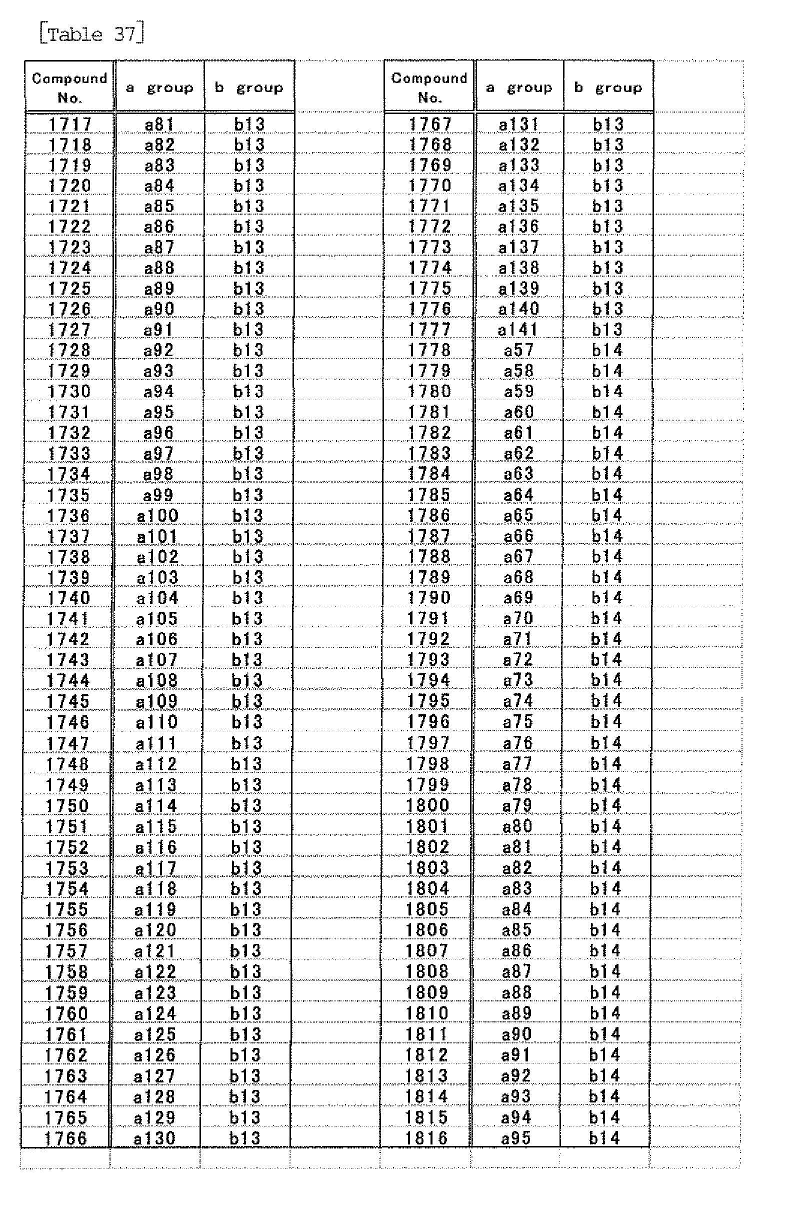

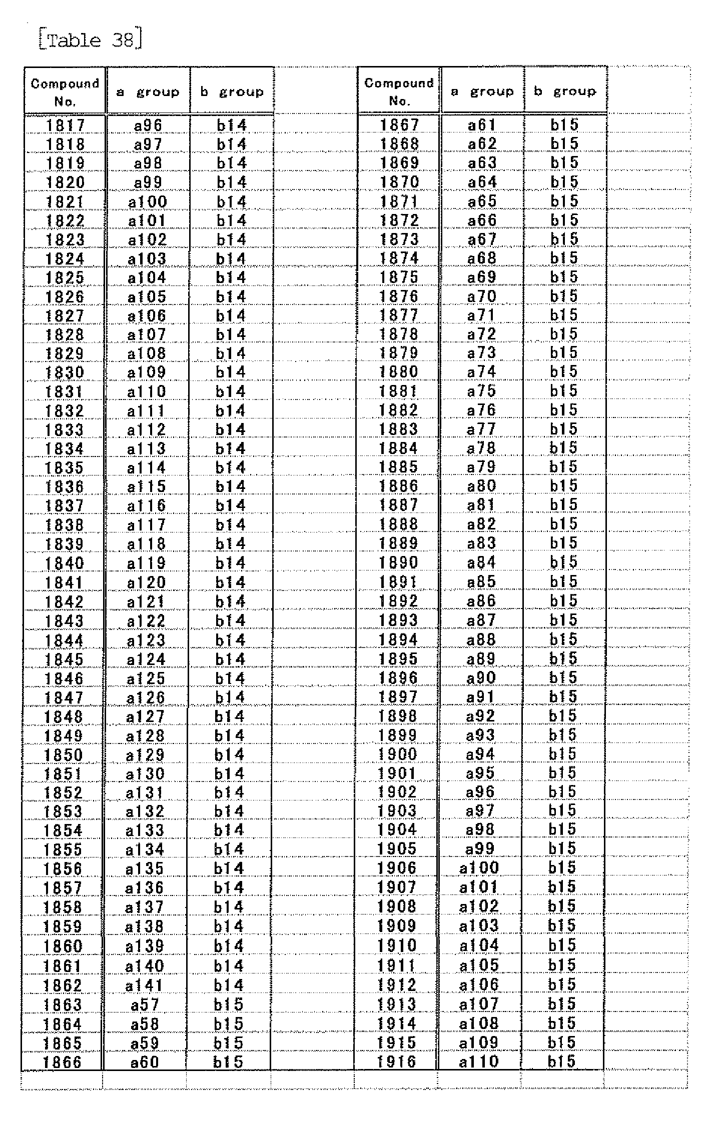

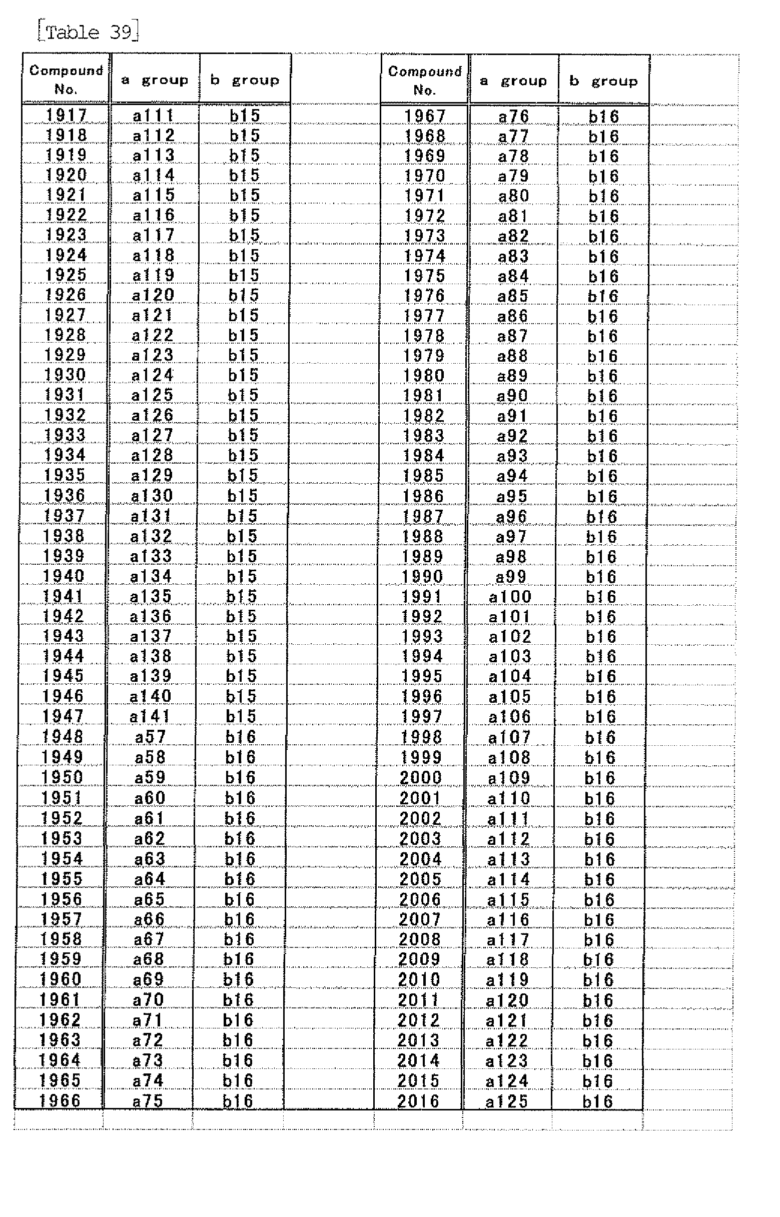

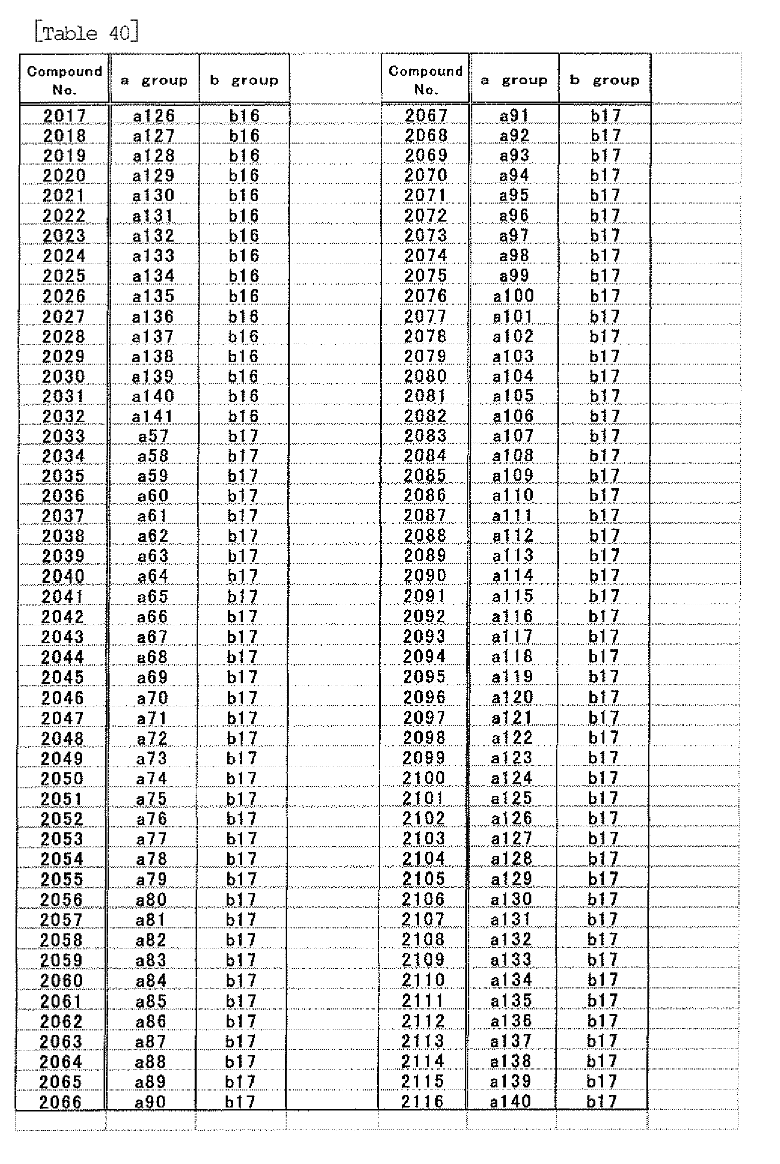

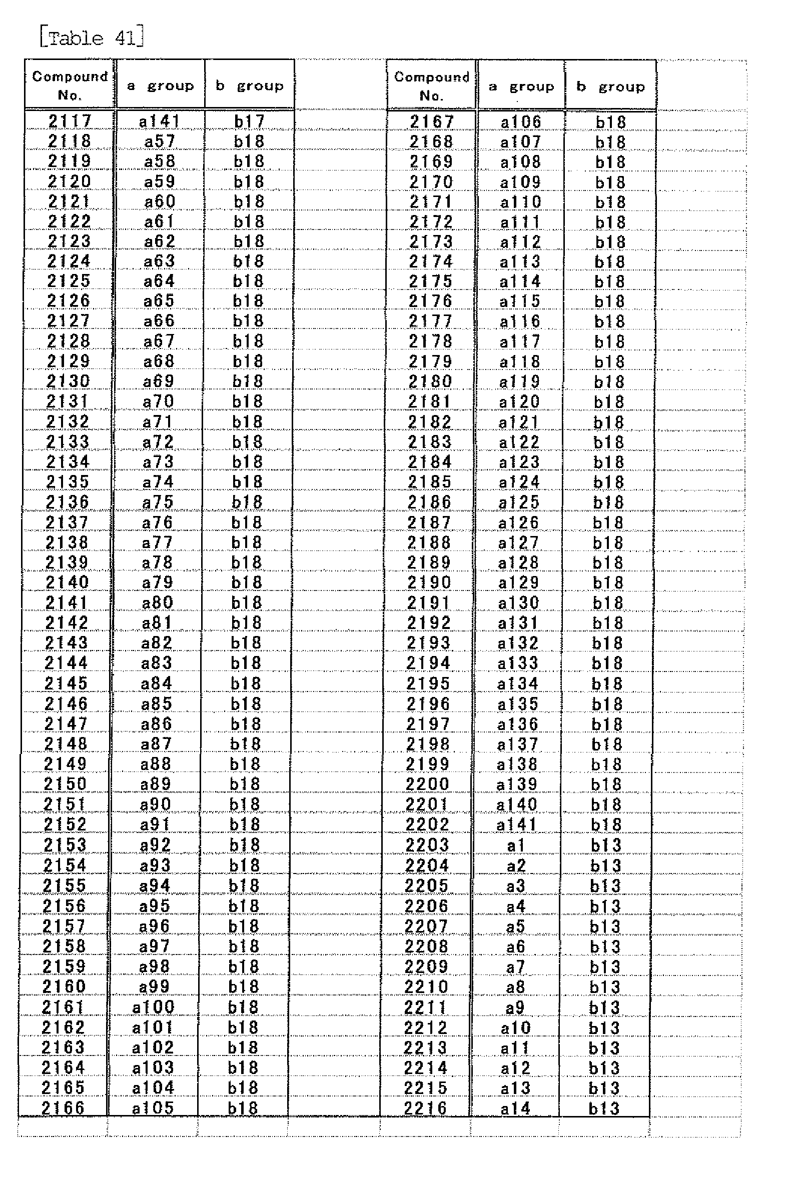

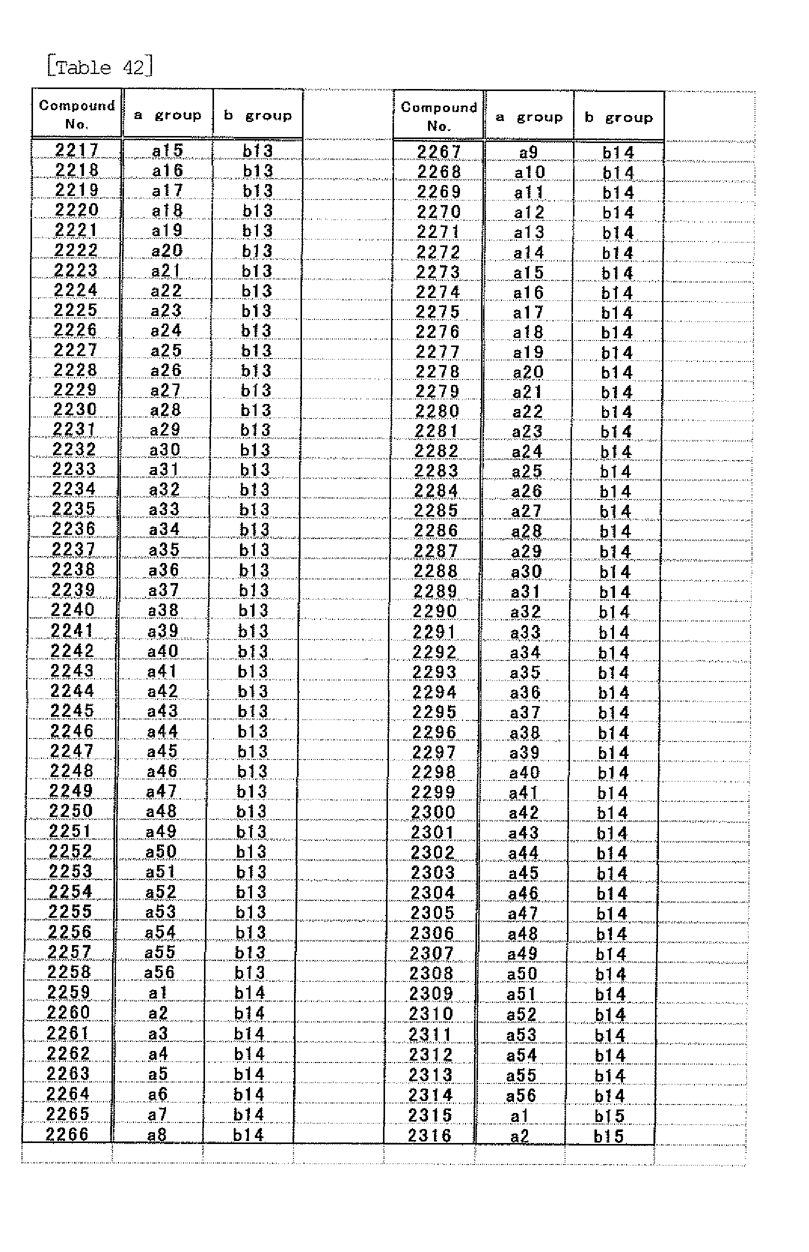

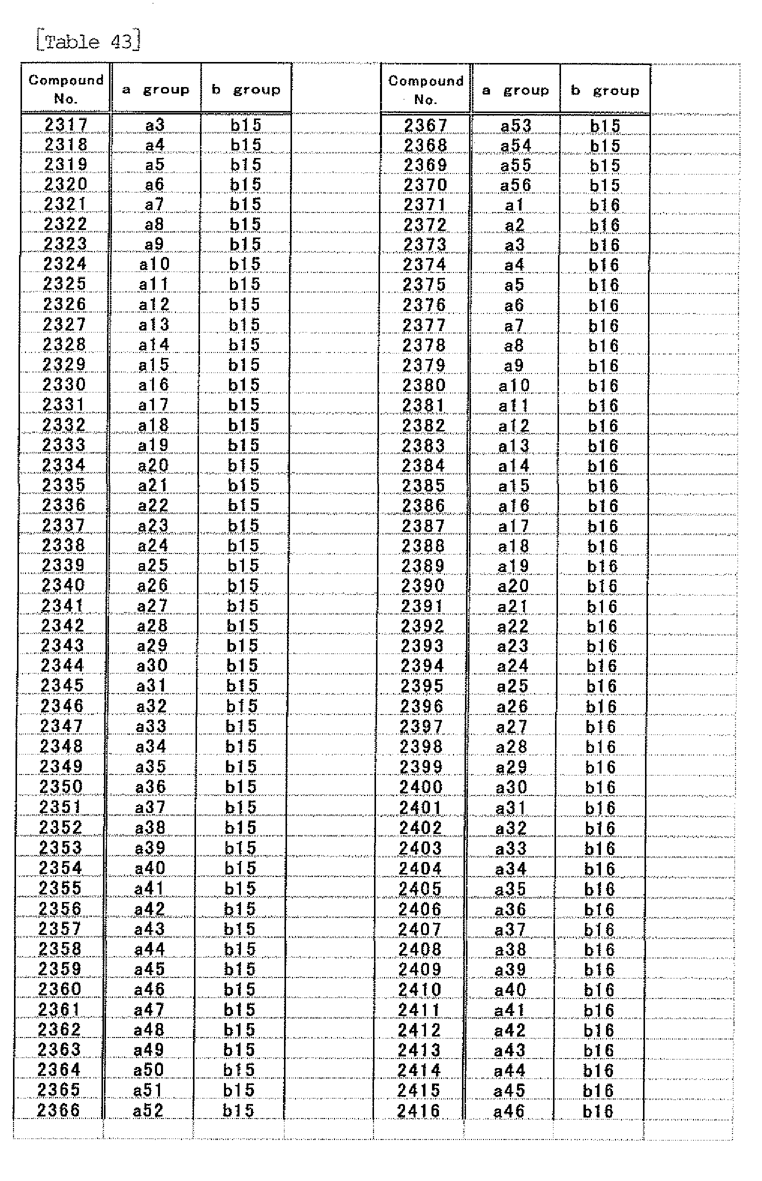

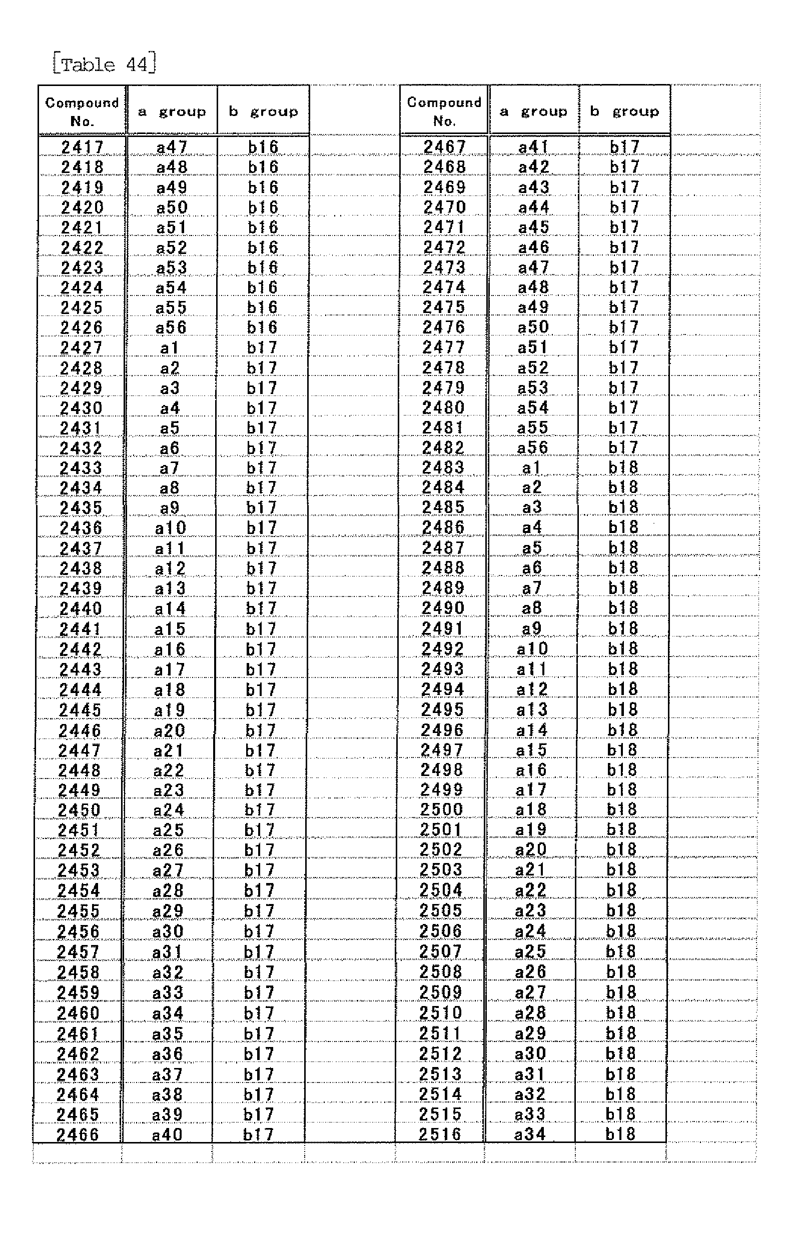

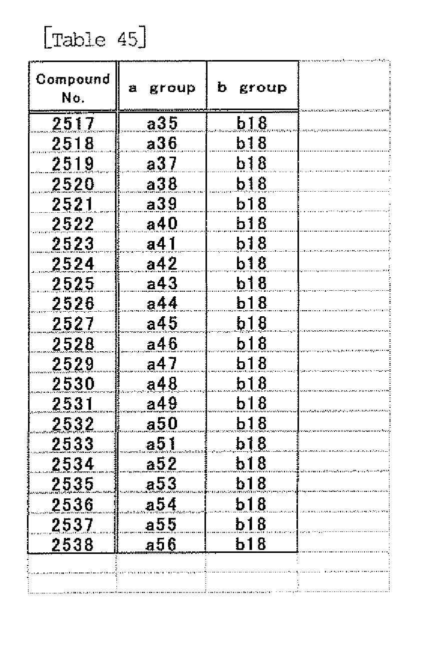

## Claims

1. A compound represented by formula (I) : 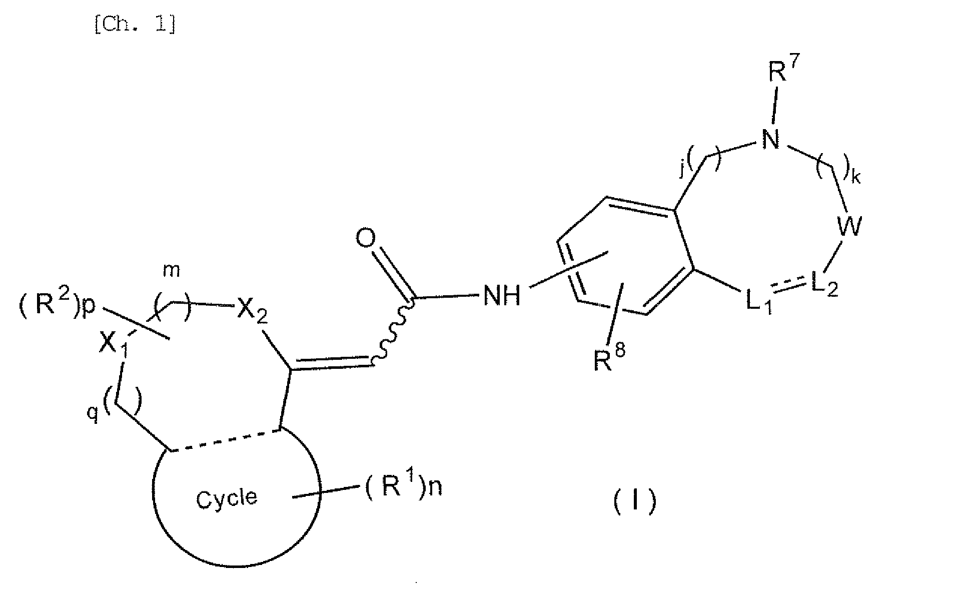 (wherein k, m, n, and p each independently represent an integer of 0 to 2; j and q represents an integer of 0 or 1; R¹ represents a group selected from a halogen atom, a substituted or unsubstituted hydrocarbon group, a substituted or unsubstituted heterocyclic group, a substituted or unsubstituted C₁₋₆ alkoxy group, a substituted or unsubstituted C₁₋₆ alkoxycarbonyl group, an amino group which may be mono- or di-substituted with a substituted or unsubstituted C₁₋₆ alkyl group, a protected or unprotected hydroxyl group, a protected or unprotected carboxyl group, a carbamoyl group which may be mono- or di-substituted with a substituted or unsubstituted C₁₋₆ alkyl group, a C₁₋₆ alkanoyl group, a C₁₋₆ alkylthio group, a C₁₋₆ alkylsulfinyl group, a C₁₋₆ alkylsulfonyl group, a sulfamoyl group which may be mono- or di-substituted with a substituted or unsubstituted C₁₋₆ alkyl group, a cyano group, and a nitro group; R² represents a group selected from a halogen atom, a substituted or unsubstituted amino group, a substituted or unsubstituted hydrocarbon group, a substituted or unsubstituted aromatic heterocyclic group, and an oxo group, or two geminal or vicinal R² may bind to each other to form a C₂₋₆ alkylene group, and form a cyclo ring group together with the carbon atom to which the two R² are bonded, or the cyclo ring group may form non-aromatic heterocyclic groups containing an oxygen atom or a nitrogen atom; X₁ represents an oxygen atom, -NR³- (wherein R³ is a hydrogen atom, a substituted or unsubstituted hydrocarbon group, a substituted or unsubstituted heterocyclic group, or a substituted or unsubstituted acyl group), or -S(O)r- (wherein r is an integer of 0 to 2); X₂ represents a methylene group, an oxygen atom, -NR³- (wherein R³ is a hydrogen atom, a substituted or unsubstituted hydrocarbon group, a substituted or unsubstituted heterocyclic group, or a substituted or unsubstituted acyl group) or -S(O)r- (wherein r is an integer of 0 to 2); W represents a methylene group, a carbonyl group or a sulfonyl group;
R⁷ represents a hydrogen atom, a substituted or unsubstituted hydrocarbon group, a substituted or unsubstituted heterocyclic group, or a substituted or unsubstituted acyl group; R⁸, R^{9A} and R^{9B} each independently represent a hydrogen atom, a halogen atom, a substituted or unsubstituted hydrocarbon group, a substituted or unsubstituted heterocyclic group, a substituted or unsubstituted C₁₋₆ alkoxyl group, a substituted or unsubstituted C₁₋₆ alkoxycarbonyl group, an amino group which may be mono- or di-substituted by a substituted or unsubstituted C₁₋₆ alkyl group, a protected or unprotected hydroxyl grop, a protected or unprotected carboxyl group, a carbamoyl group which may be mono- or di-substituted by a substituted or unsubstituted C₁₋₆ alkyl group, a C₁₋₆ alkanoyl group, C₁₋₆ alkylthio group, a C₁₋₆ alkylsulfinyl group, C₁₋₆ alkylsulfonyl group, a sulfamoyl group which may be mono- or di-substituted by a substituted or unsubstituted C₁₋₆ alkyl group, a cyano group or a nitro group; L₁ and L₂ each independently represent a single bond, a -CR^{9A}R^{9B}-, an oxygen atom; -NP¹⁰- (R¹⁰ represents a hydrogen atom, a substituted or unsubstituted hydrocarbon group, a substituted or unsubstituted heterocyclic group or a substituted or unsubstituted acyl group) or -S(O)t- (t is an integer of 0 to 2), the broken line in the ring containing X₁ and X₂ represents a condensation of two rings; Cycle moiety represents a five- or six-membered aryl ring or heteroaryl ring; and the solid line and the broken line between L₁ and L₂ is a single bond or double bond, and the wavy line represents an E-isomer or a Z-isomer), provided that when W represents a methylene group L₁ is an oxygen atom and L₂ is a -CR^{9A}R^{9B}-, and that each of (E)-2-(8-trifluoromethyl-3,4-dihydrobenzo[b]oxepin-5(2H)-ylidene)-N-(3-hydroxy-2-oxo-1,2,3,4-tetrahydroquinolin-5-yl)acetamide; (E)-2-(7-t-rifluoromethyl-2,3-dihydro-1-pentanoylquinolin-4(1H)-ylidene)-N-(3,4-dihydro-3-hydroxy(1H)quinolin-2-on-5-yl)acetamide; (E)-N-(3-hydroxy-2-oxo-1,2,3,4-tetrahydroquinolin-5-yl)-2-(7-trifluoromethyl-chroman-4-ylidene) acetamide; (E)-2-(8-trifluoromethyl-3,4-dihydrobenzo[b]oxepin-5-(2H)- ylidene)-N-(3,4-dihydro-1H-quinolin-2-on-7-yl)acetamide; (E)-2-(8-trifluoromethyl-3,4-dihydrobenzo[b]oxepin-5(2H)- ylidene)-N-(2-quinolon-7-yl)acetamide; (E)-2-(8-trifluoromethyl-3,4-dihydrobenzo[b]oxepin-5(2H)- ylidene)-N-(2-oxoindolin-6-yl)acetamide; (E)-2-(8-trifluoromethyl-3,4-dihydrobenzo[b]oxepin-5(2H)- ylidene)-N-(2H-benzo[1,4]oxazine-3(4H)-on-6-yl)acetamide; (E)-2-(8-trifluoromethyl-3,4-dihydrobenzo[b]oxepin-5(2H)- ylidene)-N-(3,4-dihydro-1H-quinolin-2-on-6-yl)acetamide; (E)-2-(8-trifluoromethyl-3,4-dihydrobenzo[b]oxepin-5(2H)- ylidene)-N-(2,3-dihydro-isoindol-1-on-6-yl)acetamide; (E)-2-(8-trifluoromethyl-3,4-dihydrobenzo[b]oxepin-5(2H)- ylidene)-N-(2-quinolon-8-yl)acetamide; (E)-2-(8-trifluoromethyl-3,4-dihydrobenzo[b]oxepin-5(2H)-ylidene)-N-(2-oxo-1,2,3,4-tetrahydroquinolin-8-yl)acetamide; (E)-2-(8-trifluoromethyl-3,4-dihydrobenzo[b]oxepin-5(2H)- ylidene)-N-(2-hydroxyethyl-2,3-dihydro-isoindol-1-on-6-yl) acetamide; (E)-2-(8-trifluoromethyl-3,4-dihydrobenzo[b]oxepin-5(2H)-ylidene)-N-(3,4-dihydro-(2H)-isoquinolin-1-on-7-yl) acetamide; (E)-2-(8-trifluoromethyl-3,4-dihydrobenzo[b]oxepin-5(2H)- ylidene)-N-(1-methyl-2-oxo-1,2,3,4-tetrahydroquinolin-7-yl) acetamide, (E)-2-(1-(2,2-difluorobutanoyl)-7-trifluoromethyl-2,3-dihydroquinolin-4(1H)-ylidene)-N-(3-hydroxy-2-oxo-1,2,3,4-tetrahydroquinolin-5-yl)acetamide ; (E)-2-(8-trifluorometyl-3,4-dihydrobenzo[b]oxepin-5(2H)-ylidene)-N-(4-(2-hydroxyethyl)-2H-1,4-benzoxazin-3(4H)-on-6-yl) acetamide ; (E)-2-(8-trifluoromethyl-3,4-dihydrobenzo[b]oxepin-5(2H)-ylidene-N-(4-(2-hydroxyacetyl)-3,4-dihydro-2H-benzo[b][1,4]oxazin-6-yl)acetamide; (E)-2-(8-trifluoromethyl-3,4-dihydrobenzo[b]oxepin-5(2H)-ylidene-N-(4-(2-hydroxypropanoyl)-3,4-dihydro-2H-benzo[b][1,4]oxazin-6-yl)acetamide; and (E)-2-(8-trifluoromethyl-3,4-dihydrobenzo[b]oxepin-5(2H)-ylidene-N-(4-(2-hydroxyethyl)-3,4-dihydro-2H-benzo[b][1,4]oxazin-6-yl)acetamide; is eliminated), a salt thereof, and solvates thereof.

2. A pharmaceutical composition comprising, as an active ingredient, at least one of the compound represented by formula (I) according to Claim 1, a pharmaceutically acceptable salt of the compound, and a solvate of the compound or the salt.

3. A transient receptor potential type I (TRPV1) receptor antagonist comprising, as an active ingredient, at least one of the compound represented by formula (I) according to Claim 1, a pharmaceutically acceptable salt of the compound, and a solvate of the compound or the salt.
